(19) Europäisches Patentamt / European Patent Office / Office européen des brevets

(11) **EP 3 058 085 B1**

(12) **EUROPEAN PATENT SPECIFICATION**

(45) Date of publication and mention of the grant of the patent:
**17.02.2021 Bulletin 2021/07**

(21) Application number: **15727472.1**

(22) Date of filing: **28.04.2015**

(51) Int Cl.:
*C12Q 1/6883* (2018.01)   *C12Q 1/02* (2006.01)
*A61B 5/00* (2006.01)   *C12Q 1/04* (2006.01)
*G01N 33/68* (2006.01)   *A61K 35/66* (2015.01)

(86) International application number:
**PCT/IL2015/050442**

(87) International publication number:
**WO 2015/166492 (05.11.2015 Gazette 2015/44)**

(54) **MICROBIOME RESPONSE TO AGENTS**

MIKROBIOMREAKTION AUF MITTEL

RÉPONSE DU MICROBIOME À DES AGENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.04.2014   US 201461984944 P
09.09.2014   US 201462048065 P
16.09.2014   US 201462050939 P**

(43) Date of publication of application:
**24.08.2016   Bulletin 2016/34**

(73) Proprietor: **Yeda Research and Development Co.,
Ltd.
7610002 Rehovot (IL)**

(72) Inventors:
• **ELINAV, Eran
7680400 Mazkeret Batia (IL)**
• **SEGAL, Eran
4730168 Ramat-HaSharon (IL)**

(74) Representative: **Dennemeyer & Associates S.A.
Postfach 70 04 25
81304 München (DE)**

(56) References cited:
**US-A1- 2010 172 874**

• **A. N. PAYNE ET AL: "Gut microbial adaptation to dietary consumption of fructose, artificial sweeteners and sugar alcohols: implications for host-microbe interactions contributing to obesity", OBESITY REVIEWS, vol. 13, no. 9, 11 June 2012 (2012-06-11), pages 799-809, XP055205060, ISSN: 1467-7881, DOI: 10.1111/j.1467-789X.2012.01009.x**
• **THERESA E COWAN ET AL: "Artificial sweetener consumption differentially affects the gut microbiota-host metabolic interactions -- Cowan et al. 27 (1): 224.7 -- The FASEB Journal", THE FASEB JOURNAL, vol. 27, 1 April 2013 (2013-04-01), page 224.7, XP055205066, ISSN: 0892-6638 cited in the application**
• **THOMPSON-CHAGOYAN O C ET AL: "Faecal microbiota and short-chain fatty acid levels in faeces from infants with cow's milk protein allergy", INTERNATIONAL ARCHIVES OF ALLERGY AND IMMUNOLOGY, KARGER AG, CH, vol. 156, no. 3, 1 January 2011 (2011-01-01), pages 325-332, XP009166971, ISSN: 1018-2438**
• **CAO SEVERINE ET AL: "The role of commensal bacteria in the regulation of sensitization to food allergens", FEBS LETTERS, ELSEVIER, AMSTERDAM, NL, vol. 588, no. 22, 1 May 2014 (2014-05-01), pages 4258-4266, XP029083055, ISSN: 0014-5793, DOI: 10.1016/J.FEBSLET.2014.04.026**
• **JOTHAM SUEZ ET AL: "Artificial sweeteners induce glucose intolerance by altering the gut microbiota", NATURE, 17 September 2014 (2014-09-17), XP055205484, ISSN: 0028-0836, DOI: 10.1038/nature13793**

EP 3 058 085 B1

**(Cont. next page)**

- SUEZ J ET AL: "Non-caloric artificial sweeteners and the microbiome: Findings and challenges", GUT MICROBES 20150401 TAYLOR AND FRANCIS INC. USA, vol. 6, no. 2, 1 April 2015 (2015-04-01), pages 149-155, XP008177160, ISSN: 1949-0976, DOI: 10.1080/19490976.2015.1017700
- MARIE S. A. PALMNÄS ET AL: "Low-Dose Aspartame Consumption Differentially Affects Gut Microbiota-Host Metabolic Interactions in the Diet-Induced Obese Rat", PLOS ONE, vol. 9, no. 10, 14 October 2014 (2014-10-14), page e109841, XP055205532, DOI: 10.1371/journal.pone.0109841
- THAISS CHRISTOPH A ET AL: "Transkingdom Control of Microbiota Diurnal Oscillations Promotes Metabolic Homeostasis", CELL, CELL PRESS, US, vol. 159, no. 3, 16 October 2014 (2014-10-16), pages 514-529, XP029084863, ISSN: 0092-8674, DOI: 10.1016/J.CELL.2014.09.048
- THAISS C A ET AL: "A day in the life of the meta-organism: Diurnal rhythms of the intestinal microbiome and its host", GUT MICROBES 20150422 TAYLOR AND FRANCIS INC. USA, vol. 6, no. 2, 22 April 2015 (2015-04-22), pages 137-142, XP008177161, ISSN: 1949-0976, DOI: 10.1080/19490976.2015.1016690
- THAISS C A ET AL: "A day in the life of the meta-organism: Diurnal rhythms of the intestinal microbiome and its host", GUT MICROBES, LANDES BIOSCIENCE, UNITED STATES, vol. 6, no. 2, 22 April 2015 (2015-04-22), pages 137-142, XP008177161, ISSN: 1949-0976, DOI: 10.1080/19490976.2015.1016690

**Description**

The present disclosure, in some aspects thereof, relates to the microbiome and, more particularly, but not exclusively, to a method and apparatus for predicting a response of a subject to one or more agents by analysis of the microbiome.

The human in intestine carries a vast and diverse microbial ecosystem that has co-evolved with our species and is essential for human health. Mammals possess an 'extended genome' of millions of microbial genes located in the intestine: the microbiome. This multigenomic symbiosis is expressed at the proteomic and metabolic levels in the host and it has therefore been proposed that humans represent a vastly complex biological 'superorganism' in which part of the responsibility for host metabolic regulation is devolved to the microbial symbionts. Modern interpretation of the gut microbiome is based on a culture-independent, molecular view of the intestine provided by high-throughput genomic screening technologies. Also, the gut microbiome has been directly implicated in the etiopathogenesis of a number of pathological states as diverse as obesity, circulatory disease, inflammatory bowel diseases (IBDs) and autism. The gut microbiota also influences drug metabolism and toxicity, dietary calorific bioavailability, immune system conditioning and response, and post-surgical recovery. The implication is that quantitative analysis of the gut microbiome and its activities is essential for the generation of future personalized healthcare strategies and that the gut microbiome represents a fertile ground for the development of the next generation of therapeutic drug targets. It also implies that the gut microbiome may be directly modulated for the benefit of the host organism.

The gut microbiota therefore perform a large number of important roles that define the physiology of the host, such as immune system maturation, the intestinal response to epithelial cell injury, and xenobiotic and energy metabolism. In most mammals, the gut microbiome is dominated by four bacterial phyla that perform these tasks: Firmicutes, Bacteroidetes, Actinobacteria and Proteobacteria. The phylotype composition can be specific and stable in an individual, and in a 2-year interval an individual conserves over 60% of phylotypes of the gut microbiome. This implies that each host has a unique biological relationship with its gut microbiota, and by definition that this influences an individual's risk of disease.

Background art includes Payne et al., obesity reviews (2012) 13, 799-809; and Cowen et al The FASEB Journal. 2013;27:224.

US Patent Application No. 20100172874 relates to the gut microbiome as a biomarker and therapeutic target for energy harvesting, weight loss or gain, and/or obesity in a subject. In particular, it provides methods of altering and monitoring the relative abundance of Bacteroides and Firmicutes in the gut microbiome of a subject.

A. N. PAYNE ET AL, relates to "Gut microbial adaptation to dietary consumption of fructose, artificial sweeteners and sugar alcohols: implications for host-microbe interactions contributing to obesity", OBESITY REVIEWS, (20120611), vol. 13, no. 9, doi:10.1111/j.1467-789X.2012.01009.x, ISSN 1467-7881, pages 799 - 809.

THERESA E COWAN ET AL, relates to "Artificial sweetener consumption differentially affects the gut microbiota-host metabolic interactions -- Cowan et al. 27 (1): 224.7 -- The FASEB Journal", THE FASEB JOURNAL, (20130401), vol. 27, ISSN 0892-6638, page 224.7.

THOMPSON-CHAGOYAN O C ET AL, relates to "Faecal microbiota and short-chain fatty acid levels in faeces from infants with cow's milk protein allergy", INTERNATIONAL ARCHIVES OF ALLERGY AND IMMUNOLOGY, KARGER AG, CH, (20110101), vol. 156, no. 3, ISSN 1018-2438, pages 325 - 332.

CAO SEVERINE ET AL, relates to "The role of commensal bacteria in the regulation of sensitization to food allergens", FEBS LETTERS, ELSEVIER, AMSTERDAM, NL, (20140501), vol. 588, no. 22, doi:10.1016/J.FEBSLET.2014.04.026, ISSN 0014-5793, pages 4258-4266.

JOTHAM SUEZ ET AL, relates to "Artificial sweeteners induce glucose intolerance by altering the gut microbiota", NATURE, (20140917), doi:10.1038/nature13793, ISSN 0028-0836.

SUEZ J ET AL, relates to "Non-caloric artificial sweeteners and the microbiome: Findings and challenges", GUT MICROBES 20150401 TAYLOR AND FRANCIS INC. USA, (20150401), vol. 6, no. 2, doi:10.1080/19490976.2015.1017700, ISSN 1949-0976, pages 149 - 155.

MARIE S. A. PALMNÄS ET AL, relates to "Low-Dose Aspartame Consumption Differentially Affects Gut Microbiota-Host Metabolic Interactions in the Diet-Induced Obese Rat", PLOS ONE, (20141014), vol. 9, no. 10, doi:10.1371/journal.pone.0109841, page e109841.

THAISS CHRISTOPH A ET AL, relates to "Transkingdom Control of Microbiota Diurnal Oscillations Promotes Metabolic Homeostasis", CELL, CELL PRESS, US, (20141016), vol. 159, no. 3, doi:10.1016/J.CELL.2014.09.048, ISSN 0092-8674, pages 514 - 529.

THAISS C A ET AL, relates to "A day in the life of the meta-organism: Diurnal rhythms of the intestinal microbiome and its host", GUT MICROBES 20150422 TAYLOR AND FRANCIS INC. USA, (20150422), vol. 6, no. 2, doi:10.1080/19490976.2015.1016690, ISSN 1949-0976, pages 137 - 142.

## SUMMARY OF THE INVENTION

The present invention relates to a method of determining tolerance to a non-caloric substance in a healthy subject, comprising:

(a) determining a signature of a gut microbiome in a sample of the healthy subject who has been subjected to the non-caloric substance;
(b) comparing said signature of said gut microbiome of the healthy subject to at least one reference signature of a pathological gut microbiome, wherein when said signature of the gut microbiome of the healthy subject is statistically significantly similar to said reference signature of said pathological gut microbiome, it is indicative that the healthy subject is intolerant to the non-caloric substance; and
(c) providing a recommendation about the amount of said non-caloric substance that can be tolerated by the healthy subject.

Preferably, the method further comprises comparing said signature of said gut microbiome of the healthy subject to at least one non-pathological reference signature, wherein when said signature of said gut microbiome of the healthy subject is statistically significantly different to said at least one non-pathological reference signature, it is indicative that the healthy subject is intolerant to the non-caloric substance.

Preferably, said pathological gut microbiome is derived from a healthy subject who is intolerant to the non-caloric substance.

Preferably, said non-pathological gut microbiome is derived from a healthy subject who is tolerant to the non-caloric substance.

Preferably, the healthy subject has been subjected to the noncaloric substance at least one month prior to said determining.

Preferably, the healthy subject has been subjected to the noncaloric substance at least once a day.

Preferably, the healthy subject has been subjected to the noncaloric substance for at least one week.

## BRIEF DESCRIPTION OF THE SEVERAL VIEWS OF THE DRAWINGS

Some aspects of the disclosure are herein described, by way of example only, with reference to the accompanying drawings. With specific reference now to the drawings in detail, it is stressed that the particulars shown are by way of example and for purposes of illustrative discussion of aspects of the disclosure. In this regard, the description taken with the drawings makes apparent to those skilled in the art how aspects of the disclosure may be practiced.

In the drawings:

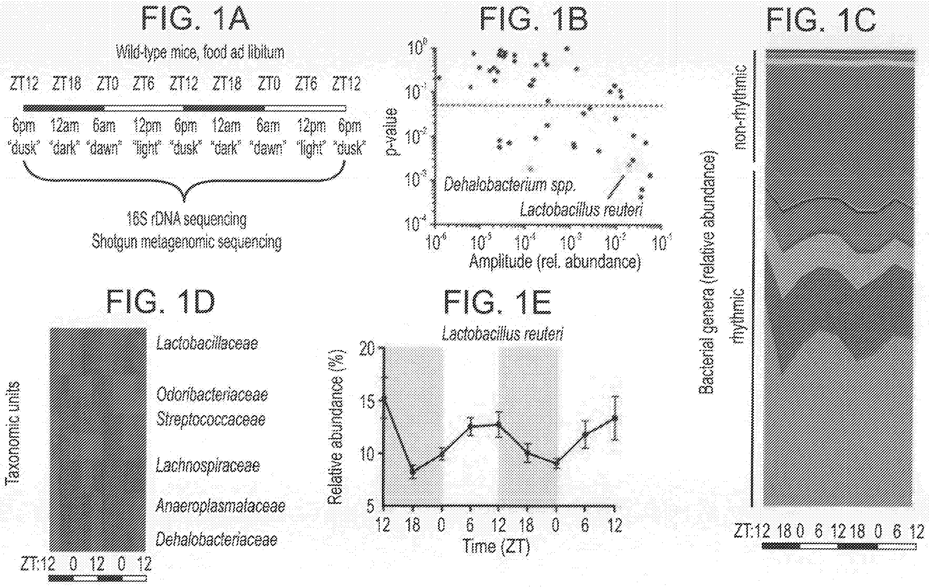

FIGs. 1A-K Intestinal microbiota exerts diurnal oscillations.

(A) Schematic showing sampling times of microbiota over the course of two light-dark cycles.
(B) OTUs showing diurnal oscillations. OTUs with p<l are shown and fluctuation amplitudes are indicated. Dashed line indicates $p<0.05$, JTK cycle; n = 10 individual mice at each Zeitgeber time (ZT).
(C) Taxonomic composition of fecal microbiota over the course of 48 hours.
(D) Histogram representation of bacterial genera oscillating with $p < 0.05$, JTK cycle. (E, F) Representative examples of diurnal oscillations in the abundance of microbiota members; n = 10 mice at each ZT.
(G, H) Histogram showing the distribution of standard deviation in gene occurrence of flagellar genes (G) and glycosaminoglycan (GAG) degradation genes versus other genes, normalized to the number of reads mapped to each gene.
(I) KEGG pathways showing diurnal oscillations. Only pathways with gene coverage >0.2 and p<l are shown. Dashed line indicates $p<0.05$, JTK cycle; n= 2 individual mice at each ZT.
(J) Representative examples of anti-phasic diurnal oscillations in the abundance of functional KEGG pathways; n = 2 individual mice at each ZT.
(K) Histogram representation of the 16 most significantly oscillating KEGG pathways, as identified by JTK cycle.

The results shown are representative of four experiments (1A-F) or two experiments (1G-K).

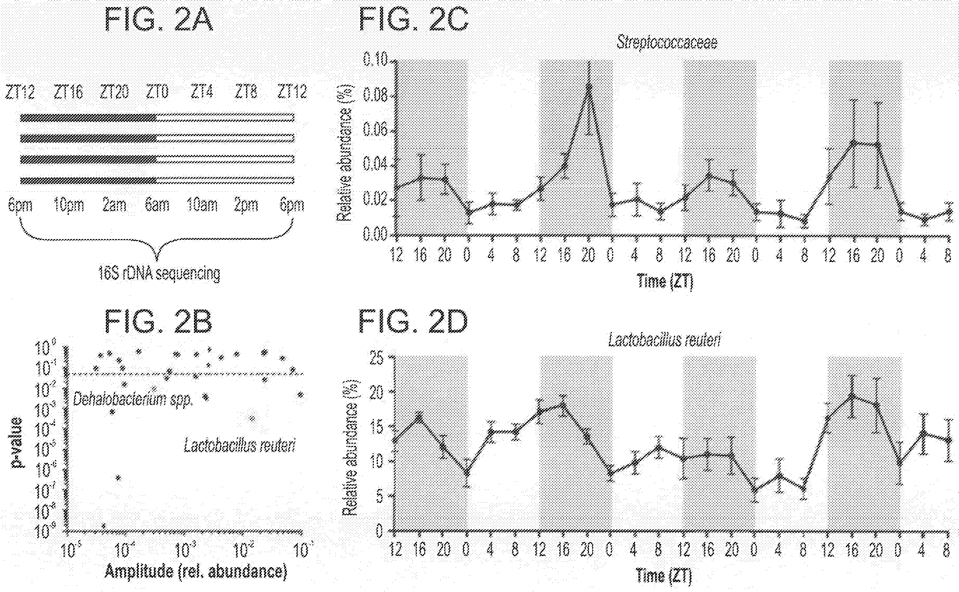

FIGs. 2A-H. Diurnal microbiota oscillations in composition and function, related to Figure 1A-K.

(A) Schematic showing sampling times of microbiota every four hours over the course of four light-dark cycles.
(B) OTUs showing diurnal oscillations. Only OTUs with p<l are shown. Dashed line indicates $p<0.05$, JTK_cycle; n = 8 individual mice at each Zeitgeber time (ZT).

(C-D) Representative examples of diurnal oscillations in the abundance of microbiota members; n = 8 mice at each ZT.

(E-G) Representative example of diurnal oscillations in the abundance of functional KEGG pathways; n = 2 individual mice at each ZT.

(H) Pathway depiction of genes involved in flagellar assembly, bacterial chemotyxis, and type III secretion. Colors indicate differential abundance of genes at different ZTs.

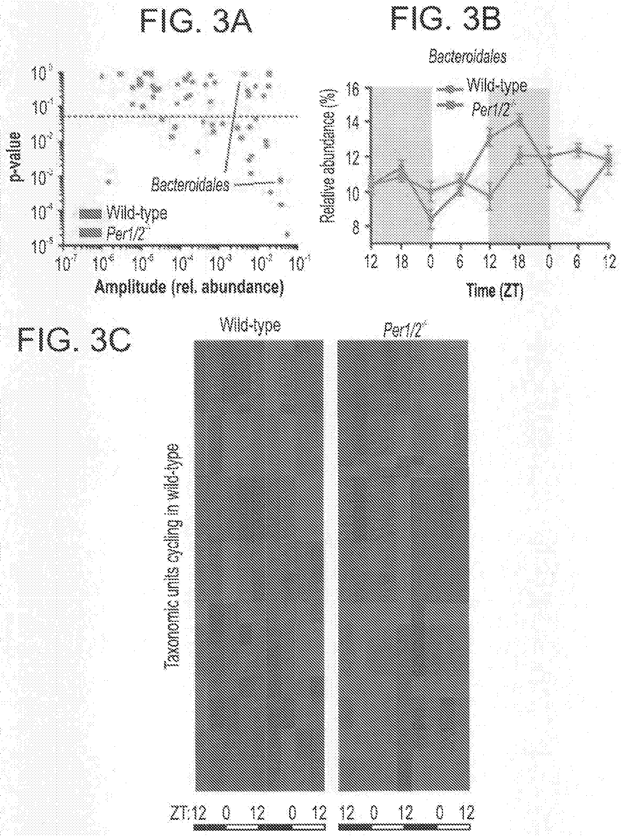

FIGs. 3A-H Loss of diurnal microbiota rhythms in *Per1/2*-deficient mice.

(A) OTUs showing diurnal oscillations in wild-type and *Per1/2*-deficient mice. Only OTUs with p<l are shown. Dashed line indicates p<0.05, JTK cycle; n = 10 individual mice in each group at each Zeitgeber time (ZT).

(B) Representative example of diurnal oscillations in wild-type mice, which are absent in *Per1/2*-deficient mice; n = 10 mice at each ZT.

(C) Histogram representation of bacterial genera oscillating with p<0.05, JTK cycle, in wild-type mice compared to *Per1/2*-deficient mice; n = 10 mice at each ZT.

(D) Histogram representation of diurnal fluctuations of KEGG pathways in microbiota from wild-type mice, which are absent in microbiota from *Per1/2*-deficient mice; metagenomics analysis was performed in a total of three mice at each ZT and only pathways with a coverage >0.2 were compared.

(E) KEGG pathways showing diurnal oscillations in wild-type compared to *Per1/2*-deficient mice. Only pathways with gene coverage >0.2 and p<l are shown. Dashed line indicates p<0.05, JTK cycle.

(F-H) Diurnal variations in genes belonging to the indicated functional pathways in wild-type and *Per1/2*-deficient mice. Metagenomics analysis was performed in a total of three mice at each ZT.

The results shown are representative of two experiments.

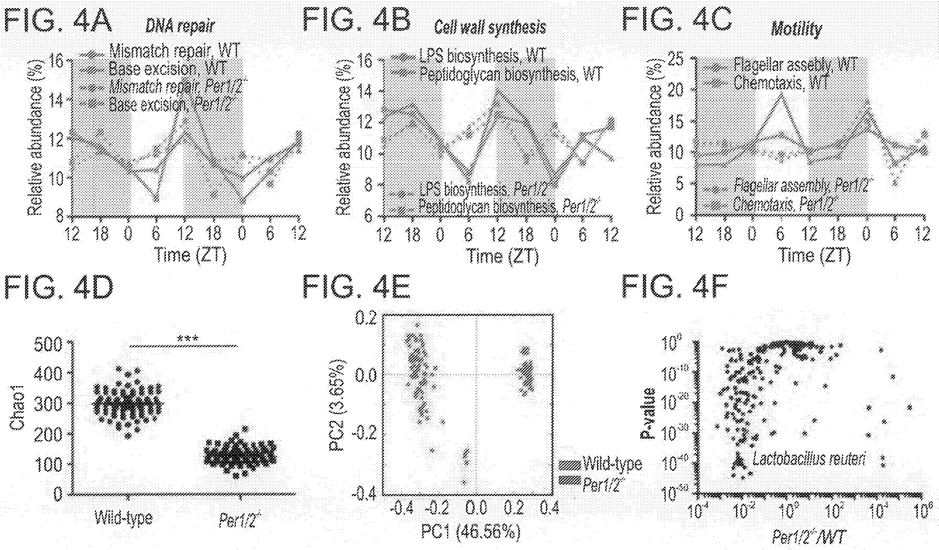

FIGs. 4A-L Dysbiosis in *Per1/2*-deficient mice, Related to Figures 3A-H.

(A-C) Diurnal variations in genes belonging to the indicated functional pathways in wild-type and *Per1/2*-deficient mice. Metagenomics analysis was performed in a total of three mice at each ZT.

(D, E) Alpha- (D) and beta-diversity (E) of fecal microbiota from wild-type and *Per1/2*-deficient mice. Samples per genotype are from different times of the day; n = 90 in each group.

(F) Differential abundance of OTUs between fecal microbiota from wild-type and *Per1/2*-deficient mice; n = 90 samples.

(G, H) Alpha- (G) and beta-diversity (H) of fecal microbiota from wild-type and *ASC*-deficient mice.

(I) OTUs showing diurnal oscillations in *ASC*-deficient mice. Only OTUs with p<l are shown. Dashed line indicates p<0.05, JTK_cycle; n = 8 individual mice at each ZT.

(J) Representative example of diurnal oscillations in the microbiota of *ASC*-deficient mice; n = 8 individual mice at each ZT.

(K, L) Diurnal feeding pattern in wild-type (K) and *Per1/2*-deficient mice (L). Mice were fed ad libitum and followed over three dark-light cycles. Examples shown are representative of 8 individual mice.

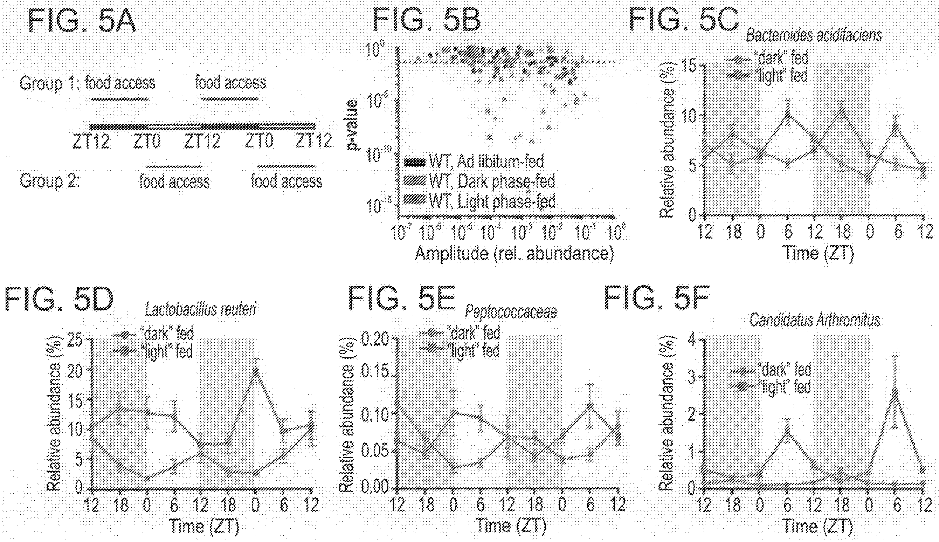

FIGs. 5A-L Feeding rhythms direct diurnal microbiota oscillations.

(A) Schematic showing timed feeding protocol.

(B) OTUs showing diurnal oscillations in wild-type mice on different feeding schedules. Only OTUs with p<l are shown. Dashed line indicates p<0.05, JTK_cycle; n = 10 individual mice at each ZT.

(C-F) Representative examples of phase shift in bacterial oscillations between dark phase-fed and light-phase fed wild-type mice; n= 10 mice at each ZT.

(G) OTUs showing diurnal oscillations in *Per1/2*-deficient mice on different feeding schedules. Only OTUs with p<l are shown. Dashed line indicates p<0.05, JTK_cycle; n = 10 individual mice at each ZT.

(H-I) Representative examples of phase shift in bacterial oscillations between dark phase-fed and light-phase fed *Per1/2*-deficient mice; n= 10 mice at each ZT.

(J) Quantification of oscillating OTUs with p<0.05, JTK cycle, in wild-type and *Per1/2*-deficient mice on different feeding schedules.

(K) Schematic showing fecal transplantation of microbiota from *Per1/2*-deficient mice (arrhythmic microbiota) into germ-free recipients (gain of rhythmicity).

(L) OTUs showing diurnal oscillations in microbiota from *Per1/2*-deficient mice before and after transplantation into germ-free mice. Only OTUs with p<l are shown. Dashed line indicates p<0.05, JTK_cycle; n = 10 individual mice at each ZT in each group. The results are representative of two independent experiments.

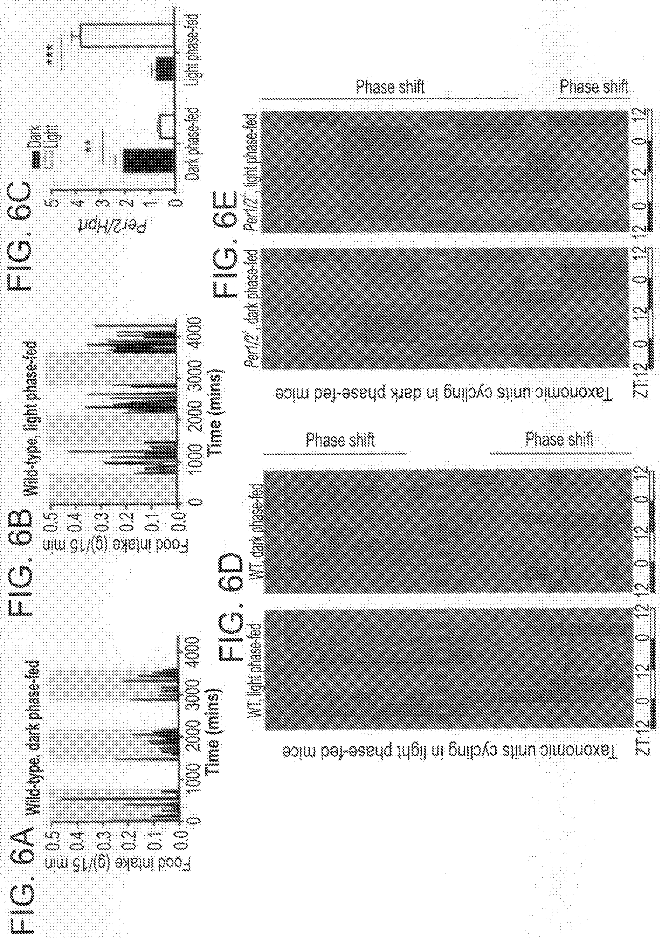

FIGs. 6A-H. The impact of feeding rhythms on diurnal microbiota oscillations, Related to Figures 5A-L.

(A, B) Feeding times in dark phase-fed (A), and light phase-fed (B) mice. Graphs shown are representative of four individual mice measured.

(C) Colonic expression of Per2 in dark phase-fed or light phase-fed mice during the dark phase and light phase shows reprogramming of the intestinal clock by feeding rhythms; n = 10 mice in each group. ** p < 0.01, *** p < 0.001.

(D, E) Histogram representation of bacterial genera oscillating with p<0.05, JTK cycle, in wild-type mice (D) and *Per1/2*-deficient mice (E) fed during the dark phase or light phase only; n = 10 mice at each ZT. Phase shifts in cycling OTUs are highlighted.

(F-H) Physical activity (F, H) and $VCO_2$ over the course of three dark-light cycles in germ-free mice transplanted with microbiota from *Per1/2*-deficient mice (F, G) or from wild-type mice (H). Measurements were taken one week after transplantation. The graph is representative of eight individual mice measured.

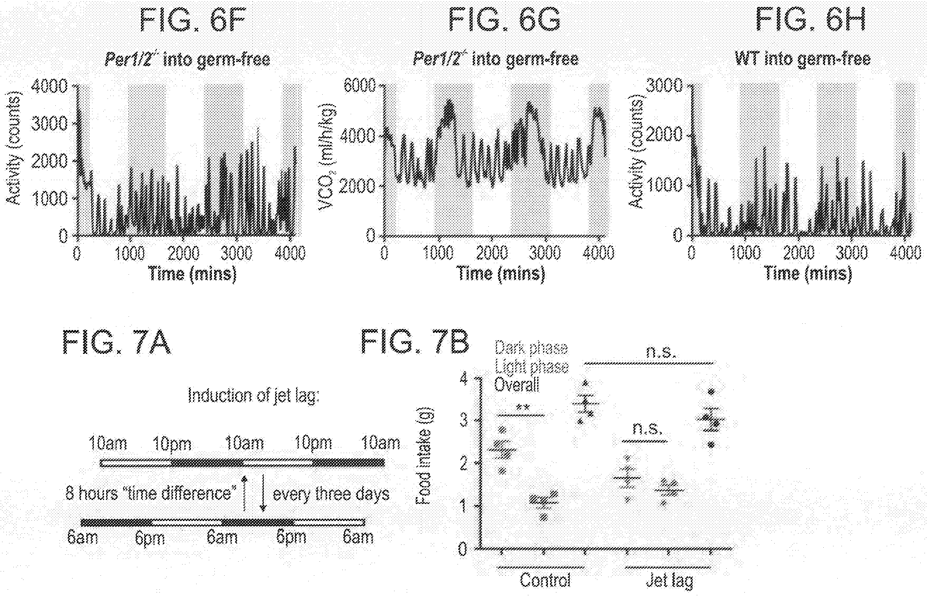

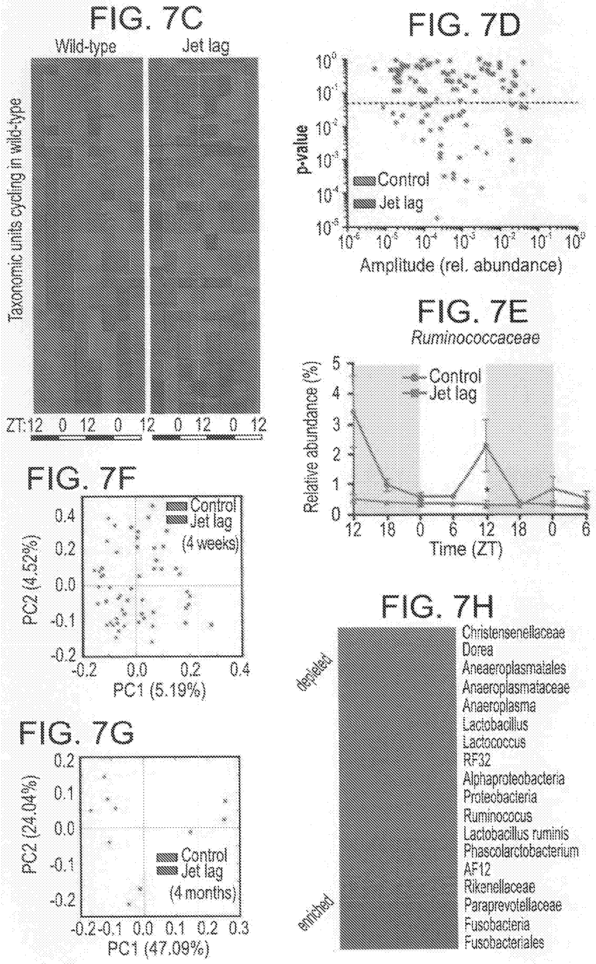

FIGs. 7A-H. Jet lag leads to loss of diurnal microbiota oscillations and dysbiosis.

(A) Schematic showing induction of jet lag by constant time shifting by 8 hours. Every three days, mice were subjected to a forward or backward shift of 8 hours. Controls remained under constant light-dark cycle conditions.

(B) Food intake of control and jet lag mice during the dark phase, light phase, and combined.** p<0.01, n.s. not significant.

(C) Heatmap representation of bacterial genera oscillating with p<0.05, JTK cycle, in control mice compared to jet lagged mice; n=5 mice at each time point.

(D) OTUs showing diurnal oscillations in control and jet lag mice. Dashed line indicates p<0.05, JTK_cycle; n=5 individual mice at each time point.

(E) Representative example of bacterial oscillations in wild-type mice which are lost under jet lag; n=5 mice at each time point.

(F) Beta-diversity of gut microbial communities in control and jet lag mice after four weeks of time shifts. Samples are pooled from different times of the day.

(G) Beta-diversity of gut microbial communities in control and jet lag mice after four months of time shifts.

(H) Heatmap representation of changes in microbial composition induced by jet lag.

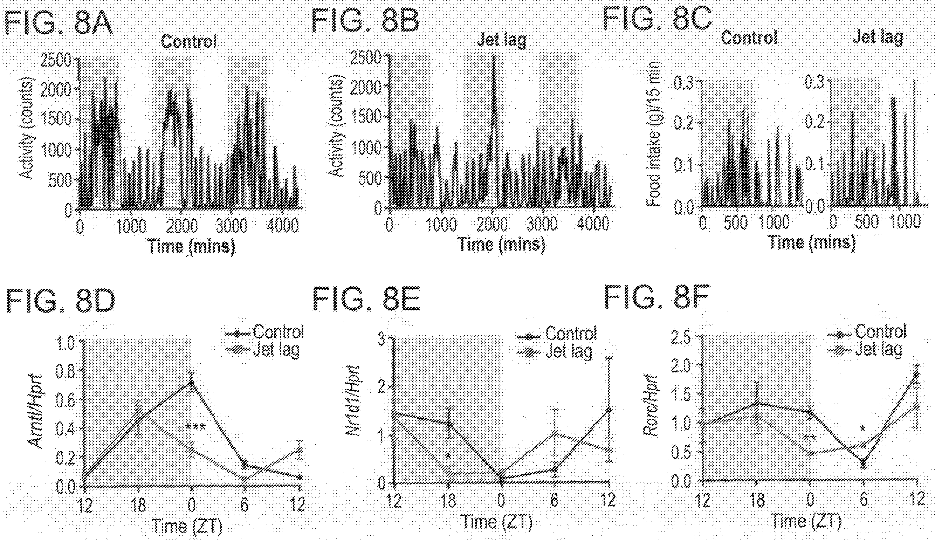

FIGs. 8A-F Circadian misalignment during jet lag, Related to Figures 7A-H.

(A, B) Physical activity over the course of three dark-light cycles in control and jet lag mice. Rhythmic activity of a control mouse (A) is converted into a random pattern by induction of jet lag (B). The results shown are representative of 16 individual mice measured.

(C) Diurnal variations in food intake of control and jet lag mice over the course of a dark-light cycle. The results shown are representative of 16 individual mice measured.

(D-F) Rhythmic expression patterns of Bmal (D), Rev-erbα (E) and RORγt (F) in the colon is altered upon jet lag induction; n = 4-5 mice at each ZT.

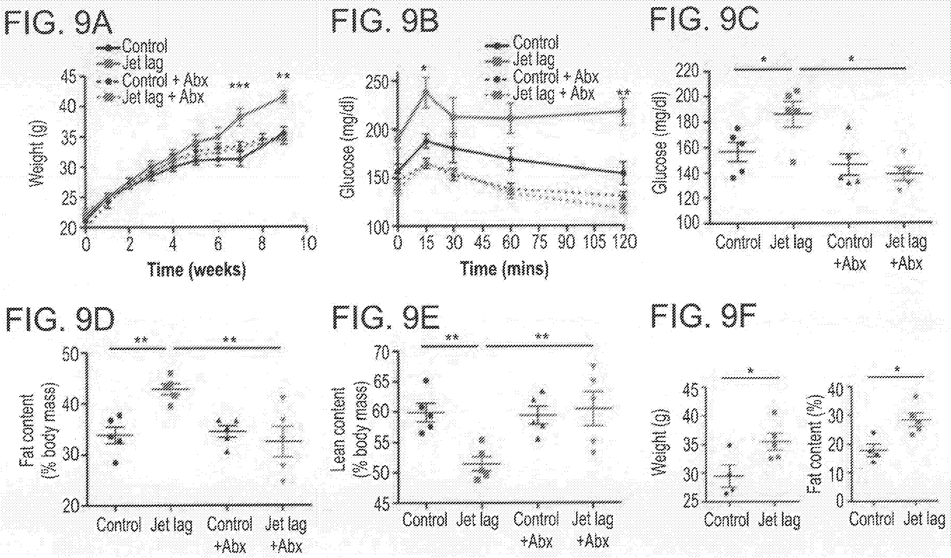

FIGs. 9A-K. Jet lag-induced dysbiosis promotes metabolic derangements.

(A-E) Mice underwent time shift-induced jet lag and were fed a high fat diet. Half of the mice were treated with antibiotics; n = 10 mice in each group.

(A) Weight gain over 9 weeks of high-fat feeding. ** p<0.01, *** p<0.001.

(B) Oral glucose tolerance test performed 8 weeks after initiation of jet lag. * p<0.05, ** p<0.01.

(C) Fasting glucose levels of control and jet lag mice, with or without Abx treatment, after 8 weeks of jet lag. * p<0.05.

(D, E) Fat (D) and lean (E) body mass of control and jet lag mice, with or without Abx treatment, after 8 weeks of jet lag. ** p<0.01.

(F) Weight and fat content of control and jet lag mice after 4 months of time shifts in the jet lag group. * p<0.05.

(G-I) Microbiota from control or jet lag mice was transplanted into germ-free (GF) mice; n = 4-6 mice in each group.

(G) Weight gain over 4 weeks. * p<0.05

(H) Oral glucose tolerance test performed on day 3 post fecal transfer. * p<0.05

(I) Fat and lean body mass in recipient mice one month post fecal transfer. ** p<0.01

(J) $T_2$-weighted MR images of control and jet lag mice after 8 weeks of jet lag. Above, coronal images; below, axial images.

(K) $T_2$-weighted MR images of recipient mice one month post fecal transfer. Above, coronal images; below, axial images.

The results shown are representative of three (9A-E) and two (9G-I) independent experiments.

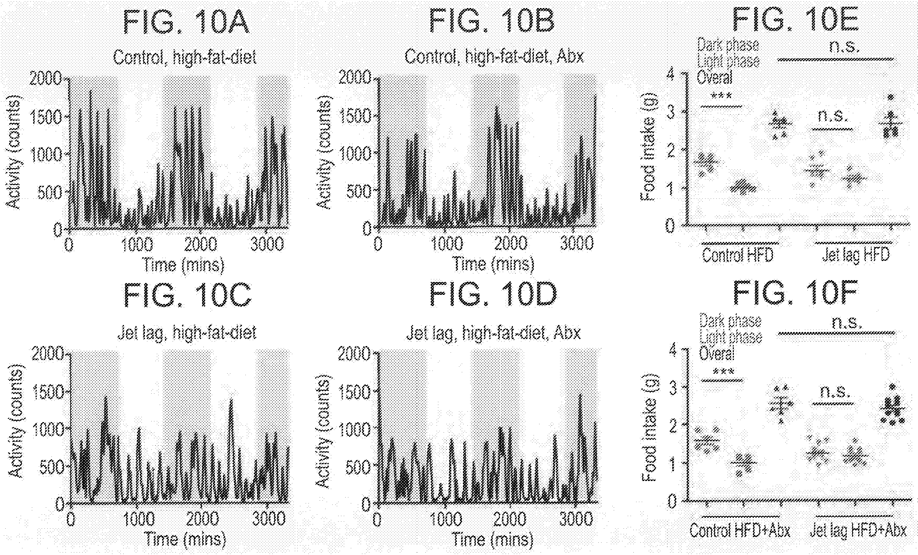

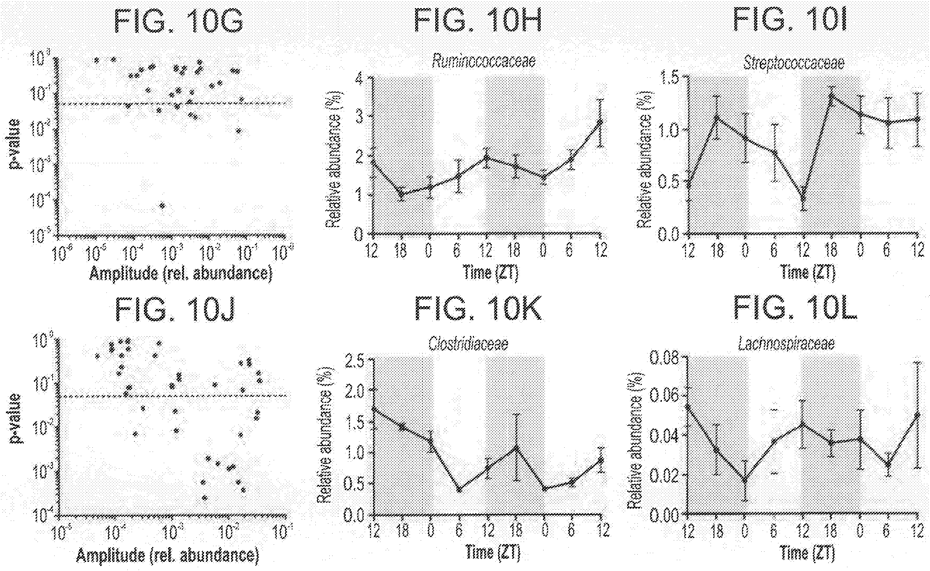

FIGs. 10A-L. The effect of high-fat diet and antibiotic treatment on diurnal behavior and microbiota oscillations, Related to Figures 9A-K.

(A-D) Physical activity over the course of 2.5 dark-light cycles in control (A, B) and jet lag mice (C, D) fed a high-fat diet. (B) and (D) are additionally treated with antibiotics. The results shown are representative of 32 individual mice measured.

(E, F) Diurnal variations in food intake of control and jet lag mice over the course of a dark-light cycle. All mice were fed a high-fat diet. In (F), mice were treated with antibiotics.

(G) OTUs showing diurnal oscillations in wild-type mice after one week on high-fat diet. Only OTUs with p<l are shown. Dashed line indicates p<0.05, JTK_cycle; n = 10 individual mice at each Zeitgeber time (ZT).

(H, I) Representative examples of diurnal oscillations in the abundance of microbiota members in mice on high-fat diet; n = 10 mice at each ZT.

(J) OTUs showing diurnal oscillations in wild-type mice after one week of antibiotic treatment. Only OTUs with p<l are shown. Dashed line indicates p<0.05, JTK cycle; n = 10 individual mice at each Zeitgeber time (ZT).

(K, L) Representative example of diurnal oscillations in the abundance of microbiota members in mice on antibiotics; n = 10 mice at each ZT.

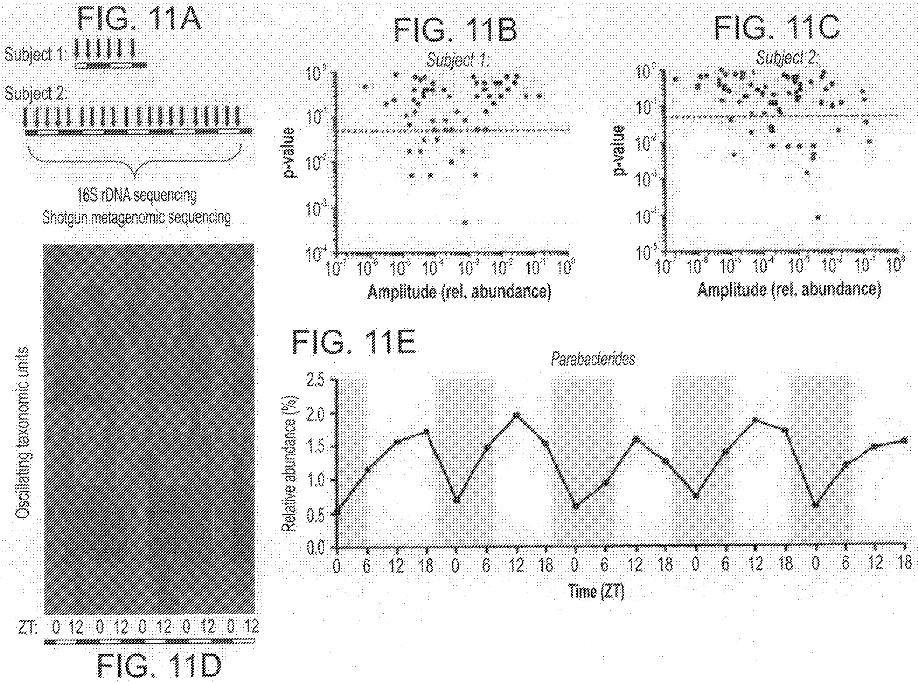

FIGs. 11A-I. Human microbiota undergoes diurnal oscillations in composition and function.

(A) Schematic showing sampling times of human microbiota from two subjects over the course of multiple light-dark cycles.

(B, C) OTUs showing diurnal oscillations in two human subjects. Only OTUs with p<l are shown. Dashed line indicates p<0.05, JTK cycle.

(D) Histogram representation of bacterial genera from one human subject oscillating with p<0.05, JTK cycle.

(E) Representative example of diurnal bacterial oscillations over five consecutive days.

(F) KEGG pathways showing diurnal oscillations. Only pathways with gene coverage >0.2 and p<l are shown. Dashed line indicates p<0.05, JTK_cycle.

(G, H) Examples of anti-phasic abundance peaks in KEGG pathways from human microbiota. ZT data are pooled from five consecutive days.

(I) Histogram representation of oscillations in KEGG pathways.

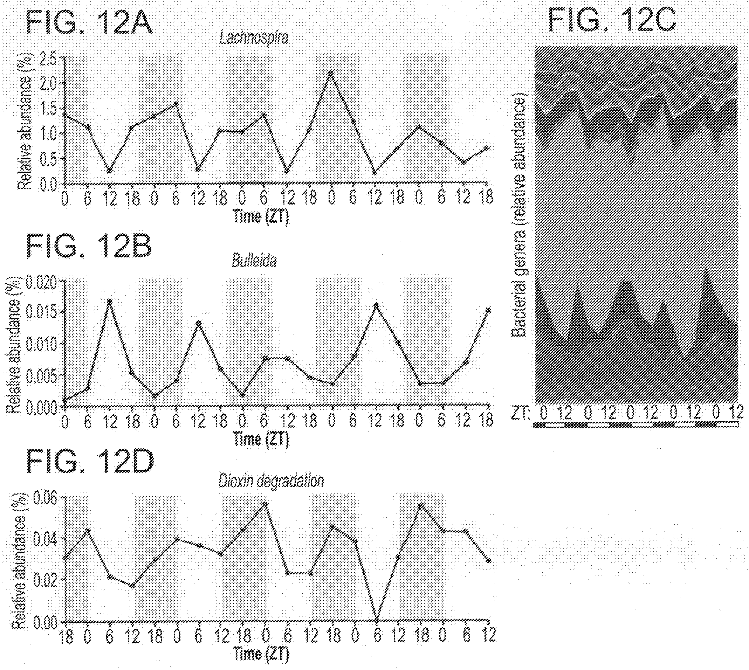

FIGs. 12A-D. Diurnal oscillations in the composition and function of human microbiota, Related to Figures 11A-I.

(A, B) Representative examples of diurnal fluctuations in the relative abundance of members of the commensal microbiota from one human subject over the course of five consecutive days.

(C) Oscillations in taxonomic composition of human fecal microbiota over the course of five days.

(D) Representative examples of diurnal fluctuations in functional pathways from human microbiota over the course of five consecutive days.

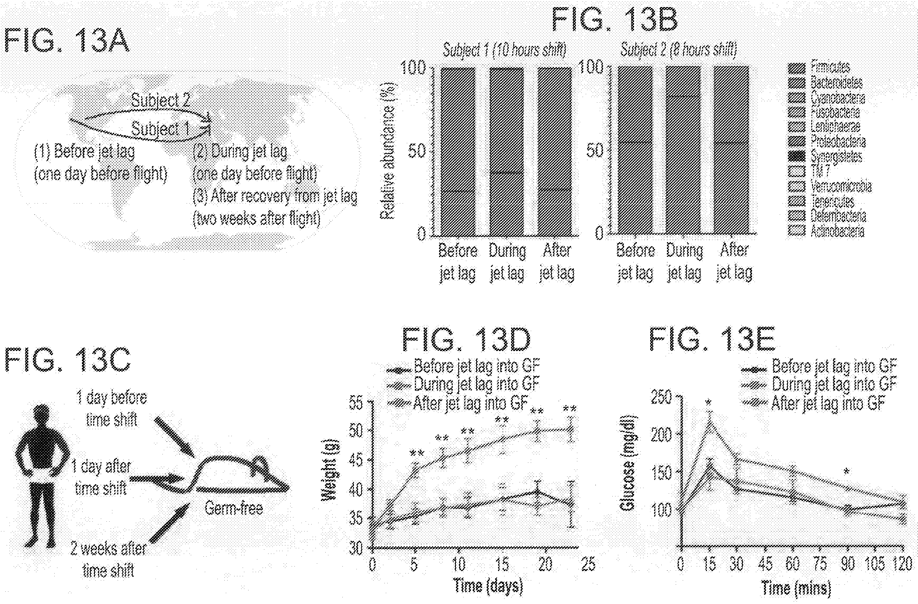

FIGs. 13A-F. Jet lag in humans is associated with dysbiosis that drives metabolic derangements.

(A) Schematic showing times of microbiota sampling from subjects before, during, and after jet lag induced by an 8-10-hour time shift.

(B) Phylum level composition of microbiota from two human subjects corresponding to the sampling times shown in (A).

(C) Schematic of fecal transplantation from human subjects before, during, and after jet lag into germ-free mice.

(D) Weight gain of recipient mice over three weeks; n = 5 mice in each group. ** p<0.01

(E) Oral glucose tolerance test of recipient mice performed on day 3 post fecal transfer; n = 5 mice in each group. * p<0.05

(F) $T_2$-weightd MR images of recipient mice performed three weeks after fecal transfer.

Above, coronal images; below, axial images.

The results shown are representative of 2 independent experiments (C-F).

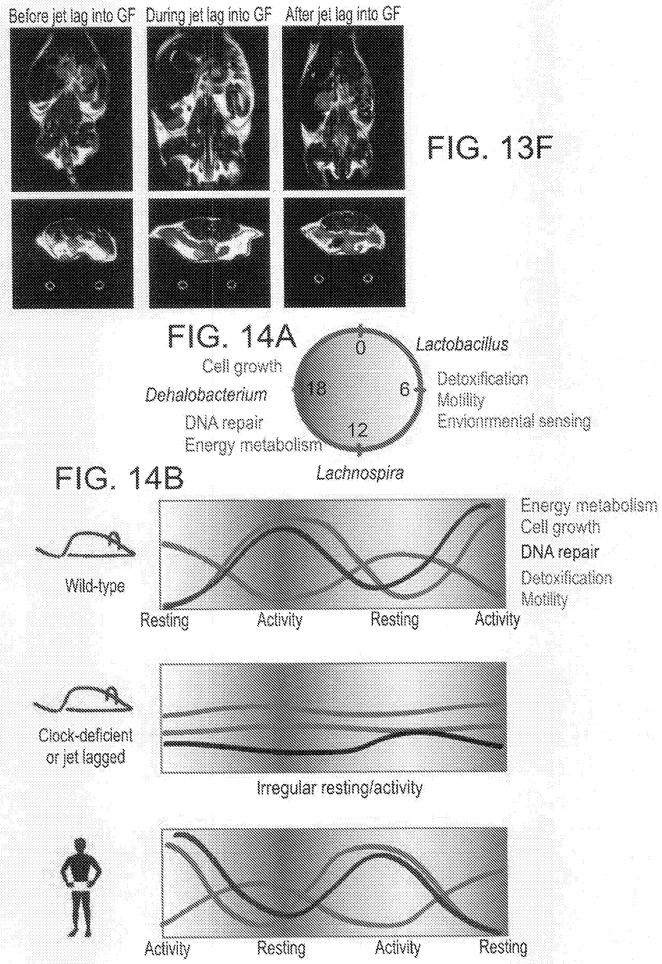

FIGs. 14A-B. Schematic of diurnal oscillations in microbiota composition and function Related to Figures 13A-F.

(A) Schematic showing "hallmark" taxonomic units and functional pathways with preferential abundance at certain times during a 24-hours light-dark cycle.

(B) Schematic depicting diurnal microbiota pathway activity in nocturnal wild-type mice, loss of oscillations in clock-disrupted mice, and phase-reversed fluctuations in humans.

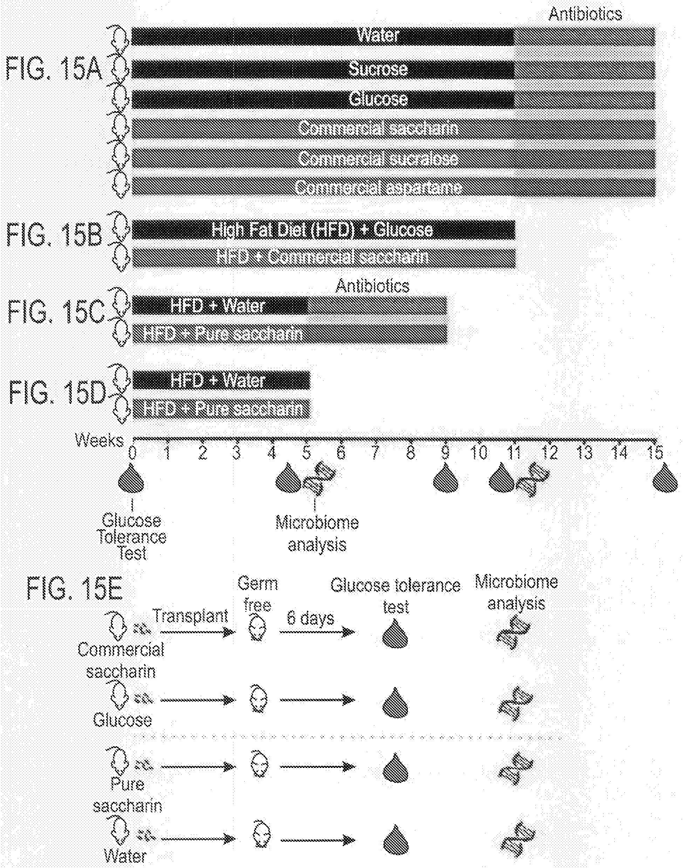

FIGs. 15A-E. Experimental scheme. 10 weeks old C57B1/6 male mice were treated with the following dietary regimes: (A) Drinking commercially available non-caloric artificial sweeteners (NAS; saccharin, sucralose and aspartame) and fed a normal chow (NC) diet (B) Drinking commercially available saccharin or glucose as control and fed a High-fat diet (HFD) (C) Drinking pure saccharin or water and fed HFD (D) as in III but with outbred Swiss-Webster mice. Glucose tolerance tests, microbiome analysis and supplementation of drinking water with antibiotics were performed on the indicated time points. (E) Schematic of fecal transplant experiments.

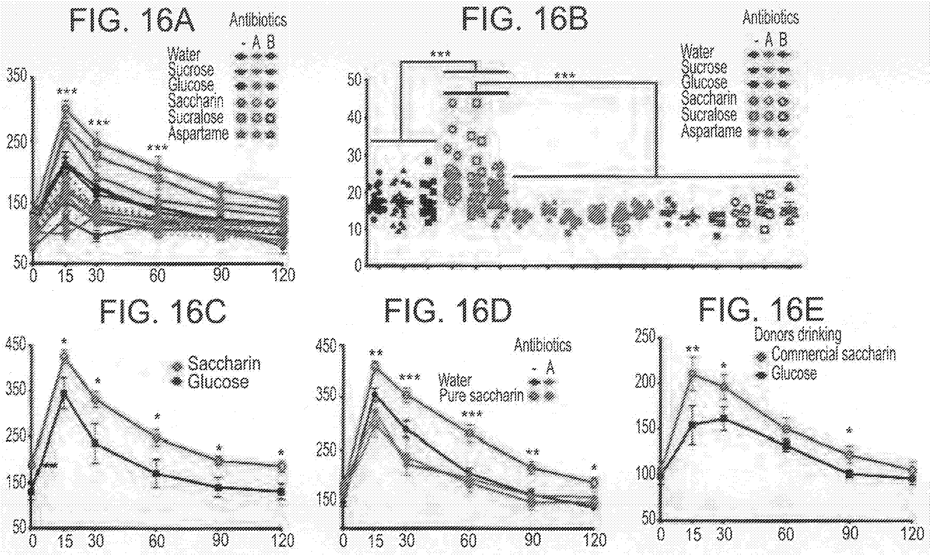

FIGs. 16A-H. Artificial sweeteners induce glucose intolerance transferrable to GF mice. (A-B) OGTT & AUC in NC-fed mice drinking commercial NAS for 11 weeks before (N=20) and after antibiotics: ciprofloxacin and metronidazole, ('Antibiotics A', N=10); or vancomycin ('Antibiotics B', N=5). (C) OGTT in mice fed HFD and commercial saccharin (N=10) or glucose (N=9). (D) OGTT of HFD-fed mice drinking 0.1 mgml$^{-1}$ saccharin or water for 5 weeks (N=20), followed by 'Antibiotics A' (N=10). (E-F) OGTT of GF mice 6 days following transplant of microbiota from (E) commercial saccharin- (N=12) and glucose-fed mice (N=11); or (F) pure saccharin- (N=16) and water-fed (N=16) donors. Symbols (GTT) or horizontal lines (AUC)- mean, error bars-S.E.M. *, $p<0.05$, **, $p<0.01$, ***, $p<0.001$, GTT: ANOVA & Bonferroni, AUC: ANOVA and Tukey post hoc analysis. Every experiment was repeated twice. (G) 16s rRNA analysis from: Saccharin-consuming mice at baseline (W0, black hexagons) and W11 (blue triangles); water controls (black circles for W11 & W0); glucose (black squares for W11 & W0); or sucrose (black triangles for W11 & W0). N=5 in each group. (H) PCoA of taxa in GF recipients according to donor identity in 1E.

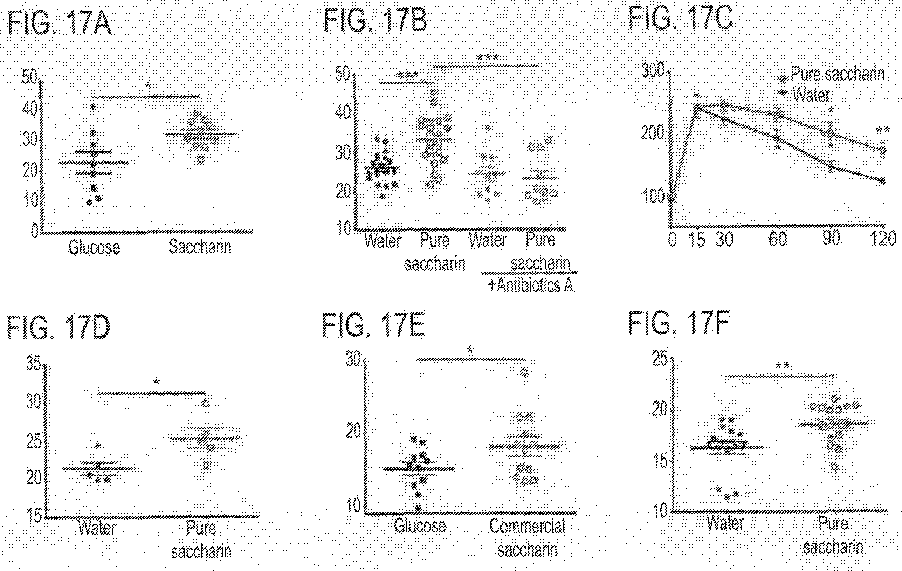

FIGs. 17A-F. Artificial sweeteners induce glucose intolerance. (A) AUC of mice fed HFD and commercial saccharin (N=10) or glucose (N=9). (B) AUC of HFD-fed mice drinking 0.1 mgml$^{-1}$ saccharin or water for 5 weeks (N=20), followed by 'Antibiotics A' (N=10). (C-D) OGTT & AUC of HFD-fed outbred Swiss-Webster mice (N=5) drinking pure saccharin or water. (E-F) Fecal samples were transferred from donor mice (N=10) drinking commercially available, pure saccharin, glucose or water controls into 8-week-old male Swiss-Webster germ-free recipient mice. AUC of GF mice 6 days following transplant of microbiota from (E) commercial saccharin- (N=12) and glucose-fed mice (N=11); or (F) pure saccharin- (N=16) and water-fed (N=16) donors. Symbols (GTT) or horizontal lines (AUC)- mean, error bars- S.E.M. *, $p<0.05$, **, $p<0.01$, ***, $p<0.001$, ANOVA and Tukey post hoc analysis (GTT) or unpaired two-sided Student *t-test* (AUC). Every experiment was repeated twice.

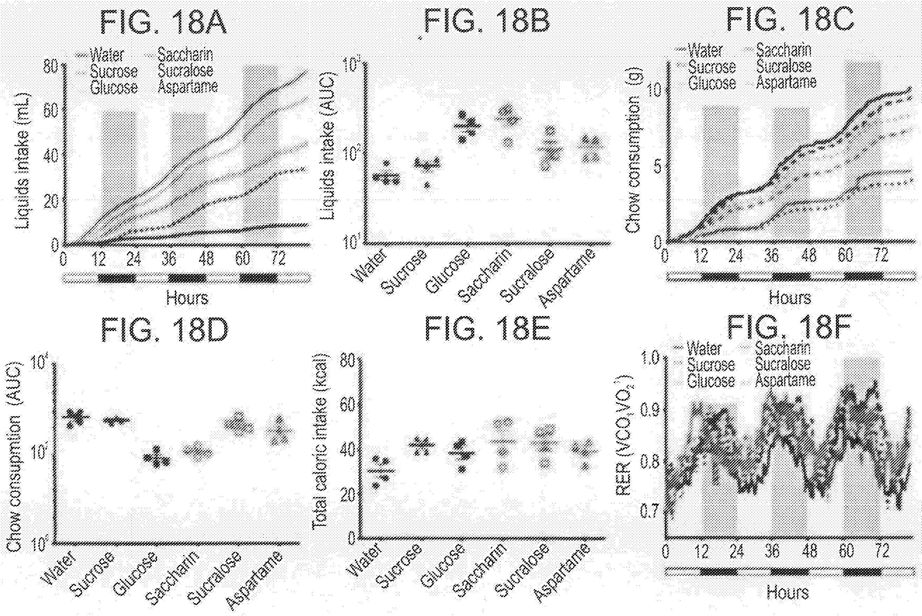

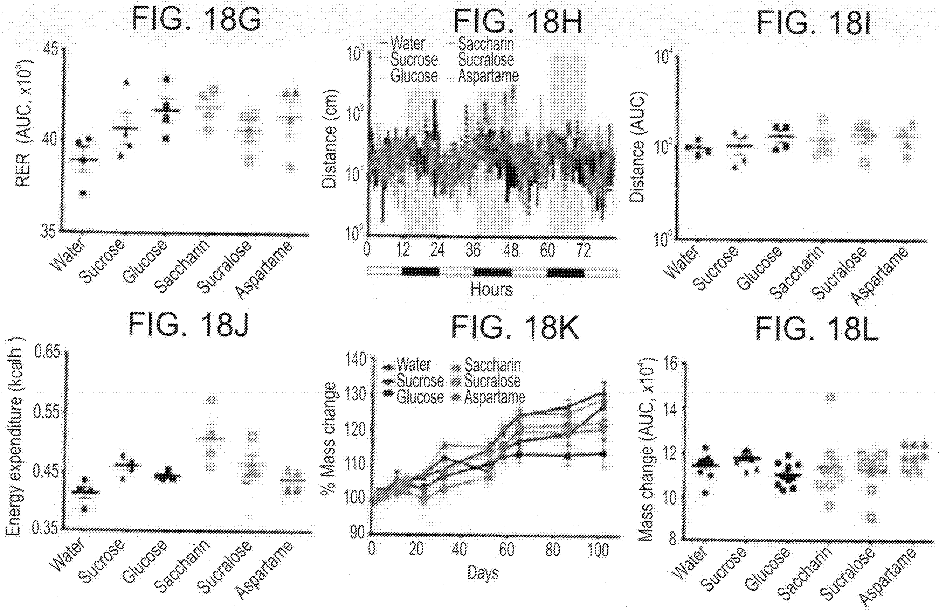

FIGs. 18A-L. Metabolic characterization of mice consuming commercial NAS formulations. 10 weeks old C57B1/6 mice (N=4) were given commercially available artificial sweeteners (saccharin, sucralose and aspartame) or controls (water, sucrose or glucose, N=4 in each group) and fed NC diet. After 11 weeks, metabolic parameters were characterized using the PhenoMaster metabolic cages system for 80 hours. Light and dark phases are denoted by white and black rectangles on the X-axis, respectively, and gray bars for the dark phase. (A) Liquids intake. (B) AUC of A. (C) Chow consumption. (D) AUC of C. (E) Total caloric intake from chow and liquid during 72 hours (see methods for calculation). (F) Respiratory exchange rate (RER). (G) AUC of F. (H) Physical activity as distance (I) AUC of H. (J) Energy expenditure. (K) Mass change compared to original mouse weight during 15 weeks (N=10). (L) AUC of K. The metabolic cages characterization and weight gain monitoring were repeated twice.

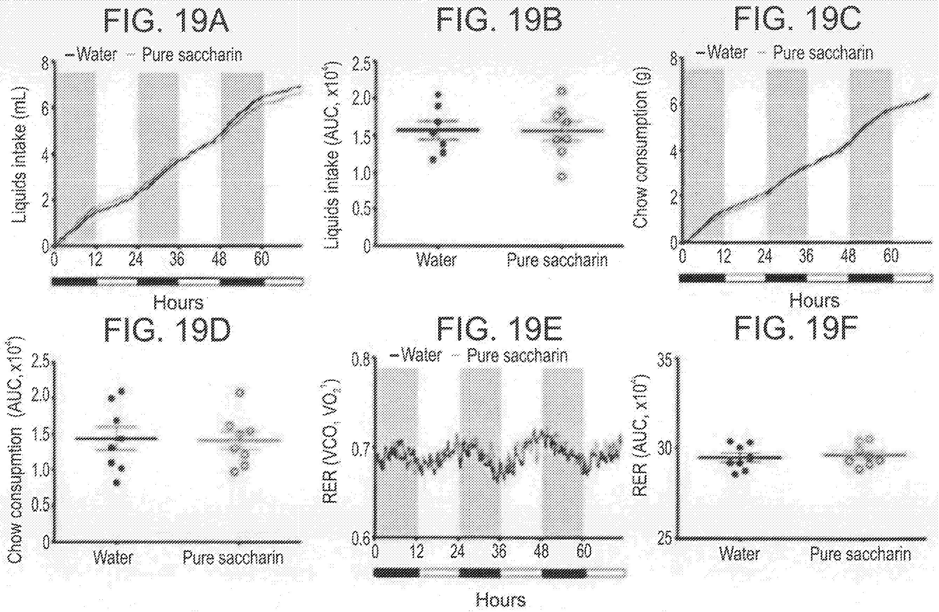

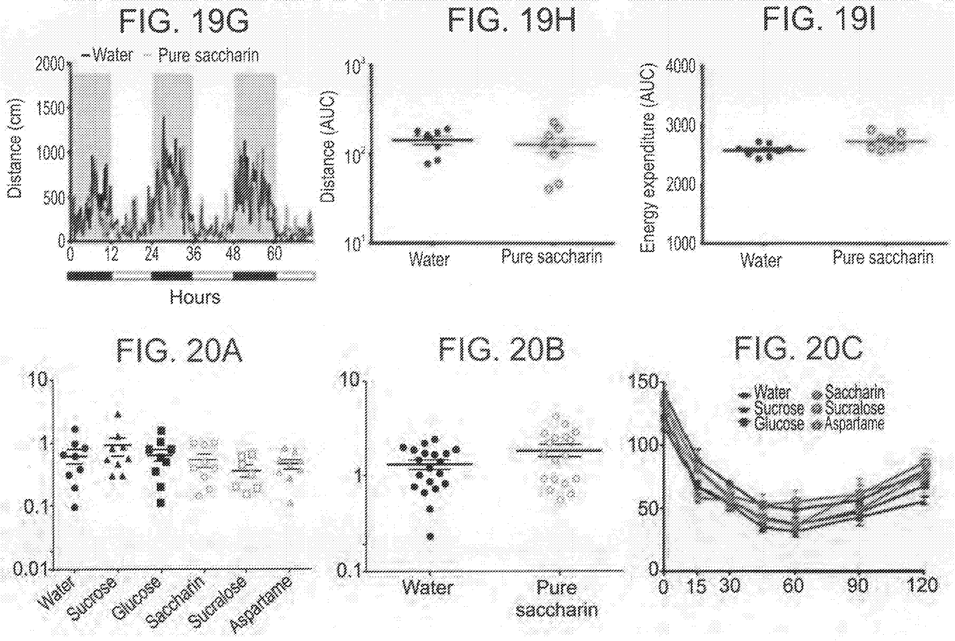

FIGs. 19A-I. Metabolic characterization of mice consuming HFD and pure saccharin or water. 10 weeks old C57B1/6 mice (N=8) were fed HFD, with or without supplementing drinking water with 0.1mgml$^{-1}$ pure saccharin. After 5 weeks, metabolic parameters were characterized using the PhenoMaster metabolic cages system for 70 hours. Light and dark phases are denoted by white and black rectangles on the X-axis, respectively, and gray bars for the dark phase. (A) Liquids intake. (B) AUC of A. (C) Chow consumption. (D) AUC of C. (E) Respiratory exchange rate (RER). (F) AUC of F. (G) Physical activity as distance. (H) AUC of H. (I) Energy expenditure. The metabolic cages characterization was repeated twice.

FIGs. 20A-C. Glucose intolerant NAS-drinking mice display normal insulin levels and tolerance. (A) Fasting plasma insulin measured after 11 weeks of commercial NAS or controls (N=10). (B) Same as A, but measured after 5 weeks of HFD and pure saccharin or water (N=20). (C) Insulin tolerance test performed after 12 weeks of commercial NAS or controls (N=10). All measurements were performed on two independent cohorts.

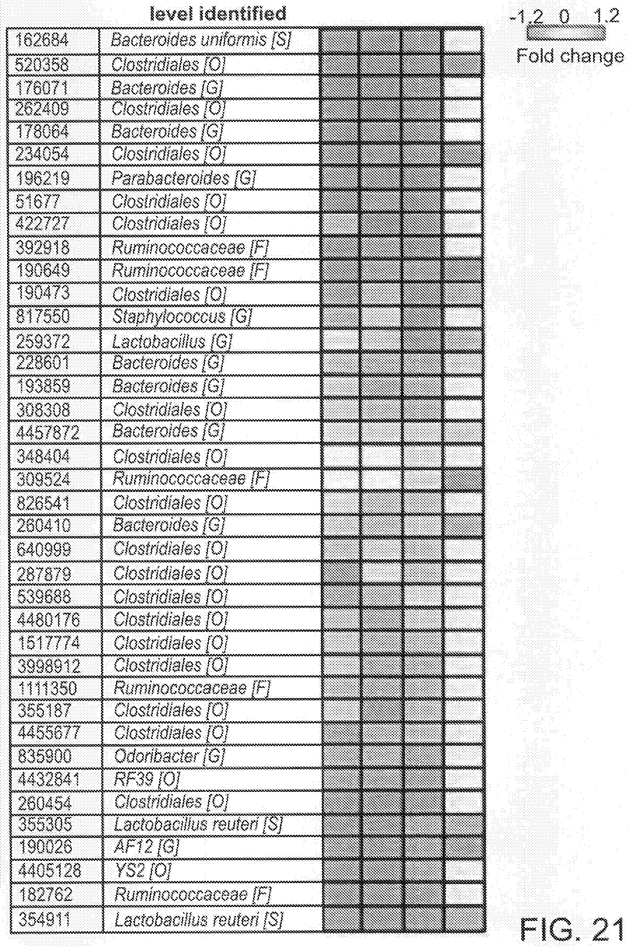

FIG. 21. Dysbiosis in saccharin-consuming mice and GF recipients. Heat-map representing W11 logarithmic-scale fold taxonomic differences between commercial saccharin and water or caloric sweetener consumers (N=5 in each group). Right column- taxonomical differences in GF mice following fecal transplantation from commercial saccharin- (recipients N=15) or glucose-consuming mice (N=13). OTU number (GreenGenes) and the lowest taxonomic level identified is denoted.

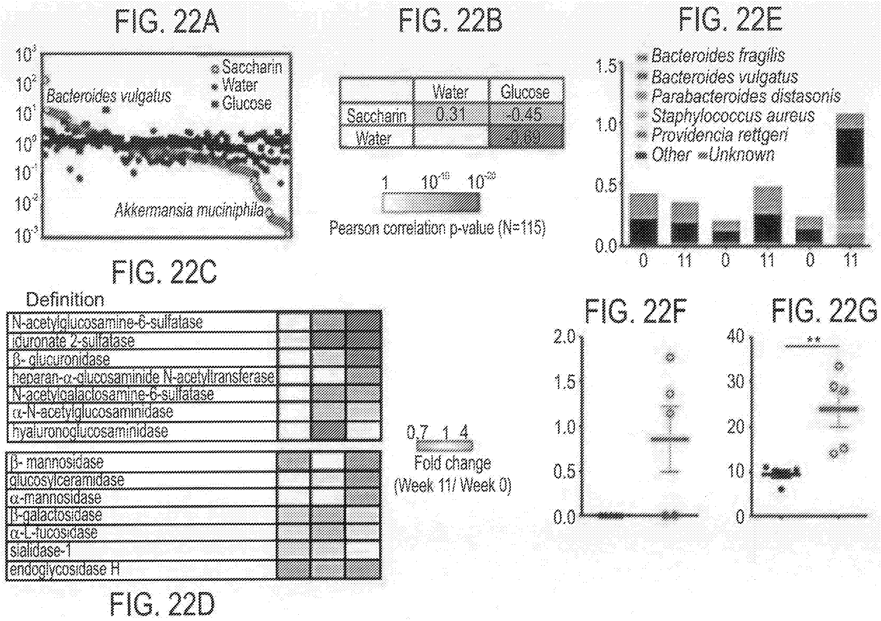

FIGs. 22A-G. Functional characterization of Saccharin-modulated microbiota. (A) Species alterations in mice consuming commercial saccharin, water or glucose for 11 weeks (N=4) shown by shotgun sequencing. (B) Pairwise correlations between changes in 115 KEGG pathways across mice receiving different treatments. (c-d) Fold change in relative abundance of (C) glycosaminoglycan or (D) other glycan degradation pathways genes. (E) Higher glycan degradation attributed to five taxa in the commercial saccharin setting. (F-G) Levels of fecal acetate and propionate at W11 in mice drinking commercial saccharin or glucose (N=5). Horizontal lines- mean, error bars- S.E.M. **, p<0.01 by two-sided Student *t-test.* SCFA measurements were performed on two independent cohorts.

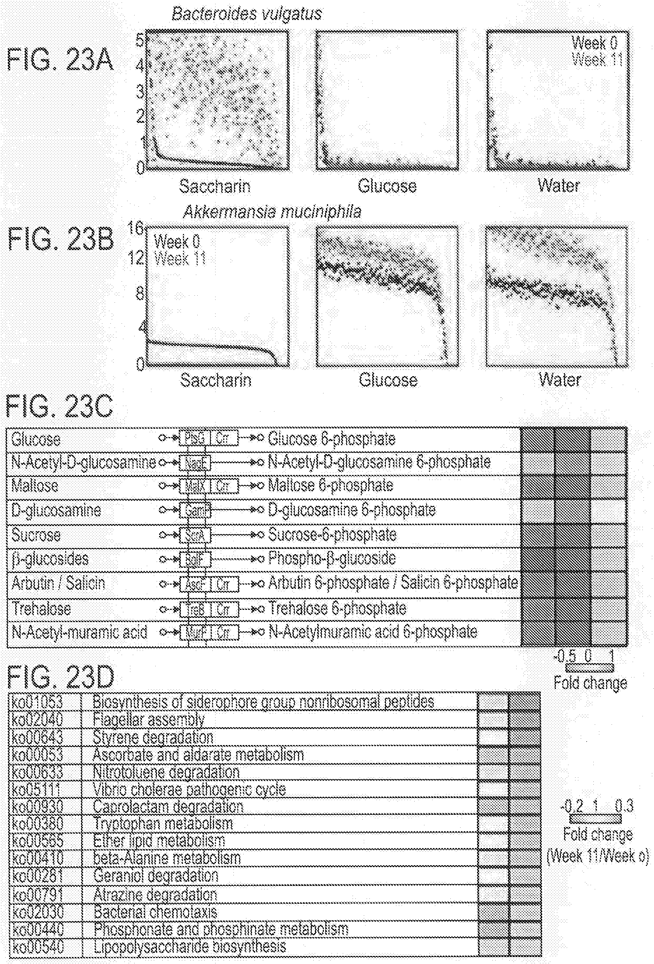

FIGs. 23A-D. Functional analysis of saccharin-modulated microbiota. (A-B) Changes in bacterial relative abundance occur throughout the bacterial genome. Shown are changes in sequencing coverage along 10,000 bp genomic regions of (A) *Bacteroides vulgatus.* (B) *Akkermansia muciniphila,* with bins ordered by abundance in week 0 of saccharin treated mice. (C) Fold change in relative abundance of modules belonging to phosphotransferase systems (PTS) between week 11 and week 0 in mice drinking commercial saccharin, glucose or water. Module diagram source: KEGG database (D) Enriched KEGG pathways (Fold change > 1.38 as cutoff) in mice consuming HFD and pure saccharin vs. water compared to the fold change in relative abundance of the same pathways in mice consuming commercial saccharin (week 11/week 0).

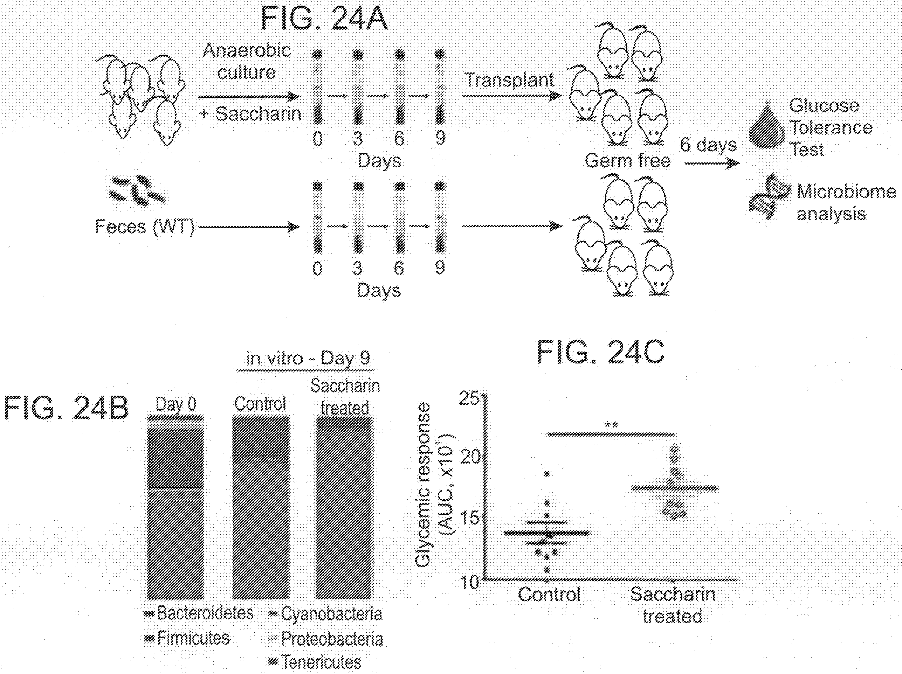

FIGs. 24A-C. Saccharin directly modulates the microbiota. (a) Experimental Schematic (b) Relative taxonomic abundance of anaerobically cultured microbiota. (c) AUC of germ-free mice 6 days following transplantation with saccharin-enriched or control fecal cultures (N=10 & N=9, respectively). Horizontal lines- mean, error bars-S.E.M. **, p<0.01, unpaired two-sided Student *t-test.* The experiment was repeated twice.

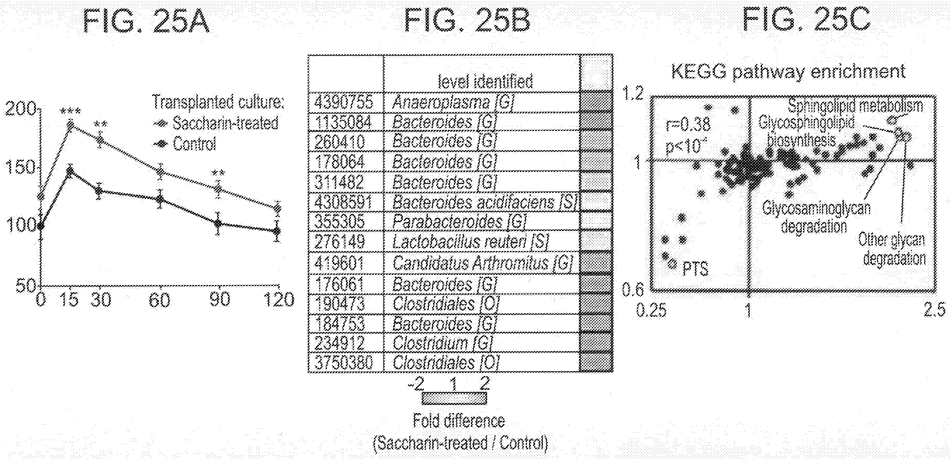

FIGs. 25A-C. Saccharin directly modulates the microbiota. (A) OGTT of germ-free mice 6 days following transplantation with saccharin-enriched or control fecal cultures (N=10 & N=9, respectively). Symbols- mean, error bars- S.E.M. **, p<0.01, ***, p<0.001, ANOVA & Bonferroni post-hoc analysis. Experiments were repeated twice. (B) Taxa representation in germ-free mice from a. (C) Comparison of KEGG pathway abundance between W11 saccharin-consuming mice (compared to W0, x-axis) and GF mice transplanted with microbiomes anaerobically cultured with 5mgml$^{-1}$ saccharin (compared to PBS, y-axis).

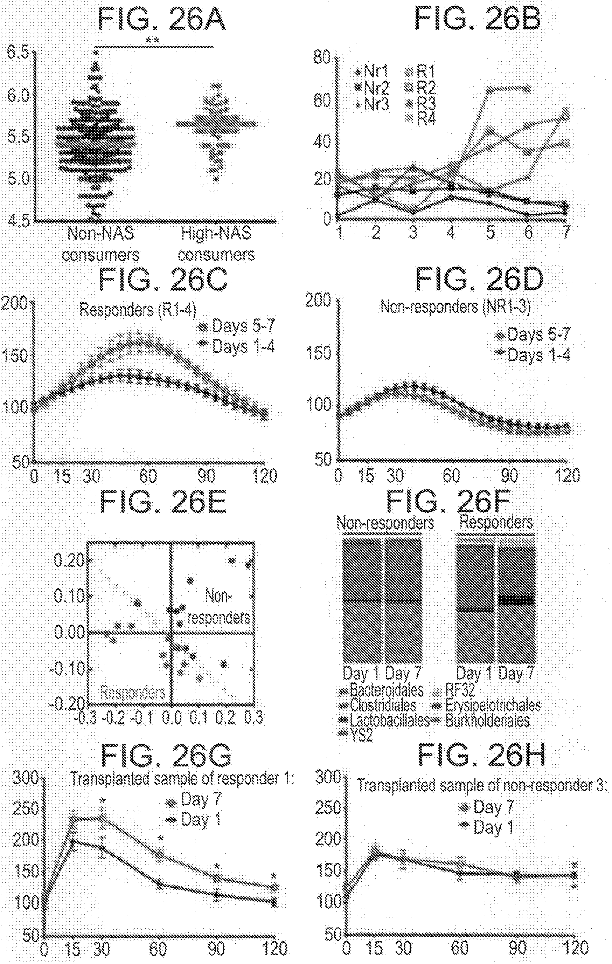

FIGs. 26A-H. Acute saccharin consumption impairs glycemic control in humans by inducing dysbiosis. (A) HbAlC% of high NAS consumers (N=40) vs. non-consumers (N=236). **, Ranksum p-value< 0.002. (B) Daily incremental area under the curve (iAUC) of OGTT in 4 responders (R) and 3 non-responders (NR). (C-D) OGTT of days 1-4 vs. days 5-7 in (C) 4 responders or (D) 3 non-responders. (E) PCoA of 16S rRNA sequences from responders (N samples=16) vs. non-responders (N=9) during days 1-4. (F) Order-level relative abundance of taxa samples from days 1 and 7 of responders and non-responders. (G-H) OGTT in GF mice (N=6) 6 days following fecal transplantation of D1 or D7 samples of (G) responder 1 (R1) or (H) non-responder 3 (NR3). Symbols- mean, error bars- S.E.M. *, p<0.05, **, p<0.01, ANOVA & Bonferroni post-hoc analysis.

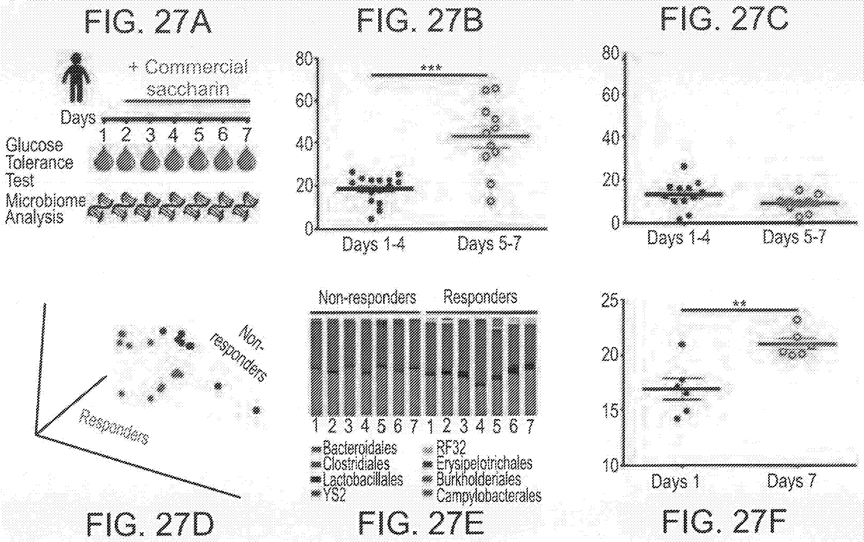

FIGs. 27A-L. Impaired glycemic control associated with acute saccharin consumption in humans is transferable to GF mice. (A) Experimental schematic (N=7). (b-c) iAUC of days 1-4 vs. days 5-7 in (B) 4 responders or (C) 3 non-responders. (D) Principal coordinates analysis (PCoA) of weighted UniFrac distances of 16S rRNA sequences demonstrating separation on principal coordinates 2 (PC2), 3 (PC3) and 4 (PC4) of microbiota from responders (N samples=12) vs. non-responders (N=8) during days 5-7. (E) Order-level relative abundance of taxa samples from days 1-7 of responders and non-responders. (F) AUC in GF mice (N=6) 6 days following fecal transplantation from samples of responder 1 (R1) collected before and after 7 days of saccharin consumption. (G-H) OGTT and AUC in GF mice (N=5) 6 days after receiving fecal samples collected from responder 4 (R4) before and after 7 days of saccharin consumption. (I) AUC in GF mice (N=5) 6 days following fecal transplantation from samples of non-responder 3 (NR3) collected before and after 7 days of saccharin consumption. (J-K) OGTT and AUC in GF mice (N=5) 6 days after receiving fecal samples collected from non-responder 2 (NR2) before and after 7 days of saccharin consumption. (L) Fold taxonomical abundance changes of selected OTUs, altered in GF recipients of D7 vs. D1 microbiomes from R1. Dot color corresponds to panel 10f Bacterial order. Symbols (GTT) or horizontal lines (AUC)- mean, error bars-S.E.M. *, p<0.05, **, p<0.01, ***, p<0.001, two-way ANOVA and Bonferroni post-hoc analysis (GTT), unpaired two-sided Student *t-test* (AUC).

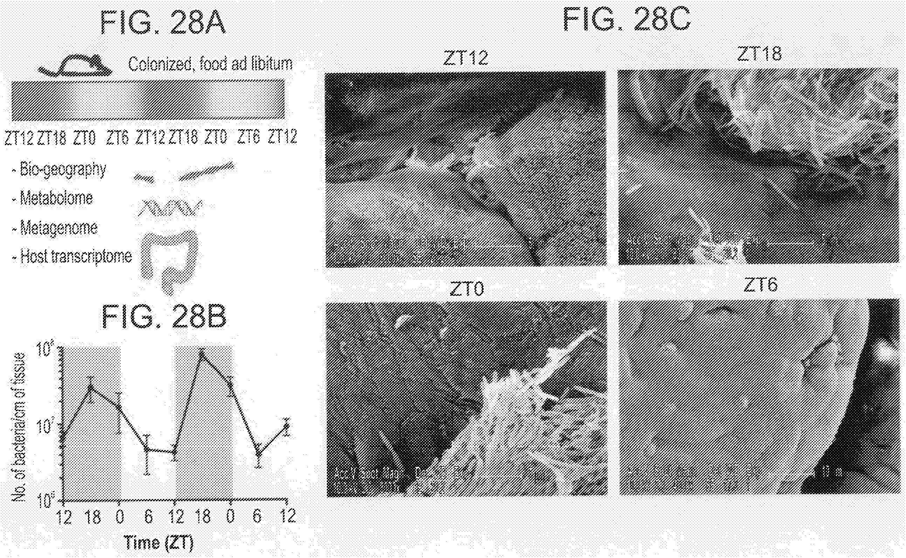

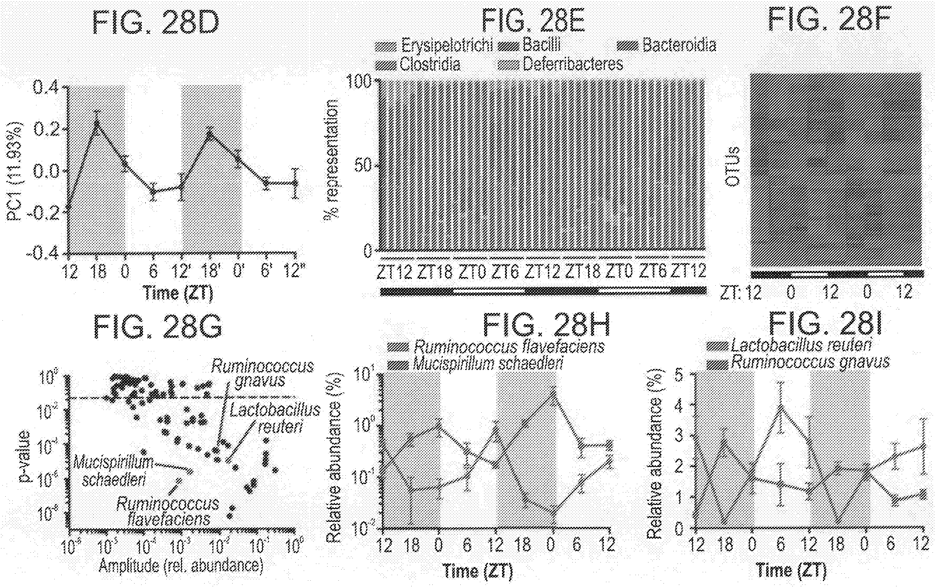

FIGs. 28A-I. Microbiota adherence to the colonic epithelium undergoes diurnal fluctuations.

(A) Schematic showing sampling times for microbiota epithelial attachment, metabolome, metagenome, and colonic transcriptome. (B) Diurnal fluctuations in the number of bacteria attached to colonic epithelium over two dark-light cycles as determined by bacterial qPCR of adherent communities. (C) SEM images showing diurnal fluctuations in epithelial colonization by bacteria. Images are representative of 10 randomly chosen views per mouse. (D) Beta-diversity of mucosal-adherent bacteria over the course of two light-dark cycles. (E) Relative taxonomic composition of mucosal-adherent bacteria over the course of two light-dark cycles. (F) Heatmap representation of the most significantly oscillating mucosal-adherent operational taxonomic units (OTUs), p<0.05 and q<0.2, JTK_cycle. (G) Epithelial-adherent OTUs showing diurnal oscillations in relative abundance. Fluctuation amplitudes are depicted. Dashed line indicates p<0.05, JTK cycle. (H, I) Examples of bacterial species showing fluctuating relative abundance in mucosal-adherent communities.

Data are representative of two independent experiments with N=45 mice.

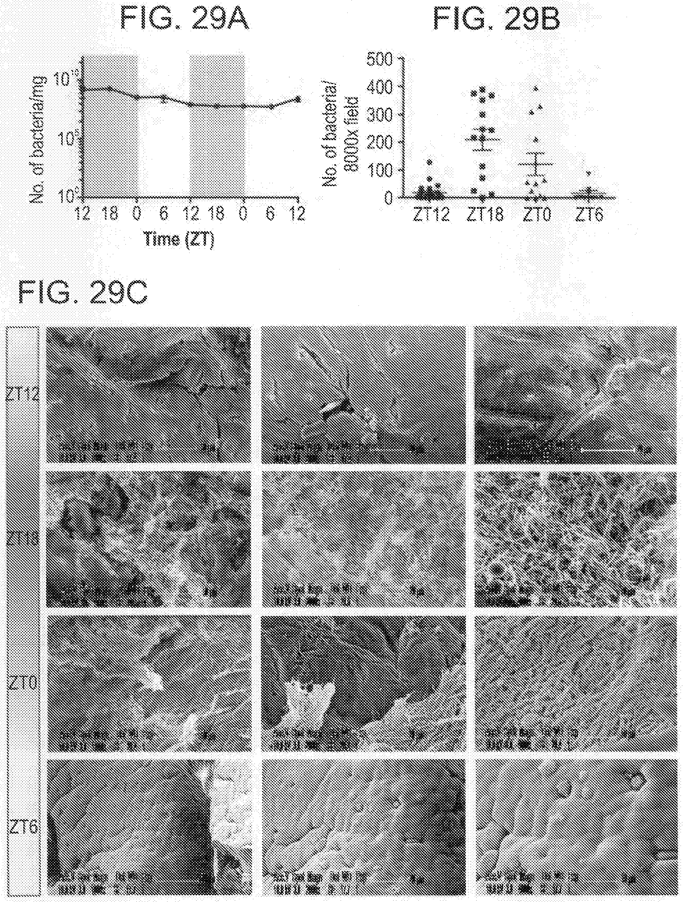

FIGs. 29A-C. Diurnal fluctuations in the number and composition of mucosal-associated commensals.

(A) Total number of luminal bacteria of the large intestine over the course of two days. (B, C) Quantification (B) and representative SEM images (C) showing diurnal fluctuations in epithelial colonization by bacteria over the course of a day. Quantification was done on 10 randomly selected images per mouse.

Data are representative of two independent experiments with N=45 mice.

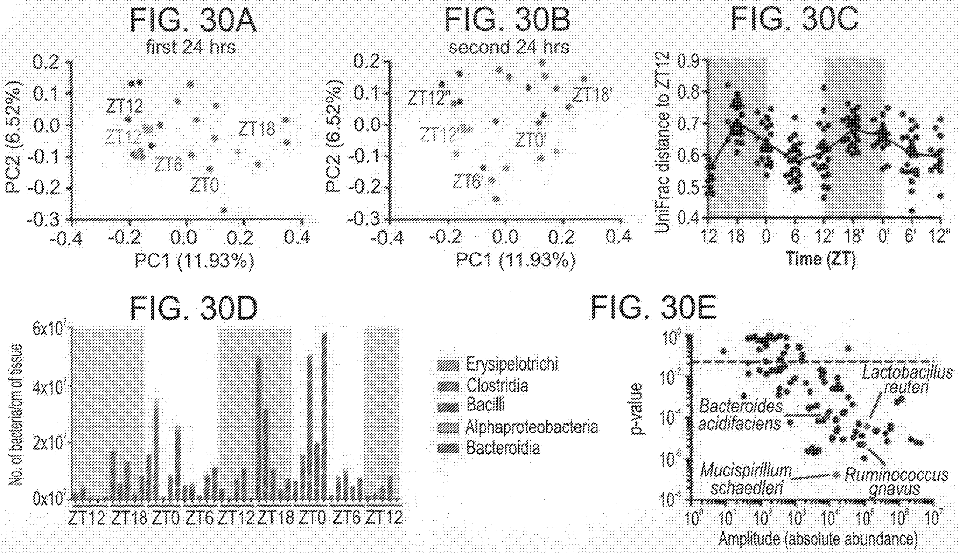

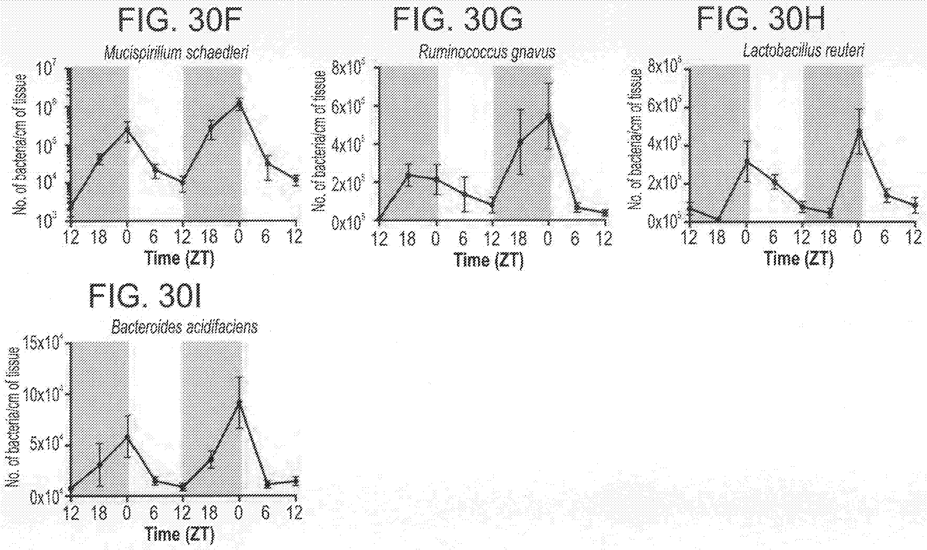

FIGs. 30A-I. Diurnal fluctuations in the number and composition of mucosal-associated commensals.

(A, B) Diurnal rhythmicity of beta-diversity of mucosal-adherent bacteria, as shown by principal coordinate analysis of samples obtained from two consecutive dark-light phases. (C) UniFrac distance of the initial time point compared to all other time points over the course of two light-dark cycles. (D) Total number of different mucosal-adherent bacterial classes over a course of two days. (E) Epithelial-adherent OTUs showing diurnal oscillations in total numbers. Fluctuation amplitudes are depicted. Dashed line indicates p<0.05, JTK_cycle. (F-I) Examples of mucosal-adherent bacterial species undergoing rhythmic fluctuations in total numbers over the course of two days. Data are representative of two independent experiments with N=45 mice.

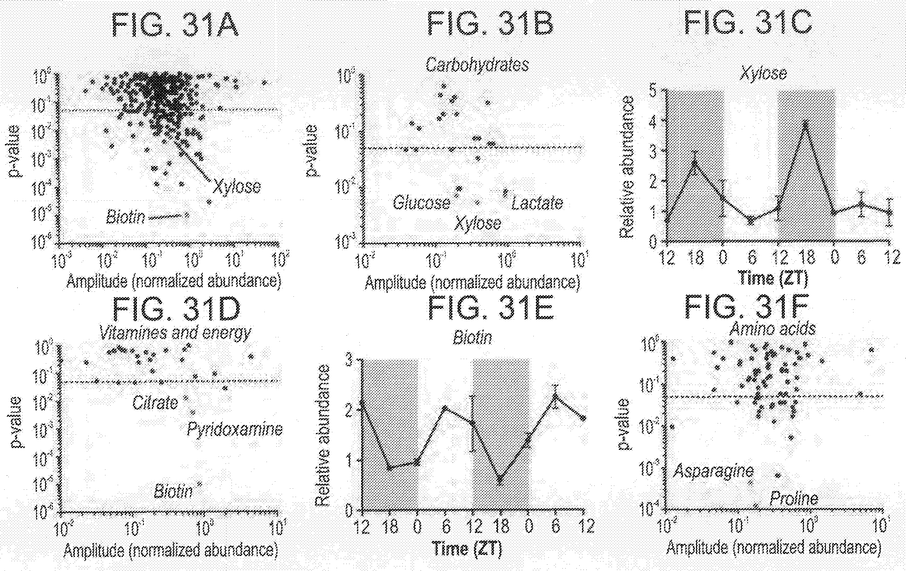

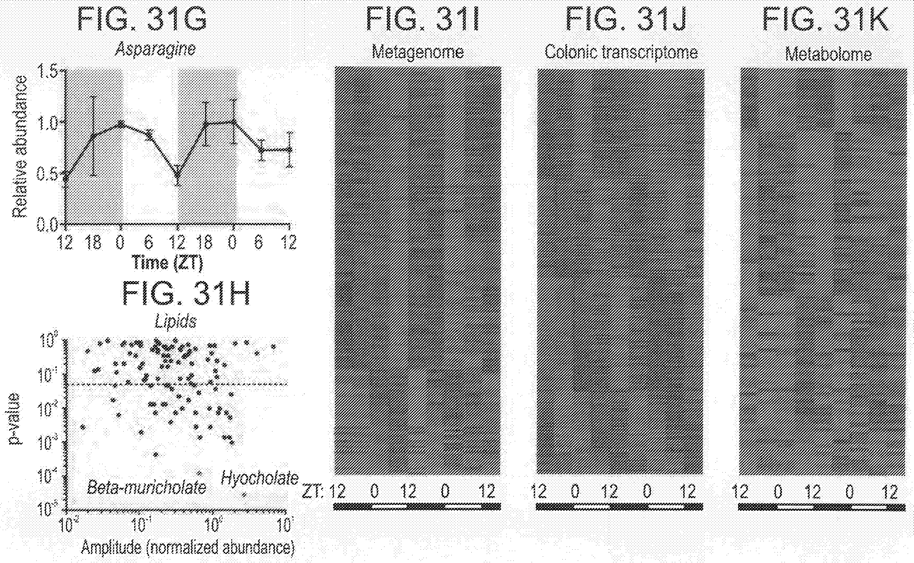

FIGs. 31A-K. The intestinal metabolome undergoes diurnal oscillations.

(A) Metabolites showing diurnal oscillations. Fluctuation amplitudes are depicted. Dashed line indicates p<0.05, JTK_cycle. (B, D, F, H) Metabolites of the indicated chemical groups showing diurnal oscillations in fecal matter. Fluctuation amplitudes are depicted. Dashed line indicates p<0.05, JTK cycle. (C, E, G) Examples for rhythmicity in intestinal metabolites belonging to different chemical groups. (I-K) Heatmap representations of the most significantly oscillating bacterial genes (I), colonic transcripts (J), and intestinal luminal metabolites (K), with p<0.05, q<0.2, JTK cycle. Data are representative of 1-2 independent experiments with N=18-27 mice.

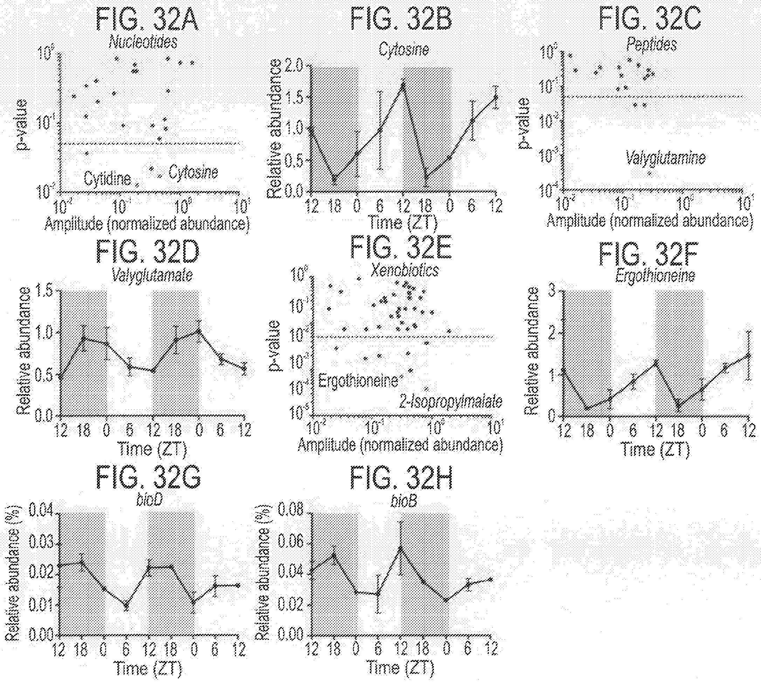

FIGs. 32A-H. Diurnal rhythms of the microbiota metabolome.

(A, C, E) Metabolites of the indicated chemical groups showing diurnal oscillations in fecal matter. Fluctuation amplitudes are depicted. Dashed line indicates p<0.05, JTK cycle. (B, F, D) Examples for rhythmicity in intestinal metabolites belonging to different chemical groups. (G, H) Examples for rhythmicity in two microbial genes, *bioD* and *bioB,* which are involved in biotin synthesis. Data are representative of 1-2 independent experiments with N=18-27 samples in each group.

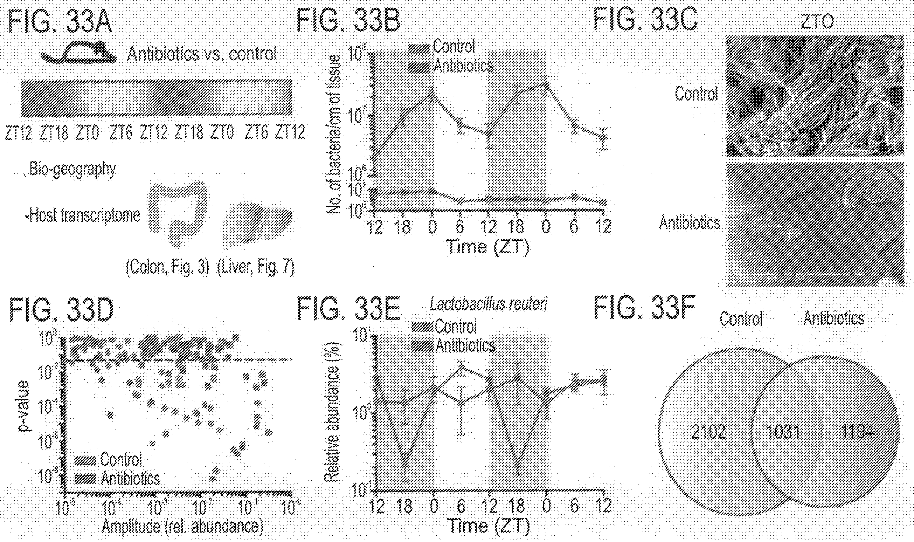

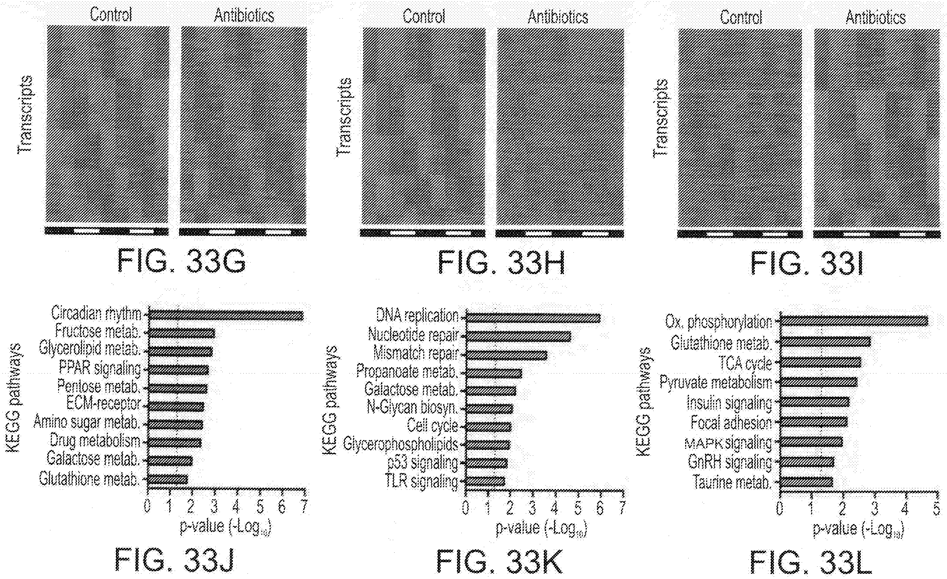

FIGs. 33A-L. Antibiotic treatment abrogates microbial adherence rhythms and reprograms intestinal transcriptome oscillations. (A) Schematic showing feeding pattern and sampling times for microbiota epithelial attachment and colonic transcriptome in antibiotics-treated mice and controls. (B) Diurnal fluctuations in the number of bacteria attached to colonic epithelium over the course of two dark-light cycles in antibiotics-treated mice and controls. (C) Representative SEM images showing epithelial colonization by bacteria at ZT0 in antibiotics-treated mice and controls. (D) Epithelial-adherent OTUs showing diurnal oscillations in relative abundance in antibiotics-treated mice and controls. Fluctuation amplitudes are depicted. Dashed line indicates p<0.05, JTK_cycle. (E) Relative abundance in epithelial-adherent communities of *Lactobacillus reuteri* in antibiotics-treated mice and controls over the course of two dark-light cycles. (F) Venn diagram of shared and unique oscillating colonic transcripts of antibiotics-treated mice compared to controls, p<0.05 and q<0.2, JTK cycle. (G-I) Heatmap representation of shared cycling colonic transcripts between antibiotics-treated mice and controls (G), of transcripts uniquely cycling in control mice (H), and of transcripts uniquely oscillating in antibiotics-treated mice (I), p<0.05 and q<0.2, JTK_cycle. (J-L) KEGG analysis of shared cycling colonic transcripts between antibiotics-treated mice and controls (J), of transcripts uniquely cycling in control mice (K), and of transcripts uniquely oscillating in antibiotics-treated mice (L). Data are representative of two independent experiments with N=45 samples in each group.

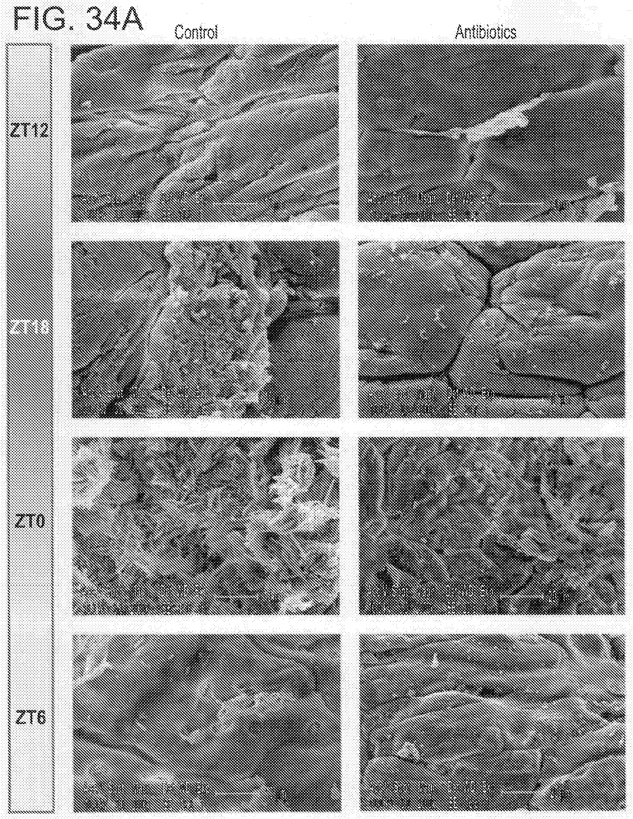

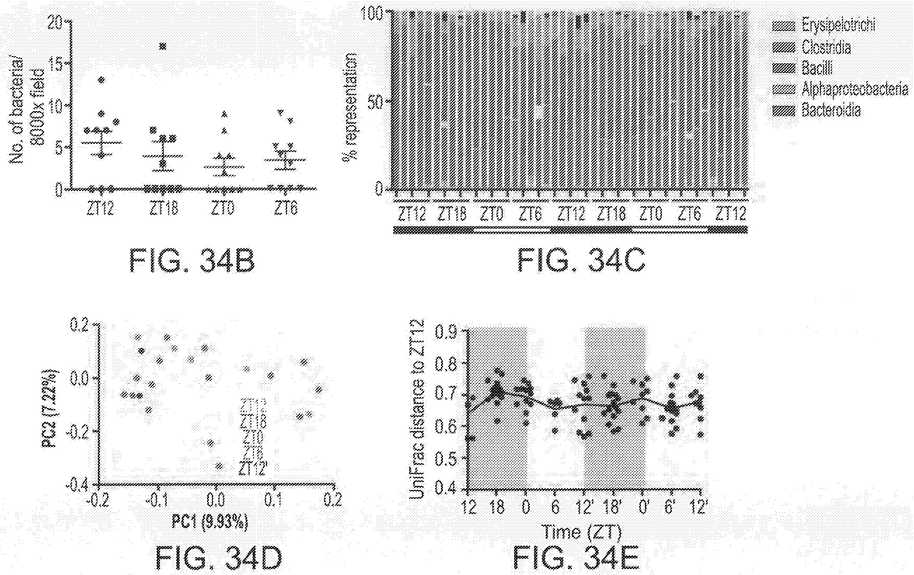

FIGs. 34A-E. Antibiotic treatment abrogates microbial adherence rhythms. (A, B) Representative SEM images (A) and quantification (B) of diurnal fluctuations in epithelial colonization by bacteria over the course of a day in antibiotics-treated and control mice (C) Relative taxonomic composition of mucosal-adherent bacteria over the course of two light-dark cycles after antibiotic treatment. (D) Principal coordinate analysis of mucosal-adherent communities in antibiotics-treated mice every 6 hours over the course of one day. (E) UniFrac distance of the initial time point compared to all other time points over the course of two light-dark cycles in antibiotics-treated mice.

Data are representative of two independent experiments with N=36 mice.

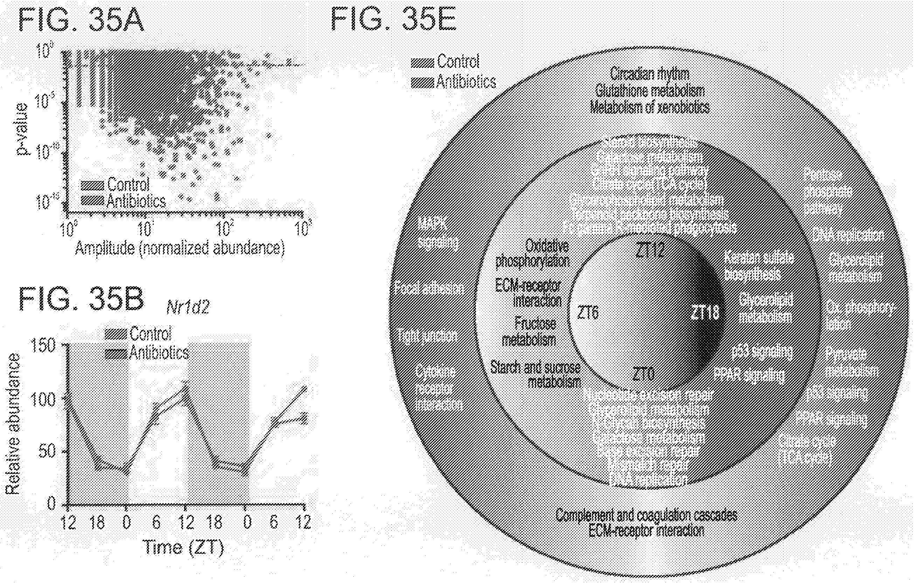

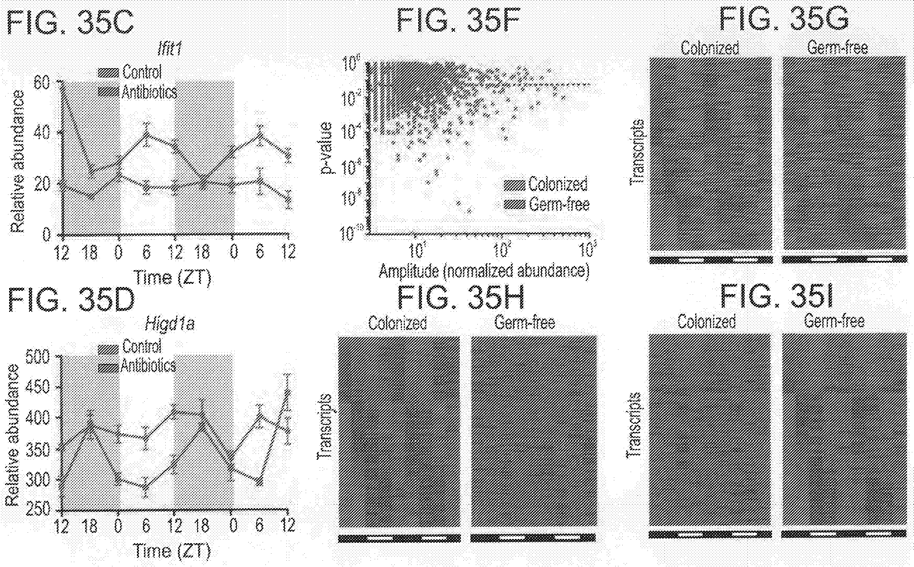

FIGs. 35A-I. The microbiota is required for coordinated oscillations in the intestinal transcriptome.

(A) Colonic transcripts showing diurnal oscillations in antibiotics-treated mice and controls. Fluctuation amplitudes are depicted. Dashed line indicates p<0.05, JTK cycle.(B-D) Representative examples of colonic transcripts with shared rhythmicity between antibiotics-treated mice and controls (B), loss of rhythmicity upon antibiotic treatment (C), and *de-novo* rhythmicity in antibiotics-treated mice (D).

(E) Schematic representation of peak metabolic activities in the colon of antibiotics-treated mice compared to controls. Each oscillating transcript was assigned an acrophase ZTs, and peak profiles were determined by KEGG analysis for each ZT. Note that peak metabolic activities differ between both groups. (F) Colonic transcripts showing diurnal oscillations in germ-free mice and controls. Fluctuation amplitudes are depicted. Dashed line indicates p<0.05, JTK_cycle. (G-I) Heatmap representation of shared cycling transcripts between germ-free mice and controls (G), of transcripts uniquely cycling in control mice (H), and of transcripts uniquely oscillating in germ-free mice (I), p<0.05, JTK cycle. Data are representative of 1-2 experiments with N=27-45 mice in each group.

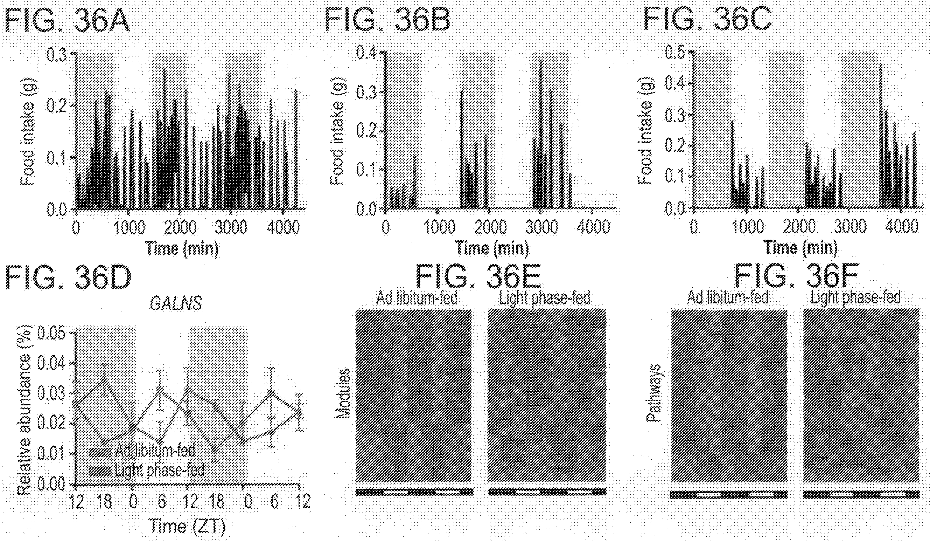

FIGs. 36A-J. Feeding times control oscillations of the metagenome and colonic transcriptome.
(A-C) Distribution of food intake over the course of three dark-light phases in mice fed ad libitum (A), fed only during the dark phase (B), or only during the light phase (C). (D) Example of bacterial genes showing a phase shift upon light phase feeding. (E, F) Heatmap representation of oscillating metagenomic KEGG modules (E) and pathways (F) showing a phase shift upon reversal of feeding times. (F) Heatmap representation of oscillating metagenomic KEGG pathways showing a phase shift upon reversal of feeding times. (G) Example of phase shift in a metagenomic module related to bacterial mucus degradation upon light phase feeding of mice. (H) Heatmap representation of selected KEGG modules and pathways involved in bacterial motility showing a phase shift upon reversal of feeding times. (I, J) Examples of phase reversal of oscillating transcripts in dark phase-fed versus light phase-fed mice. Data are representative of 1-2 independent experiments with N=18-27 samples in each group.

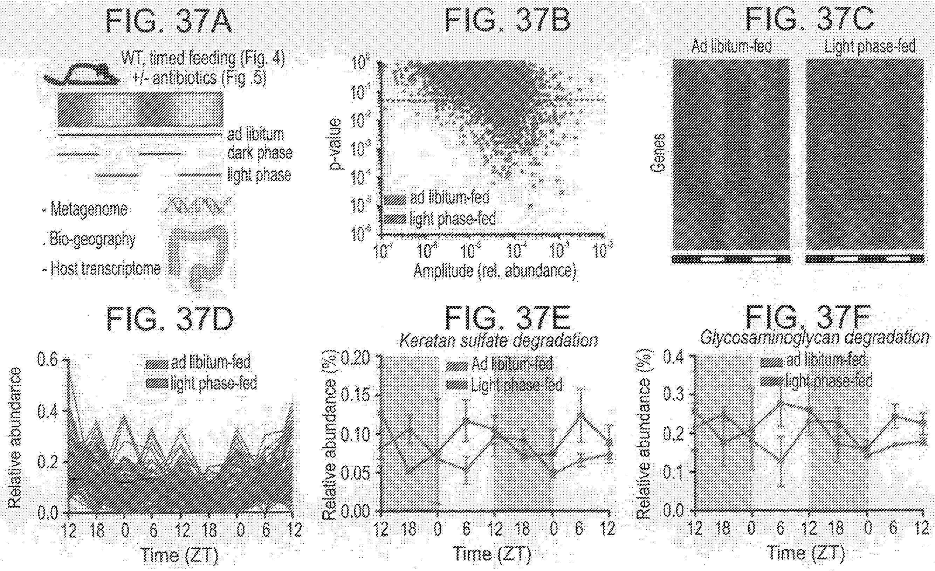

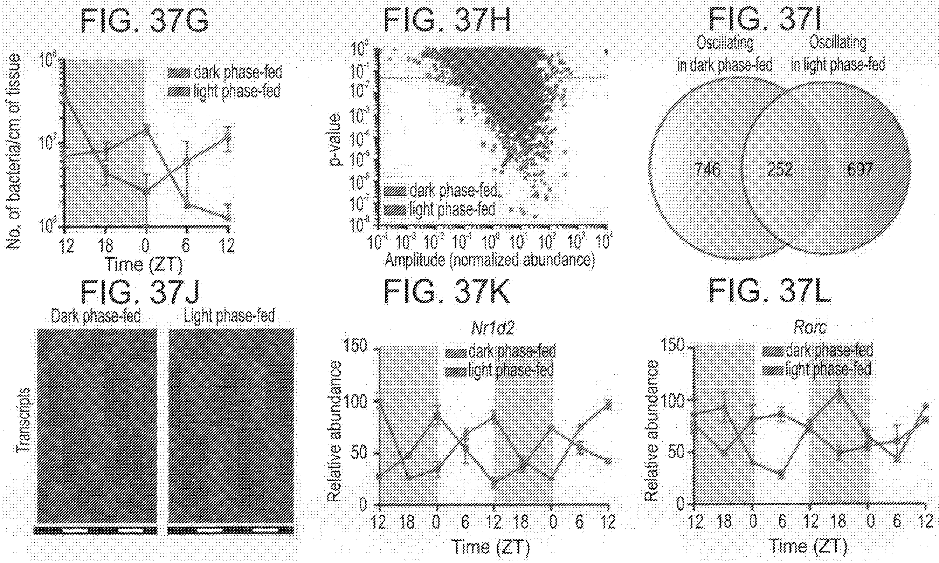

FIGs. 37A-L. Feeding times control metagenomic oscillations, epithelial adherence, and the host transcriptome. (A) Schematic showing feeding pattern and sampling times for microbiota metagenome, epithelial attachment, and colonic transcriptome. (B) Microbial genes showing diurnal oscillations in ad libitum-fed versus light phase-fed mice. Fluctuation amplitudes are depicted. Dashed line indicates p<0.05, JTK_cycle. (C) Heatmap representation of cycling bacterial genes that undergo a phase shift upon light phase feeding. (D) Phase shift of oscillations in microbial genes peaking in abundance at ZT12 in ad libitum-fed mice. (E, F) Example of phase shift in metagenomic modules (E) and pathways (F) related to bacterial mucus degradation upon light phase feeding of mice. (G) Diurnal fluctuations in the number of bacteria attached to colonic epithelium in mice on scheduled feeding. (H) Colonic transcripts showing diurnal oscillations in dark phase-fed versus light phase-fed mice. Fluctuation amplitudes are depicted. Dashed line indicates p<0.05, JTK cycle. (I) Venn diagram of shared and unique oscillating colonic transcripts of dark phase-fed mice compared to light phase-fed mice, p<0.05 and q<0.2, JTK cycle.
(J) Heatmap representation of shared cycling transcripts between dark phase-fed versus light phase-fed mice. Note the phase shift between oscillations in both groups.
(K, L) Examples of phase reversal of oscillating transcripts in dark phase-fed versus light phase-fed mice. Data are representative of two independent experiments with N=18-27 samples in each group.

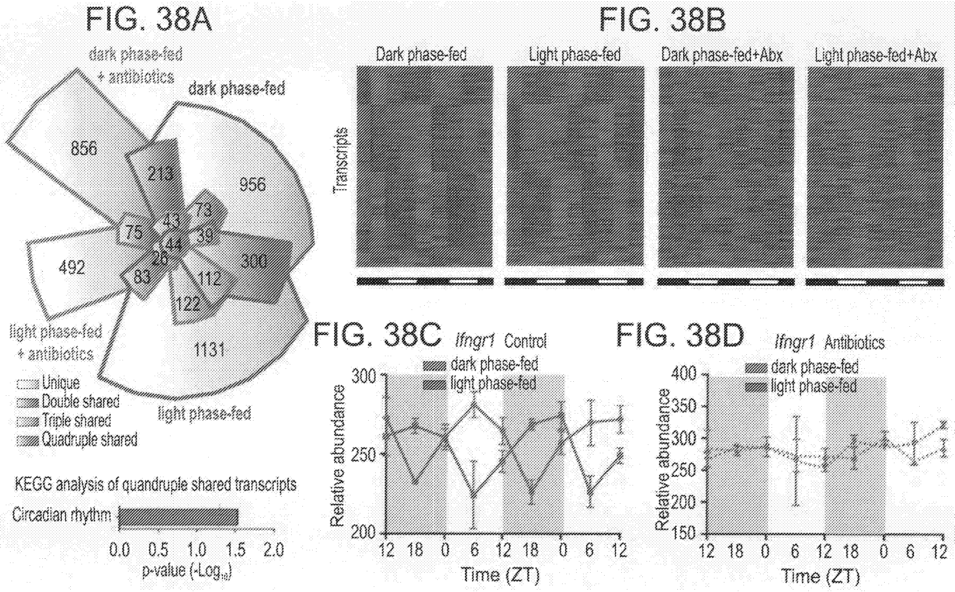

FIGs. 38A-J. Combinatorial reprogramming of intestinal transcriptome oscillations by feeding times and the microbiome. (A) Chow-Ruskey diagram showing the four-way overlap of colonic oscillations in dark phase-fed and light phase-fed mice, with and without antibiotic treatment. Transcripts were counted as oscillating for p<0.05, JTK cycle. The area colors indicate the degree of overlap. The boundary colors indicate the respective experimental group. The areas of each domain are proportional to the number of transcripts. KEGG assignment of the oscillating transcripts shared by all four conditions is shown below. (B) Heatmap representation of oscillating genes (p<0.05, q<0.2, JTK cycle) which are phase-shifted upon feeding time reversal and lose oscillations upon antibiotics treatment. (C-F) *Ifngr1* and *Cldn2* as examples of colonic transcripts which are oscillating in dark phase-fed and light phase-fed mice (C, E), but not in antibiotics-treated groups (D, F). (G-J) *Cry1* and *Per2* as examples of colonic transcripts which are cycling in dark phase-fed and light phase-fed mice, both without (G, I) and with antibiotic treatment (H, J). Note that the phase difference between dark phase-fed and light phase-fed groups is less pronounced upon antibiotic treatment. N=27 samples in each group.

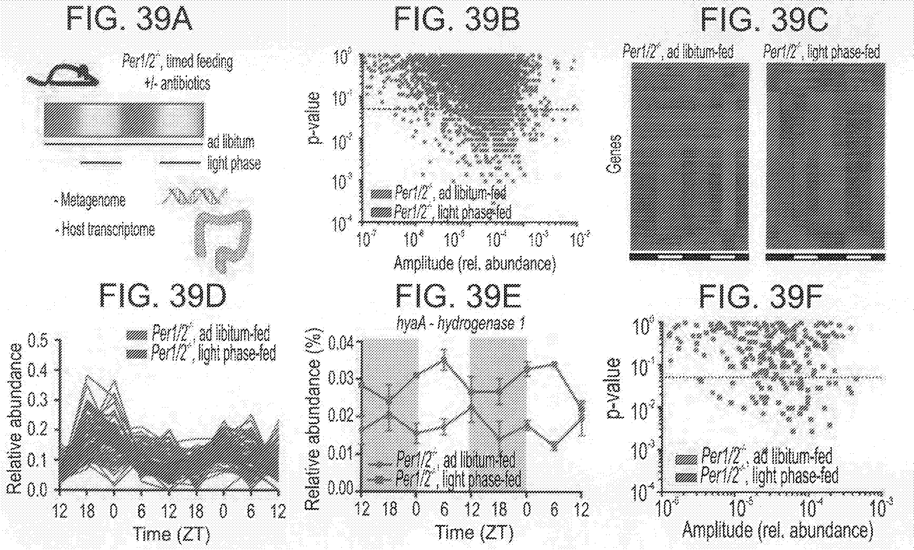

FIGs. 39A-G. Induction of transcriptome oscillations in *Per1/2*-deficient mice is driven by microbiota rhythmicity. (A) Schematic showing feeding pattern and sampling for metagenome and colonic transcriptome in antibiotics-treated *Per1/2*-deficient mice and controls. (B) Microbial genes showing diurnal oscillations in ad libitum-fed and light phase-fed *Per1/2*-deficient mice. Fluctuation amplitudes are depicted. Dashed line indicates p<0.05, JTK_cycle.(C) Heatmap representation of restored microbial gene oscillations in light phase-fed *Per1/2*-deficient mice. (D) Induction of oscillations in microbial genes peaking in abundance during the dark phase in light phase-fed *Per1/2*-deficient mice.
(E) Example of gain of rhythmicity in a microbial gene upon light phase feeding of *Per1/2*-deficient mice compared to ad libitum-fed controls. (F) Microbial KEGG modules showing diurnal oscillations in ad libitum-fed and light phase-fed *Per1/2*-deficient mice. Fluctuation amplitudes are depicted. Dashed line indicates p<0.05, JTK_cycle. (G) Heatmap representation of colonic transcripts in *Per1/2*-deficient mice that gain rhythmicity upon light phase feeding, but not in antibiotics-treated mice.
N=18-27 samples in each group.

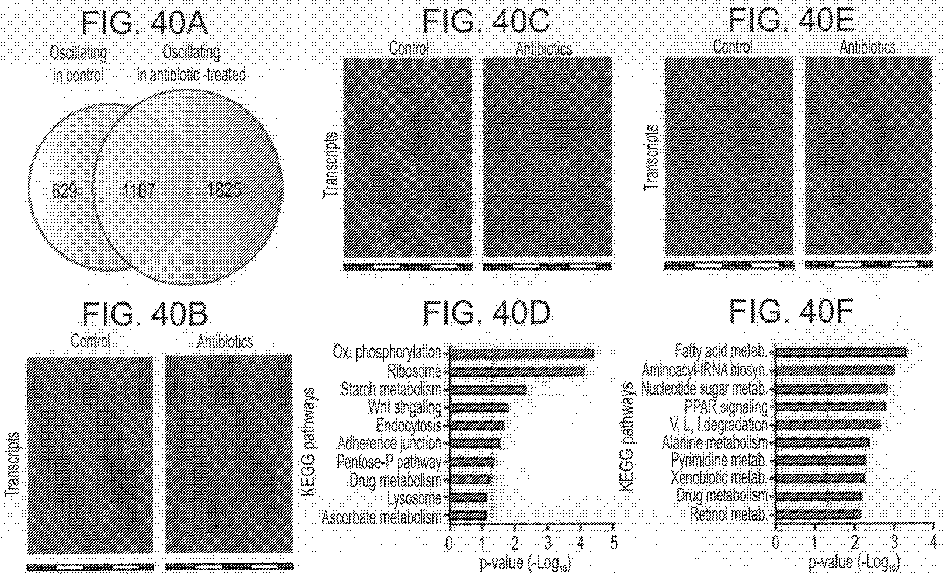

FIGs. 40A-H. Reprogramming of the hepatic transcriptional clock by antibiotic treatment. (A) Venn diagram depicting unique and shared oscillating hepatic transcripts between control and antibiotics-treated mice with p<0.05 and q<0.2, JTK_cycle. (B) Heatmap representation of hepatic transcripts with shared oscillations between antibiotics-treated mice and controls. (C, D) Heatmap representation (C) and KEGG pathway analysis (D) of transcripts uniquely oscillating in control mice. (E, F) Heatmap representation (E) and KEGG pathway analysis (F) of transcripts uniquely oscillating in antibiotics-treated mice. (G) Schematic representation of peak metabolic activities in the liver of antibiotics-treated and control mice. Each oscillating transcript was assigned an acrophase ZTs, and peak profiles were determined by KEGG analysis for each ZT. N=36 samples in each group. (H) Schematic summarizing rhythmic activity and mucosal adherence of the microbiome. Upon disruption of the microbiome, the host transcriptome is reprogrammed, including loss of normal oscillations and de-novo genesis of oscillations.

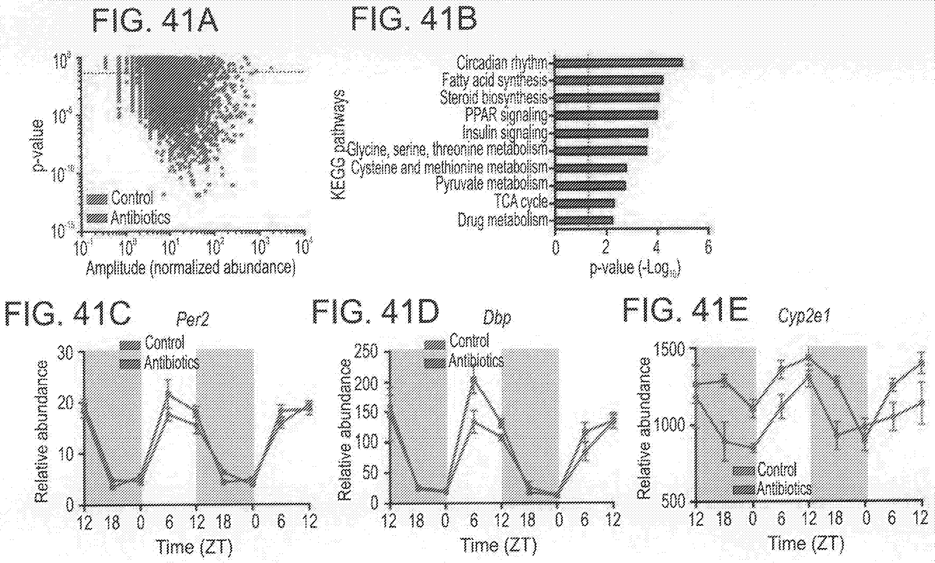

FIGs. 41A-H. The impact of antibiotics treatment on hepatic transcriptome oscillations.

(A) Hepatic transcripts showing diurnal oscillations in antibiotics-treated mice and controls. Fluctuation amplitudes are depicted. Dashed line indicates p<0.05, JTK_cycle.
(B) KEGG pathway analysis of hepatic cycling transcripts shared between antibiotics-treated mice and controls. (C-F) Examples of hepatic transcript oscillations shared between antibiotics-treated mice and controls. (G) Example of hepatic transcript oscillations unique to control mice. (H) Schematic of suggested model for interaction of genetic and environmental factor in determining transcriptome oscillations. The network of transcription factors constituting the molecular clock integrates signals coming from diet and the microbiota, which determine rhythmic activation of target genes. This, in turn, determines the portion of the transcriptome that undergoes cyclic oscillations.N=45 samples in each group.

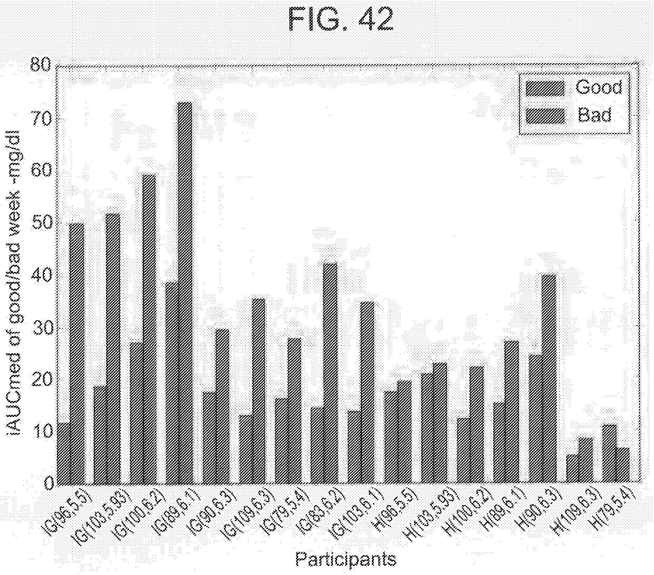

FIG. 42 is a bar graph illustrating that the average glycemic response in the good week are lower compared to the bad week. Average iAUCmed level of 16 participants in the good (green) and bad (red) weeks. iAUCmed is the incremental area under the curve (AUC) above the median glucose level 15 minutes before the meal was consumed. The iAUCmed level of a participant is the average iAUCmed of all its breakfasts, lunches and dinners. In the x-axis, IG signifies an impaired glucose participant and H signifies a healthy participant. The first number after the symbol IG/H in the brackets is the average wakeup glucose level of 6 days of experiment and the second number in the brackets is the HbA1C at the beginning of the experiment.

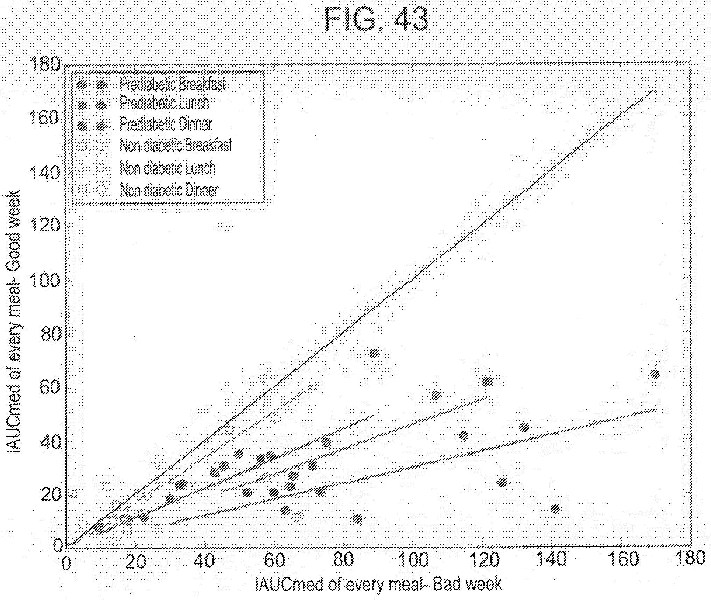

FIG. 43 is a graph illustrating that the glycemic response to meals follows a diurnal pattern, with breakfasts having the lowest response, followed by lunch, and dinner, with the highest. Every dot represents the average iAUCmed of meals in the bad week (x-axis) compared to the good week (y-axis). The majority of points are below the x=y line, meaning that on average AUC in the bad week is higher compared to the good week. Shown are iAUCmed levels of breakfasts (red), lunch (green) and dinner (blue). Filled dots correspond to impaired glucose participants and empty dots correspond to healthy individuals. The slope of the linear fit of all breakfasts in impaired glucose participants (red line) is highest compared to lunch (green line) followed by dinner (green line). Dashed lines correspond to healthy participants and complete lines to glucose impaired participants.

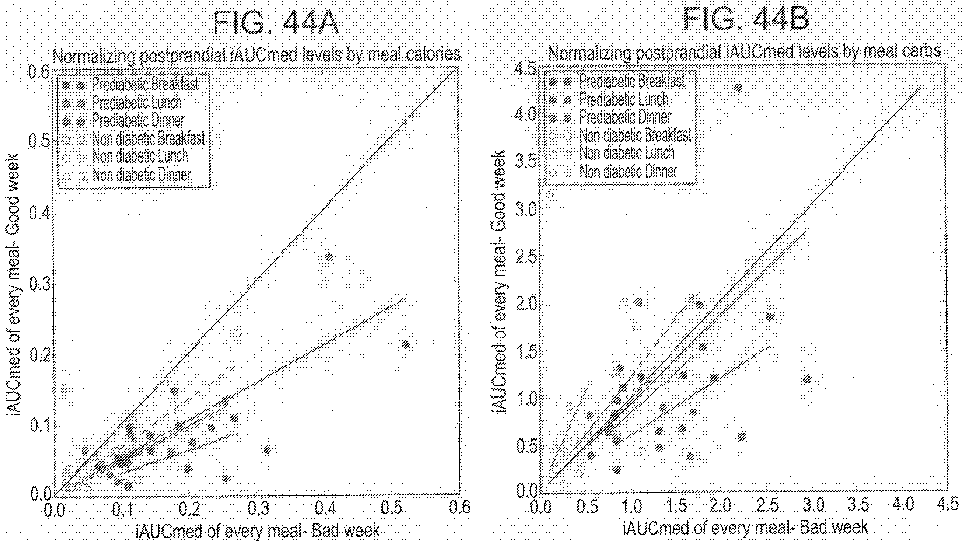

FIGs. 44A-B are graphs illustrating that AUC following meals show a diurnal pattern after normalizing by meal calories and carbohydrate levels. Figure 44A shows normalize iAUCmed by meal calorie content and Figure 44B shows normalized iAUCmed by meal carbohydrate content.

## DESCRIPTION OF SPECIFIC ASPECTS OF THE DISCLOSURE

The present disclosure, in some aspects thereof, relates to the microbiome and, more particularly, but not exclusively, to a method and apparatus for predicting a response of a subject to one or more agents by analysis of the microbiome.

Before explaining at least one aspect of the disclosure in detail, it is to be understood that the disclosure is not necessarily limited in its application to the details set forth in the following description or exemplified by the Examples. The disclosure is capable of other aspects or of being practiced or carried out in various ways.

The gut microbiome is in constant flux, continuously changing its microbial composition in response to external stimuli such as food intake, antibiotic intake and disease. As such, the phylogenetic compositions of microbiomes vary from one individual to another. Such differences have been associated with diseases such as colon cancer and inflammatory bowel disease, susceptibility to obesity, the severity of autism spectrum disorders, and differences in responses to medical treatments.

The present inventors have now found that the level of toxicity of an agent or a condition to a particular person can be measured by analyzing its affect on his microbiome. More specifically, an agent which is toxic to a particular individual will drive the composition of his microbiome to be more similar to a pathological microbiome (e.g. a microbiome from a diseased subject), whereas a non-toxic agent will not have this effect. Thus, his microbiome can be used as a barometer to gauge the toxicity of external stimuli.

Whilst reducing the present disclosure to practice, the present inventors have demonstrated that consumption of commonly used artificial sweetener formulations drives the development of glucose intolerance in particular subjects, through induction of compositional and functional alterations to the intestinal microbiota. Whilst some subjects seem to be more tolerant to the effects of artificial sweeteners, others are less tolerant. The individual response to artificial sweeteners was shown to be due to differences in the microbiome of the tested subjects.

In addition, the present inventors have found that the bacterial content of the gut microbiome is vulnerable to the ravages of circadian rhythm alterations. Disruption to the circadian rhythm caused the microbial content of the gut microbiome to be more similar to a microbiome of an obese or glucose intolerant subject. Thus, when jet-lagged microbes were transferred from either mice or humans into germ-free mice, the rodents became more susceptible to glucose intolerance and diabetes.

Whilst further reducing the present disclosure to practice, the present inventors showed that the gut microbiome has its own innate circadian rhythm which impacts the daily local and systemic transcriptome oscillations of the host. This diurnal meta-organismal activity is adapted to food intake, whose timing determines the phase of microbiome activity and synchronization with the host. The present inventors showed that microbiota disruption by antibiotic treatment or in germ-free mice reprograms the intestinal and hepatic host transcriptome to feature both massive loss and de-novo genesis of oscillations, resulting in temporal reorganization of metabolic pathways.

Accordingly, the present inventors propose that the natural circadian rhythm of the microbiome of the host should be taken into account (i.e. care should be taken so as to not disrupt the circadian rhythm of the microbiome) when determining antibiotic or probiotic treatment regimens.

Thus, according to one aspect of the present disclosure there is provided a method of determining an effect of an agent on a microbiome of a subject comprising:

(a) exposing the microbiome to the agent;
(b) comparing the signature of the microbiome following the exposing with reference signature of a pathological microbiome, wherein when the signature of the microbiome is statistically significantly similar to the pathological microbiome reference signature, it is indicative that the agent has a deleterious effect on the microbiome.

As used herein, the term "microbiome" refers to the totality of microbes (bacteria, fungae, protists), their genetic elements (genomes) in a defined environment.

The microbiome may be a gut microbiome, an oral microbiome, a bronchial microbiome, a skin microbiome or a vaginal microbiome.

According to a particular aspect, the microbiome is a gut microbiome (i.e. intestinal microbiome).

The present aspects encompass the recognition that microbial signatures can be relied upon as proxy for microbiome composition and/or activity. Microbial signatures comprise data points that are indicators of microbiome composition and/or activity. Thus, according to the present disclosure, changes in microbiomes can be detected and/or analyzed through detection of one or more features of microbial signatures.

In some aspects, a microbial signature includes information relating to absolute amount of one or more types of microbes, and/or products thereof. In some aspects, a microbial signature includes information relating to relative amounts of five, ten, twenty or more types of microbes and/or products thereof.

Examples of microbial products include, but are not limited to mRNAs, polypeptides, carbohydrates and metabolites.

In some aspects, a microbial signature includes information relating to presence, level, and/or activity of at least ten types of microbes. In some aspects, a microbial signature includes information relating to presence, level, and/or activity of between 5 and 100 types of microbes. In some aspects, a microbial signature includes information relating to presence, level, and/or activity of between 100 and 1000 or more types of microbes. In some aspects, a microbial signature includes information relating to presence, level, and/or activity of substantially all types of bacteria within the microbiome. In some aspects, a microbial signature includes information relating to presence, level, and/or activity of substantially all types of microbes within the microbiome.

In some aspects, a microbial signature includes information relating to presence, level, and/or activity of metabolites of at least ten types of microbes. In some aspects, a microbial signature includes information relating to presence, level, and/or activity of metabolites of between 5 and 100 types of microbes. In some aspects, a microbial signature includes information relating to presence, level, and/or activity of metabolites of between 100 and 1000 or more types of microbes. In some aspects, a microbial signature includes information relating to presence, level, and/or activity of substantially metabolites of all types of bacteria within the microbiome. In some aspects, a microbial signature includes information relating to presence, level, and/or activity of metabolites of substantially all types of microbes within the microbiome.

According to this aspect of the present disclosure the microbiome signature includes a presence or level of at least one, at least 10, at least 20, at least 50, at least 100, at least 200, at least 300, at least 400, at least 500, at least 600, at least 700, at least 800, at least 900, at least 1000, at least 1200, at least 1500 or all the species of microbes of the microbiome.

In some aspects, a microbiome signature comprises a level or set of levels of at least one, or at least five, or at least ten or more types of microbes (e.g. bacteria) or components or by-products thereof. In some aspects, a microbial signature comprises a level or set of levels of at least one or at least five or at least ten or more DNA sequences. In some aspects, a microbial signature comprises a level or set of levels of ten or more 16S rRNA gene sequences. In some aspects, a microbial signature comprises a level or set of levels of 18S rRNA gene sequences. In some aspects, a microbial signature comprises a level or set of levels of at least five or at least ten or more RNA transcripts. In some aspects, a microbial signature comprises a level or set of levels of at least five or at least ten or more proteins. In some aspects, a microbial signature comprises a level or set of levels of at least one or at least five or at least ten or more metabolites.

16S and 18S rRNA gene sequences encode small subunit components of prokaryotic and eukaryotic ribosomes respectively. rRNA genes are particularly useful in distinguishing between types of microbes because, although sequences of these genes differs between microbial species, the genes have highly conserved regions for primer binding. This specificity between conserved primer binding regions allows the rRNA genes of many different types of microbes to be amplified with a single set of primers and then to be distinguished by amplified sequences.

According to one aspect, the subject under analysis is a healthy subject (i.e. one who has not been diagnosed with a disease known to affect the microbiome). Thus, according to one aspect, the subject under analysis does not have a metabolic disease (e.g. is not diabetic or prediabetic, does not have Crohn's disease) or cancer. The subjects are typically mammals (e.g. humans).

According to one aspect, the microbiome profile of the subject under analysis is included in a subject specific database, and the profile of the reference microbiome (pathological microbiome) derived from a non-healthy subject is included in a second database. The second database may comprise profiles of more than one pathological microbiome and may comprise average data from a plurality of pathological microbiomes.

Both the subject-specific database and the second database may be stored in a computer readable format on a computer readable medium, and is optionally and preferably accessed by a data processor, such as a general purpose computer or dedicated circuitry.

The subject-specific database may comprise additional data describing the subject. Representative examples of types of data other than the microbiome profile or signature include without limitation responses to foods, blood chemistry of the subject, partial blood chemistry of the subject, genetic profile of the subject, metabolomic data associated with the subject, the medical condition of the subject, sleep patterns of the subject, food intake habits of the subject (e.g. does the subject use artificial sweeteners or not), and the like. The subject-specific database may also comprise data pertaining to the frequency of intake or exposure to the agent, the time of intake or exposure to the agent etc. These and other types of data are described in more detail below.

In order to analyze the microbiome, samples are taken from a subject. Thus, for example stool samples may be taken to analyze the gut microbiome, bronchial samples may be taken to analyze the bronchial microbiome etc. According to a particular aspect, the microbiome of a subject is determined from a stool sample of the subject. It will be appreciated that microbiomes of the same source are compared (i.e. the gut microbiome of the subject is compared with the gut microbiome of a second subject or group of subjects).

The present inventors have shown that changes in eating patterns (e.g. due to circadian misalignment) affect the composition of the microbiome. Therefore, preferably samples are taken at a fixed time in the day.

Agents which are analyzed according to this aspect of the present disclosure may be substances or conditions.

Substances which may be analyzed are typically non-caloric substances (i.e. have less than 3 calories per 100 g).

Exemplary non-caloric substances include food additives.

The food additive may be classified according to the European Union with an E number. Thus, for example a substance having an E number between 100-199 is a color, a substance having an E number between 200-299 is a preservative, a substance having an E number between 300-399 is an antioxidant, a substance having an E number between 400-499 is a thickener, stabilizer or emulsifier, a substance having an E number between 500-599 is a acidity regulator or anti-caking agent, a substance having an E number between 600-199 is a flavor enhancer, a substance having an E number between 700-799 is an antibiotic, a substance having an E number between 900-999 is a glazing agent or sweetener. Additional chemicals have E numbers between 1000-1599.

Food additives may be categorized into the following groups: Acids, Acidity regulators, Anticaking agents, Antifoaming agents, Antioxidants, Bulking agents, Colorings, Emulsifiers, Flavors, Flavor enhancers, Glazing agents, Humectants, Preservatives, stabilizers, artificial sweeteners and thickeners.

According to a particular aspect, the substance is caffeine.

According to another aspect, the substance is an artificial sweetener.

Examples of artificial sweeteners include, but are not limited to aspartame, acesulfame, steviol, saccharin, cyclamate, erythritol, isomalt, maltitol, lactitol, mannitol, Neohesperidine dihydrochalcone, Neotame, sorbitol, xylitol, and Sucralose.

According to another aspect, the substance is a therapeutic agent. Examples of therapeutic agents include, but are not limited to, inorganic or organic compounds; small molecules (i.e., less than 1000 Daltons) or large molecules (i.e., above 1000 Daltons); biomolecules (e.g. proteinaceous molecules, including, but not limited to, peptide, polypeptide, post-translationally modified protein, antibodies etc.) or a nucleic acid molecule (e.g. double-stranded DNA, single-stranded DNA, double-stranded RNA, single-stranded RNA, or triple helix nucleic acid molecules) or chemicals. Therapeutic agents may be natural products derived from any known organism (including, but not limited to, animals, plants, bacteria, fungi, protista, or viruses) or from a library of synthetic molecules. Therapeutic agents can be monomeric as well as polymeric compounds.

According to a particular aspect, the substance is a metabolite.

As used herein, a "metabolite" is an intermediate or product of metabolism. The term metabolite is generally restricted to small molecules and does not include polymeric compounds such as DNA or proteins. A metabolite may serve as a substrate for an enzyme of a metabolic pathway, an intermediate of such a pathway or the product obtained by the metabolic pathway.

In preferred aspects, metabolites include but are not limited to sugars, organic acids, amino acids, fatty acids, hormones, vitamins, oligopeptides (less than about 100 amino acids in length), as well as ionic fragments thereof. Cells can also be lysed in order to measure cellular products present within the cell. In particular, said metabolites are less than about 3000 Daltons in molecular weight, and more particularly from about 50 to about 3000 Daltons.

The metabolite of this aspect of the present disclosure may be a primary metabolite (i.e. essential to the microbe for growth) or a secondary metabolite (one that does not play a role in growth, development or reproduction, and is formed during the end or near the stationary phase of growth.

Representative examples of metabolic pathways in which the metabolites of the present disclosure are involved include, without limitation, citric acid cycle, respiratory chain, photosynthesis, photorespiration, glycolysis, gluconeogenesis, hexose monophosphate pathway, oxidative pentose phosphate pathway, production and $\beta$-oxidation of fatty acids, urea cycle, amino acid biosynthesis pathways, protein degradation pathways such as proteasomal degradation, amino acid degrading pathways, biosynthesis or degradation of: lipids, polyketides (including, e.g., flavonoids and isoflavonoids), isoprenoids (including, e.g., terpenes, sterols, steroids, carotenoids, xanthophylls), carbohydrates, phenylpropanoids and derivatives, alkaloids, benzenoids, indoles, indole-sulfur compounds, porphyrines, anthocyans, hormones, vitamins, cofactors such as prosthetic groups or electron carriers, lignin, glucosinolates, purines, pyrimidines, nucleosides, nucleotides and related molecules such as tRNAs, microRNAs (miRNA) or mRNAs.

According to a particular aspect, the therapeutic agent is not a food or food additive.

According to another aspect, the therapeutic agent is not for treating obesity or a disease related to glucose intolerance (e.g. diabetes).

According to still another aspect, the substance is not a therapeutic agent.

The substances may be isolated or may be incorporated in a carrier such as a food or drink.

According to one aspect, the carrier is a non-caloric carrier such as a non-caloric food or drink. Thus for example, the substance may be a diet drink or coffee without milk.

Preferably the microbiome is exposed to a similar amount of agent to which the subject under examination is routinely subjected to.

As mentioned, the agent which is tested may also be a condition.

Exemplary conditions contemplated by the present disclosure include but are not limited to altered sleep patterns, tobacco smoke exposure, radiation exposure, light exposure and food intake patterns.

The first step according to this aspect of the present disclosure involves exposing the microbiome to the agent. It will be appreciated that when the agent is a substance, this step may be affected in vivo or ex vivo. When the agent is a condition, this step is typically effected in vivo. The exposing may be a direct exposure (e.g. contacting) or may be effected via the subject (e.g. the subject is exposed to different sleep patterns).

The contacting may be carried out a single time, or may be affected on a multitude of occasions over the course of a particular time period.

It will be appreciated that the signature may be determined in a microbiome of a subject who has known to have been subjected to the agent or condition. This information may be obtained directly from the subject under analysis (e.g. using a questionnaire). Preferably, the subject has been subjected to the agent over a period of time (for example a week, a month or longer). According to a particular aspect, the subject has been subjected to the agent at least once a day, at least two times a day, at least three times a day or more. Preferably, the subject has been subjected to the agent at least 1 month prior to the analysis, at least one week prior to the analysis and optionally at least one day prior to the analysis.

Following the contacting the signature of the microbiome of the test subject is compared with a reference signature of a pathological microbiome.

As used herein, the phrase "pathological microbiome" refers to a microbiome derived from a subject who is known to have a disease (e.g. metabolic disease such as diabetes, or pre-diabetes, cancer) or has already been preclassified as having a microbiome that is intolerant to the agent.

*Quantifying Microbial Levels:* In methods in accordance with the present disclosure, a microbial signature is obtained and/or determined by quantifying microbial levels. Methods of quantifying levels of microbes of various types are described herein below.

In some aspects, determining a level or set of levels of one or more types of microbes or components or products thereof comprises determining a level or set of levels of one or more DNA sequences. In some aspects, one or more DNA sequences comprises any DNA sequence that can be used to differentiate between different microbial types. In certain aspects, one or more DNA sequences comprises 16S rRNA gene sequences. In certain aspects, one or more DNA sequences comprises 18S rRNA gene sequences. In some aspects, 1, 2, 3, 4, 5, 10, 15, 20, 25, 50, 100, 1,000, 5,000 or more sequences are amplified.

In some aspects, a microbiota sample (e.g. fecal sample) is directly assayed for a level or set of levels of one or more DNA sequences. In some aspects, DNA is isolated from a microbiota sample and isolated DNA is assayed for a level or set of levels of one or more DNA sequences. Methods of isolating microbial DNA are well known in the art. Examples include but are not limited to phenol-chloroform extraction and a wide variety of commercially available kits, including QIAamp DNA Stool Mini Kit (Qiagen, Valencia, Calif.).

In some aspects, a level or set of levels of one or more DNA sequences is determined by amplifying DNA sequences using PCR (e.g., standard PCR, semi-quantitative, or quantitative PCR). In some aspects, a level or set of levels of one or more DNA sequences is determined by amplifying DNA sequences using quantitative PCR. These and other basic DNA amplification procedures are well known to practitioners in the art and are described in Ausebel et al. (Ausubel F M, Brent R, Kingston R E, Moore D D, Seidman J G, Smith J A, Struhl K (eds). 1998. Current Protocols in Molecular Biology. Wiley: New York).

In some aspects, DNA sequences are amplified using primers specific for one or more sequence that differentiate(s) individual microbial types from other, different microbial types. In some aspects, 16S rRNA gene sequences or fragments thereof are amplified using primers specific for 16S rRNA gene sequences. In some aspects, 18S DNA sequences are amplified using primers specific for 18S DNA sequences.

In some aspects, a level or set of levels of one or more 16S rRNA gene sequences is determined using phylochip technology. Use of phylochips is well known in the art and is described in Hazen et al. ("Deep-sea oil plume enriches indigenous oil-degrading bacteria." Science, 330, 204-208, 2010). Briefly, 16S rRNA genes sequences are amplified and labeled from DNA extracted from a microbiota sample. Amplified DNA is then hybridized to an array containing probes for microbial 16S rRNA genes. Level of binding to each probe is then quantified providing a sample level of microbial type corresponding to 16S rRNA gene sequence probed. In some aspects, phylochip analysis is performed by a commercial vendor. Examples include but are not limited to Second Genome Inc. (San Francisco, Calif.).

In some aspects, determining a level or set of levels of one or more types of microbes or components or products thereof comprises determining a level or set of levels of one or more microbial RNA molecules (e.g., transcripts). Methods of quantifying levels of RNA transcripts are well known in the art and include but are not limited to northern analysis, semi-quantitative reverse transcriptase PCR, quantitative reverse transcriptase PCR, and microarray analysis.

In some aspects, determining a level or set of levels of one or more types of microbes or components or products thereof comprises determining a level or set of levels of one or more microbial polypeptides. Methods of quantifying polypeptide levels are well known in the art and include but are not limited to Western analysis and mass spectrometry. These and all other basic polypeptide detection procedures are described in Ausebel et al.

In some aspects, determining a level or set of levels of one or more types of microbes or components or products thereof comprises determining a level or set of levels of one or more microbial metabolites. In some aspects, levels of metabolites are determined by mass spectrometry. In some aspects, levels of metabolites are determined by nuclear magnetic resonance spectroscopy. In some aspects, levels of metabolites are determined by enzyme-linked immunosorbent assay (ELISA). In some aspects, levels of metabolites are determined by colorimetry. In some aspects, levels of metabolites are determined by spectrophotometry.

Methods of analyzing SCFAs are known in the art. An exemplary method is described in the Examples section herein below.

It will be appreciated that the pathological microbiome reference signature is selected so as to correspond with the microbiome signature of the subject. For example, if the microbiome signature of the subject comprises amounts of microbe metabolites, then the pathological microbiome reference signature also comprises amounts of microbe metabolites. If the microbiome signature of the subject comprises expression data for a group of genes involved in glycosaminoglycan synthesis, then the pathological microbiome reference signature also comprises expression data for the group of genes involved in glycosaminoglycan synthesis.

According to one aspect of the present disclosure two microbiome signatures can be have a statistically significant similar signature when they comprise at least 50 % of the same microbes, at least 60 % of the same microbes, at least 70 % of the same microbes, at least 80 % of the same microbes, at least 90 % of the same microbes, at least 91 % of the same microbes, at least 92 % of the same microbes, at least 93 % of the same microbes, at least 94 % of the same microbes, at least 95 % of the same microbes, at least 96 % of the same microbes, at least 97 % of the same microbes, at least 98 % of the same microbes, at least 99 % of the same microbes or 100 % of the same microbes.

Additionally, or alternatively, microbiomes may have a statistically significant similar signature when the quantity (e.g. occurrence) in the microbiome of at least one microbe of interest is identical. According to another aspect, microbiomes may have a statistically significant similar signature when the relative ratio in the microbiome of at least 10 % of its microbes are identical. According to another aspect, microbiomes may have a statistically significant similar signature when the relative ratio in the microbiome of at least 20 % of its microbes are identical. According to another aspect, microbiomes may have a statistically significant similar signature when the relative ratio in the microbiome of at least 30 % of its microbes are identical. According to another aspect, microbiomes may have a statistically significant similar signature when the relative ratio in the microbiome of at least 40 % of its microbes are identical. According to another aspect, microbiomes may have a statistically significant similar signature when the relative ratio in the microbiome of at least 50 % of its microbes are identical. According to another aspect, microbiomes may have a statistically significant similar signature when the relative ratio in the microbiome of at least 60 % of its microbes are identical. According to another aspect, microbiomes may have a statistically significant similar signature when the relative ratio in the microbiome of at least 70 % of its microbes are identical. According to another aspect, microbiomes may have a statistically significant similar signature when the relative ratio in the microbiome of at least 80 % of its microbes are identical. According to another aspect, microbiomes may have a statistically significant similar signature when the relative ratio in the microbiome of at least 90 % of its microbes are identical. Thus, the fractional percentage of microbes (e.g. relative amount, ratio, distribution, frequency, percentage, etc.) of the total may be statistically similar.

According to another aspect, in order to classify a microbe as belonging to a particular genus, family, order, class or phylum, it must comprise at least 90 % sequence homology, at least 91 % sequence homology, at least 92 % sequence homology, at least 93 % sequence homology, at least 94 % sequence homology, at least 95 % sequence homology, at least 96 % sequence homology, at least 97 % sequence homology, at least 98 % sequence homology, at least 99 % sequence homology to a reference microbe known to belong to the particular genus. According to a particular aspect, the sequence homology is at least 95 %.

According to another aspect, in order to classify a microbe as belonging to a particular species, it must comprise at least 90 % sequence homology, at least 91 % sequence homology, at least 92 % sequence homology, at least 93 % sequence homology, at least 94 % sequence homology, at least 95 % sequence homology, at least 96 % sequence homology, at least 97 % sequence homology, at least 98 % sequence homology, at least 99 % sequence homology to a reference microbe known to belong to the particular species. According to a particular aspect, the sequence homology is at least 97 %.

In determining whether a nucleic acid or protein is substantially homologous or shares a certain percentage of sequence identity with a sequence of the disclosure, sequence similarity may be defined by conventional algorithms, which typically allow introduction of a small number of gaps in order to achieve the best fit. In particular, "percent identity" of two polypeptides or two nucleic acid sequences is determined using the algorithm of Karlin and Altschul (Proc. Natl. Acad. Sci. USA 87:2264-2268, 1993). Such an algorithm is incorporated into the BLASTN and BLASTX programs of Altschul et al. (J. Mol. Biol. 215:403-410, 1990). BLAST nucleotide searches may be performed with the BLASTN program to obtain nucleotide sequences homologous to a nucleic acid molecule of the disclosure. Equally, BLAST protein searches may be performed with the BLASTX program to obtain amino acid sequences that are homologous to a polypeptide of the disclosure. To obtain gapped alignments for comparison purposes, Gapped BLAST is utilized as described in Altschul et al. (Nucleic Acids Res. 25:3389-3402, 1997). When utilizing BLAST and Gapped BLAST programs, the default parameters of the respective programs (e.g., BLASTX and BLASTN) are employed. See wwwdotncbidotnlmdotnihdotgov for more details.

According to still another aspect, two microbiome signatures can be classified as being similar, if the relative number of genes belonging to a particular pathway is similar. Such pathways are described herein below.

According to still another aspect, two microbiome signatures can be classified as being similar, if the relative amount of a product generated by the microbes is similar. Such products are described herein below.

As well as comparing the microbiome signature of the subject under analysis to the pathological reference microbiome, the microbiome signature of the subject may also (or alternatively) be compared to a non-pathological reference microbiome.

Thus, according to another aspect, the method further comprises comparing the signature of the microbiome following the exposing with a non-pathological microbiome reference signature, wherein when the signature of the microbiome is statistically significantly different to the non-pathological microbiome reference signature, it is indicative that the agent has a deleterious effect on the microbiome. Additionally, when the signature of the microbiome is statistically significantly similar to the non-pathological microbiome reference signature, it is indicative that the agent has a non-deleterious effect on the microbiome.

Non-pathological microbiomes are typically derived from healthy subjects (i.e. do not have any diseases, especially metabolic diseases). Further, the non-pathological microbiomes are typically derived from healthy subjects that do not chronically ingest agents which are known to adversely affect the microbiome. Thus, for example, the non-pathological microbiome is typically derived from a healthy subject that does not chronically ingest artificial sweeteners.

Further steps may be added to any of the methods described herein which are used to assess an agent's effect on the microbiome including for example administering to the subject the agent prior to the analyzing of the microbiome signature and/or preparing the sample for analysis (e.g. removal of fecal matter, making a protein extract, preparing a nucleic acid sample etc.).

Any of the analytical methods described herein can be embodied in many forms. For example, it can be embodied in on a tangible medium such as a computer for performing the method operations. It can be embodied on a computer readable medium, comprising computer readable instructions for carrying out the method operations. It can also be embodied in electronic device having digital computer capabilities arranged to run the computer program on the tangible medium or execute the instruction on a computer readable medium.

Computer programs implementing the analytical method of the present aspects can commonly be distributed to users on a distribution medium such as, but not limited to, CD-ROMs or flash memory media. From the distribution medium, the computer programs can be copied to a hard disk or a similar intermediate storage medium. In some aspects of the present disclosure, computer programs implementing the method of the present aspects can be distributed to users by allowing the user to download the programs from a remote location, via a communication network, e.g., the internet. The computer programs can be run by loading the computer instructions either from their distribution medium or their intermediate storage medium into the execution memory of the computer, configuring the computer to act in accordance with the method of this disclosure. All these operations are well-known to those skilled in the art of computer systems.

By carrying out the methods described herein, it is possible to determine the tolerance of a subject to the agent. It then becomes possible to provide recommendations as to the amount of a substance which can be tolerated (e.g. ingested), or the amount or level of a condition that can be tolerated by a subject.

Following are two examples of agents that were shown to have an effect on the microbiome.

*Artificial Sweeteners:* The present inventors have demonstrated that consumption of commonly used artificial sweetener formulations drives the development of glucose intolerance in particular subjects, through induction of compositional and functional alterations to the intestinal microbiota. Whilst some subjects seem to be more tolerant to the effects of artificial sweeteners, others are less tolerant. The individual response to artificial sweeteners was shown to be due to differences in the microbiome of the tested subjects.

The phrase "tolerance to artificial sweeteners" as used herein refers to the ability to ingest (either eat or drink) the FDA's maximal acceptable daily intake (ADI) of artificial sweetener (e.g. commercial saccharin 5mgkg$^{-1}$) without showing a clinical parameter that is associated with disturbed glucose metabolism. Thus, for example a person may be considered tolerant to an artificial sweetener if he does not fail a glucose tolerance test. For example, a person may be considered as failing a glucose tolerance test if 2 hours following ingestion of 75 grams of glucose, his blood glucose level is less than 140 mg/dl, and all values between 0 and 2 hours are less than 200 mg/dl. Additionally, a person may be considered tolerant to an artificial sweetener if his fasting glucose levels are within the normal range.

Typically, the subjects who are tested for their tolerance to artificial sweeteners are not diabetic or pre-diabetic. Preferably they do not suffer from a metabolic disorder.

Particularly relevant microbiome signatures when testing for tolerance to artificial sweeteners include: level of microbes belonging to the order bacteroidales, Clostridilales, Bactobacillales, YS2, RF32, Erysipelotrichales, Burkholderiales, Lactobacillales, Anaeroplasmatales, Enterobacteriales and/or Campylobacterales.

According to a particular aspect, the microbiome signature comprises a level of microbes belonging to the order bacteroidales, Clostridilales, Bactobacillales, YS2, RF32, Erysipelotrichales, Burkholderiales and/or Campylobacterales, when testing for tolerance to artificial sweeteners.

According to another aspect, the microbiome signature comprises a level of microbes belonging to the phylum Bacteroidetes, Firmicutes and/or Tenericutes, when testing for tolerance to artificial sweeteners.

According to another aspect, the microbiome signature comprises a level of microbes belonging to the class Bacteroidia, Bacilli, Clostridia, Mollicutes and/or Gammaproteobacteria, when testing for tolerance to artificial sweeteners.

According to another aspect, the microbiome signature comprises a level of microbes belonging to the order Bacteroidales, Lactobacillales, Clostridiales, Anaeroplasmatales and/or Enterobacteriales, when testing for tolerance to artificial sweeteners.

According to another aspect, the microbiome signature comprises a level of microbes belonging to the family Bacteroidaceae, Lactobacillaceae, Porphyromonadaceae, Anaeroplasmataceae, Clostridiaceae, Odoribacteraceae, Ruminococcaceae, Streptococcaceae, Dehalobacteriaceae, Enterobacteriaceae and/or S24-7, when testing for tolerance to artificial sweeteners.

According to another aspect, the microbiome signature comprises a level of microbes belonging to the genus, Bacteroides, Lactobacillus, Parabacteroides, Anaeroplasma, Candidatus Arthromitus, Odoribacter, Lactococcus and/or Dehalobacterium, when testing for tolerance to artificial sweeteners.

According to yet another aspect, the microbiome signature comprises a level of microbes of at least one of the species set forth in Table 4 herein below, when testing for tolerance to artificial sweeteners.

As mentioned herein above, the microbiome signature may refer to the level of particular genes expressed in the microbes of the microbiome.

More specifically, the microbiome signature may comprise levels of genes belonging to the glycan degradation pathway (e.g. the glycosaminoglycan pathway), when testing for tolerance to artificial sweeteners.

According to still another aspect, the microbiome signature may comprise levels of microbe genes including for example genes involved in starch and sucrose metabolism, genes involved in fructose and mannose metabolism, genes involved in folate biosynthesis, genes involved in glycerolipid-biosynthesis, genes involved in fatty acid biosynthesis, genes involved in glucose transport pathways, genes involved in ascorbate and aldarate metabolism, genes involved in lipopolysaccharide biosynthesis and/or genes involved in bacterial chemotaxis, when testing for tolerance to artificial sweeteners.

As mentioned herein above, the microbiome signature may refer to the level of a product or bi-product generated by microbes of the microbiome - for example a metabolite.

An exemplary metabolite which may be analyzed in the sample are short chain fatty acids (SCFAs), when testing for tolerance to artificial sweeteners.

According to one aspect of the present disclosure two microbiome signatures can be classified as being similar if the relative level of microbes belonging to the order bacteroidales, Clostridilales, Bactobacillales, YS2, RF32, Erysipelotrichales, Burkholderiales, Lactobacillales, Anaeroplasmatales, Enterobacteriales and/or Campylobacterales is statistically similar.

According to one aspect of the present disclosure two microbiome signatures can be classified as being similar if the relative level of microbes belonging to the order bacteroidales, Clostridilales, Bactobacillales, YS2, RF32, Erysipelotrichales, Burkholderiales and/or Campylobacterales is statistically similar.

According to one aspect of the present disclosure two microbiome signatures can be classified as being similar if the relative level of microbes belonging to the phylum Bacteroidetes, Firmicutes and/or Tenericutes is statistically similar.

According to one aspect of the present disclosure two microbiome signatures can be classified as being similar if the relative level of microbes belonging to the class Bacteroidia, Bacilli, Clostridia, Mollicutes and/or Gammaproteobacteria is statistically similar.

According to one aspect of the present disclosure two microbiome signatures can be classified as being similar if the relative level of microbes belonging to the order Bacteroidales, Lactobacillales, Clostridiales, Anaeroplasmatales and/or Enterobacteriales is statistically similar.

According to one aspect of the present disclosure two microbiome signatures can be classified as being similar if the relative level of microbes belonging to the family Bacteroidaceae, Lactobacillaceae, Porphyromonadaceae, Anaeroplasmataceae, Clostridiaceae, Odoribacteraceae, Ruminococcaceae, Streptococcaceae, Dehalobacteriaceae, Enterobacteriaceae and/or S24-7 is statistically similar.

According to one aspect of the present disclosure two microbiome signatures can be classified as being similar if the relative level of microbes belonging to the genus, Bacteroides, Lactobacillus, Parabacteroides, Anaeroplasma, Candidatus Arthromitus, Odoribacter, Lactococcus and/or Dehalobacterium is statistically similar.

According to one aspect of the present disclosure two microbiome signatures can be classified as being similar if the relative level of microbes belonging to the species set forth in Table 4 herein below is statistically similar.

According to another aspect of the present disclosure there is provided a method of determining tolerance to an artificial sweetener in a subject comprising analyzing the amount of microbes belonging to an order selected from the group consisting of bacteroidales order, Clostridilales order, Bactobacillales order, YS2 order, RF32 order, Erysipelotrichales order, Burkholderiales order and/or Campylobacterales order in a microbiome of the subject, wherein an amount of microbes of the Bacteroidales, Clostridilales, Bactobacillales and/or YS2 order above a predetermined level is indicative of a subject being tolerant to the artificial sweetener and an amount of microbes of the RF32, Erysipelotrichales, Burkholderiales and/ or Campylobacterales order above a predetermined level is indicative of a subject being intolerant to the artificial sweetener.

Determining the amount of microbes belonging to a particular order has been described herein above.

According to this aspect the relative amount of microbes belonging to at least one of the orders, two of the orders, three of the orders, four of the orders, five of the orders, six of the orders, seven of the orders, eight of the orders, nine of the orders, ten of the orders, eleven of the orders or all of the above mentioned orders are analyzed.

Preferably the increase in the level is at least 1.5 fold, 2 fold, 5 fold or greater.

According to yet another aspect of the present disclosure there is provided a method of determining tolerance to an artificial sweetener in a subject comprising analyzing the amount of at least one microbe or class of microbes as set forth in Table 4 in a microbiome of the subject, wherein the amount of at least one of the microbes or the class of microbes above a predetermined level is indicative of a subject being intolerant to the artificial sweetener.

According to this aspect of the present disclosure at least one of the microbes described in Table 4 is analyzed, at least 5 % of the microbes described in Table 4 are analyzed, at least 10 % of the microbes described in Table 4 are analyzed, at least 20 % of the microbes described in Table 4 are analyzed, at least 30 % of the microbes described in Table 4 are analyzed, at least 40 % of the microbes described in Table 4 are analyzed, at least 50 % of the microbes described in Table 4 are analyzed.

Preferably the increase in the level is at least 1.5 fold, 2 fold, 5 fold or greater.

Further steps may be added to any of the methods described herein which are used to assess a subject's tolerance to an artificial sweetener including for example administering to the subject the artificial sweetener prior to the analyzing of the microbiome signature and preparing the sample for analysis (e.g. removal of fecal matter, making a protein extract, preparing a nucleic acid sample etc.).

*Altered Circadian rhythm*

The present inventors have shown that subjects with circadian misalignment (e.g. those suffering from jet-lag) have microbiomes that are statistically significantly similar to pathological microbiomes and suggest that the resulting microbial community may contribute to metabolic imbalances.

Thus, the present inventors propose determining if a subject is tolerant to having his circadian rhythm altered (e.g. changing time zones, performing night shifts etc.) by analyzing his microbiome. If his microbiome is statistically significantly similar to a pathological microbiome then it is indicative that he is intolerant to these conditions.

The present disclosure contemplates kits for analyzing a person's microbiome in order to determine his tolerance to different agents or conditions.

Thus, according to another aspect of the present disclosure there is provided a kit for determining whether a subject is tolerant to an agent comprising:

(i) an agent which is capable of determining an amount of at least one microbiome component, wherein the level of the at least one microbiome component is significantly different in an agent-tolerant microbiome and an agent-intolerant microbiome; and
(ii) a pathological microbiome.

In one aspect, the kit comprises an agent which is capable of determining an amount of at least one microbiome component, wherein the level of the at least one microbiome component is significantly different in an artificial sweetener-tolerant and artificial sweetener-intolerant subject.

The microbiome component (e.g. biomolecule) may be enriched, depleted, up-regulated, down-regulated, degraded, or stabilized in the agent-tolerant microbiome as compared to the agent-intolerant microbiome.

As mentioned herein above the microbiome component (i.e. biomolecule) may be a nucleic acid, an oligonucleic acid, an amino acid, a peptide, a polypeptide, a protein, a lipid, a carbohydrate, a metabolite, or a fragment thereof. Nucleic acids may include RNA, DNA, and naturally occurring or synthetically created derivatives. The microbiome related component may be present in, produced by, or modified by a microorganism within the gut.

The biomolecule may allow for the analysis of a particular species or class of microbes.

In the case of a microbe, the agent may be a primer or set of primers for amplifying 16S rRNA or 18S rRNA. An example of such a primer set is provided in the Examples section herein below. The kit of this aspect may comprise additional reagents required for subsequent sequencing reactions.

In the case of a gene (DNA) or RNA, the agent may be an oligonucleotide which hybridizes specifically to the DNA or RNA of interest.

The oligonucleotide may be in the form of an amplification primer. In this case, the kit may comprise additional components to perform an amplification reaction such as enzymes, salts and buffers. Typically, the kit comprises oligonucleotides for amplifying at least two genes known to be differentially expressed in artificial sweetener tolerant/intolerant microbiomes. According to a particular aspect, the two genes are part of a pathway known to be involved in artificial sweetener tolerance - such as the glycan degradation pathway (e.g. the glycosaminoglycan pathway).

Additionally, or alternatively, the primers may amplify genes involved in at least one process or pathway known to be up-regulated or down-regulated in an agent-tolerant microbiome as compared to an agent-intolerant microbiome. Thus, in the case of artificial sweeteners, the primer may amplify genes in one or more of the following processes or pathways: starch and sucrose metabolism, fructose and mannose metabolism, folate biosynthesis, glycerolipid-biosynthesis, fatty acid biosynthesis glucose transport pathways, ascorbate and aldarate metabolism, lipopolysaccharide biosynthesis and/or bacterial chemotaxis.

Alternatively, the oligonucleotide may be attached to a solid surface (i.e. array). Several substrates suitable for the construction of arrays are known in the art, and one skilled in the art will appreciate that other substrates may become available as the art progresses. The substrate may be a material that may be modified to contain discrete individual sites appropriate for the attachment or association of the oligonucleotide and is amenable to at least one detection method. Non-limiting examples of substrate materials include glass, modified or functionalized glass, plastics (including acrylics, polystyrene and copolymers of styrene and other materials, polypropylene, polyethylene, polybutylene, polyurethanes, TeflonJ, etc.), nylon or nitrocellulose, polysaccharides, nylon, resins, silica or silica-based materials including silicon and modified silicon, carbon, metals, inorganic glasses and plastics. In an exemplary aspect, the substrates may allow optical detection without appreciably fluorescing.

A substrate may be planar, a substrate may be a well, i.e. a 364 well plate, or alternatively, a substrate may be a bead. Additionally, the substrate may be the inner surface of a tube for flow-through sample analysis to minimize sample volume.

Similarly, the substrate may be flexible, such as a flexible foam, including closed cell foams made of particular plastics.

The oligonucleotide or oligonucleotides may be attached to the substrate in a wide variety of ways, as will be appreciated by those in the art. The oligonucleotide may either be synthesized first, with subsequent attachment to the substrate, or may be directly synthesized on the substrate. The substrate and the oligonucleotide may be derivatized with chemical functional groups for subsequent attachment of the two. For example, the substrate may be derivatized with a chemical functional group including, but not limited to, amino groups, carboxyl groups, oxo groups or thiol groups. Using these functional groups, the oligonucleotide may be attached using functional groups on the oligonucleotide either directly or indirectly using linkers.

The oligonucleotide may also be attached to the substrate non-covalently. For example, a biotinylated oligonucleotide can be prepared, which may bind to surfaces covalently coated with streptavidin, resulting in attachment. Alternatively, an oligonucleotide or oligonucleotides may be synthesized on the surface using techniques such as photopolymerization and photolithography. Additional methods of attaching oligonucleotides to arrays and methods of synthesizing oligonucleotides on substrates are well known in the art, i.e. VLSIPS technology from Affymetrix (e.g., see U.S. Pat. No. 6,566,495, and Rockett and Dix, "DNA arrays: technology, options and toxicological applications," Xenobiotica 30(2):155-177).

In one aspect, the oligonucleotide or oligonucleotides attached to the substrate are located at a spatially defined address of the array. Arrays may comprise from about 1 to about several hundred thousand addresses or more. In one aspect, the array may be comprised of less than 10,000 addresses. In another alternative aspect, the array may be comprised of at least 10,000 addresses. In yet another alternative aspect, the array may be comprised of less than 5,000 addresses. In still another alternative aspect, the array may be comprised of at least 5,000 addresses. In a further aspect, the array may be comprised of less than 500 addresses. In yet a further aspect, the array may be comprised of at least 500 addresses.

An oligonucleotide may be represented more than once on a given array. In other words, more than one address of an array may be comprised of the same oligonucleotide. In some aspects, two, three, or more than three addresses of the array may be comprised of the same oligonucleotide. In certain aspects, the array may comprise control oligonucleotides and/or control addresses. The controls may be internal controls, positive controls, negative controls, or background controls.

The array may be comprised of oligonucleotides which hybridize with DNA or RNA which are indicative of an artificial sweetener tolerant or non-tolerant microbiome.

In one aspect, the array may comprise an agent which can quantify or qualify the presence of a biomolecule enriched in the agent tolerant host microbiome compared to the agent intolerant host microbiome. In another aspect, the array may comprise an agent which can quantify or qualify the presence of a biomolecule depleted in the agent tolerant host microbiome compared to the agent intolerant host microbiome. In yet another aspect, the array may comprise an agent which can quantify or qualify the presence of a biomolecule up-regulated in the agent tolerant host microbiome compared to the agent intolerant host microbiome. In still another aspect, the array may comprise an agent which can quantify or qualify the presence a biomolecule down-regulated in the agent tolerant host microbiome compared to the agent intolerant host microbiome. In still yet another aspect, the array may comprise an agent which can quantify or qualify the presence of a biomolecule degraded in the agent tolerant host microbiome compared to the agent intolerant host microbiome. In an alternative aspect, the array may comprise an agent which can quantify or qualify the presence of a biomolecule stabilized in the agent tolerant host microbiome compared to the agent intolerant host microbiome.

For example, when the agent is an artificial sweetener, the array may comprise oligonucleotides that hybridize with DNA/RNA sequences that encode polypeptides involved in the glycan degradation pathway (e.g. the glycosaminoglycan pathway).

Additionally, or alternatively, when the agent is an artificial sweetener, the array may comprise oligonucleotides that hybridize with DNA/RNA sequences that encode polypeptides involved in at least one more of the following processes or pathways: starch and sucrose metabolism, fructose and mannose metabolism, folate biosynthesis, glycerolipid-biosynthesis, fatty acid biosynthesis glucose transport pathways, ascorbate and aldarate metabolism, lipopolysaccharide biosynthesis and/or bacterial chemotaxis.

Preferably, at least 2, 5, 10, 15, 20 genes of a particular pathway are represented on the array.

In one aspect, the array comprises oligonucleotides that hybridize with at least 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 275, 280, 285, 290, 295, 300, 305, 310, 315, 320, 325, 330, 335, 340, 345, 350, 355, 360, 365, 370, 375, 380, 385, 390, 395, or 400 oligonucleotides indicative of, or modulated in, agent tolerant host microbiome compared to an agent-intolerant host microbiome. In another aspect, the array comprises oligonucleotides that specifically identify at least 200, at least 300, at least 400, at least 500, at least 600, at least 700, at least 800, or at least 900 biomolecules indicative of, or modulated in, an agent-tolerant host microbiome compared to an agent-intolerant host microbiome.

The kits described herein may also comprise control samples. According to one aspect, the kit comprises a positive control e.g. a pathological microbiome.

Additionally, or alternatively, the kit comprises a negative control e.g. a non pathological microbiome.

The pathological and/or non-pathological microbiome may be processed. Thus, the control microbiomes may be represented as isolated polynucleotides or proteins.

Alternatively, the control microbiome may be represented by microbes.

The control samples may be in any suitable form, for example in a powdered dry form. In addition, the control samples may have undergone processing in order for it to increase its survival. For example, the microorganism may be coated or encapsulated in a polysaccharide, fat, starch, protein or in a sugar matrix. Standard encapsulation techniques known in the art can be used. For example, techniques discussed in U.S. Pat. No. 6,190,591, may be used.

According to still another aspect of the present disclosure there is provided a method of restoring the tolerance of a subject to an agent comprising administering to the subject an effective amount of a probiotic composition which comprises statistically significantly similar microbes to the non-pathological microbiome, thereby restoring the subjects tolerance to the agent.

For example, the present disclosure contemplates microbial compositions (e.g. probiotic compositions), wherein a majority of the microbes of the composition are microbes of the bacteroidales order, the Clostridilales order, the Bactobacillales order and/or the YS2 order for increasing tolerance to artificial sweeteners.

The present disclosure further contemplates microbial compositions (e.g. probiotic or antibiotic compositions) for increasing tolerance to circadian misalignment.

For example, the present inventors have shown that microbiota samples obtained during jet lag showed a higher relative representation of *Firmicutes*, which was reversed upon recovery from jet lag. Thus agents which can reduce the level of Firmicutes in the microbiome may be effective at restoring a subject's tolerance to circadian misalignment.

As used herein, the term "probiotic" refers to any microbial type that is associated with health benefits in a host organism and/or reduction of risk and/or symptoms of a disease, disorder, condition, or event in a host organism. In some aspects, probiotics are formulated in a food product, functional food or nutraceutical. In some aspects, probiotics are types of bacteria.

The microbial compositions of this aspect of the present disclosure may be statistically significantly similar to a microbiome of a subject who has found to be tolerant of the agent.

The microbial compositions may be taken from a microbiota sample of the microbiome.

A microbiota sample comprises a sample of microbes and or components or products thereof from a microbiome.

In some aspects, a microbiota sample is collected by any means that allows recovery of the microbes and without disturbing the relative amounts of microbes or components or products thereof of a microbiome. The particular method of recovery should be adapted to the microbiome source.

Alternatively, the microbial composition may be artificially created by adding known amounts of different microbes.

It will be appreciated that the microbial composition which is derived from the microbiota sample of a subject may be manipulated prior to administrating by increasing the amount of a particular strain or depleting the amount of a particular strain. Alternatively, the microbial compositions are treated in such a way so as not to alter the relative balance between the microbial species and taxa comprised therein. In some aspects, the microbial composition is expanded ex vivo using known culturing methods prior to administration. In other aspects, the microbial composition is not expanded ex vivo prior to administration.

According to one aspect, the microbial composition is not derived from fecal material.

According to still another aspect, the microbial composition is devoid (or comprises only trace quantities) of fecal material (e.g, fiber).

The probiotic microorganism may be in any suitable form, for example in a powdered dry form. In addition, the probiotic microorganism may have undergone processing in order for it to increase its survival. For example, the microorganism may be coated or encapsulated in a polysaccharide, fat, starch, protein or in a sugar matrix. Standard encapsulation techniques known in the art can be used. For example, techniques discussed in U.S. Pat. No. 6,190,591, may be used.

According to a particular aspect, the probiotic microorganism composition is formulated in a food product, functional food or nutraceutical.

In some aspects, a food product, functional food or nutraceutical is or comprises a dairy product. In some aspects, a dairy product is or comprises a yogurt product. In some aspects, a dairy product is or comprises a milk product. In some aspects, a dairy product is or comprises a cheese product. In some aspects, a food product, functional food or nutraceutical is or comprises a juice or other product derived from fruit. In some aspects, a food product, functional food or nutraceutical is or comprises a product derived from vegetables. In some aspects, a food product, functional food or nutraceutical is or comprises a grain product, including but not limited to cereal, crackers, bread, and/or oatmeal. In some aspects, a food product, functional food or nutraceutical is or comprises a rice product. In some aspects, a food product, functional food or nutraceutical is or comprises a meat product.

Prior to administration, the subject may be pretreated with an agent which reduces the number of naturally occurring microbes in the microbiome (e.g. by antibiotic treatment). According to a particular aspect, the treatment significantly eliminates the naturally occurring gut microflora by at least 20 %, 30 % 40 %, 50 %, 60 %, 70 %, 80 % or even 90 %.

In some aspects, administering comprises any means of administering an effective (e.g., therapeutically effective) or otherwise desirable amount of a composition to an individual. In some aspects, administering a composition comprises administration by any route, including for example parenteral and non-parenteral routes of administration. Parenteral routes include, e.g., intraarterial, intracerebroventricular, intracranial, intramuscular, intraperitoneal, intrapleural, intraportal, intraspinal, intrathecal, intravenous, subcutaneous, or other routes of injection. Non-parenteral routes include, e.g., buccal, nasal, ocular, oral, pulmonary, rectal, transdermal, or vaginal. Administration may also be by continuous infusion, local administration, sustained release from implants (gels, membranes or the like), and/or intravenous injection.

In some aspects, a composition is administered in an amount and/or according to a dosing regimen that is correlated with a particular desired outcome (e.g., with a particular change in microbiome composition and/or signature that correlates with an outcome of interest). In some aspects, the desired outcome is enhanced tolerance to artificial sweeteners, as described above. In some aspects, the desired outcome is tolerance to jet-lag or night shift work.

Particular doses or amounts to be administered in accordance with the present disclosure may vary, for example, depending on the nature and/or extent of the desired outcome, on particulars of route and/or timing of administration, and/or on one or more characteristics (e.g., weight, age, personal history, genetic characteristic, lifestyle parameter, severity of diabetes and/or level of risk of diabetes, etc., or combinations thereof). Such doses or amounts can be determined by those of ordinary skill. In some aspects, an appropriate dose or amount is determined in accordance with standard clinical techniques. Alternatively or additionally, in some aspects, an appropriate dose or amount is determined through use of one or more in vitro or in vivo assays to help identify desirable or optimal dosage ranges or amounts to be administered.

In some particular aspects, appropriate doses or amounts to be administered may be extrapolated from dose-response curves derived from in vitro or animal model test systems. The effective dose or amount to be administered for a particular individual can be varied (e.g., increased or decreased) over time, depending on the needs of the individual. In some aspects, where bacteria are administered, an appropriate dosage comprises at least about 100, 200, 300, 400, 500, 600, 700, 800, 900, 1000 or more bacterial cells. In some aspects, the present disclosure encompasses the recognition that greater benefit may be achieved by providing numbers of bacterial cells greater than about 1000 or more (e.g., than about 1500, 2000, 2500, 3000, 35000, 4000, 4500, 5000, 5500, 6000, 7000, 8000, 9000, 10,000, 15,000, 20,000, 25,000, 30,000, 40,000, 50,000, 75,000, 100,000, 200,000, 300,000, 400,000, 500,000, 600,000, 700,000, 800,000, 900,000, $1 \times 10^6$, $2 \times 10^6$, $3 \times 10^6$, $4 \times 10^6$, $5 \times 10^6$, $6 \times 10^6$, $7 \times 10^6$, $8 \times 10^6$, $9 \times 10^6$, $1 \times 10^7$, $1 \times 10^8$, $1 \times 10^9$, $1 \times 10^{10}$, $1 \times 10^{11}$, $1 \times 10^{12}$, $1 \times 10^{13}$ or more bacteria.

As well as treating a subject with probiotic compositions, the present disclosure also contemplates treating subjects with anti-microbial compositions whose presence are known to cause intolerance to an agent.

As used herein, the term "antibiotic agent" refers to a group of chemical substances, isolated from natural sources or derived from antibiotic agents isolated from natural sources, having a capacity to inhibit growth of, or to destroy bacteria, and other microorganisms, used chiefly in treatment of infectious diseases. Examples of antibiotic agents include, but are not limited to; Amikacin; Amoxicillin; Ampicillin; Azithromycin; Azlocillin; Aztreonam; Aztreonam; Carbenicillin; Cefaclor; Cefepime; Cefetamet; Cefinetazole; Cefixime; Cefonicid; Cefoperazone; Cefotaxime; Cefotetan; Cefoxitin; Cefpodoxime; Cefprozil; Cefsulodin; Ceftazidime; Ceftizoxime; Ceftriaxone; Cefuroxime; Cephalexin; Cephalothin; Cethromycin; Chloramphenicol; Cinoxacin; Ciprofloxacin; Clarithromycin; Clindamycin; Cloxacillin; Co-amoxiclavuanate; Dalbavancin; Daptomycin; Dicloxacillin; Doxycycline; Enoxacin; Erythromycin estolate; Erythromycin ethyl succinate; Erythromycin glucoheptonate; Erythromycin lactobionate; Erythromycin stearate; Erythromycin; Fidaxomicin; Fleroxacin; Gentamicin; Imipenem; Kanamycin; Lomefloxacin; Loracarbef; Methicillin; Metronidazole; Mezlocillin; Minocycline; Mupirocin; Nafcillin; Nalidixic acid; Netilmicin; Nitrofurantoin; Norfloxacin; Ofloxacin; Oxacillin; Penicillin G; Piperacillin; Retapamulin; Rifaxamin, Rifampin; Roxithromycin; Streptomycin; Sulfamethoxazole; Teicoplanin; Tetracycline; Ticarcillin; Tigecycline; Tobramycin; Trimethoprim; Vancomycin; combinations of Piperacillin and Tazobactam; and their various salts, acids, bases, and other derivatives. Anti-bacterial antibiotic agents include, but are not limited to, aminoglycosides, carbacephems, carbapenems, cephalosporins, cephamycins, fluoroquinolones, glycopeptides, lincosamides, macrolides, monobactams, penicillins, quinolones, sulfonamides, and tetracyclines.

Antibacterial agents also include antibacterial peptides. Examples include but are not limited to abaecin; andropin; apidaecins; bombinin; brevinins; buforin II; CAP18; cecropins; ceratotoxin; defensins; dermaseptin; dermcidin; drosomycin; esculentins; indolicidin; LL37; magainin; maximum H5; melittin; moricin; prophenin; protegrin; and or tachyplesins.

According to a particular aspect, the antibiotic is a non-absorbable antibiotic.

Thus, for example, the present disclosure contemplates treating a subject with an antibiotic that reduces the microbes of the RF32, Erysipelotrichales, Burkholderiales and/ or Campylobacterales order in order to enhance a subject's tolerance to an artificial sweetener. Preferably, the antibiotic does not have efficacy (or has less efficacy) against microbes which are of the Bacteroidales, Clostridilales, Bactobacillales and/or YS2 order.

The present inventors have found that the oscillating patterns of the microbiome impact the daily local and systemic transcriptome oscillations of the host. The present inventors showed that microbiota disruption by antibiotic treatment or in germ-free mice reprogrammed the intestinal and hepatic host transcriptome to feature both massive loss and de-novo genesis of oscillations, resulting in temporal reorganization of metabolic pathways. Accordingly, the present inventors propose that analysis of the rhythm of the microbiome over the course of a day may shed important information as to the dose or regime of administration of an antibiotic or probiotic agent. Thus, for example if the analysis of the rhythm of the microbiome shows that a particular microbe is at a peak in the morning hours and at a trough in the evening hours, then it may be recommended that a probiotic agent which comprises this microbe is administered in the morning and not the evening so as not to alter the natural circadian rhythm of the microbiome. If the analysis of the rhythm of the microbiome shows that a particular microbe is at a peak in the morning hours and at a trough in the evening hours, then it may be recommended that an antibiotic agent which downregulates this microbe is administered in the evening and not the morning so as not to alter the natural circadian rhythm of the microbiome.

Analysis of the microbiome may be performed by analyzing the level of microbes themselves or products (e.g. metabolites) thereof. Analysis of the microbiome is further described herein above.

In order to analyze the rhythm of the microbiome, at least two samples, at least 3 samples, at least 4 samples, at least 5 samples, at least 6 samples or more of the microbiome should be measured during the course of a 24 hour period.

It is to be understood that while the above types of additional information were described separately, the present aspects contemplate any combination of two or more types of information for the databases.

As used herein the term "about" refers to $\pm$ 10 %.

The word "exemplary" is used herein to mean "serving as an example, instance or illustration." Any aspect described as "exemplary" is not necessarily to be construed as preferred or advantageous over other aspects and/or to exclude the incorporation of features from other aspects.

The word "optionally" is used herein to mean "is provided in some aspects and not provided in other aspects." Any particular aspect of the disclosure may include a plurality of "optional" features unless such features conflict.

The terms "comprises", "comprising", "includes", "including", "having" and their conjugates mean "including but not limited to".

The term "consisting of' means "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various aspects of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Various aspects and aspects of the present disclosure as delineated hereinabove and as claimed in the claims section below find experimental support in the following examples.

## EXAMPLES

Reference is now made to the following examples, which together with the above descriptions illustrate some aspects of the disclosure in a non limiting fashion.

Generally, the nomenclature used herein and the laboratory procedures utilized in the present disclosure include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

## EXAMPLE 1

### *Trans-kingdom control of microbiota diurnal oscillations promotes metabolic homeostasis*

## MATERIALS AND METHODS

*Mice* - C57B1/6 mice were purchased from Harlan and allowed to acclimatize to the local animal facility for two weeks before used for experimentation. Unless otherwise specified, mice were kept under strict light-dark cycles, with lights being turned on at 6am and turned off at 6pm. In all experiments, age- and gender-matched were used. Mice were 8-9 weeks of age at the beginning of experiments. For experiments involving high-fat diet, only male mice were used. For all other experiments, both male and female mice were used. Stool samples were collected fresh and on the basis of individual mice. Fresh pellets were collected in tubes, immediately frozen in liquid nitrogen upon collection, and stored at -80°C until DNA isolation.

Unless stated otherwise, 10 mice per experimental group were used for the collection of fecal material from each individual mouse. For the induction of jet lag, mice were shifted between control light conditions (lights turned on at 6am and turned off at 6pm) and an 8-hour time difference (lights turned on at 10pm and turned off at 10am) every three days. Experiments performed on jet lagged mice were done when these mice were in the same light-dark cycle as control mice, and Zeitgeber times (ZTs) were synchronized (i.e. ZT0 of jet lag mice corresponded to ZT0 of control mice, as all mice were exposed to the same light-dark conditions at the onset of sample collection).

In food restriction experiments, mice were housed under standard light-dark conditions (6am to 6pm), but had access to food only during the light or dark phase, respectively, for two weeks. For antibiotic treatment, mice were given a combination of vancomycin (1 g/l), ampicillin (1 g/l), kanamycin (1 g/l), and metronidazole (1 g/l) in their drinking water (Rakoff-Nahoum et al., 2004). All antibiotics were obtained from Sigma Aldrich. Antibiotics were given for the entire duration of experiments, i.e. starting at the onset of jet lag induction until the experimental endpoint. For experiments involving gnotobiotic mice, germ-free Swiss Webster mice were housed in sterile isolators. For fecal transplantation experiments, 100mg of stool was resuspended in 1ml of PBS, homogenized, and filtered through a 70µm strainer. Recipient mice were gavaged with 200µl of the filtrate. All experimental procedures were approved by the local IACUC.

*Human samples* - Stool collection from humans was performed using sterile cotton swabs and stored at room temperature until arrival at the laboratory, where DNA extraction was performed. Collection of human stool was approved by the Tel Aviv Sourasky Medical Center Institutional Review Board. In experiments involving human stool collection at multiple times of the day, subjects ate four meals per day, and fecal samples were collected after food intake.

*Taxonomic microbiota analysis* - Frozen fecal samples were processed for DNA isolation using the MoBio PowerSoil kit according to the manufacturer's instructions. 1ng of the purified fecal DNA was used for PCR amplification and sequencing of the bacterial 16S rRNA gene. Amplicons spanning the variable region 2 (V2) of the 16S rRNA gene were generated by using the following barcoded primers: Fwd 5'-NNNNNNNNNAGAGTTTGATCCTGGCTCAG-3' (SEQ ID NO: 1), Rev 5'-TGCTGCCTCCCGTAGGAGT-3' (SEQ ID NO: 2), where N represents a barcode base. The reactions were subsequently pooled in an equimolar ratio, purified (PCR clean kit, Promega), and used for Illumina MiSeq sequencing. 500 bp paired-end sequencing was employed. Reads were then processed using the QIIME (Quantitative Insights Into Microbial Ecology, wwwdotqiimedotorg) analysis pipeline as described (Caporaso et al., 2010; Elinav et al., 2011). In brief, fasta quality files and a mapping file indicating the barcode sequence corresponding to each sample were used as inputs, reads were split by samples according to the barcode, taxonomical classification was performed using the RDP-classifier, a de-novo taxonomic tree of the sequences was built based on sequence similarity, and an OTU table was created. After chimera removal, the average number of reads per fecal sample was 34,847. Sequences sharing 97% nucleotide sequence identity in the V2 region were binned into operational taxonomic units (97% ID OTUs) using uclust, chimeric sequences were removed using ChimeraSlayer.

*Metagenomic sequence mapping.* Illumina sequencing reads were mapped to a gut microbial gene catalogue [20203603] using GEM mapper [23103880] with the following parameters:
-m 0.08 -s 0 -q offset-33 -gem-quality-threshold 26

*Functional assignment.* Reads mapped to the gut microbial gene catalogue were assigned a KEGG [10592173, 24214961] identification number, according to the gene to category mapping that accompanied each of these databases. Genes were subsequently mapped to KEGG modules and pathways. For the KEGG pathway analysis, only pathways whose gene coverage was above 0.2 were included. KEGG pathways were then tested by JTK cycle to daily oscillations.

*Glucose tolerance test* - Mice were fasted for 6 hours and subsequently given 200µl of a 0.2g/ml glucose solution (JT Baker) by oral gavage. Blood glucose was determined at 0, 15, 30, 60, 90, and 120 minutes after glucose challenge (Contour™ blood glucose meter, Bayer, Switzerland).

*Magnetic resonance imaging* - Mice were anesthetized with isofluorane (5% for induction, 1-2% for maintenance) mixed with oxygen (1 liter/min) and delivered through a nasal mask. Once anesthetized, the animals were placed in a head-holder to assure reproducible positioning inside the magnet. Respiration rate was monitored and kept throughout the experimental period around 60-80 breaths per minute. MRI experiments were performed on 9.4 Tesla BioSpec Magnet 94/20 USR system (Bruker, Germany) equipped with gradient coils system capable of producing pulse gradient of up to 40 gauss/cm in each of the three directions. All MR images had been acquired with a quadrature resonator coil (Bruker). The MRI protocol included two sets of coronal and axial multi-slices T2-weighted MR images. The T2-weighted images acquired using the multi-slice RARE sequence (TR = 2500 ms, TE = 35 ms, RARE factor = 8), and matrix size was 256 x 256, four averages, corresponding to an image acquisition time of 2 min 40 sec per set. The first set was used to acquire 21 axial slices with 1.00mm slice thickness (no gap). The field of view was selected with 4.2 x 4.2 cm$^2$. The second set was used to acquire 17 coronal slices with 1.00mm slice thickness (no gap). The field of view was selected with 7.0 x 5.0 cm$^2$.

Total fat and lean mass of mice were measured by EchoMRI-100™ (Echo Medical Systems, Houston, TX).

*Metabolic studies* - Food intake and locomotor activity were measured using the PhenoMaster system (TSE-Systems, Bad Homburg, Germany), which consists of a combination of sensitive feeding sensors for automated measurement and a photobeam-based activity monitoring system detects and records ambulatory movements, including rearing and climbing, in each cage. All parameters were measured continuously and simultaneously. Mice were trained singly-housed in identical cages prior to data acquisition.

*Gene expression analysis* - Tissues were preserved in RNAlater solution (Ambion) and subsequently homogenized in Trizol reagent (Invitrogen). Cells sorted by FACS were resuspended in Trizol reagent. RNA was purified according to the manufacturer's instructions. One microgram of total RNA was used to generate cDNA (HighCapacity cDNA Reverse Transcription kit; Applied Biosystems). RealTime-PCR was performed using gene-specific primer/probe sets (Applied Biosystems) and Kapa Probe Fast qPCR kit (Kapa Biosystems) on a Viia7 instrument (Applied Biosystems). PCR conditions were 95°C for 20 s, followed by 40 cycles of 95°C for 3 s and 60°C for 30 s. Data were analyzed using the deltaCt method with hprtl serving as the reference housekeeping gene.

*Statistical Analysis* - Data are expressed as mean ± SEM. For the analysis of rhythmic oscillations and their amplitudes, the non-parametric test JTK_cycle was used (Hughes et al., 2010), incorporating a window of 18-24 hours for the determination of circadian periodicity. P values < 0.05 were considered significant. The Benjamini-Hochberg procedure was used to control the false discovery rate. JTK cycle results are provided in supplemental tables. Differences in metabolic data were analyzed by ANOVA, and post-hoc analysis for multiple group comparison was performed. Pairwise comparison between host transcript data was performed using student's t-test. ANOVA and t-test were performed using GraphPad Prism software.

**RESULTS**

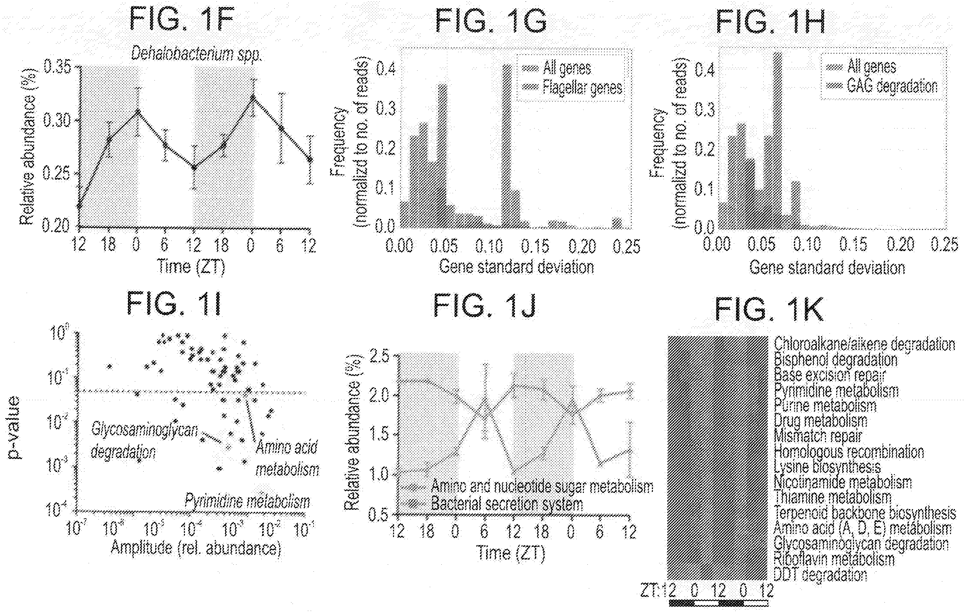

To determine the longitudinal changes of microbiota composition over the course of a day, taxonomic analysis of fecal microbiota from mice was performed every 6 hours for two light-dark cycles (Figure 1A). All mice were fed ad libitum, housed under strict 24-hour dark-light conditions, with lights being kept on for 12 hours. Samples were taken at the time points of changing light conditions (Zeitgeber times (ZT) 12 and 0, i.e. "dusk" and "dawn", respectively) and at the midpoint of the dark and light phases (ZT 18 and 6, respectively). The commonly used non-parametric algorithm JTK_cycle was used to detect rhythmic elements in the taxonomic data set (Hughes et al., 2010). Strikingly, significant ($p<0.05$) diurnal fluctuations were detected in the abundance of more than 15% of all bacterial operational taxonomic units (OTUs) (Figure 1B). Groups of fluctuating bacteria featured distinctive acrophase and bathyphase times with a 24-hour period. Bacterial genera rhythmically oscillating in a 24-hour cycle belonged to abundant taxonomical orders, namely *Clostridiales, Lactobacillales,* and *Bacteroidales,* such that rhythmically oscillating OTUs accounted for about 60% of the microbiota composition and resulted in time of the day-specific taxonomic configurations (Figures 1C and 1D). Highly robust circadian fluctuations were found, for instance, in *Lactobacillus reuteri* and *Dehalobacterium spp.* (Figures 1E and 1F). The rhythmicity was reproducible regardless of housing conditions or cage effects (data not shown). These results were confirmed with a finer sampling resolution over a longer sampling period, with fecal samples being collected every 4 hours for 4 consecutive days (Figures 2A-D).

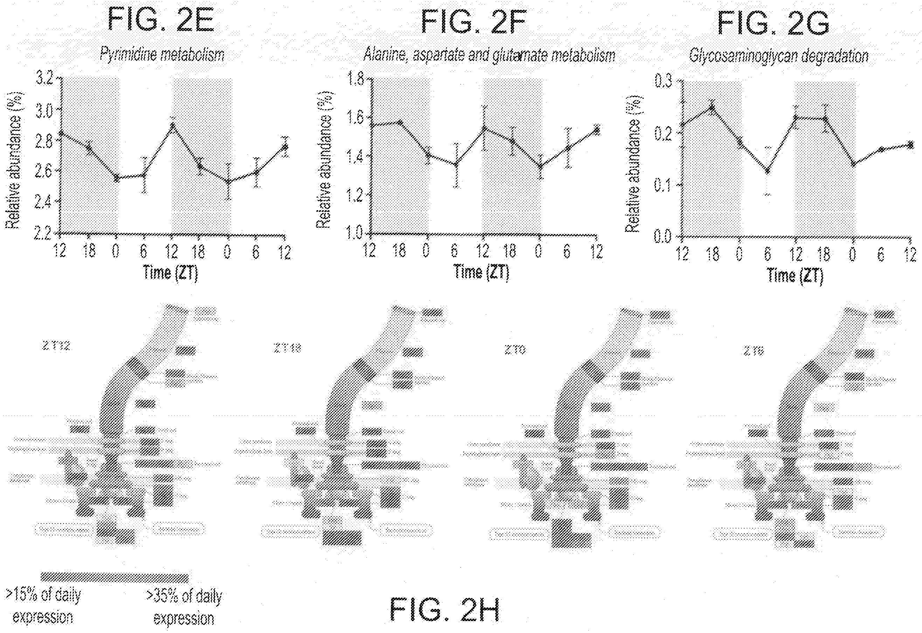

The present inventors next analyzed whether these diurnal oscillations in microbiota composition have consequences for the functional capacities of the intestinal microbial community over the course of a day. Shotgun metagenomic sequencing of fecal samples collected every 6 hours over the course of two light-dark cycles was performed and the metagenomic reads were mapped to a gut microbial gene catalogue (Qin et al., 2010). While the majority of genes showed a stable level over the course of a day, certain groups of genes (such as genes involved in flagellar assembly and glycosaminoglycan degradation, Figures 1G and 1H) featured a stronger variation in abundance. To test whether such fluctuations in genes belonging to functional entities follow diurnal rhythms, genes were grouped into KEGG pathways (Kanehisa and Goto, 2000; Kanehisa et al., 2014) and the JTK_cycle algorithm was employed to detect oscillations that occur with a 24-hour rhythm. Interestingly, 23% of all pathways with gene coverage above 0.2 featured diurnal rhythmicity (Figure 1I). Among these were pathways involved in nucleic acid homeostasis, including nucleotide metabolism (Figure 2E), amino acid metabolism (Figure 2F), and mucus degradation (Figure 2G).

These results suggest the existence of day time-specific profiles of microbiota functionality. Interestingly, it appeared that distinct functional groups exhibited coordinated anti-phasic fluctuations (Figure 1J). For instance, functions involved in energy metabolism, DNA repair, and cell growth were favorably performed during the dark phase (Figure 1K), while the light phase featured higher abundance of "maintenance" pathways involved in detoxification, motility and environmental sensing. For instance, genes performing functions in flagellar assembly, bacterial chemotaxis, and type III secretion were most abundant during the light phase (Figure 2H).

Together, these results uncover fluctuations in microbiota composition and function on the scale of hours, which follow 24-hour rhythmicity, and which result in robust oscillations and time of day-specific configurations.


***A functional circadian clock of the host is required for diurnal microbiota oscillations***

Importantly, the observed gut microbiota diurnal rhythmicity was present despite the lack of direct microbial exposure to environmental light-dark alterations. The present inventors thus sought to determine how these rhythmic fluctuations in microbiota composition are generated in a 24-hour period. The biological clock of the host is synchronized to environmental day-night variations by the molecular components of the circadian clock. To test whether the circadian clock of the host is required for diurnal rhythmicity in microbiota composition, *Per1/2*-/- mice were used, which are deficient in a functional host clock (Adamovich et al., 2014). A taxonomic comparison between the microbiota of wild-type and *Per1/2*-/- mice was performed at each phase of the dark-light cycle over 48 hours, and the JTK_cycle algorithm was then used to identify rhythmic elements. Notably, *Per1/2*-/- mice demonstrated a near complete loss of rhythmic fluctuations in commensal bacterial abundance (Figure 3A), as exemplified by *Bacteroidales* (Figure 3B). The rhythmic pattern observed in wild-type mice was replaced by a random abundance fluctuation in clock-deficient mice with a reduction in the number of oscillating bacterial taxonomic units (Figure 3C).

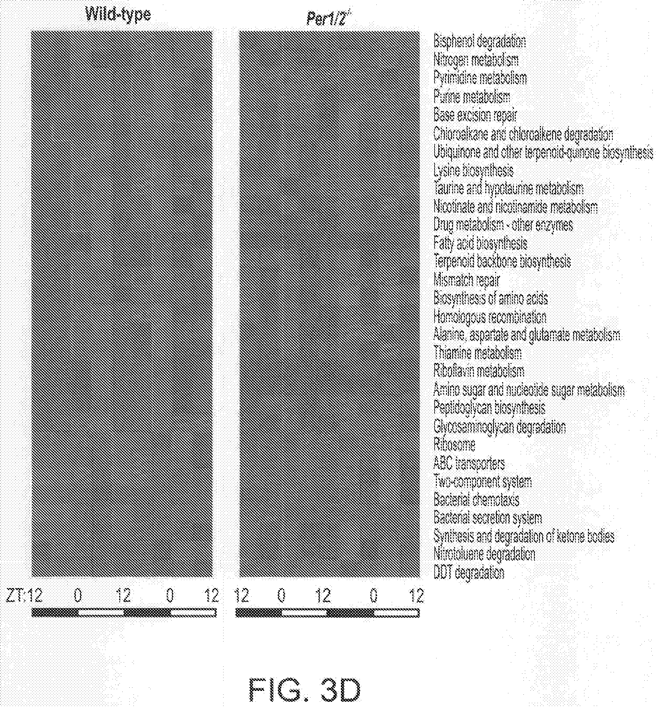

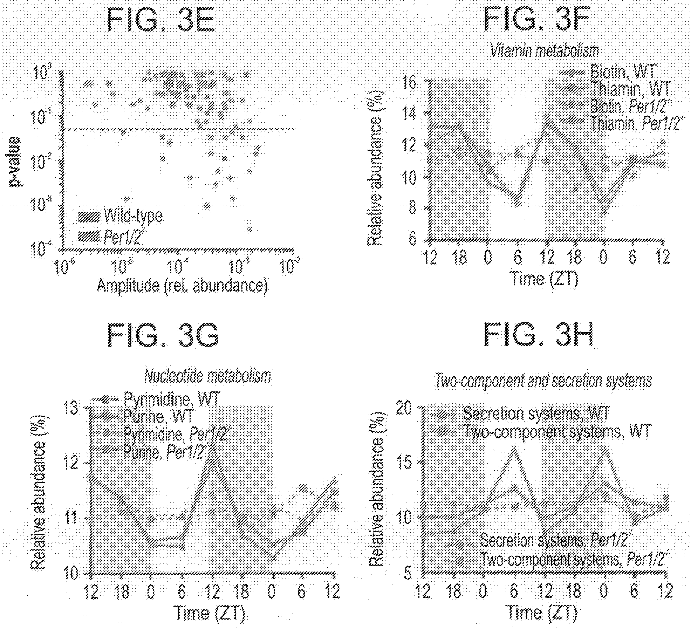

To determine whether the loss of compositional oscillations has any consequences for the diurnal metagenomic profile shotgun sequencing of microbiota from *Per1/2*-/- mice was performed and the results were compared to wild-type mice at each phase of the day. The diurnal patterns in metagenomic pathways observed in wild-type mice was non-existent in *Per1/2*-deficient mice (Figures 3D and 3E), and were instead replaced by mostly invariant levels of pathway activity throughout the light-dark cycle. The preferential activity of certain functionalities during the light or dark phase was therefore lost in *Per1/2*-/- mice. For instance, pathways involved in vitamin metabolism (Figure 3F), nucleotide metabolism (Figure 3G), two-component as well as secretion systems (Figure 3H), DNA repair (Figure 4A), cell wall synthesis (Figure 4B), and motility (Figure 4C) lost their diurnal rhythmicity in *Per1/2*-/- mice. Together, these data indicate that a functional circadian clock of the host is required for the generation of diurnal fluctuations in the composition and function of the intestinal microbiota.

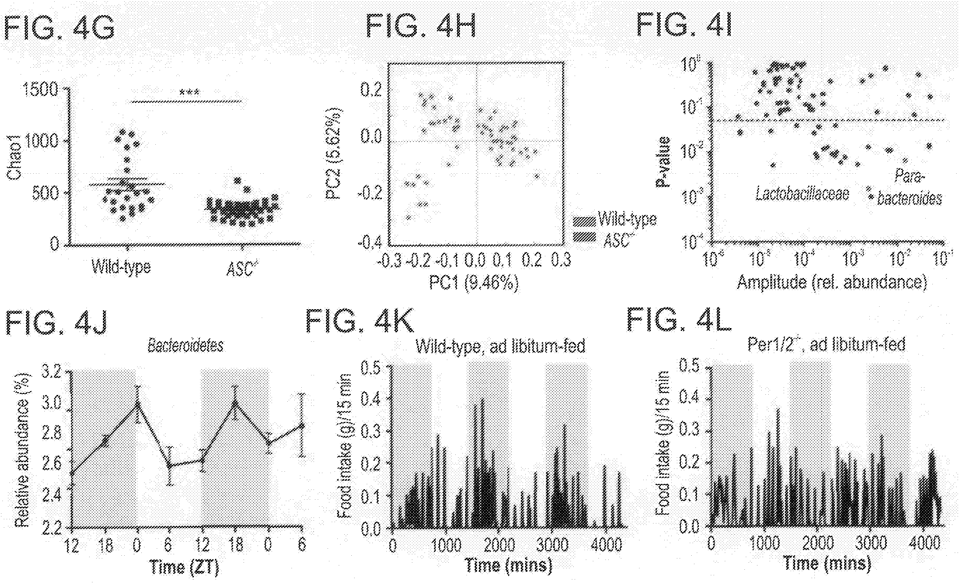

Importantly, the present inventors also noted dysbiosis in Per1/2-deficient mice, as evident from lower alpha-diversity (Figure 4D) and featured distinct intestinal community composition when compared to controls (Figure 4E). Some of the biggest differences in microbiota composition between wild-type and Per1/2-deficient mice were found in bacterial genera which undergo diurnal fluctuations in wild-type mice (Figures 4F). To rule out the possibility that dysbiosis and loss of diurnal microbiota oscillations are inherently interconnected, other genetically modified, dysbiotic mice were analyzed for the existence of diurnal microbiota oscillations. Mice deficient in the inflammasome adaptor ASC were selected, a model which has recently been described to feature a functionally-important and well-defined dysbiosis (Elinav et al., 2011; Henao-Mejia et al., 2012). Indeed, fecal communities of wild-type and $ASC^{-/-}$ mice differed by alpha- and beta-diversity (Figures 4G and 4H). Nonetheless, bacterial OTUs from $ASC^{-/-}$ mice displayed significant compositional oscillations, as identified by JTK_cycle (Figures 4I and 4J). Thus, it may be concluded that microbiota diurnal oscillations are present at different microbiota configurations, and that compositional dysbiosis and loss of diurnal rhythmicity may occur independently of each other.

### Microbiota diurnal oscillations are controlled by feeding time

The present inventors next set out to determine the mechanism by which the circadian clock of the host is involved in generating microbial compositional oscillations in the intestine. The host circadian clock controls the rhythmicity of many physiological functions, including food consumption (Turek et al., 2005). Conversely, feeding times are central in entraining and synchronizing peripheral clocks (Asher et al., 2010; Hoogerwerf et al., 2007; Stokkan et al., 2001). Rodents are nocturnal animals that eat preferentially during the dark phase (Figure 4K). In contrast, $Per1/2^{-/-}$ mice feature a greatly attenuated diurnal feeding rhythm, and consume food continuously throughout the day (Figure 4L). It is therefore plausible that microbiota rhythmicity in a normal wild-type host is driven by its diurnal eating habits, while the diminished microbiota rhythmicity in $Per1/2^{-/-}$ mice is secondary to its profoundly altered food consumption timing. To this end, a timed feeding experiment was performed in which wild-type mice were given access to food only during the light phase or only during the dark phase (Figures 5A, 6A and 6B). In line with the ability of scheduled feeding to entrain peripheral clocks, this reversal of feeding habits inverted the expression pattern of intestinal clock genes (Figure 6C). After two weeks of continuous scheduled feeding, fecal microbiota samples were collected every 6 hours for two consecutive light-dark cycles. Using the JTK_cycle algorithm, it was found that the microbiota oscillations in the dark phase-fed group was similar to ad libitum-fed mice, reflecting the normal mainly nocturnal feeding habits of rodents, Figures 5B-5F, 6D). In contrast, cycling OTUs often featured distinct phases between dark phase-fed and light-phase fed groups (Figures 5C-5F and 6D). Most cycling OTUs appeared to exhibit a phase shift of about 12 hours upon modification of feeding times, suggesting direct control of microbiota rhythms by feeding times. Such phase shift was, for instance, observed in the case of *Bacteroides acidifaciens* (Figure 5C), *Lactobacillus reuteri* (Figure 5D), and *Peptococcaceae* (Figure 5E). The present inventors also observed cases of de-novo or enhanced rhythmicity in the light phase-fed groups, as exemplified by *Candidatus Arthromitus* (Figure 5F). These results suggest that feeding times influence daily fluctuations in microbiota composition, and that the oscillations in abundance of commensal bacteria can be controlled by scheduled feeding.

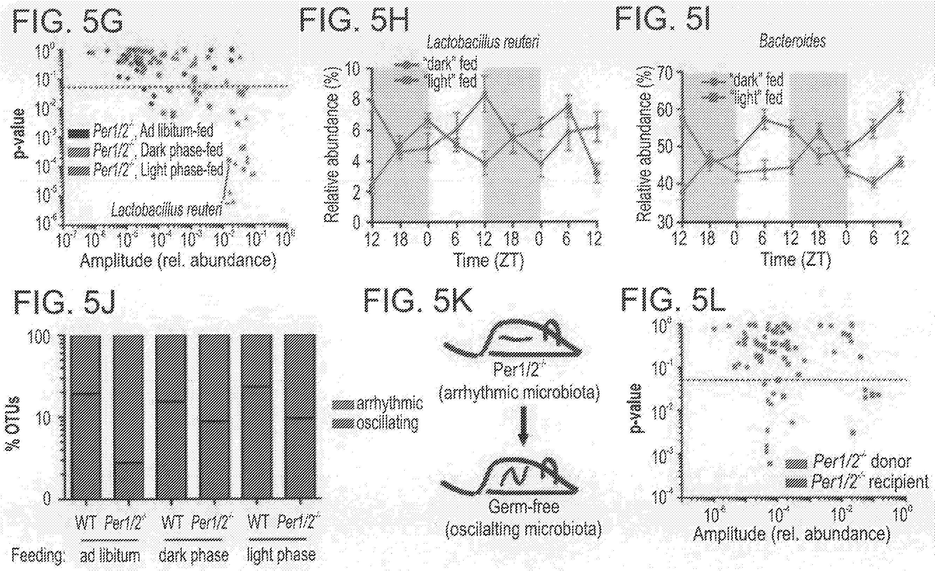

Consequently, if feeding times are directly controlling diurnal fluctuations in microbiota composition, then timed feeding should rescue the loss of such fluctuations in mice deficient in the circadian clock. The present inventors therefore performed a similar food restriction experiment on $Per1/2^{-/-}$ mice and microbiota samples were analyzed every 6 hours over two light-dark cycles after two weeks of scheduled feeding. Indeed, both light phase-fed and dark phase-fed, but not ad libitum-fed $Per1/2^{-/-}$ mice featured significantly oscillating bacterial OTUs, demonstrating de-novo rhythmicity generation in a formerly arrhythmic community composition (Figure 5G and 6E).

Similar to wild-type mice undergoing timed feeding, the phase of microbiota oscillations followed the feeding time in $Per1/2^{-/-}$ mice, and oscillating OTUs showed phase shifts between dark phase-fed and light phase-fed mice (Figure 6E). For instance, the oscillations in *Lactobacillus reuteri* (Figure 5H) and *Bacteroides* (Figure 5I) observed in dark phase-fed $Per1/2^{-/-}$ mice followed the patterns observed in ad libitum-fed or dark phase-fed wild-type mice, while the light phase-fed group exhibited opposite cycles. These results demonstrate that rhythmic feeding can reconstitute OTU oscillations in $Per1/2^{-/-}$ mice (Figure 5J).

To further corroborate the centrality of host feeding rhythmicity in controlling microbiota oscillations, microbiota from $Per1/2^{-/-}$ mice (lacking diurnal fluctuations) were transplanted into germ-free mice that were housed under normal light-dark conditions (Figure 5K). Upon fecal transplantation, colonized germ-free mice exhibited regular nocturnal activity and metabolic patterns (Figure 6F and 6G). This was also observed when control transplantations with microbiota from wild-type mice were performed (Figure 6H). Notably, one week after transplantation into the germ-free host, fecal microbiota from $Per1/2^{-/-}$ mice featured a normalized diurnal rhythmicity (Figures 5L). Taken together, these results show that rhythmicity of food intake dictates daily oscillations in microbiota composition, and that microbiota rhythmicity is a flexible process that can be lost or regained in response to changed feeding behaviors. Thereby, feeding times couple the circadian patterns of host behavior to diurnal fluctuations in microbiota composition and function.

***Environmental disruption of normal sleep patterns induces loss of microbiota diurnal rhythmicity and dysbiosis***

The present inventors next sought to test the physiological relevance of microbiota diurnal rhythmicity. In humans, disturbances of the circadian clock often occur in the setting of shift work and chronic jet lag, where external light conditions change frequently and impair the ability of the molecular clock to adapt to a stable rhythm. This situation was mimicked in mice by using a jet lag model in which mice were exposed to an 8-hour time shift every three days (Figure 7A). This model simulates the jet lag situation induced by frequent flying between countries with an 8-hour time difference and likewise mimics a scenario of regular switching between day and night shift-work (Huang et al., 2011; Yamaguchi et al., 2013). After four weeks of jet lag induction, mice returned to the starting light cycle conditions and were analyzed one day after the last time shift. Induction of jet lag resulted in the loss of host rhythmic physical activity (Figures 8A and 8B). Similar to humans, jet lag also led to an irregular pattern of food intake rhythms, resulting in a loss of day-night variations in food consumption (Figure 7B and 8C). Nonetheless, the overall daily amount of food intake was not affected between control and jet lagged mice (Figure 7B). Successful induction of jet lag was also confirmed by a shift in peripheral clock transcript oscillations (Figures 8D-8F).

Given the finding that rhythmic food intake induces diurnal fluctuations in the microbiota, the present inventors examined whether these disruptions of rhythmic behavior by jet lag would also impair diurnal oscillations in microbiota composition. To this end, a taxonomic analysis of microbiota composition was made every 6 hours in jet lagged mice and rhythmicity was tested by JTK cycle. Analogous to mice deficient in the circadian clock, jet lagged mice featured an abrogation of bacterial rhythms with a reduced number of oscillating bacterial taxonomic units (Figures 7C-E). Together, similar to genetic disruption of the circadian clock, environmentally-induced abrogation of daily oscillatory patterns is associated with loss of diurnal rhythmicity in microbiota composition.

Since dysbiosis was observed in genetically clock-deficient mice, the community composition of "jet lagged" mice was analyzed after 4 weeks of time shifts. Indeed, microbiota composition slightly differed between control and jet lagged mice (Figure 7F). When mice were followed for a period of 16 weeks of continuous time shifting, dysbiosis was enhanced (Figure 7G) and taxonomic units that were found to be oscillating in wild-type mice were affected (Figure 7H). Altogether, these data suggest that chronic environmental or genetic disruption of the mammalian dark-light cycle manifests as significant alterations in feeding rhythms and as a failure to maintain microbiota rhythmicity and composition.

***Dysbiosis associated with environmental clock disruption drives metabolic disease***

Chronic jet lag and shift work are behavioral patterns that have become widespread in humans only recently, following the industrial revolution. These newly introduced behavioral patterns are associated with increased risk for obesity, diabetes, and cardiovascular disease, all disease states that have emerged in parallel in modern human populations (Archer et al., 2014; Buxton et al., 2012; Fonken et al., 2010; Scheer et al., 2009; Suwazono et al., 2008). Since loss of microbiota oscillations and dysbiosis were found to be associated with jet lag in mice, the present inventors set out to test whether the microbiota involved in metabolic imbalances is associated with altered circadian rhythms. They first established that jet lag is associated with manifestations of the metabolic syndrome. Jet lagged and control mice were fed a high-fat diet, containing 60% of caloric energy from fat, thereby mimicking human dietary habits predisposing to the metabolic syndrome. Indeed, as early as 6 weeks after instating of high-fat diet, time-shifted mice exhibited enhanced weight gain and exacerbated glucose intolerance as compared to mice maintained on normal circadian rhythmicity (Figures 9A-9C).

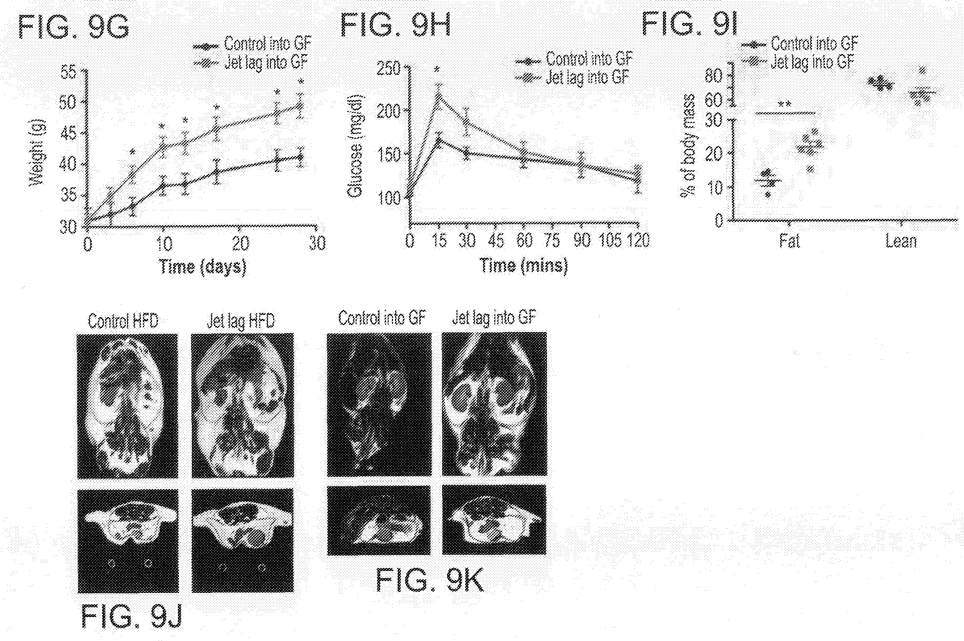

Since the overall food intake was not different between wild-type and jet lagged mice (Figure 7B), it was hypothesized that alterations in microbiota composition may contribute to this metabolic phenotype. Indeed, wide spectrum antibiotic treatment for the duration of jet lag induction (vancomycin, ampicillin, kanamycin, and metronidazole in the drinking water, (Fagarasan et al., 2002; Rakoff-Nahoum et al., 2004)), abrogated obesity and glucose intolerance in jet lagged mice (Figures 9A-9C). Obesity in time-shifted mice was associated with higher fat mass, which was rescued by antibiotic treatment (Figures 9D and 9E). Magnetic resonance imaging (MRI) revealed that this accumulation of fat mass resulted in increased subcutaneous and visceral fat deposition in mice that underwent chronic time shifting (Figure 9J).

Of note, glucose tolerance by itself underlies circadian variation (Kaasik et al., 2013; So et al., 2009). Nevertheless, diurnal differences in glucose intolerance between jet lagged and control groups persisted irrespective of daily time of measurement (data not shown). Disruption of nocturnal behavior and feeding patterns in jet lagged mice was unaffected by high-fat diet or antibiotics treatment (Figure 10A-10F). While high-fat feeding did to some extend reduce the amount of oscillating OTUs (Figure 10G-I), microbiota oscillations persisted after one week of antibiotics treatment (Figure 10J-K).

To further highlight that jet lag-induced adverse effect on weight homeostasis was independent of the dietary composition, jet lagged mice maintained on regular chow diet for four months featured higher body weight and increased body fat mass as compared to their non-jet lagged controls (Figure 9F).

To further corroborate the role of the altered microbiota in the metabolic imbalances observed in jet lagged mice, fecal transfer of control or "jet lagged" microbiota configurations into germ-free Swiss Webster mice was performed. Recipients of the time-shifted microbiota exhibited enhanced weight gain and glucose intolerance as compared to control microbiota recipients (Figures 9G and 9H).

Furthermore, similar to their respective donors, recipients of microbiota from time-shifted mice featured a significant increase in body adiposity (Figure 9I). MRI scanning showed an increase in body fat in germ-free mice that had received microbiota from jet lagged donors (Figure 9K). Collectively, these results demonstrate that jet lag-associated metabolic derangements are transmissible by the microbiota.

### *Human microbiota exhibits diurnal oscillations and time shift-associated dysbiosis with metabolic consequences*

Finally, the present inventors examined whether the findings in animal models may apply to humans. They first determined microbiota community variations in human fecal samples from two subjects collected at multiple time points during the day for several consecutive days (Figure 11A). Using 16S sequencing, diurnal fluctuations in the abundance of up to 10% of all bacterial OTUs (Figure 11B and 11C) were found.

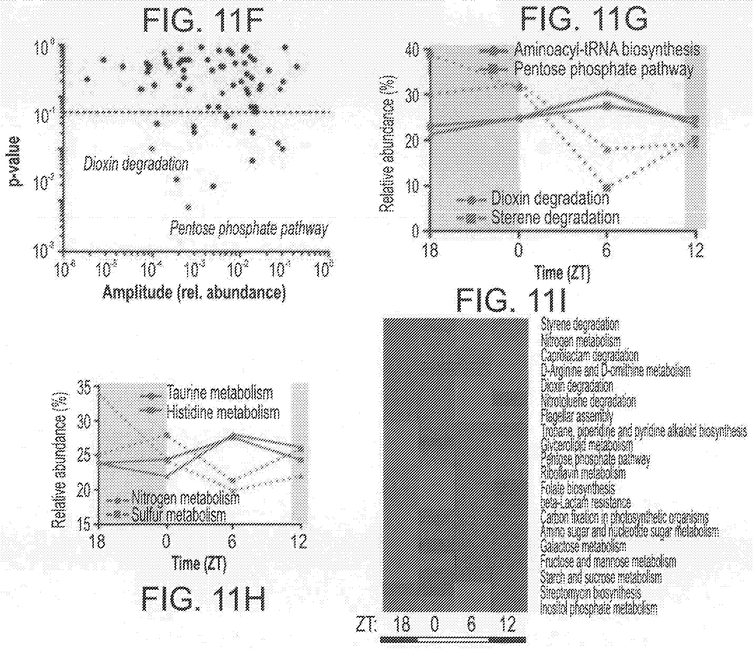

Similar to what was found in mice, oscillating OTUs features distinct acrophases and bathyphases over the course of a day (Figure 11D). Robust oscillations were found, for instance, in *Paraacteroides* (Figure 11E), *Lachnospira* (Figure 12A), and *Bulleida* (Figure 12B). The diurnal rhythmicity in OTU abundance resulted in time of the day-specific microbiota community configurations with a repetitive pattern over the observed time period (Figure 12C). Metagenomic analysis of human samples was performed at multiple times of a day. It was found that about 20% of all pathways with a gene coverage higher than 0.2 exhibited a diurnal abundance pattern (Figures 11F), as exemplified by genes belonging to dioxin degradation pathways (Figure 12D). Analogous to the findings in mice, distinct functional entities featured preferential abundance at different times of the day. For example, energy metabolism and protein production was preferentially performed during the light phase, while detoxification pathways were mostly active during the night (Figures 11G and Figure 11H). The peak phases of pathway activity occurred at opposite times of the day compared to mouse microbiota (Figure 11I), as would be expected from diurnal versus nocturnal behavior of the host. Together, these data suggest that like in mice, components of the human intestinal microbiota may undergo diurnal variations in composition and function.

Furthermore, the data in mice suggests that disruption of the circadian clock by aberrant sleep-activity cycles leads to aberrant microbiota composition. The time shift model which was applied in mice corresponds to the jet lag induced by flying between countries with an 8-hour time difference. The present inventors therefore collected fecal samples from two healthy human donors who underwent such a flight-induced time shift of 8-10 hours (flying from central or western United States time zones to Israel) and performed a taxonomic analysis one day before the induction of travel-induced jet lag, during jet lag (one day after landing), and after recovery from jet lag (two weeks after landing) (Figure 13A). Indeed, microbiota communities showed a time shift-induced change in composition, detected 24 hours into jet lag (Figure 13B and Tables A and B). Microbiota samples obtained during jet lag showed a higher relative representation of *Firmicutes,* which was reversed upon recovery from jet lag.

Interestingly, *Firmicutes* have been associated with a higher propensity for obesity and metabolic disease in multiple human studies (Ley et al., 2006; Ridaura et al., 2013). To analyze whether the microbiota changes in jet lagged individuals were associated with increased susceptibility to metabolic disease, fecal transfer experiments into germ-free mice of human samples obtained from individual subjects before jet lag, 24 hours into jet lag, and following recovery from jet lag were performed (Figure 13C).

Germ-free mice colonized with microbiota from jet lagged individuals displayed enhanced weight gain and featured higher blood glucose levels after oral glucose challenge compared to samples taken before the time-shift (Figure 13D and 13E). This metabolic effect was abrogated following recovery from jet lag (Figure 13D and 13E).

Furthermore, germ-free recipients of microbiota from the jet lagged state accumulated more body fat than mice receiving microbiota from the same subjects before or after jet lag (Figure 13F). Together, albeit preliminary, these data suggest that some commensal representatives of the human microbiota may undergo diurnal oscillations, that circadian misalignment in humans is associated with dysbiosis, and that the resulting microbial community may contribute to metabolic imbalances.

*Table A*

| two weeks after flight | one day after flight | one day before flight | Taxon |
|---|---|---|---|
| 0.711608729 | 0.613459201 | 0.719298246 | k_Bacteria;p_Bacteroidetes |
| 0.004089705 | 0.005229496 | 0.004099033 | k_Bacteria;p_Cyanobacteria |
| 0 | 0 | 0 | k_Bacteria;p_Deferribacteres |
| 0.279172673 | 0.374215882 | 0.271132376 | k_Bacteria;p_Firmicutes |
| 0 | 0 | 0 | k_Bacteria;p_Fusobacteria |
| 0.000100567 | 0 | 1.49E-05 | k_Bacteria;p_Lentisphaerae |
| 0.002447119 | 0.001927303 | 0.001266974 | k_Bacteria;p_Proteobacteria |
| 0 | 0.001264409 | 0.000223584 | k_Bacteria;p_Synergistetes |
| 3.35E-05 | 0.000184137 | 5.96E-05 | k_Bacteria;p_TM7 |
| 0.002547685 | 0.003719571 | 0.00390526 | k_Bacteria;p_Tenericutes |
| 0 | 0 | 0 | k_Bacteria;p_Verrucomicrobia |
| 0.711541685 | 0.613422374 | 0.719253529 | k_Bacteria;p_Bacteroidetes;c_Bacteroidia |
| 0 | 0 | 0 | k_Bacteria;p_Bacteroidetes;c_Flavobacteriia |
| 6.70E-05 | 3.68E-05 | 4.47E-05 | k_Bacteria;p_Bacteroidetes;c_Sphingobacteriia |
| 0.004089705 | 0.005229496 | 0.004084127 | k_Bacteria;p_Cyanobacteria;c_4C0d-2 |
| 0 | 0 | 1.49E-05 | k_Bacteria;p_Cyanobacteria;c_Chloroplast |
| 0 | 0 | 0 | k_Bacteria;p_Cyanobacteria;c_Nostocophycideae |
| 0 | 0 | 0 | k_Bacteria;p_Deferribacteres;c_Deferribacteres |
| 0.01910764 | 0.047519672 | 0.0280821 | k_Bacteria;p_Firmicutes;c_Bacilli |
| 0.260065033 | 0.326671659 | 0.243035371 | k_Bacteria;p_Firmicutes;c_Clostridia |
| 0 | 2.46E-05 | 1.49E-05 | k_Bacteria;p_Firmicutes;_Erysipelotrichi |
| 0 | 0 | 0 | k_Bacteria;p_Fusobacteria;c_Fusobacteriia |
| 0.000100567 | 0 | 1.49E-05 | k_Bacteria;p_Lentisphaerae;c_[Lentisphaeria] |
| 0.000435788 | 0.001559028 | 0.000924146 | k_Bacteria;p_Proteobacteria;c_Alphaproteobacteria |
| 3.35E-05 | 0.000331447 | 8.94E-05 | k_Bacteria;p_Proteobacteria;c_Betaproteobacteria |
| 0 | 0 | 0 | k_Bacteria;p_Proteobacteria;c_Deltaproteobacteria |
| 0.001977808 | 0 | 0.000253395 | k_Bacteria;p_Proteobacteria;c_Epsilonproteobacteria |
| 0 | 3.68E-05 | 0 | k_Bacteria;p_Proteobacteria;c_Gammaproteobacteria |
| 0 | 0.001264409 | 0.000223584 | k_Bacteria;p_Synergistetes;c_Synergistia |
| 3.35E-05 | 0.000184137 | 5.96E-05 | k_Bacteria;p_TM7;c_TM7-3 |
| 0.00184372 | 0.003707296 | 0.00390526 | k_Bacteria;p_Tenericutes;c_Mollicutes |
| 0.000703966 | 1.23E-05 | 0 | k_Bacteria;p_Tenericutes;c_RF3 |
| 0 | 0 | 0 | k_Bacteria;p_Verrucomicrobia;c_Verrucomicrobiae |
| 0.711541685 | 0.613422374 | 0.719253529 | k_Bacteria;p_Bacteroidetes;c_Bacteroidia;o_Bacteroidales |
| 0 | 0 | 0 | k_Bacteria;p_Bacteroidetes;c_Flavobacteriia;o_Flavobacteriales |
| 6.70E-05 | 3.68E-05 | 4.47E-05 | k_Bacteria;p_Bacteroidetes;c_Sphingobacteriia;o_Sphingobacteriales |

(continued)

| two weeks after flight | one day after flight | one day before flight | Taxon |
|---|---|---|---|
| 0.004089705 | 0.005229496 | 0.004084127 | k_Bacteria;p_Cyanobacteria;c_4C0d-2;o_YS2 |
| 0 | 0 | 1.49E-05 | k_Bacteria;p_Cyanobacteria;c_Chloroplast;o_Streptophyta |
| 0 | 0 | 0 | k_Bacteria;p_Cyanobacteria;c_Nostocophycideae;o_Nostocales |
| 0 | 0 | 0 | k_Bacteria;p_Deferribacteres;c_Deferribacteres;o_Deferribacterales |
| 3.35E-05 | 2.46E-05 | 1.49E-05 | k_Bacteria;p_Firmicutes;c_Bacilli;o_Bacillales |
| 0 | 1.23E-05 | 0 | k_Bacteria;p_Firmicutes;c_Bacilli;o_Gemellales |
| 0.019074118 | 0.047482845 | 0.028067194 | k_Bacteria;p_Firmicutes;c_Bacilli;o_Lactobacillales |
| 0.260065033 | 0.325456353 | 0.242781976 | k_Bacteria;p_Firmicutes;c_Clostridia;o_Clostridiales |
| 0 | 0.001215305 | 0.000253395 | k_Bacteria;p_Firmicutes;c_Clostridia;o_SHA-98 |
| 0 | 2.46E-05 | 1.49E-05 | k_Bacteria;p_Firmicutes;c_Erysipelotrichi;o_Erysipelotrichales |
| 0 | 0 | 0 | k_Bacteria;p_Fusobacteria;c_Fusobacteriia;o_Fusobacteriales |
| 0.000100567 | 0 | 1.49E-05 | k_Bacteria;p_Lentisphaerae;c_[Lentisphaeria];o_Victiv allales |
| 3.35E-05 | 0 | 0 | k_Bacteria;p_Proteobacteria;c_Alphaproteobacteria;o_ |
| 0.000100567 | 0.000871583 | 0.000506789 | k_Bacteria;p_Proteobacteria;c_Alphaproteobacteria;o_Caulobacterales |
| 0.0003017 | 0.000405102 | 0.000417356 | k_Bacteria;p_Proteobacteria;c_Alphaproteobacteria;o_RF32 |
| 0 | 0.00014731 | 0 | k_Bacteria;p_Proteobacteria;c_Alphaproteobacteria;o_Rhizobiales |
| 0 | 0 | 0 | k_Bacteria;p_Proteobacteria;c_Alphaproteobacteria;o_Rhodobacterales |
| 0 | 2.46E-05 | 0 | k_Bacteria;p_Proteobacteria;c_Alphaproteobacteria;o_Rickettsiales |
| 0 | 0.000110482 | 0 | k_Bacteria;p_Proteobacteria;c_Alphaproteobacteria;o_Sphingomonadales |
| 3.35E-05 | 0.000331447 | 8.94E-05 | k_Bacteria;p_Proteobacteria;c_Betaproteobacteria;o_Burkholderiales |
| 0 | 0 | 0 | k_Bacteria;p_Proteobacteria;c_Betaproteobacteria;o_Neisseriales |
| 0 | 0 | 0 | k_Bacteria;p_Proteobacteria;c_Deltaproteobacteria;o_Desulfovibrionales |
| 0.001977808 | 0 | 0.000253395 | k_Bacteria;p_Proteobacteria;c_Epsilonproteobacteria;o_Campylobacterales |
| 0 | 2.46E-05 | 0 | k_Bacteria;p_Proteobacteria;c_Gammaproteobacteria;o_Enterobacteriales |
| 0 | 0 | 0 | k_Bacteria;p_Proteobacteria;c_Gammaproteobacteria;o_Legionellales |
| 0 | 0 | 0 | k_Bacteria;p_Proteobacteria;c_Gammaproteobacteria;o_Pasteurellales |
| 0 | 1.23E-05 | 0 | k_Bacteria;p_Proteobacteria;c_Gammaproteobacteria;o_Pseudomonadales |

(continued)

| two weeks after flight | one day after flight | one day before flight | Taxon |
|---:|---:|---:|---|
| 0 | 0.001264409 | 0.000223584 | k_Bacteria;p_Synergistetes;c_Synergistia;o_Synergistales |
| 3.35E-05 | 0.000171861 | 5.96E-05 | k_Bacteria;p_TM7;c_TM7-3;o_ |
| 0 | 1.23E-05 | 0 | k_Bacteria;p_TM7;c_TM7-3;o_CW040 |
| 0.000201133 | 0.000724273 | 0.000298111 | k_Bacteria;p_Tenericutes;c_Mollicutes;o_Anaeroplasmatales |
| 0.001642587 | 0.002983023 | 0.003607149 | k_Bacteria;p_Tenericutes;c_Mollicutes;o_RF39 |
| 0.000703966 | 1.23E-05 | 0 | k_Bacteria;p_Tenericutes;c_RF3;o_ML615J-28 |
| 0 | 0 | 0 | k_Bacteria;p_Verrucomicrobia;c_Verrucomicrobiae;o_Verrucomicrobiales |
| 0 | 0 | 0 | k_Bacteria;p_Bacteroidetes;c_Bacteroidia;o_Bacteroid ales;f_ |
| 0.527706078 | 0.51508 0836 | 0.598995 364 | k_Bacteria;p_Bacteroidetes;c_Bacteroidia;o_Bacteroidales;f_Bacteroidaceae |
| 0.039120378 | 0.05436 9576 | 0.078194 637 | k_Bacteria;p_Bacteroidetes;c_Bacteroidia;o_Bacteroidales;f_Porphyromonadaceae |
| 0.058362107 | 0.00108 0272 | 0.001475 652 | k_Bacteria;p_Bacteroidetes;c_Bacteroidia;o_Bacteroidales;f_Prevotellaceae |
| 0.075357849 | 0.02906 9125 | 0.026338 148 | k_Bacteria;p_Bacteroidetes;c_Bacteroidia;o_Bacteroidales;f_Rikenellaceae |
| 0.0003017 | 0.00033 1447 | 0.000387 545 | k_Bacteria;p_Bacteroidetes;c_Bacteroidia;o_Bacteroidales;f_S24-7_ |
| 0.003955617 | 0.00596 6045 | 0.006811 847 | k_Bacteria;p_Bacteroidetes;c_Bacteroidia;o_Bacteroidales;f_[Bamesiellaceae] |
| 0.006436258 | 0.00748 8246 | 0.007050 336 | k_Bacteria;p_Bacteroidetes;c_Bacteroidia;o_Bacteroidales;f_[Odoribacteraccae] |
| 0.0003017 | 3.68E-05 | 0 | k_Bacteria;p_Bacteroidetes;c_Bacteroidia;o_Bacteroidales;f_[Paraprevotellaceae] |
| 0 | 0 | 0 | k_Bacteria;p_Bacteroidetes;c_Flavobacteriia;o_Flavobacteriales;f_Flavobacteriaceae |
| 0 | 0 | 0 | k_Bacteria;p_Bacteroidetes;c_Flavobacteriia;o_Flavobacteriales;f_[Weeksellaceae]_ |
| 6.70E-05 | 3.68E-05 | 4.47E-05 | k_Bacteria;p_Bacteroidetes;c_Sphingobacteriia;o_Sphingobacteriales;f_Sphingobacteriaceae |
| 0.004089705 | 0.00522 9496 | 0.004084 127 | k_Bacteria;p_Cyanobacteria;c_4C0d-2;o_YS2;f_ |
| 0 | 0 | 1.49E-05 | k_Bacteria;p_Cyanobacteria;c_Chloroplast;o_Streptophyta;f_ |
| 0 | 0 | 0 | k_Bacteria;p_Cyanobacteria;c_Nostocophycideae;o_Nostocales;f_Nostocaceae |
| 0 | 0 | 0 | k_Bacteria;p_Deferribacteres;c_Deferribacteres;o_Deferribacterales;f_Deferribacteraceae |
| 3.35E-05 | 1.23E-05 | 1.49E-05 | k_Bacteria;p_Firmicutes;c_Bacilli;o_Bacillales;f_Bacillaceae |
| 0 | 1.23E-05 | 0 | k_Bacteria;p_Firmicutes;c_Bacilli;o_Bacillales;f_Paenibacillaceae |
| 0 | 0 | 0 | k_Bacteria;p_Firmicutes;c_Bacilli;o_Bacillales;f_Staphylococcaceae |

(continued)

| two weeks after flight | one day after flight | one day before flight | Taxon |
|---|---|---|---|
| 0 | 1.23E-05 | 0 | k_Bacteria;p_Firmicutes;c_Bacilli;o_Gemellales;f_Gemellaceae |
| 0 | 0 | 2.98E-05 | k_Bacteria;p_Firmicutes;c_Bacilli;o_Lactobacillales;f_ |
| 0 | 0 | 0 | k_Bacteria;p_Firmicutes;c_Bacilli;o_Lactobacillales;f_Aerococcaceae |
| 0 | 0 | 0 | k_Bacteria;p_Firmicutes;c_Bacilli;o_Lactobacillales;f_Enterococcaceae |
| 0.018169019 | 0.02603 6999 | 0.023342 128 | k_Bacteria;p_Firmicutes;c_Bacilli;o_Lactobacillales;f_Lactobacillaceae |
| 0 | 8.59E-05 | 2.98E-05 | k_Bacteria;p_Firmicutes;c_Bacilli;o_Lactobacillales;f_Leuconostocaceae |
| 0.000905099 | 0.02135 9915 | 0.004665 444 | k_Bacteria;p_Firmicutes;c_Bacilli;o_Lactobacillales;f_Streptococcaceae |
| 0.02913077 | 0.090755085 | 0.053660063 | k_Bacteria;p_Firmicutes;c_Clostridia;o_Clostridiales;f_ |
| 0.000100567 | 0.000257792 | 0.000119245 | k_Bacteria;p_Firmicutes;c_Clostridia;o_Clostridiales;f_Christensenellaceae |
| 0.048540109 | 0.014252219 | 0.01067239 | k_Bacteria;p_Firmicutes;c_Clostridia;o_Clostridiales;f_Clostridiaceae |
| 0.000201133 | 0.000319171 | 0.000313017 | k_Bacteria;p_Firmicutes;c_Clostridia;o_Clostridiales;f_Dehalobacteriaceae |
| 0 | 4.91E-05 | 0 | k_Bacteria;p_Firmicutes;c_Clostridia;o_Clostridiales;f_Eubacteriaceae |
| 0.022493379 | 0.06802028 | 0.051200644 | k_Bacteria;p_Firmicutes;c_Clostridia;o_Clostridiales;f_Lachnospiraceae |
| 0.000871577 | 8.59E-05 | 2.98E-05 | k_Bacteria;p_Firmicutes;c_Clostridia;o_Clostridiales;f_Peptococcaceae |
| 0.00077101 | 0.004063294 | 0.001878102 | k_Bacteria;p_Firmicutes;c_Clostridia;o_Clostridiales;f_Peptostreptococcaceae |
| 0.137809661 | 0.114742024 | 0.095723591 | k_Bacteria;p_Firmicutes;c_Clostridia;o_Clostridiales;f_Ruminococcaceae |
| 0.019979216 | 0.017628067 | 0.020286485 | k_Bacteria;p_Firmicutes;c_Clostridia;o_Clostridiales;f_Veillonellaceae |
| 0.000167611 | 0.015283387 | 0.008868816 | k_Bacteria;p_Firmicutes;c_Clostridia;o_Clostridiales;f_[Mogibacteriaceae] |
| 0 | 0 | 2.98E-05 | k_Bacteria;p_Firmicutes;c_Clostridia;o_Clostridiales;f_[Tissierellaceae] |
| 0 | 0.001215305 | 0.000253395 | k_Bacteria;p_Firmicutes;c_Clostridia;o_SHA-98;f |
| 0 | 2.46E-05 | 1.49E-05 | k_Bacteria;p_Firmicutes;c_Erysipelotrichi;o_Erysipelo trichales;f_Erysipelotrichaceae |
| 0 | 0 | 0 | k_Bacteria;p_Fusobacteria;c_Fusobacteriia;o_Fusobact eriales;f_Fusobacteriaceae |
| 0.000100567 | 0 | 1.49E-05 | k_Bacteria;p_Lentisphaerae;c_[Lentisphaeria];o_Victivallales;f_Victivallaceae |
| 3.35E-05 | 0 | 0 | k_Bacteria;p_Proteobacteria;c_Alphaproteobacteria;o_; f_ |

(continued)

| two weeks after flight | one day after flight | one day before flight | Taxon |
|---|---|---|---|
| 0.000100567 | 0.000871583 | 0.000506789 | k_Bacteria;p_Proteobacteria;c_Alphaproteobacteria;o_Caulobacterales;f_Caulobacteraceae |
| 0.0003017 | 0.000405102 | 0.000417356 | k_Bacteria;p_Proteobacteria;c_Alphaproteobacteria;o_RF32;f_ |
| 0 | 2.46E-05 | 0 | k_Bacteria;p_Proteobacteria;c_Alphaproteobacteria;o_Rhizobiales;f_Hyphomicrobiaceae |
| 0 | 1.23E-05 | 0 | k_Bacteria;p_Proteobacteria;c_Alphaproteobacteria;o_Rhizobiales;f_Phyllobacteriaceae |
| 0 | 0.000110482 | 0 | k_Bacteria;p_Proteobacteria;c_Alphaproteobacteria;o_Rhizobiales;f_Rhizobiaceae |
| 0 | 0 | 0 | k_Bacteria;p_Proteobacteria;c_Alphaproteobacteria;o_Rhizobiales;f_Xanthobacteraceae |
| 0 | 0 | 0 | k_Bacteria;p_Proteobacteria;c_Alphaproteobacteria;o_Rhodobacterales;f_Rhodobacteraceae |
| 0 | 2.46E-05 | 0 | k_Bacteria;p_Proteobacteria;c_Alphaproteobacteria;o_Rickettsiales;f_ |
| 0 | 3.68E-05 | 0 | k_Bacteria;p_Proteobacteria;c_Alphaproteobacteria;o_Sphingomonadales;f_ |
| 0 | 0 | 0 | k_Bacteria;p_Proteobacteria;c_Alphaproteobacteria;o_Sphingomonadales;f_Erythrobacteraceae |
| 0 | 7.37E-05 | 0 | k_Bacteria;p_Proteobacteria;c_Alphaproteobacteria;o_Sphingomonadales;f_Sphingomonadaceae |
| 0 | 0 | 0 | k_Bacteria;p_Proteobacteria;c_etaproteobacteria;o_Burkholderiales;f_ |
| 0 | 0 | 0 | k_Bacteria;p_Proteobacteria;c_Betaproteobacteria;o_Burkholderiales;f_Alcaligenaceae |
| 3.35E-05 | 0.000331447 | 8.94E-05 | k_Bacteria;p_Proteobacteria;c_Betaproteobacteria;o_Burkholderiales;f_Comamonadaceae |
| 0 | 0 | 0 | k_Bacteria;p_Proteobacteria;c_Betaproteobacteria;o_Burkholderiales;f_Oxalobacteraceae |
| 0 | 0 | 0 | k_Bacteria;p_Proteobacteria;c_Betaproteobacteria;o_Neisseriales;f_Neisseriaceae |
| 0 | 0 | 0 | k_Bacteria;p_Proteobacteria;c_Deltaproteobacteria;o_Desulfovibrionales;f_Desulfovibrionaceae |
| 0.001944286 | 0 | 0.000253395 | k_Bacteria;p_Proteobacteria;c_Epsilonproteobacteria;o__Campylobacterales;f_Campylobacteraceae |
| 3.35E-05 | 0 | 0 | k_Bacteria;p_Proteobacteria;c_Epsilonproteobacteria;o_Campylobacterales;f_Helicobacteraceae |
| 0 | 2.46E-05 | 0 | k_Bacteria;p_Proteobacteria;c_Gammaproteobacteria;o_Enterobacteriales;f_Enterobacteriaceae |
| 0 | 0 | 0 | k_Bacteria;p_Proteobacteria;c_Gammaproteobacteria;o_Legionellales;f_Legionellaceae |
| 0 | 0 | 0 | k_Bacteria;p_Proteobacteria;c_Gammaproteobacteria;o_Pasteurellales;f_Pasteurellaceae |

(continued)

| two weeks after flight | one day after flight | one day before flight | Taxon |
|---|---|---|---|
| 0 | 1.23E-05 | 0 | k_Bacteria;p_Proteobacteria;c_Gammaproteobacteria;o_ Pseudomonadales;f_Pseudomonadaceae |
| 0 | 0.001264409 | 0.000223584 | k_Bacteria;p_Synergistetes;c_Synergistia;o_Synergista les;f_ Synergistaceae |
| 3.35E-05 | 0.000171861 | 5.96E-05 | k_Bacteria;p_TM7;c_TM7-3;o_;f_ |
| 0 | 1.23E-05 | 0 | k_Bacteria;p_TM7;c_TM7-3;o_CW040;f_ |
| 0.000201133 | 0.000724273 | 0.000298111 | kBacteria;p_Tenericutes;c_Mollicutes;o_Anaeroplasmatales;f_ Anaeroplasmataceae |
| 0.001642587 | 0.002983023 | 0.003607149 | k_Bacteria;p_Tenericutes;c_Mollicutes;o_RF39;f |
| 0.000703966 | 1.23E-05 | 0 | k_Bacteria;p_Tenericutes;c_RF3;o_ML615J-28;f_ |
| 0 | 0 | 0 | k_Bacteria;p_Verrucomicrobia;c_Verrucomicrobiae;o_ Verrucomicrobiales;f_Verrucomicrobiaceae |
| 0 | 0 | 0 | k_Bacteria;p_Bacteroidetes;c_Bacteroidia;o_Bacteroidales;f_;g |
| 0.527706078 | 0.515080836 | 0.598995364 | k_Bacteria;p_Bacteroidetes;c_Bacteroidia;o_Bacteroidales;f_ Bacteroidaceae;g_Bacteroides |
| 0.039120378 | 0.054369576 | 0.078194637 | k_Bacteria;p_Bacteroidetes;c_Bacteroidia;o_Bacteroidales;f_ Porphyromonadaceae;g_Parabacteroides |
| 0.058362107 | 0.001080272 | 0.001475652 | k_Bacteria;p_Bacteroidetes;c_Bacteroidia;o_Bacteroid ales;f_ Prevotellaceae;g_Prevotella |
| 0.074720928 | 0.028418507 | 0.025712114 | k_Bacteria;p_Bacteroidetes;c_Bacteroidia;o_Bacteroidales;f_ Rikenellaceae;g_ |
| 0.000636921 | 0.000650618 | 0.000626034 | k_Bacteria;p_Bacteroidetes;c_Bacteroidia;o_Bacteroidales;f_ Rikenellaceae;g_AF12 |
| 0.0003017 | 0.000331447 | 0.000387545 | k_Bacteria;p_Bacteroidetes;c_Bacteroidia;o_Bacteroidales;f_ S24-7;g__ |
| 0.003955617 | 0.005966045 | 0.006811847 | k_Bacteria;p_Bacteroidetes;c_Bacteroidia;o_Bacteroidales;f_ [Barnesiellaceae];g_ |
| 0.000167611 | 0 | 0 | k_Bacteria;p_Bacteroidetes;c_Bacteroidia;o_Bacteroidales;f_ [Odoribacteraceae];g_Butyricimonas |
| 0.006268647 | 0.007488246 | 0.007050336 | k_Bacteria;p_Bacteroidetes;c_Bacteroidia;o_Bacteroidales;f_ [Odoribacteraceae];g_Odoribacter |
| 0.0003017 | 3.68E-05 | 0 | k_Bacteria;p_Bacteroidetes;c_Bacteroidia;o_Bacteroidales;f_ [Paraprevotellaceael;g_Paraprevotella |
| 0 | 0 | 0 | k_Bacteria;p_Bacteroidetes;c_Flavobacteriia;o_ Flavobacteriales;f_Flavobacteriaceae;g_Flavobacterium |
| 0 | 0 | 0 | k_Bacteria;p_Bacteroidetes;c_Flavobacteriia;o_ Flavobacteriales;f_[Weeksellaceae];g_Chryseobacterium |
| 0 | 0 | 0 | k_Bacteria;p_Bacteroidetes;c_Flavobacteriia;o_ Flavobacteriales;f_[Weeksellaceae];g_Cloacibacterium |
| 0 | 0 | 0 | k_Bacteria;p_Bacteroidetes;c_Sphingobacteriia;o_ Sphingobacteriales;f_Sphingobacteriaceae;g_Pedobacter |

(continued)

| two weeks after flight | one day after flight | one day before flight | Taxon |
|---|---|---|---|
| 6.70E-05 | 3.68E-05 | 4.47E-05 | k_Bacteria;p_Bacteroidetes;c_Sphingobacteriia;o_Sphingobacteriales;f_Sphingobacteriaceae;g_Sphingobacterium |
| 0.004089705 | 0.005229496 | 0.004084127 | k_Bacteria;p_Cyanobacteria;c_4C0d-2;o_YS2;f_;g_ |
| 0 | 0 | 1.49E-05 | k_Bacteria;p_Cyanobacteria;c_Chloroplast;o_Streptophyta;f_;g_ |
| 0 | 0 | 0 | k_Bacteria;p_Cyanobacteria;c_Nostocophycideae;o_Nostocales;f_Nostocaceae;g_Tolypothrix |
| 0 | 0 | 0 | k_Bacteria;p_Deferribacteres;c_Deferribacteres;o_Deferribacterales;f_Deferribacteraceae;g_Mucispirillum |
| 3.35E-05 | 1.23E-05 | 1.49E-05 | k_Bacteria;p_Firmicutes;c_Bacilli;o_Bacillales;f_Bacillaceae;g_Bacillus |
| 0 | 1.23E-05 | 0 | k_Bacteria;p_Firmicutes;c_Bacilli;o_Bacillales;f_Paenibacillaceae;g_Paenibacillus |
| 0 | 0 | 0 | k_Bacteria;p_Firmicutes;c_Bacilli;o_Bacillales;f_Staphylococcaceae;g_Staphylococcus |
| 0 | 1.23E-05 | 0 | k_Bacteria;p_Firmicutes;c_Bacilli;o_Gemellales;f_Gemellaceae;g_ |
| 0 | 0 | 0 | k_Bacteria;p_Firmicutes;c_Bacilli;o_Gemellales;f_Gemellaceae;g_Gemella |
| 0 | 0 | 2.98E-05 | k_Bacteria;p_Firmicutes;c_Bacilli;o_Lactobacillales;f_;g_ |
| 0 | 0 | 0 | k_Bacteria;p_Firmicutes;c_Bacilli;o_Lactobacillales;f_Aerococcaceae;g_Aerococcus |
| 0 | 0 | 0 | k_Bacteria;p_Firmicutes;c_Bacilli;o_Lactobacillales;f_Enterococcaceae;g_Enterococcus |
| 0 | 0 | 0 | k_Bacteria;p_Firmicutes;c_Bacilli;o_Lactobacillales;f__Lactobacillaceae;g_ |
| 0.018169019 | 0.026036999 | 0.023342128 | k_Bacteria;p_Firmicutes;c_Bacilli;o_Lactobacillales;f__Lactobacillaceae;g_Lactobacillus |
| 0 | 0 | 0 | k_Bacteria;p_Firmicutes;c_Bacilli;o_Lactobacillales;f_Leuconostocaceae;g_ |
| 0 | 8.59E-05 | 2.98E-05 | k_Bacteria;p_Firmicutes;c_Bacilli;o_Lactobacillales;f_Leuconostocaceae;g_Leuconostoc |
| 0 | 0 | 0 | k_Bacteria;p_Firmicutes;c_Bacilli;o_Lactobacillales;f_Leuconostocaceae;g_Weissella |
| 6.70E-05 | 0.000785652 | 0.000581317 | k_Bacteria;p_Firmicutes;c_Bacilli;o_Lactobacillales;f_Streptococcaceae;g_Lactococcus |
| 0.000838054 | 0.020574263 | 0.004084127 | k_Bacteria;p_Firmicutes;c_Bacilli;o_Lactobacillales;f_Streptococcaceae;g_Streptococcus |
| 0.02913077 | 0.090755085 | 0.053660063 | k_Bacteria;p_Firmicutes;c_Clostridia;o_Clostridiales;f_;g_ |
| 0.000100567 | 0.000159586 | 8.94E-05 | k_Bacteria;p_Firmicutes;c_Clostridia;o_Clostridiales;f_Christensenellaceae;g_ |

(continued)

| two weeks after flight | one day after flight | one day before flight | Taxon |
|---|---|---|---|
| 0 | 9.82E-05 | 2.98E-05 | k_Bacteria;p_Firmicutes;c_Clostridia;o_Clostridiales;f_ Christensenellaceae;g_Christensenella |
| 0.0391539 | 0.005401358 | 0.002668098 | k_Bacteria;p_Firmicutes;c_Clostridia;o_Clostridiales;f_ Clostridiaceae;g_ |
| 6.70E-05 | 0.000208689 | 0.0002683 | k_Bacteria;p_Firmicutes;c_Clostridia;o_Clostridiales;f_ Clostridiaceae;g_Candidatus Arthromitus |
| 0.007978278 | 0.004001915 | 0.004441861 | k_Bacteria;p_Firmicutes;c_Clostridia;o_Clostridiales;f_ Clostridiaceae;g_Clostridium |
| 0.001340887 | 0.004640257 | 0.003294132 | k_Bacteria;p_Firmicutes;c_Clostridia;o_Clostridiales;f_ Clostridiaceae;g_SMB53 |
| 0.000201133 | 0.000319171 | 0.000313017 | k_Bacteria;p_Firmicutes;c_Clostridia;o_Clostridiales;f_ Dehalobacteriaceae;g_Dehalobacterium |
| 0 | 4.91E-05 | 0 | k_Bacteria;p_Firmicutes;c_Clostridia;o_Clostridiales;f_ Eubacteriaceae;g_Pseudoramibacter_Eubacterium |
| 0.006268647 | 0.032285388 | 0.028618701 | k_Bacteria;p_Firmicutes;c_Clostridia;o_Clostridiales; f701_Lachnospiraceae;g_ |
| 0.000905099 | 0.004578878 | 0.000953957 | k_Bacteria;p_Firmicutes;c_Clostridia;o_Clostridiales;f_ Lachnospiraceae;g_Anaerostipes |
| 0.004123228 | 0.00128896 | 0.000536601 | k_Bacteria;p_Firmicutes;c_Clostridia;o_Clostridiales;f_ Lachnospiraceae;g_Blautia |
| 0.000167611 | 0.000159586 | 0.000298111 | k_Bacteria;p_Firmicutes;c_Clostridia;o_Clostridiales;f_ Lachnospiraceae;g_Butyrivibrio |
| 0.002346552 | 0.018487374 | 0.017871782 | k_Bacteria;p_Firmicutes;c_Clostridia;o_Clostridiales;f_ Lachnospiraceae;g_Coprococcus |
| 0.000201133 | 0.000441929 | 0.000104339 | k_Bacteria;p_Firmicutes;c_Clostridia;o_Clostridiales;f_ Lachnospiraceae;g_Dorea |
| 6.70E-05 | 4.91E-05 | 7.45E-05 | k_Bacteria;p_Firmicutes;c_Clostridia;o_Clostridiales;f_ Lachnospiraceae; g_Lachnobacterium |
| 0.00046931 | 0.009759271 | 0.002280553 | k_Bacteria;p_Firmicutes;c_Clostridia;o_Clostridiales;f_ Lachnospiraceae;g_Lachnospira |
| 0 | 0 | 0 | k_Bacteria;p_Firmicutes;c_Clostridia;o_Clostridiales;f_ Lachnospiraceae;_g_Oribacterium |
| 0.007106701 | 0.000110482 | 0.000104339 | k_Bacteria;p_Firmicutes;c_Clostridia;o_Clostridiales;f_ Lachnospiraceae;g_Roseburia |
| 0.000838054 | 0.000859307 | 0.000357734 | k_Bacteria;p_Firmicutes;c_Clostridia;o_Clostridiales;f_ Lachnospiraceae;g_[Ruminococcus] |
| 0.000804532 | 0 | 0 | k_Bacteria;p_Firmicutes;c_Clostridia;o_Clostridiales;f_ Peptococcaceae;g_ |
| 0 | 0 | 0 | k_Bacteria;p_Firmicutes;c_Clostridia;o_Clostridiales; fPeptococcaceae;g_Desulfotomaculum |
| 6.70E-05 | 8.59E-05 | 2.98E-05 | k_Bacteria;p_Firmicutes;c_Clostridia;o_Clostridiales;f_ Peptococcaceae;g_rc4-4 |
| 0.00077101 | 0.004063294 | 0.001878102 | k_Bacteria;p_Firmicutes;c_Clostridia;o_Clostridiales;f_ Peptostreptococcaceae;g_ |

(continued)

| two weeks after flight | one day after flight | one day before flight | Taxon |
|---|---|---|---|
| 0 | 0 | 0 | k_Bacteria;p_Firmicutes;c__Clostridia;o_Clostridiales;f_Peptostreptococcaceae;g_Filifactor |
| 0.121517884 | 0.105473785 | 0.085095917 | k_Bacteria;p_Firmicutes;c_Clostridia;o_Clostridiales;f_Ruminococcaceae;g_ |
| 3.35E-05 | 0 | 2.98E-05 | k_Bacteria;p_Firmicutes;c_Clostridia;o_Clostridiales;f_Ruminococcaceae;g_Faecalibacterium |
| 0.011799806 | 0.00023324 | 0.000417356 | k_Bacteria;p_Firmicutes;c_Clostridia;o_Clostridiales;f_Ruminococcaceae;g_Oscillospira |
| 0.004458449 | 0.009034998 | 0.010180506 | k_Bacteria;p_Firmicutes;c_Clostridia;o_Clostridiales;f_Ruminococcaceae;g_Ruminococcus |
| 0 | 0 | 0 | k_Bacteria;p_Firmicutes;c_Clostridia;o_Clostridiales;f_Veillonellaceae;g_ |
| 0.013643525 | 0.00929279 | 0.011894647 | k_Bacteria;p_Firmicutes;c_Clostridia;o_Clostridiales;f_Veillonellaceae;g_Dialister |
| 0.005966947 | 2.46E-05 | 0 | k_Bacteria;p_Firmicutes;c_Clostridia;o_Clostridiales;f_Veillonellaceae;g_Megamonas |
| 0 | 0 | 0 | k_Bacteria;p_Firmicutes;c_Clostridia;o_Clostridiales;f_Veillonellaceae;g_Megasphaera |
| 0.000100567 | 0.007611004 | 0.007721087 | k_Bacteria;p_Firmicutes;c_Clostridia;o_Clostridiales;f_Veillonellaceae;g_Phascolarctobacterium |
| 0.000268177 | 0.000699721 | 0.000670751 | k_Bacteria;p_Firmicutes;c_Clostridia;o_Clostridiales;f_Veillonellaceae;g_Veillonella |
| 0.000167611 | 0.015283387 | 0.008868816 | k_Bacteria;p_Firmicutes;c_Clostridia;o_Clostridiales;f_[Mogibacteriaceae];g_ |
| 0 | 0 | 1.49E-05 | k_Bacteria;p_Firmicutes;c_Clostridia;o_Clostridiales;f_[Tissierellaceae];g_Anaerococcus |
| 0 | 0 | 1.49E-05 | k_Bacteria;p_Firmicutes;c_Clostridia;o_Clostridiales;f_[Tissierellaceae];g_Finegoldia |
| 0 | 0 | 0 | k_Bacteria;p_Firmicutes;c_Clostridia;o_Clostridiales;f_[Tissierellaceae];g_Parvimonas |
| 0 | 0.001215305 | 0.000253395 | k_Bacteria;p_Firmicutes;c_Clostridia;o_SHA-98;f_;g_ |
| 0 | 0 | 0 | k_Bacteria;p_Firmicutes;c_Erysipelotrichi;o_Erysipelo trichales;f_Erysipelotrichaceae;g_ |
| 0 | 2.46E-05 | 1.49E-05 | k_Bacteria;p_Firmicutes;c_Erysipelotrichi;o_Erysipelo trichales;f_Erysipelotrichaceae;g_Bulleidia |
| 0 | 0 | 0 | k_Bacteria;p_Firmicutes;c_Erysipelotrichi;o_Erysipelotrichales;f_Erysipelotrichaceae;g_Coprobacillus |
| 0 | 0 | 0 | k_Bacteria;p_Fusobacteria;c_Fusobacteriia;o_Fusobacteriales;f_Fusobacteriaceae;g_Fusobacterium |
| 0.000100567 | 0 | 1.49E-05 | k_Bacteria;p_Lentisphaerae;c_[Lentisphaeria];o_Victiv allales;f_Victivallaceae;g_Victivallis |
| 3.35E-05 | 0 | 0 | k_Bacteria;p_Proteobacteria;c_Alphaproteobacteria;o_;f_;g_ |

(continued)

| two weeks after flight | one day after flight | one day before flight | Taxon |
|---|---|---|---|
| 0.000100567 | 0.000871583 | 0.000506789 | k_Bacteria;p_Proteobacteria;c_Alphaproteobacteria;o_ Caulobacterales;f_Caulobacteraceae;g_ |
| 0 | 0 | 0 | k_Bacteria;p_Proteobacteria;c_Alphaproteobacteria;o_ Caulobacterales;f_Caulobacteraceae;g_Mycoplana |
| 0.0003017 | 0.000405102 | 0.000417356 | k_Bacteria;p_Proteobacteria;c_Alphaproteobacteria;o_RF32;f_; g_ |
| 0 | 1.23E-05 | 0 | k_Bacteria;p_Proteobacteria;c_Alphaproteobacteria;o_ Rhizobiales;f_Hyphomicrobiaceae;g_Devosia |
| 0 | 1.23E-05 | 0 | k_Bacteria;p_Proteobacteria;c_Alphaproteobacteria;o_ Rhizobiales;f_Hyphomicrobiaceae;g_Hyphomicrobium |
| 0 | 1.23E-05 | 0 | k_Bacteria;p_Proteobacteria;c_Alphaproteobacteria;o_ Rhizobiales;f_Phyllobacteriaceae;g_ |
| 0 | 0.000110482 | 0 | k_Bacteria;p_Proteobacteria;c_Alphaproteobacteria;o_ Rhizobiales;f_Rhizobiaceae;g_Agrobacterium |
| 0 | 0 | 0 | k_Bacteria;p_Proteobacteria;c_Alphaproteobacteria;o_ Rhizobiales;f_Xanthobacteraceae;g_ |
| 0 | 0 | 0 | k_Bacteria;p_Proteobacteria;c_Alphaproteobacteria;o_ Rhodobacterales;f_Rhodobacteraceae;g_ |
| 0 | 2.46E-05 | 0 | k_Bacteria;p_Proteobacteria;c_Alphaproteobacteria;o_ Rickettsiales;f_;g_ |
| 0 | 3.68E-05 | 0 | k_Bacteria;p_Proteobacteria;c_Alphaproteobacteria;o_ Sphingomonadales;f_;g_ |
| 0 | 0 | 0 | k_Bacteria;p_Proteobacteria;c_Alphaproteobacteria;o_ Sphingomonadales;f_Erythrobacteraceae;g_ |
| 0 | 1.23E-05 | 0 | k_Bacteria;p_Proteobacteria;c_Alphaproteobacteria;o_ Sphingomonadales;f_Sphingomonadaceae;g_ |
| 0 | 6.14E-05 | 0 | k_Bacteria;p_Proteobacteria;c_Alphaproteobacteria;o_ Sphingomonadales;f_Sphingomonadaceae;g_Sphingopyxis |
| 0 | 0 | 0 | k_Bacteria;p_Proteobacteria;c_Betaproteobacteria;o_ Burkholderiales;f_;g_ |
| 0 | 0 | 0 | k_Bacteria;p_Proteobacteria;c_Betaproteobacteria;o_ Burkholderiales;f_Alcaligenaceae;g_Sutterella |
| 3.35E-05 | 0.000331447 | 8.94E-05 | k_Bacteria;p_Proteobacteria;c_Betaproteobacteria;o_ Burkholderiales;f_Comamonadaceae;g_Comamonas |
| 0 | 0 | 0 | k_Bacteria;p_Proteobacteria;c_Betaproteobacteria;o_ Burkholderiales;f_Oxalobacteraceae;g_Ralstonia |
| 0 | 0 | 0 | k_Bacteria;p_Proteobacteria;c_Betaproteobacteria;o_ Neisseriales;f_Neisseriaceae;g_Neisseria |
| 0 | 0 | 0 | k_Bacteria;p_Proteobacteria;c_Deltaproteobacteria;o_ Desulfovibrionales;f_Desulfovibrionaceae;g_Desulfovibrio |
| 0.001944286 | 0 | 0.000253395 | k_Bacteria;p_Proteobacteria;c_Epsilonproteobacteria;o_ Campylobacterales;f_Campylobacteraceae;g_Campylobacter |
| 3.35E-05 | 0 | 0 | k_Bacteria;p_Proteobacteria;c_Epsilonproteobacteria;o_ Campylobacterales;f_Helicobacteraceae;g_Helicobacter |

(continued)

| two weeks after flight | one day after flight | one day before flight | Taxon |
|---|---|---|---|
| 0 | 2.46E-05 | 0 | k_Bacteria;p_Proteobacteria;c_Gammaproteobacteria;o_Enterobacteriales;f_Enterobacteriaceae;g_ |
| 0 | 0 | 0 | k_Bacteria;p_Proteobacteria;c_Gammaprotcobacteria;o__Legionellales;f_Legionellaceae;g_Legionella |
| 0 | 0 | 0 | k_Bacteria;p_Proteobacteria;c_Gammaproteobacteria;o_Pasteurellales;f_Pasteurellaceae;g_Haemophilus |
| 0 | 1.23E-05 | 0 | k_Bacteria;p_Proteobacteria;c_Gammaproteobacteria;o_Pseudomonadales;f_Pseudomonadaceae;g_ |
| 0 | 0.001264409 | 0.000223584 | k_Bacteria;p_Synergistetes;c_Synergistia;o_Synergistales;f_Synergistaceae;g_Synergistes |
| 3.35E-05 | 0.000171861 | 5.96E-05 | k_Bacteria;p_TM7;c_TM7-3;o_;f_;g_ |
| 0 | 1.23E-05 | 0 | k_Bacteria;p_TM7;c_TM7-3;o_CW040;f_;g_ |
| 0.000201133 | 0.000724273 | 0.000298111 | k_Bacteria;p_Tenericutes;c_Mollicutes;o_Anaeroplas matales;f_Anaeroplasmataceae;g_Anaeroplasma |
| 0.001642587 | 0.002983023 | 0.003607149 | k_Bacteria;p_Tenericutes;c_Mollicutes;o_RF39;f_;g_ |
| 0.000703966 | 1.23E-05 | 0 | k_Bacteria;p_Tenericutes;c_RF3;o_ML615J-28;f_;g_ |
| 0 | 0 | 0 | k_Bacteria;p_Verrucomicrobia;c_Verrucomicrobiae;o_Verrucomicrobiales;f_Verrucomicrobiaceae;g_Akkermansia |
| 0 | 0 | 0 | k_Bacteria;p_Bacteroidetes;c_Bacteroidia;o_Bacteroidales;f_;g_;s_ |
| 0.271797794 | 0.306428843 | 0.381105695 | k_Bacteria;p_Bacteroidetes;c_Bacteroidia;o_Bacteroidales;f_Bacteroidaceae;g_Bacteroides;s_ |
| 0.047098656 | 0.04802298 | 0.057729285 | k_Bacteria;p_Bacteroidetes;c_Bacteroidia;o_Bacteroidales;f_Bacteroidaceae;g_Bacteroides;s_acidifaciens |
| 0.091850759 | 0.005634598 | 0.006454113 | k_Bacteria;p_Bacteroidetes;c_Bacteroidia;o_Bacteroidales;fBacteroidaceae;g_Bacteroides;s_caccae |
| 0.001709631 | 0.149286161 | 0.145433678 | k_Bacteria;p_Bacteroidetes;c_Bacteroidia;o_Bacteroidales;f_Bacteroidaceae;g_Bacteroides;s_eggerthii |
| 0.11343904 | 0.003842329 | 0.005038084 | k_Bacteria;p_Bacteroidetes;c_Bacteroidia;o_Bacteroidales;f_Bacteroidaceae;g_Bacteroides;s_fragilis |
| 0.000636921 | 0.000626066 | 0.001013579 | k_Bacteria;p_Bacteroidetes;c_Bacteroidia;o_Bacteroidales;f_Bacteroidaceae;g_Bacteroides;s_ovatus |
| 0.001173276 | 0.001239857 | 0.00222093 | k_Bacteria;p_Bacteroidetes;c_Bacteroidia;o_Bacteroidales;f_Bacteroidaceae;g_Bacteroides;s_uniformis |
| 0.033857397 | 0.052024895 | 0.073424854 | k_Bacteria;p_Bacteroidetes;c_Bacteroidia;o_Bacteroidales;f_Porphyromonadaceae;g_Parabacteroides;s_ |
| 0.005262981 | 0.00234468 | 0.004769783 | k_Bacteria;p_Bacteroidetes;c_Bacteroidia;o_Bacteroidales;f_Porphyromonadaceae;g_Parabacteroides;s_distasonis |
| 0.00955382 | 4.91E-05 | 0.000342828 | k_Bacteria;p_Bacteroidetes;c_Bacteroidia;o_Bacteroidales;f_Prevotellaceae;g_Prevotella;s_ |
| 0.045724247 | 0.001018892 | 0.001117918 | k_Bacteria;p_Bacteroidetes;c_Bacteroidia;o_Bacteroidales;f_Prevotellaceae;g_Prevotella;s_copri |

(continued)

| two weeks after flight | one day after flight | one day before flight | Taxon |
|---|---|---|---|
| 0 | 0 | 0 | k_Bacteria;p_Bacteroidetes;c_Bacteroidia;o_Bacteroidales;f_ Prevotellaceae;g_Prevotella;s_melaninogenica |
| 0.00308404 | 1.23E-05 | 1.49E-05 | k_Bacteria;p_Bacteroidetes;c_Bacteroidia;o_Bacteroidales;f_ Prevotellaceae;g_Prevotella;s_stercorea |
| 0.074720928 | 0.028418507 | 0.025712114 | k_Bacteria;p_Bacteroidetes;c_Bacteroidia;o_Bacteroid ales;f_ Rikenellaceae;g_;s_ |
| 0.000636921 | 0.000650618 | 0.000626034 | k_Bacteria;p_Bacteroidetes;c_Bacteroidia;o_Bacteroidales;f_ Rikenellaceae;g_AF12;s_ |
| 0.0003017 | 0.000331447 | 0.000387545 | k_Bacteria;p_Bacteroidetes;c_Bacteroidia;o_Bacteroid ales;f_ S24-7;g_;s_ |
| 0.003955617 | 0.005966045 | 0.006811847 | k_Bacteria;p_Bacteroidetes;c_Bacteroidia;o_Bacteroidales;f_ [Barnesiellaceae];g_;s_ |
| 0.000167611 | 0 | 0 | k_Bacteria;p_Bacteroidetes;c_Bacteroidia;o_Bacteroidales;f_ [Odoribacteraceae];g_Butyricimonas;s_ |
| 0.006268647 | 0.007488246 | 0.007050336 | k_Bacteria;p_Bacteroidetes;c_Bacteroidia;o_Bacteroidales;f_ [Odoribacteraceael;g_Odoribacter;s_ |
| 0.0003017 | 3.68E-05 | 0 | k_Bacteria;p_Bacteroidetes;c_Bacteroidia;o_Bacteroidales;f_ [Paraprevotellaceae];g_Paraprevotella;s_ |
| 0 | 0 | 0 | k_Bacteria;p_Bacteroidetes;c_Flavobacteriia;o_ Flavobacteriales;f_Flavobacteriaceae;g_Flavobacterium;s_ succinicans |
| 0 | 0 | 0 | k_Bacteria;p_Bacteroidetes;c_Flavobacteriia;o_ Flavobacteriales;f_[Weeksellaceae];g_Chryscobacterium;s_ |
| 0 | 0 | 0 | k_Bacteria;p_Bacteroidetes;c_Flavobacteriia;o_ Flavobacteriales;f_[Weeksellaceae];g_Cloacibacterium;s_ |
| 0 | 0 | 0 | k_Bacteria;p_Bacteroidetes;c_Sphingobacteriia;o_ Sphingobacteriales;f_Sphingobacteriaceae;g_Pedobacter;s_ |
| 6.70E-05 | 3.68E-05 | 4.47E-05 | k_Bacteria;p_Bacteroidetes;c_Sphingobacteriia;o_ Sphingobacteriales;f_Sphingobacteriaceae;g_ Sphingobacterium;s_ |
| 0.004089705 | 0.005229496 | 0.004084127 | k_Bacteria;p_Cyanobacteria;c_4C0d-2;o_YS2;f_;g_;s_ |
| 0 | 0 | 1.49E-05 | k_Bacteria;p_Cyanobacteria;c_Chloroplast;o_Streptophyta;f_; g_;s_ |
| 0 | 0 | 0 | k_Bacteria;p_Cyanobacteria;c_Nostocophycideae;o_ Nostocales;f_Nostocaceae;g_Tolypothrix;s_distorta |
| 0 | 0 | 0 | k_Bacteria;p_Deferribacteres;c_Deferribacteres;o_ Deferribacterales;f_Deferribacteraceae;g__Mucispirillum;s_ schaedleri |
| 3.35E-05 | 1.23E-05 | 1.49E-05 | k_Bacteria;p_Firmicutes;c_Bacilli;o_Bacillales;f_Bacillaceae;g_ Bacillus;s_ |
| 0 | 1.23E-05 | 0 | k_Bacteria;p_Firmicutes;c_Bacilli;o_Bacillales;f_ Paenibacillaceae;g_Paenibacillus;s_ |
| 0 | 0 | 0 | k_Bacteria;p_Firmicutes;c_Bacilli;o_Bacillales;f_ Staphylococcaceae;g_Staphylococcus;s_ |

(continued)

| two weeks after flight | one day after flight | one day before flight | Taxon |
|---|---|---|---|
| 0 | 1.23E-05 | 0 | k_Bacteria;p_Firmicutes;c_Bacilli;o_Gemellales;f_Ge mellaceae;g_;s_ |
| 0 | 0 | 0 | k_Bacteria;p_Firmicutes;c_Bacilli;o_Gemellales;f_Ge mellaceae;g_Gemella;s_ |
| 0 | 0 | 2.98E-05 | k_Bacteria;p_Firmicutes;c_Bacilli;o_Lactobacillales;f__;g_;s_ |
| 0 | 0 | 0 | k_Bacteria;p_Firmicutes;c_Bacilli;o_Lactobacillales;f__ Aerococcaceae;g_Aerococcus;s_ |
| 0 | 0 | 0 | k_Bacteria;p_Firmicutes;c_Bacilli;o_Lactobacillales;f__ Enterococcaceae;g_Enterococcus;s_ |
| 0 | 0 | 0 | k_Bacteria;p_Firmicutes;c_Bacilli;o_Lactobacillales;f__ Lactobacillaceae;g_;s_ |
| 0.014414535 | 0.0188802 | 0.018020838 | k_Bacteria;p_Firmicutes;c_Bacilli;o_Lactobacillales;f__ Lactobacillaceae;g_Lactobacillus;s_ |
| 0 | 0 | 0 | k_Bacteria;p_Firmicutes;c_Bacilli;o_Lactobacillales;f_ Lactobacillaceae;g_Lactobacillus;s_delbrueckii |
| 0 | 0 | 0.000193772 | k_Bacteria;p_Firmicutes;c_Bacilli;o_Lactobacillales;f_ Lactobacillaceae;g_Lactobacillus;s_iners |
| 0.003720961 | 0.003339021 | 0.003815827 | k_Bacteria;p_Firmicutes;c_Bacilli;o_Lactobacillales;f_ Lactobacillaceae;g_Lactobacillus;s_reuteri |
| 3.35E-05 | 0.003731847 | 0.001296785 | k_Bacteria;p_Firmicutes;c_Bacilli;o_Lactobacillales;f__ Lactobacillaceae;g_Lactobacillus;s_ruminis |
| 0 | 0 | 0 | k_Bacteria;p_Firmicutes;c_Bacilli;o_Lactobacillales;f_ Lactobacillaceae;g_Lactobacillus;s_vaginalis |
| 0 | 8.59E-05 | 1.49E-05 | k_Bacteria;p_Firmicutes;c_Bacilli;o_Lactobacillales;f_ Lactobacillaceae;g_Lactobacillus;s_zeae |
| 0 | 0 | 0 | k_Bacteria;p_Firmicutes;c_Bacilli;o_Lactobacillales;f_ Leuconostocaceae;g_;s |
| 0 | 0 | 0 | k_Bacteria;p_Firmicutes;c_Bacilli;o_Lactobacillales;f_ Leuconostocaceae;g_Leuconostoc;s_ |
| 0 | 8.59E-05 | 2.98E-05 | k_Bacteria;p_Firmicutes;c_Bacilli;o_Lactobacillales;f_ Leuconostocaceae;g_Leuconostoc;s_mesenteroides |
| 0 | 0 | 0 | k_Bacteria;p_Firmicutes;c_Bacilli;o_Lactobacillales;f__ Leuconostocaceae;g_Weissella;s_cibaria |
| 6.70E-05 | 0.000748825 | 0.000521695 | k_Bacteria;p_Firmicutes;c_Bacilli;o_Lactobacillales;f_ Streptococcaceae;g_Lactococcus;s, |
| 0 | 3.68E-05 | 5.96E-05 | k_Bacteria;p_Firmicutes;c_Bacilli;o_Lactobacillales;f__ Streptococcaceae;g_Lactococcus;s_garvieae |
| 0.000838054 | 0.020574263 | 0.004084127 | k_Bacteria;p_Firmicutes;c_Bacilli;o_Lactobacillales;f__ Streptococcaceae;g_Streptococcus;s_ |
| 0 | 0 | 0 | k_Bacteria;p_Firmicutes;c_Bacilli;o_Lactobacillales;f_ Streptococcaceae;g_Streptococcus;s_sobrinus |
| 0.02913077 | 0.090755085 | 0.053660063 | k_Bacteria;p_Firmicutes;c_Clostridia;o_Clostridiales;f_;g_;s |

(continued)

| two weeks after flight | one day after flight | one day before flight | Taxon |
|---|---|---|---|
| 0.000100567 | 0.000159586 | 8.94E-05 | k_Bacteria;p_Firmicutes;c_Clostridia;o_Clostridiales;f_Christensenellaceae;g_;s_ |
| 0 | 9.82E-05 | 2.98E-05 | k_Bacteria;p_Firmicutes;c_Clostridia;o_Clostridiales;f Christensenellaceae;g_Christensenella;s_ |
| 0.0391539 | 0.005401358 | 0.002668098 | k_Bacteria;p_Firmicutes;c_Clostridia;o_Clostridiales;f_Clostridiaceae;g_;s_ |
| 6.70E-05 | 0.000208689 | 0.0002683 | k_Bacteria;p_Firmicutes;c_Clostridia;o_Clostridiales;f_Clostridiaceae;g_CandidatusArthromitus;s_ |
| 0.007944755 | 0.003793226 | 0.004352427 | k_Bacteria;p_Firmicutes;c_Clostridia;o_Clostridiales;f_Clostridiaceae;g_Clostridium;s_ |
| 3.35E-05 | 0.000208689 | 8.94E-05 | k_Bacteria;p_Firmicutes;c__Clostridia;o_Clostridiales;f_Clostridiaceae;g_Clostridium;s_hiranonis |
| 0.001340887 | 0.004640257 | 0.003294132 | k_Bacteria;p_Firmicutes;c_Clostridia;o_Clostridiales;f_Clostridiaceae;g_SMB53;s_ |
| 0.000201133 | 0.000319171 | 0.000313017 | k_Bacteria;p_Firmicutes;c_Clostridia;o_Clostridiales;fDehalobacteriaceae;g_Dehalobacterium;s_ |
| 0 | 4.91E-05 | 0 | k_Bacteria;p_Firmicutes;c_Clostridia;o_Clostridiales;f_Eubacteriaceae;g_Pseudoramibacter Eubacterium;s |
| 0.006268647 | 0.032285388 | 0.028618701 | k_Bacteria;p,_Firmicutes;c_Clostridia;o_Clostridiales;f_Lachnospiraceae;g_;s_ |
| 0.000905099 | 0.004578878 | 0.000953957 | k_Bacteria;p_Firmicutes;c_Clostridia;o_Clostridiales;f_Lachnospiraceae;g_Anaerostipes;s_ |
| 0.004123228 | 0.00128896 | 0.000536601 | k_Bacteria;p_Firmicutes;c Clostridia;o Clostridiales;f Lachnospiraceae;g Blauria;s_ |
| 0.000167611 | 0.000159586 | 0.000298111 | k_Bacteria;p_Firmicutes;c_Clostridia;o_Clostridiales;f_Lachnospiraceae;g_Butyrivibrio;s_ |
| 0.002346552 | 0.018487374 | 0.017871782 | k_Bacteria;p_Firmicutes;c_Clostridia;o_Clostridiales;f_Lachnospiraceae;g_Coprococcus;s_ |
| 0 | 0 | 0 | k_Bacteria;p_Firmicutes;c_Clostridia;o_Clostridiales;f_Lachnospiraceae;g_Coprococcus;s_eutactus |
| 0.000201133 | 0.000441929 | 0.000104339 | k_Bacteria;p_Firmicutes;c_Clostridia;o_Clostridiales;f_Lachnospiraceae;g_Dorea;s_ |
| 0 | 0 | 0 | k_Bacteria;p_Firmicutes;c_Clostridia;o_Clostridiales;f_Lachnospiraceae;g_Dorea;s_formicigenerans |
| 6.70E-05 | 4.91E-05 | 7.45E-05 | k_Bacteria;p_Firmicutes;c_Clostridia;o_Clostridiales;f_Lachnospiraceae;g_Lachnobacterium;s_ |
| 0.00046931 | 0.009759271 | 0.002280553 | k_Bacteria;p_Firmicutes;c_Clostridia;o_Clostridiales;f_Lachnospiraceae;g_Lachnospira;s_ |
| 0 | 0 | 0 | k_Bacteria;p_Firmicutes;c_Clostridia;o_Clostridiales;f_Lachnospiraceae;g_Oribacterium;s_ |
| 0.007106701 | 0.000110482 | 0.000104339 | k_Bacteria;p_Firmicutes;c_Clostridia;o_Clostridiales;f_Lachnospiraceae;g_Roseburia;s_ |
| 0 | 0 | 0 | k_Bacteria;p_Firmicutes;c_Clostridia;o_Clostridiales;f_Lachnospiraceae;g_Roseburia;s_faecis |

(continued)

| two weeks after flight | one day after flight | one day before flight | Taxon |
|---|---|---|---|
| 0.000636921 | 0.000491033 | 0.000298111 | k_Bacteria;p_Firmicutes;c_Clostridia;o_Clostridiales;f_Lachnospiraceae;g_[Ruminococcus];s_ |
| 0.000201133 | 0.000368274 | 5.96E-05 | k_Bacteria;p_Firmicutes;c_Clostridia;o_Clostridiales;f_Lachnospiraceae;g_[Ruminococcus];s_gnavus |
| 0.000804532 | 0 | 0 | k_Bacteria;p_Firmicutes;c_Clostridia;o_Clostridiales;f_Peptococcaceae;g_;s_ |
| 0 | 0 | 0 | k_Bacteria;p_Firmicutes;c_Clostridia;o_Clostridiales;f_Peptococcaceae;g_Desulfotomaculum;s_ |
| 6.70E-05 | 8.59E-05 | 2.98E-05 | k_Bacteria;p_Firmicutes;c_Clostridia;o_Clostridiales;f_Peptococcaceae;g_rc4-4;s_ |
| 0.00077101 | 0.004063294 | 0.001878102 | k_Bacteria;p_Firmicutes;c_Clostridia;o_Clostridiales;f_Peptostreptococcaceae;g_;s_ |
| 0 | 0 | 0 | k_Bacteria;p_Firmicutes;c_Clostridia;o_Clostridiales;f_Peptostreptococcaceae;g_Filifactor;s_ |
| 0.121517884 | 0.105473785 | 0.085095917 | k_Bacteria;p_Firmicutes;c_Clostridia;o_Clostridiales;f_Ruminococcaceae;g_;s_ |
| 3.35E-05 | 0 | 2.98E-05 | k_Bacteria;p_Firmicutes;c_Clostridia;o_Clostridiales;f_Ruminococcaceae;g_Faecalibacterium;s_prausnitzii |
| 0.011799806 | 0.00023324 | 0.000417356 | k_Bacteria;p_Firmicutes;c_Clostridia;o_Clostridiales;f_Ruminococcaceae;g_Oscillospira;s_ |
| 0.004458449 | 0.007807417 | 0.009017872 | k_Bacteria;p_Firmicutes;c_Clostridia;o_Clostridiales;f_Ruminococcaceae;g_Ruminococcus;s_ |
| 0 | 0 | 2.98E-05 | k_Bacteria;p_Firmicutes;c_Clostridia;o_Clostridiales;f_Ruminococcaceae;g_Ruminococcus;s_callidus |
| 0 | 0.001227581 | 0.001132824 | k_Bacteria;p_Firmicutes;c_Clostridia;o_Clostridiales;fRuminococcaceae;g_Ruminococcus;s_flavefaciens |
| 0 | 0 | 0 | k_Bacteria;p_Firmicutes;c_Clostridia;o_Clostridiales;f_Veillonellaceae;g_;s_ |
| 0.013643525 | 0.00929279 | 0.011894647 | k_Bacteria;p_Firmicutes;c_Clostridia;o_Clostridiales;f_Veillonellaceae;g_Dialister;s |
| 0.005966947 | 2.46E-05 | 0 | k_Bacteria;p_Firmicutes;c_Clostridia;o_Clostridiales;f_Veillonellaceae;g_Megamonas;s_ |
| 0 | 0 | 0 | k_Bacteria;p_Firmicutes;c_Clostridia;o_Clostridiales;f_Veillonellaceae;g_Megasphaera;s_ |
| 0.000100567 | 0.007611004 | 0.007721087 | k_Bacteria;p_Firmicutes;c_Clostridia;o_Clostridiales;f_Veillonellaceae;g_Phascolarctobacteriutn;s_ |
| 0 | 4.91E-05 | 1.49E-05 | k_Bacteria;p_Firmicutes;c_Clostridia;o_Clostridiales;f_Veillonellaceae;g_Veillonella;s |
| 0.000201133 | 0.000515584 | 0.000491884 | k_Bacteria;p_Firmicutes;c_Clostridia;o_Clostridiales;f_Veillonellaceae;g_Veillonella;s_dispar |
| 6.70E-05 | 0.000135034 | 0.000163961 | k_Bacteria;p_Firmicutes;c_Clostridia;o_Clostridiales;f_Veillonellaceae;g_Veillonella;s,_parvula |
| 0.000167611 | 0.015283387 | 0.008868816 | k_Bacteria;p_Firmicutes;c Clostridia;o_Clostridiales;f_[Mogibacteriaceae];g_;s_ |

(continued)

| two weeks after flight | one day after flight | one day before flight | Taxon |
|---|---|---|---|
| 0 | 0 | 1.49E-05 | k_Bacteria;p_Firmicutes;c_Clostridia;o_Clostridiales;f_ [Tissierellaceae];g_Anaerococcus;s_ |
| 0 | 0 | 1.49E-05 | k_Bacteria;p_Firmicutes;c__Clostridia;o_Clostridiales;f_ [Tissierellaceae];g_Finegoldia;s_ |
| 0 | 0 | 0 | k_Bacteria;p_Firmicutes;c_Clostridia;o_Clostridiales;f_ [Tissierellaceae];g_Parvimonas;s_ |
| 0 | 0.001215305 | 0.000253395 | k_Bacteria;p_Firmicutes;c_Clostridia;o_SHA-98;f_;g_;s_ |
| 0 | 0 | 0 | k_Bacteria;p_Firmicutes;c_Erysipelotrichi;o_Erysipelo trichales; f__Erysipelotrichaceae;g_;s_ |
| 0 | 2.46E-05 | 1.49E-05 | k_Bacteria;p_Firmicutes;c_Erysipelotrichi;o_Erysipelotrichales; f_Erysipelotrichaceae;g_Bulleidia;s_moorei |
| 0 | 0 | 0 | k_Bacteria;p_Firmicutes;c_Erysipelotrichi;o_Erysipelotrichales; f_Erysipelotrichaceae;g_Coprobacillus;s_ |
| 0 | 0 | 0 | k_Bacteria;p_Fusobacteria;c_Fusobacteriia;o_Fusobacteriales; f_Fusobacteriaceae;g_Fusobacterium;s_ |
| 0.000100567 | 0 | 1.49E-05 | k_Bacteria;p_Lentisphaerae;c_[Lentisphaeria];o_Victivallales;f_ Victivallaceae;g_Victivallis;s_vadensis |
| 3.35E-05 | 0 | 0 | k_Bacteria;p_Proteobacteria;c_Alphaproteobacteria;o_;f_;g_;s_ |
| 0.000100567 | 0.000871583 | 0.000506789 | k_Bacteria;p_Proteobacteria;c_Alphaproteobacteria;o_ Caulobacterales;f_Caulobacteraceae;g_;s_ |
| 0 | 0 | 0 | k_Bacteria;p_Proteobacteria;c_Alphaproteobacteria;o_ Caulobacterales;f_Caulobacteraceae;g_Mycoplana;s_ |
| 0.0003017 | 0.000405102 | 0.000417356 | k_Bacteria;p_Proteobacteria;c_Alphaproteobacteria;o_RF32;f_; g_;s_ |
| 0 | 1.23E-05 | 0 | k_Bacteria;p_Proteobacteria;c_Alphaproteobacteria;o_ Rhizobiales;f_Hyphomicrobiaceae;g_Devosia;s_ |
| 0 | 1.23E-05 | 0 | k_Bacteria;p_Proteobacteria;c_Alphaproteobacteria;o_ Rhizobiales;f_Hyphomicrobiaceae;g_Hyphomicrobium;s_ |
| 0 | 1.23E-05 | 0 | k_Bacteria;p_Proteobacteria;c_Alphaproteobacteria;o_ Rhizobiales;f_Phyllobacteriaceae;g_;s_ |
| 0 | 0.000110482 | 0 | k_Bacteria;p_Proteobacteria;c_Alphaproteobacteria;o_ Rhizobiales;f_Rhizobiaceae;g_Agrobacteriutn;s_ |
| 0 | 0 | 0 | k_Bacteria;p_Proteobacteria;c_Alphaproteobacteria;o_ Rhizobiales;f_Xanthobacteraccae;g_;s_ |
| 0 | 0 | 0 | k_Bacteria;p_Proteobacteria;c_Alphaproteobacteria;o_ Rhodobacterales;f_Rhodobacteraceae;g_;s_ |
| 0 | 2.46E-05 | 0 | k_Bacteria;p_Proteobacteria;c__Alphaproteobacteria;o_ Rickettsiales;f_;g_;s_ |
| 0 | 3.68E-05 | 0 | k_Bacteria;p_Proteobacteria;c_Alphaproteobacteria;o_ Sphingomonadales;f_;g_;s_ |
| 0 | 0 | 0 | k_Bacteria;p_Proteobacteria;c__Alphaproteobacteria;o_ Sphingomonadales;f_Erythrobacteraceae;g_;s_ |

(continued)

| two weeks after flight | one day after flight | one day before flight | Taxon |
|---|---|---|---|
| 0 | 1.23E-05 | 0 | k_Bacteria;p_Proteobacteria;c_Alphaproteobacteria;o_Sphingomonadales;f_Sphingomonadaceae;g_;s_ |
| 0 | 6.14E-05 | 0 | k_Bacteria;p_Proteobacteria;c__Alphaproteobacteria;o_Sphingomonadales;f_Sphingomonadaceae;g_Sphingopyxis;s_ |
| 0 | 0 | 0 | k_Bacteria;p_Proteobacteria;c_Betaproteobacteria;o_Burkholderiales;f_;g_;s_ |
| 0 | 0 | 0 | k_Bacteria;p_Proteobacteria;c_Betaproteobacteria;o_B rkholderiales;f_Alcaligenaceae;g_Sutterella;s_ |
| 3.35E-05 | 0.000331447 | 8.94E-05 | k_Bacteria;p_Proteobacteria;c_Betaproteobacteria;o_Burkholderiales;f_Comamonadaceae;g_Comamonas;s_ |
| 0 | 0 | 0 | k_Bacteria;p_Proteobacteria;c_Betaproteobacteria;o_Burkholderiales;f_Oxalobacteraceae;g__Ralstonia;s_ |
| 0 | 0 | 0 | k_Bacteria;p_Proteobacteria;c_Betaproteobacteria;o_Neisseriales;f_Neisseriaceae;g_Neisseria;s_subflava |
| 0 | 0 | 0 | k_Bacteria;p_Proteobacteria;c_Deltaproteobacteria;o_Desulfovibrionales;f_Desulfovibrionaceae;g_Desulfovibrio;s_C21_c20 |
| 0 | 0 | 0 | k_Bacteria;p_Proteobacteria;c_Deltaproteobacteria;o_Desulfovibrionales;f_Desulfovibrionaceae;g_Desulfovibrio;s_D168 |
| 0.001944286 | 0 | 0.000253395 | k_Bacteria;p_Proteobacteria;c_Epsilonproteobacteria;o__Campylobacterales;f_Campylobacteraceae;g_Campylobacter;s_ |
| 0 | 0 | 0 | k_Bacteria;p_Proteobacteria;c_Epsilonproteobacteria;o__Campylobacterales;f_Campylobacteraceae;g_Campylobacter;s_ureolyticus |
| 3.35E-05 | 0 | 0 | k_Bacteria;p_Proteobacteria;c_Epsilonproteobacteria;o__Campylobacterales;f_Helicobacteraceae;g_Helicobacter;s_apodemus |
| 0 | 2.46E-05 | 0 | k_Bacteria;p_Proteobacteria;c_Gammaproteobacteria;o_Enterobacteriales;f_Enterobacteriaceae;g_;s_ |
| 0 | 0 | 0 | k_Bacteria;p_Proteobacteria;c_Gammaproteobacteria;o_Legionellales;f_Legionellaceae;g_Legionella;s_ |
| 0 | 0 | 0 | k_Bacteria;p_Proteobacteria;c_Gammaproteobacteria;o__Pasteurellales;f_Pasteurellaceae;g_Haemophilus;s_para influenzae |
| 0 | 1.23E-05 | 0 | k_Bacteria;p_Proteobacteria;c_Gammaproteobacteria;o_Pseudomonadales;f_Pseudomonadaceae;g_;s_ |
| 0 | 0.001264409 | 0.000223 584 | k_Bacteria;p_Synergistetes;c_Synergistia;o_Synergista les;f_Synergistaceae;g_Synergistes;s_ |
| 3.35E-05 | 0.000171861 | 5.96E-05 | k_Bacteria;p_TM7;c_TM7-3;o_;f_;g_;s_ |
| 0 | 1.23E-05 | 0 | k_Bacteria;p_TM7;c_TM7-3;o_CW040;f_;g_;s_ |
| 0.000201133 | 0.000724273 | 0.000298 111 | k_Bacteria;p_Tenericutes;c_Mollicutes;o_Anaeroplasmatales;f_Anaeroplasmataceae;g_Anaeroplasma;s_ |

(continued)

| two weeks after flight | one day after flight | one day before flight | Taxon |
|---|---|---|---|
| 0.001642587 | 0.002983023 | 0.003607 149 | k_Bacteria;p_Tenericutes;c_Mollicutes;o_RF39;f_; ;g_;s_ |
| 0.000703966 | 1.23E-05 | 0 | k_Bacteria;p_Tenericutes;c_RF3;o_ML615J-28;f_;g_;s_ |
| 0 | 0 | 0 | k_Bacteria;p_Verrucomicrobia;c_Verrucomicrobiae;o_ Verrucomicrobiales;f_Verrucomicrobiaceae;g_Akkermansia;s_ muciniphila |

***Table B***

| two weeks after flight | one day after flight | one day before flight | Taxon |
|---|---|---|---|
| 0.453274365 | 0.1704345 | 0.448049 738 | k_Bacteria;p_Actinobacteria |
| 0.000879024 | 0.001206945 | 0.000839 554 | k_Bacteria;p_Bacteroidetes |
| 0 | 0 | 0 | k_Bacteria;p_Cyanobacteria |
| 0.54529722 | 0.825573299 | 0.550566 132 | k_Bacteria;p_Deferribacteres |
| 0 | 0 | 0 | k_Bacteria;p_Firmicutes |
| 0.000439512 | 0.002669204 | 0.000476 504 | k_Bacteria;p_Fusobacteria |
| 0 | 6.96E-05 | 0 | k_Bacteria;p_Proteobacteria |
| 0.000109878 | 4.64E-05 | 6.81E-05 | k_Bacteria;p_TM7 |
| 0 | 0 | 0 | k_Bacteria;p_Tenericutes |
| 0.453274365 | 0.170364869 | 0.448027 047 | k_Bacteria;p_Actinobacteria;c_Coriobacteriia |
| 0 | 6.96E-05 | 2.27E-05 | k_Bacteria;p_Bacteroidetes;c_Bacteroidia |
| 0.000879024 | 0.001206945 | 0.000839 554 | k_Bacteria;p_Bacteroidetes;c_Sphingobacteriia |
| 0 | 0 | 0 | k_Bacteria;p_Cyanobacteria;c_4C0d-2 |
| 0 | 0 | 0 | k_Bacteria;p_Cyanobacteria;c_Chloroplast |
| 0.005548841 | 0.005524092 | 0.005944 952 | k_Bacteria;p_Deferribacteres;c_Deferribacteres |
| 0.533650148 | 0.819445734 | 0.540877 221 | k_Bacteria;p_Firmicutes;c_Bacilli |
| 0.006098231 | 0.000603472 | 0.003743959 | k_Bacteria;p_Firmicutes;c_Clostridia |
| 0 | 0 | 0 | k_Bacteria;p_Firmicutes;c_Erysipelotrichi |
| 0.000219756 | 0.000232105 | 0.000385741 | k_Bacteria;p_Fusobacteria;c_Fusobacteriia |
| 8.24E-05 | 0.0024371 | 6.81E-05 | k_Bacteria;p_Proteobacteria;c_Alphaproteobacteria |
| 0 | 0 | 0 | k_Bacteria;p_Proteobacteria;c_Betaproteobacteria |
| 0 | 0 | 0 | k_Bacteria;p_Proteobacteria;c_Deltaproteobacteria |
| 0.000137348 | 0 | 2.27E-05 | k_Bacteria;p_Proteobacteria;c_Epsilonproteobacteria |
| 0 | 6.96E-05 | 0 | k_Bacteria;p_Proteobacteria;c_Gammaproteobacteria |
| 0.000109878 | 4.64E-05 | 6.81E-05 | k_Bacteria;p_TM7;c_TM7-3 |
| 0 | 0 | 0 | k_Bacteria;p_Tenericutes;c_Mollicutes |
| 0.453274365 | 0.170364869 | 0.448027047 | k_Bacteria;p_Actinobacteria;c_Coriobacteriia;o_ Coriobacteriales |
| 0 | 6.96E-05 | 2.27E-05 | k_Bacteria;p_Bacteroidetes;c_Bacteroidia;o_Bacteroidales |

(continued)

| two weeks after flight | one day after flight | one day before flight | Taxon |
|---|---|---|---|
| 0.000879024 | 0.001206945 | 0.000839554 | k_Bacteria;p_Bacteroidetes;c_Sphingobacteriia;o_Sphingobacteriales |
| 0 | 0 | 0 | k_Bacteria;p_Cyanobacteria;c_4C0d-2;o_YS2 |
| 0 | 0 | 0 | k_Bacteria;p_Cyanobacteria;c_Chloroplast;o_Streptophyta |
| 2.75E-05 | 2.32E-05 | 0 | k_Bacteria;p_Deferribacteres;c_Deferribacteres;o_Deferribacterales |
| 0 | 2.32E-05 | 0 | k_Bacteria;p_Firmicutes;c_Bacilli;o_Bacillales |
| 0.005521371 | 0.005477672 | 0.005944952 | k_Bacteria;p_Firmicutes;c_Bacilli;o_Gemellales |
| 0 | 0 | 0 | k_Bacteria;p_Firmicutes;c_Bacilli;o_Lactobacillales |
| 0.533650148 | 0.819445734 | 0.540877221 | k_Bacteria;p_Firmicutes;c_Bacilli;o_Turicibacterales |
| 0 | 0 | 0 | k_Bacteria;p_Firmicutes;c_Clostridia;o_Clostridiales |
| 0.006098231 | 0.000603472 | 0.003743959 | k_Bacteria;p_Firmicutes;c_Clostridia;o_SHA-98 |
| 0 | 0 | 0 | k_Bacteria;p_Firmicutes;c_Erysipelotrichi;o_Erysipelotrichales |
| 0.000109878 | 0.000116052 | 0.000204216 | k_Bacteria;p Fusobacteria;c Fusobacteriia;o_Fusobacteriales |
| 8.24E-05 | 9.28E-05 | 0.000136144 | k_Bacteria;p_Proteobacteria;c_Alphaproteobacteria;o_Caulobacterales |
| 2.75E-05 | 0 | 2.27E-05 | k_Bacteria;p_Proteobacteria;c_Alphaproteobacteria;o_RF32 |
| 0 | 2.32E-05 | 2.27E-05 | k_Bacteria;p_Proteobacteria;c_Alphaproteobacteria;o_Rhizobiales |
| 8.24E-05 | 0.0024371 | 6.81E-05 | k_Bacteria;p_Proteobacteria;c_Alphaproteobacteria;o_Rhodobacterales |
| 0 | 0 | 0 | k_Bacteria;p_Proteobacteria;c_Betaproteobacteria;o_Burkholderiales |
| 0 | 0 | 0 | k_Bacteria;p_Proteobacteria;c_Betaproteobacteria;o_Methylophilales |
| 0 | 0 | 0 | k_Bacteria;p_Proteobacteria;c_Betaproteobacteria;o_Neisseriales |
| 0 | 0 | 0 | k_Bacteria;p_Proteobacteria;c_Deltaproteobacteria;o_Desulfovibrionales |
| 0 | 0 | 0 | k_Bacteria;p_Proteobacteria;c_Epsilonproteobacteria;o_Campylobacterales |
| 2.75E-05 | 0 | 0 | k_Bacteria;p_Proteobacteria;c_Gammaproteobacteria;o_Enterobacteriales |
| 0.000109878 | 0 | 2.27E-05 | k_Bacteria;p_Proteobacteria;c_Gammaproteobacteria;o_Pasteurellales |
| 0 | 6.96E-05 | 0 | k_Bacteria;p_Proteobacteria;c_Gammaproteobacteria;o_Pseudomonadales |
| 0.000109878 | 4.64E-05 | 6.81E-05 | k_Bacteria;p_TM7;c,_TM7-3;o_ |
| 0 | 0 | 0 | k_Bacteria;p_Tenericutes;c_Mollicutes;o_Anaeroplasmatales |
| 0 | 0 | 0 | k_Bacteria;p_Tenericutes;c_Mollicutes;o_RF39 |

(continued)

| two weeks after flight | one day after flight | one day before flight | Taxon |
|---|---|---|---|
| 0 | 0 | 0 | k_Bacteria;p_Actinobacteria;c_Coriobacteriia;o_Coriobacteriales;f_Coriobacteriaceae |
| 0.418607845 | 0.146991923 | 0.424973338 | k_Bacteria;p_Bacteroidetes;c_Bacteroidia;o_Bacteroidales;f_ |
| 0.023733656 | 0.018846904 | 0.016314583 | k_Bacteria;p_Bacteroidetes;c_Bacteroidia;o_Bacteroidales;f_Bacteroidaceae |
| 2.75E-05 | 6.96E-05 | 4.54E-05 | k_Bacteria;p_Bacteroidetes;c_Bacteroidia;o_Bacteroi dales;f_Porphyromonadaceae |
| 0.003735853 | 0.003110203 | 0.002518663 | k_Bacteria;p_Bacteroidetes;c_Bacteroidia;o_Bacteroi dales;f_Prevotellaceae |
| 0.000219756 | 0.000185684 | 0.000272288 | k_Bacteria;p_Bacteroidetes;c_Bacteroidia;o_Bacteroi dales;f_Rikenellaceae |
| 0.005109329 | 0.000139263 | 0.001429511 | k_Bacteria;p_Bacteroidetes;c_Bacteroidia;o_Bacteroi dales;f_S24-7 |
| 0.001840457 | 0.001021261 | 0.002473282 | k_Bacteria;p_Bacteroidetes;c_Bacteroidia;o_Bacteroi dales;f_[Barnesiellaceae] |
| 0 | 0 | 0 | k_Bacteria;p_Bacteroidetes;c_Bacteroidia;o_Bacteroi dales;f_[Odoribacteraceae] |
| 0 | 6.96E-05 | 2.27E-05 | k_Bacteria;p_Bacteroidetes;c_Bacteroidia;o_Bacteroi dales;f_[Paraprevotellaceae] |
| 0.000879024 | 0.001206945 | 0.000839554 | k_Bacteria;p_Bacteroidetes;c_Sphingobacteriia;o_Sphingobacteriales;f_Sphingobacteriaceae |
| 0 | 0 | 0 | k_Bacteria;p_Cyanobacteria;c_4C0d-2;o_YS2;f_ |
| 0 | 0 | 0 | k_Bacteria;p_Cyanobacteria;c_Chloroplast;o_Streptophyta;f_ |
| 2.75E-05 | 0 | 0 | k_Bacteria;p_Deferribacteres;c_Deferribacteres;o_Deferribacterales;f_Deferribacteraceae |
| 0 | 0 | 0 | k_Bacteria;p_Firmicutes;c_Bacilli;o_Bacillales;f_Bacillaceae |
| 0 | 2.32E-05 | 0 | k_Bacteria;p_Firmicutes;c_Bacilli;o_Bacillales;f_Paenibacillaceae |
| 0 | 2.32E-05 | 0 | k_Bacteria;p_Firmicutes;c_Bacilli;o_Bacillales;f_Staphylococcaceae |
| 0 | 0 | 4.54E-05 | k_Bacteria;p_Firmicutes;c_Bacilli;o_Gemellales;f_Gemellaceae |
| 0 | 0 | 0 | k_Bacteria;p_Firmicutes;c_Bacilli;o_Lactobacillales;f_ |
| 0 | 0 | 0 | k_Bacteria;p_Firmicutes;c_Bacilli;o_Lactobacillales;f_Aerococcaceae |
| 0.005274146 | 0.00538483 | 0.00585419 | k_Bacteria;p_Firmicutes;c_Bacilli;o_Lactobacillales;f_Enterococcaceae |
| 0 | 0 | 0 | k_Bacteria;p_Firmicutes;c_Bacilli;o_Lactobacillales;f_Lactobacillaceae |
| 0.000247226 | 9.28E-05 | 4.54E-05 | k_Bacteria;p_Firmicutes;c_Bacilli;o_Lactobacillales;f_Leuconostocaceae |
| 0 | 0 | 0 | k_Bacteria;p_Firmicutes;c Bacilli;o_Lactobacillales;f_Streptococcaceae |

(continued)

| two weeks after flight | one day after flight | one day before flight | Taxon |
|---|---|---|---|
| 0.049417646 | 0.103774023 | 0.045335935 | k_Bacteria;p_Firmicutes;c_Bacilli;o_Turicibacterales;f_Turicibacteraceae |
| 0 | 4.64E-05 | 0 | k_Bacteria;p_Firmicutes;c_Clostridia;o_Clostridiales;f_ |
| 0.033677618 | 0.234727509 | 0.030382791 | k_Bacteria;p_Firmicutes;c_Clostridia;o_Clostridiales;f_Christensenellaceae |
| 2.75E-05 | 2.32E-05 | 6.81E-05 | k_Bacteria;p_Firmicutes;c_Clostridia;o_Clostridiales;f_Clostridiaceae |
| 0.327656302 | 0.388148733 | 0.394794763 | k_Bacteria;p_Firmicutes;c_Clostridia;o_Clostridiales;f_Dehalobacteriaceae |
| 0 | 2.32E-05 | 4.54E-05 | k_Bacteria;p_Firmicutes;c_Clostridia;o_Clostridiales;f_Lachnospiraceae |
| 0.001236128 | 0.000394578 | 0.000158835 | k_Bacteria;p_Firmicutes;c_Clostridia;o__Clostridiales;f_Peptococcaceae |
| 0.115152181 | 0.029895089 | 0.061400921 | k_Bacteria;p_Firmicutes;c_Clostridia;o_Clostridiales;f_Peptostreptococcaceae |
| 0.006208109 | 0.062343329 | 0.008508997 | k_Bacteria;p_Firmicutes;c_Clostridia;o_Clostridiales;f_Ruminococcaceae |
| 0.000274695 | 6.96E-05 | 0.000181525 | k_Bacteria;p_Firmicutes;c_Clostridia;o_Clostridiales;f_Veillonellaceae |
| 0 | 0 | 0 | k_Bacteria;p_Firmicutes;c_Clostridia;o_Clostridiales;f_[Mogibacteriaceae] |
| 0 | 0 | 0 | k_Bacteria;p_Firmicutes;c_Clostridia;o_Clostridiales;f_[Tissierellaceae] |
| 0.006098231 | 0.000603472 | 0.003743959 | k_Bacteria;p_Firmicutes;c_Clostridia;o_SHA-98;f_ |
| 0 | 0 | 0 | k_Bacteria;p_Firmicutes;c_Erysipelotrichi;o_Erysipel otrichales;f_Erysipelotrichaceae |
| 0.000109878 | 0.000116052 | 0.000204216 | k_Bacteria;p_Fusobacteria;c_Fusobacteriia;o_Fusobac teriales;f_Fusobacteriaceae |
| 8.24E-05 | 9.28E-05 | 0.000136144 | k_Bacteria;p_Proteobacteria;c_Alphaproteobacteria;o_Caulobacterales;f_Caulobacteraceae |
| 0 | 0 | 2.27E-05 | k_Bacteria;p_Proteobacteria;c_Alphaproteobacteria;o_RF32;f_ |
| 2.75E-05 | 0 | 0 | k_Bacteria;p_Proteobacteria;c_Alphaproteobacteria;o_Rhizobiales;f_Brucellaceae |
| 0 | 2.32E-05 | 2.27E-05 | k_Bacteria;p_Proteobacteria;c_Alphaproteobacteria;o_Rhizobiales;f_Rhizobiaceae |
| 2.75E-05 | 6.96E-05 | 0 | k_Bacteria;p_Proteobacteria;c_Alphaproteobacteria;o_Rhodobacterales;f_Rhodobacteraceae |
| 2.75E-05 | 0.002297837 | 4.54E-05 | k_Bacteria;p_Proteobacteria;c_Betaproteobacteria;o_Burkholderiales;f_Alcaligenaceae |
| 2.75E-05 | 6.96E-05 | 2.27E-05 | k_Bacteria;p_Proteobacteria;c_Betaproteobacteria;o_Burkholderiales;f_Comamonadaceae |
| 0 | 0 | 0 | k_Bacteria;p_Proteobacteria;c_Betaproteobacteria;o_Burkholderiales;f_Oxalobacteraceae |

(continued)

| two weeks after flight | one day after flight | one day before flight | Taxon |
|---|---|---|---|
| 0 | 0 | 0 | k_Bacteria;p_Proteobacteria;c_Betaproteobacteria;o_Methylophilales;f_Methylophilaceae |
| 0 | 0 | 0 | k_Bacteria;p_Proteobacteria;c_Betaproteobacteria;o_Neisseriales;f_Neisseriaceae |
| 0 | 0 | 0 | k_Bacteria;p_Proteobacteria;c_Deltaproteobacteria;o_Desulfovibrionales;f_Desulfovibrionaceae |
| 0 | 0 | 0 | k_Bacteria;p_Proteobacteria;c_Epsilonproteobacteria;o_Campylobacterales;f_Campylobacteraceae |
| 0 | 0 | 0 | k_Bacteria;p_Proteobacteria;c_Epsilonproteobacteria;o_Campylobacterales;f_Helicobacteraceae |
| 2.75E-05 | 0 | 0 | k_Bacteria;p_Proteobacteria;c_Gammaproteobacteria;o_Enterobacteriales;f_Enterobacteriaceae |
| 0.000109878 | 0 | 2.27E-05 | k_Bacteria;p_Proteobacteria;c_Gammaproteobacteria;o_Pasteurellales;f_Pasteurellaceae |
| 0 | 6.96E-05 | 0 | k_Bacteria;p_Proteobacteria;c_Gammaproteobacteria;o_Pseudomonadales;f_Pseudomonadaceae |
| 0.000109878 | 4.64E-05 | 6.81E-05 | k_Bacteria;p__TM7;c_TM7-3;o_;f_ |
| 0 | 0 | 0 | k_Bacteria;p_Tenericutes;c_Mollicutes;o_Anaeroplas matales;f_Anaeroplasmataceae |
| 0 | 0 | 0 | k_Bacteria;p_Tenericutes;c_Mollicutes;o_RF39;f_ |
| 0 | 0 | 0 | k_Bacteria;p_Actinobacteria;c_Coriobacteriia;o_Coriobacteriales;f_Coriobacteriaceae;g_Adlercreutzia |
| 0.418607845 | 0.146991923 | 0.424973338 | k_Bacteria;p_Bacteroidetes;c_Bacteroidia;o_Bacteroi dales;f_;g_ |
| 0.023733656 | 0.018846904 | 0.016314583 | k_Bacteria;p_Bacteroidetes;c_Bacteroidia;o_Bacteroidales;f_Bacteroidaceae;g_Bacteroides |
| 2.75E-05 | 6.96E-05 | 4.54E-05 | k_Bacteria;p_Bacteroidetes;c_Bacteroidia;o_Bacteroidales;f_Porphyromonadaceae;g_Parabacteroides |
| 0.003625975 | 0.002994151 | 0.002337138 | k_Bacteria;p_Bacteroidetes;c_Bacteroidia;o_Bacteroidales;f_Prevotellaceae;g_Prevotella |
| 0.000109878 | 0.000116052 | 0.000181525 | k_Bacteria;p_Bacteroidetes;c_Bacteroidia;o_Bacteroi dales;f_Rikenellaceae;g_ |
| 0 | 0 | 0 | k_Bacteria;p_Bacteroidetes;c_Bacteroidia;o_Bacteroidales;f_Rikenellaceae;g_AF12 |
| 0.000219756 | 0.000185684 | 0.000272288 | k_Bacteria;p_Bacteroidetes;c_Bacteroidia;o_Bacteroidales;f_Rikenellaceae;g_Alistipes |
| 0.005109329 | 0.000139263 | 0.001429511 | k_Bacteria;p_Bacteroidetes;c_Bacteroidia;o_Bacteroidales;f_S24-7;g_ |
| 0.000466982 | 6.96E-05 | 0.000635338 | k_Bacteria;p_Bacteroidetes;c_Bacteroidia;o_Bacteroidales;f_[Barnesiellaceae];g_ |
| 0.001373475 | 0.000951629 | 0.001837943 | k_Bacteria;p_Bacteroidetes;c_Bacteroidia;o_Bacteroidales;f_[Odoribacteraceae];g_Butyricimonas |

(continued)

| two weeks after flight | one day after flight | one day before flight | Taxon |
|---|---|---|---|
| 0 | 0 | 0 | k_Bacteria;p_Bacteroidetes;c_Bacteroidia;o_Bacteroidales;f_[Odoribacteraceae];g_Odoribacter |
| 0 | 6.96E-05 | 2.27E-05 | k_Bacteria;p_Bacteroidetes;c_Bacteroidia;o_Bacteroidales;f_[Paraprevotellaceael;g_[Prevotella] |
| 0.000879024 | 0.001206945 | 0.000839554 | k_Bacteria;p_Bacteroidetes;c_Sphingobacteriia;o_Sphingobacteriales;f_Sphingobacteriaceae;g_Sphingobacterium |
| 0 | 0 | 0 | k_Bacteria;p_Cyanobacteria;c_4C0d-2;o_YS2;f_;g_ |
| 0 | 0 | 0 | k_Bacteria;p_Cyanobacteria;c_Chloroplast;o_Streptop hyta;f_;g_ |
| 2.75E-05 | 0 | 0 | k_Bacteria;p_Deferribacteres;c_Deferribacteres;o_Deferribacterales;f_Deferribacteraceae;g_Mucispirillum |
| 0 | 0 | 0 | k_Bacteria;p_Firmicutes;c_Bacilli;o_Bacillales;f_Ba cillaceae;g_Bacillus |
| 0 | 2.32E-05 | 0 | k_Bacteria;p_Firmicutes;c_Bacilli;o_Bacillales;f_Pa enibacillaceae;g_Paenibacillus |
| 0 | 2.32E-05 | 0 | k_Bacteria;p_Firmicutes;c_Bacilli;o_Bacillales;f_Staphylococcaceae;g_Macrococcus |
| 0 | 0 | 4.54E-05 | k_Bacteria;p_Firmicutes;c_Bacilli;o_Gemellales;f_Gemellaceae;g_ |
| 0 | 0 | 0 | k_Bacteria;p_Firmicutes;c_Bacilli;o_Lactobacillales;f_;g_ |
| 0 | 0 | 0 | k_Bacteria;p_Firmicutes;c_Bacilli;o_Lactobacillales;f_Aerococcaceae;g_Aerococcus |
| 0 | 0 | 0 | k_Bacteria;p_Firmicutes;c_Bacilli;o_Lactobacillales;f_Aerococcaceae;g_Facklamia |
| 0 | 0 | 0 | k_Bacteria;p_Firmicutes;c_Bacilli;o_Lactobacillales;f_Enterococcaceae;g_Enterococcus |
| 0.005274146 | 0.00538483 | 0.00585419 | k_Bacteria;p_Firmicutes;c_Bacilli;o_Lactobacillales;f_Lactobacillaceae;g |
| 0 | 0 | 0 | k_Bacteria;p_Firmicutes;c_Bacilli;o_Lactobacillales;f_Lactobacillaceae;g_Lactobacillus |
| 0 | 0 | 0 | k_Bacteria;p_Firmicutes;c_Bacilli;o_Lactobacillales;f_Leuconostocaceae;g_ |
| 0 | 0 | 0 | k_Bacteria;p_Firmicutes;c_Bacilli;o_Lactobacillales;f_Leuconostocaceae;g_Leuconostoc |
| 0 | 0 | 0 | k_Bacteria;p_Firmicutes;c_Bacilli;o_Lactobacillales;f_Leuconostocaceae;g_Weissella |
| 0.000247226 | 9.28E-05 | 4.54E-05 | k_Bacteria;p_Firmicutes;c_Bacilli;o_Lactobacillales;f_Streptococcaceae;g_Lactococcus |
| 0 | 0 | 0 | k_Bacteria;p_Firmicutes;c_Bacilli;o_Lactobacillales;f_Streptococcaceae;g_Streptococcus |
| 0.049417646 | 0.103774023 | 0.045335935 | k_Bacteria;p_Firmicutes;c_Bacilli;o_Turicibacterales; f_Turicibacteraceae;g_Turicibacter |
| 0 | 4.64E-05 | 0 | k_Bacteria;p_Firmicutes;c_Clostridia;o_Clostridiales;f_;g_ |

(continued)

| two weeks after flight | one day after flight | one day before flight | Taxon |
|---|---|---|---|
| 0.001181189 | 0.001717575 | 0.000521885 | k_Bacteria;p_Firmicutes;c_Clostridia;o_Clostridiales;f_ Christensenellaceae;g_Christensenella |
| 0 | 0 | 0 | k_Bacteria;p_Firmicutes;c_Clostridia;o_Clostridiales;f_ Clostridiaceae;g_ |
| 5.49E-05 | 4.64E-05 | 0 | k_Bacteria;p_Firmicutes;c_Clostridia;o_Clostridiales;f_ Clostridiaceae;g_02d06 |
| 0.030683441 | 0.231756569 | 0.028907899 | k_Bacteria;p_Firmicutes;c_Clostridia;o_Clostridiales;f_ Clostridiaceae;g_Candidatus Arthromitus |
| 0.001758049 | 0.001206945 | 0.000953008 | k_Bacteria;p_Firmicutes;c_Clostridia;o_Clostridiales;f_ Clostridiaceae;g_Clostridium |
| 2.75E-05 | 2.32E-05 | 6.81E-05 | k_Bacteria;p_Firmicutes;c_Clostridia;o_Clostridiales;f_ Clostridiaceae;g_SMB53 |
| 0.180199978 | 0.168183084 | 0.156951283 | k_Bacteria;p_Firmicutes;c_Clostridia;o_Clostridiales;f_ Dehalobacteriaceae;g_Dehalobacterium |
| 0.004917042 | 0.001160524 | 0.027614531 | k_Bacteria;p_Firmicutes;c_Clostridia;o_Clostridiales;f_ Lachnospiraceae;g_ |
| 0.011647072 | 0.006638195 | 0.027614531 | k_Bacteria;p_Firmicutes;c_Clostridia;o_Clostridiales;f_ Lachnospiraceae;g_Anaerostipes |
| 2.75E-05 | 2.32E-05 | 2.27E-05 | k_Bacteria;p_Firmicutes;c_Clostridia;o_Clostridiales;f_ Lachnospiraceae;g_Blautia |
| 0.000164817 | 0.000278526 | 0.000113453 | k_Bacteria;p_Firmicutes;c_Clostridia;o_Clostridiales;f_ Lachnospiraceae;g_Butyrivibrio |
| 0.04194594 | 0.110156903 | 0.039572508 | k_Bacteria;p_Firmicutes;c_Clostridia;o_Clostridiales;f_ Lachnospiraceae;g_Clostridium |
| 0.014036919 | 0.027388358 | 0.011776452 | k_Bacteria;p_Firmicutes;c_Clostridia;o_Clostridiales;f_ Lachnospiraceae;g_Coprococcus |
| 0.005329085 | 0.00392257 | 0.001520274 | k_Bacteria;p_Firmicutes;c_Clostridia;o_Clostridiales;f_ Lachnospiraceae;g_Dorea |
| 0.011399846 | 0.002831678 | 0.00308593 | k_Bacteria;p_Firmicutes;c_Clostridia;o_Clostridiales;f_ Lachnospiraceae; g_Lachnobacterium |
| 0.002582134 | 0.000858787 | 0.00240521 | k_Bacteria;p_Firmicutes;c_Clostridia;o_Clostridiales;f_ Lachnospiraceae;g_Lachnospira |
| 0.055405999 | 0.066706898 | 0.124117901 | k_Bacteria;p_Firmicutes;c_Clostridia;o_Clostridiales;f_ Lachnospiraceae;g_Roseburia |
| 0 | 0 | 0 | k_Bacteria;p_Firmicutes;c_Clostridia;o_Clostridiales;f_ Lachnospiraceae;g_[Ruminococcus] |
| 0 | 2.32E-05 | 4.54E-05 | k_Bacteria;p_Firmicutes;c_Clostridia;o_Clostridiales;f_ Peptococcaceae;g_ |
| 0.001236128 | 0.000394578 | 0.000158835 | k_Bacteria;p_Firmicutes;c_Clostridia;o_Clostridiales;f_ Peptococcaceae;g_rc4-4 |
| 0 | 0 | 0 | k_Bacteria;p_Firmicutes;c_Clostridia;o_Clostridiales;f_ Peptostreptococcaceae;g_ |
| 0.099192396 | 0.028293566 | 0.049216038 | k_Bacteria;p_Firmicutes;c_Clostridia;o_Clostridiales;f_ Peptostreptococcaceae;g_Filifactor |

(continued)

| two weeks after flight | one day after flight | one day before flight | Taxon |
|---|---|---|---|
| 0.002115152 | 6.96E-05 | 0.00102108 | k_Bacteria;p_Firmicutes;c_Clostridia;o_Clostridiales;f_Ruminococcaceae;g_ |
| 0.000109878 | 9.28E-05 | 0.000499194 | k_Bacteria;p_Firmicutes;c_Clostridia;o_Clostridiales;f_Ruminococcaceae;g_Faecalibacterium |
| 0.013734754 | 0.001439049 | 0.010664609 | k_Bacteria;p_Firmicutes;c_Clostridia;o_Clostridiales;f_Ruminococcaceae;g_Oscillospira |
| 0.000164817 | 0 | 0.000113453 | k_Bacteria;p_Firmicutes;c_Clostridia;o_Clostridiales;f_Ruminococcaceae;g_Ruminococcus |
| 0 | 4.64E-05 | 0 | k_Bacteria;p,_Firmicutes;c_Clostridia;o_Clostridiales;f_Veillonellaceae;g_Dialister |
| 0.005823536 | 0.061647015 | 0.008327472 | k_Bacteria;p_Firmicutes;c_Clostridia;o_Clostridiales;f_Veillonellaceae;g_Megasphaera |
| 0.000219756 | 0.000649893 | 6.81E-05 | k_Bacteria;p_Firmicutes;c_Clostridia;o_Clostridiales;f_Veillonellaceae;g_Phascolarctobacterium |
| 0.000274695 | 6.96E-05 | 0.000181525 | k_Bacteria;p_Firmicutes;c_Clostridia;o_Clostridiales;f_Veillonellaceae;g_Veillonella |
| 0 | 0 | 0 | k_Bacteria;p_Firmicutes;c_Clostridia;o_Clostridiales;f_[Mogibacteriaceae];g_ |
| 0 | 0 | 0 | k_Bacteria;p_Firmicutes;c_Clostridia;o_Clostridiales;f_[Tissierellaceae];g_Peptoniphilus |
| 0.005933414 | 0.000603472 | 0.003721268 | k_Bacteria;p_Firmicutes;c_Clostridia;o_SHA-98;f_;g_ |
| 8.24E-05 | 0 | 0 | k_Bacteria;p_Firmicutes;c_Erysipelotrichi;o_Erysipelotrichales;f_Erysipelotrichaceae;g_ |
| 0 | 0 | 0 | k_Bacteria;p_Firmicutes;c_Erysipelotrichi;o_Erysipelotrichales;f_Erysipelotrichaceae;g_Allobaculum |
| 0 | 0 | 0 | k_Bacteria;p_Firmicutes;c_Erysipelotrichi;o_Erysipelotrichales;f_Erysipelotrichaceae;g_Bulleidia |
| 8.24E-05 | 0 | 2.27E-05 | k_Bacteria;p_Firmicutes;c_Erysipelotrichi;o_Erysipelotrichales;f_Erysipelotrichaceae;g_Coprobacillus |
| 0 | 0 | 0 | k_Bacteria;p_Firmicutes;c_Erysipelotrichi;o_Erysipelotrichales;f_Erysipelotrichaceae;g_[Eubacterium] |
| 0.000109878 | 0.000116052 | 0.000204216 | k_Bacteria;p_Fusobacteria;c_Fusobacteriia;o_Fusobacteriales;f_Fusobacteriaceae;g_Fusobacterium |
| 8.24E-05 | 9.28E-05 | 0.000136144 | k_Bacteria;p_Proteobacteria;c_Alphaproteobacteria;o_Caulobacterales;f_Caulobacteraceae;g_ |
| 0 | 0 | 2.27E-05 | k_Bacteria;p_Proteobacteria;c_Alphaproteobacteria;o_RF32;f_;g_ |
| 0 | 0 | 0 | k_Bacteria;p_Proteobacteria;c_Alphaproteobacteria;o__Rhizobiales;f_Burucellaceae;g_Ochrobactrum |
| 2.75E-05 | 0 | 0 | k_Bacteria;p_Proteobacteria;c_Alphaproteobacteria;o_Rhizobiales;f_Rhizobiaceae;g_ |
| 0 | 2.32E-05 | 2.27E-05 | k_Bacteria;p_Proteobacteria;c_Alphaproteobacteria;o__Rhizobiales;f_Rhizobiaceae;g_Agrobacterium |

(continued)

| two weeks after flight | one day after flight | one day before flight | Taxon |
|---|---|---|---|
| 2.75E-05 | 6.96E-05 | 0 | k_Bacteria;p_Proteobacteria;c_Alphaproteobacteria;o__Rhodobacterales;f_Rhodobacteraceae;g_ |
| 2.75E-05 | 0.002297837 | 4.54E-05 | k_Bacteria;p_Proteobacteria;c_Betaproteobacteria;o_Burkholderiales;f_Alcaligenaceae;g_Sutterella |
| 0 | 6.96E-05 | 0 | k_Bacteria;p_Proteobacteria;c_Betaproteobacteria;o_Burkholderiales;f_Comamonadaceae;g_Comamonas |
| 2.75E-05 | 0 | 2.27E-05 | k_Bacteria;p_Proteobacteria;c_Betaproteobacteria;o_Burkholderiales;f_Oxalobacteraceae;g_Oxalobacter |
| 0 | 0 | 0 | k_Bacteria;p_Proteobacteria;c_Betaproteobacteria;o_Burkholderiales;f_Oxalobacteraceae;g_Ralstonia |
| 0 | 0 | 0 | k_Bacteria;p_Proteobacteria;c_Betaproteobacteria;o_Methylophilales;f_Methylophilaceae;g_ |
| 0 | 0 | 0 | k_Bacteria;p_Proteobacteria;c_Betaproteobacteria;o_Neisseriales;f_Neisseriaceae;g_Neisseria |
| 0 | 0 | 0 | k_Bacteria;p_Proteobacteria;c_Deltaproteobacteria;o_Desulfovibrionales;f_Desulfovibrionaceae;g_ |
| 0 | 0 | 0 | k_Bacteria;p_Proteobacteria;c_Deltaproteobacteria;o_Desulfovibrionales;f_Desulfovibrionaceae;g_Bilophila |
| 0 | 0 | 0 | k_Bacteria;p_Proteobacteria;c_Epsilonproteobacteria;o_Campylobacterales;f_Campylobacteraceae;g_Campylobacter |
| 0 | 0 | 0 | k_Bacteria;p_Proteobacteria;c_Epsilonproteobacteria;o_Campylobacterales;f_Helicobacteraceae;g_Helicobacter |
| 2.75E-05 | 0 | 0 | k_Bacteria;p_Proteobacteria;c_Gammaproteobacteria;o_Enterobacteriales;f_Enterobacteriaceae;g_ |
| 0.000109878 | 0 | 2.27E-05 | k_Bacteria;p_Proteobacteria;c_Gammaproteobacteria;o_Pasteurellales;f_Pasteurellaceae;g_Haemophilus |
| 0 | 6.96E-05 | 0 | k_Bacteria;p_Proteobacteria;c_Gammaproteobacteria;o_Pseudomonadales;f_Pseudomonadaceae;g_ |
| 0.000109878 | 4.64E-05 | 6.81E-05 | k_Bacteria;p_TM7;c_TM7-3;o_;f_;g_ |
| 0 | 0 | 0 | k_Bacteria;p_Tenericutes;c_Mollicutes;o_Anaeroplasmatales;f_Anaeroplasmataceae;g_Anaeroplasma |
| 0 | 0 | 0 | k_Bacteria;p_Tenericutes;c_Mollicutes;o_RF39;f_;g_ |
| 0 | 0 | 0 | k_Bacteria;p_Actinobacteria;c_Coriobacteriia;o_Coriobacteriales;f_Coriobacteriaceae;g_Adlercreutzia;s_ |
| 0.388830898 | 0.136129422 | 0.400490118 | k_Bacteria;p_Bacteroidetes;c_Bacteroidia;o_Bacteroidales;f_;g_;s |
| 0.0128832 | 0.007427351 | 0.012139502 | k_Bacteria;p_Bacteroidetes;c_Bacteroidia;o_Bacteroidales;f_Bacteroidaceae;g_Bacteroides;s_ |
| 0 | 0 | 0 | k_Bacteria;p_Bacteroidetes;c_Bacteroidia;o_Bacteroidales;f_Bacteroidaceae;g_Bacteroides;s_acidifaciens |
| 0.012910669 | 0.002088943 | 0.007555989 | k_Bacteria;p_Bacteroidetes;c_Bacteroidia;o_Bacteroidales;f_Bacteroidaceae;g_Bacteroides;s_caccae |

(continued)

| two weeks after flight | one day after flight | one day before flight | Taxon |
|---|---|---|---|
| 0.002554664 | 0.000533841 | 0.003539743 | k_Bacteria;p_Bacteroidetes;c Bacteroidia;o_Bacteroidales;f_Bacteroidaceae;g_Bacteroides;s_eggerthii |
| 0.000494451 | 0.000139263 | 0.000499194 | k_Bacteria;p_Bacteroidetes;c_Bacteroidia;o_Bacteroi dales;f Bacteroidaceae;g_Bacteroides;s_fragilis |
| 0.000933963 | 0.000673104 | 0.000748792 | k_Bacteria;p_Bacteroidetes;c_Bacteroidia;o_Bacteroidales;f_Bacteroidaceae;g_Bacteroides;s_ovatus |
| 0.020162619 | 0.017848853 | 0.012979057 | k_Bacteria;p_Bacteroidetes;c_Bacteroidia;o_Bacteroidales;f_Bacteroidaceae;g_Bacteroides;s_uniformis |
| 0.003571036 | 0.00097484 | 0.003335527 | k_Bacteria;p_Bacteroidetes;c_Bacteroidia;o_Bacteroidales;f_Porphyromonadaceae;g_Parabacteroides;s_ |
| 0 | 2.32E-05 | 0 | k_Bacteria;p_Bacteroidetes;c_Bacteroidia;o_Bacteroidales;f_Porphyromonadaceae;g_Parabacteroides;s_distasonis |
| 0 | 0 | 4.54E-05 | k_Bacteria;p_Bacteroidetes;c_Bacteroidia;o_Bacteroidales;f_Porphyromonadaceae;g_Parabacteroides;s_gordonii |
| 2.75E-05 | 6.96E-05 | 0 | k_Bacteria;p_Bacteroidetes;c_Bacteroidia;o_Bacteroidales;f_Prevotellaceae;g_Prevotella;s_ |
| 0 | 0 | 0 | k_Bacteria;p_Bacteroidetes;c_Bacteroidia;o_Bacteroidales;f_Prevotellaceae;g_Prevotella;s _copri |
| 0.003625975 | 0.002994151 | 0.002337138 | k_Bacteria;p_Bacteroidetes;c_Bacteroidia;o_Bacteroidales;f_Prevotellaceae;g_Prevotella;s_stercorea |
| 0.000109878 | 0.000116052 | 0.000181525 | k_Bacteria;p_Bacteroidetes;c_Bacteroidia;o_Bacteroidales;f_Rikenellaceae;g_;s_ |
| 0 | 0 | 0 | k_Bacteria;p_Bacteroidetes;c_Bacteroidia;o_Bacteroidales;f_Rikenellaceae;g_AF12;s_ |
| 0.000219756 | 0.000185684 | 0.000272288 | k_Bacteria;p_Bacteroidetes;c_Bacteroidia;o_Bacteroidales;f_Rikenellaceae;g_Alistipes;s_indistinctus |
| 0.005109329 | 0.000139263 | 0.001429511 | k_Bacteria;p_Bacteroidetes;c_Bacteroidia;o_Bacteroidales;f_S24-7;g_;s_ |
| 0.000466982 | 6.96E-05 | 0.000635338 | k_Bacteria;p_Bacteroidetes;c_Bacteroidia;o_Bacteroidales;f_[Barnesiellaceae];g_;s_ |
| 0.001373475 | 0.000951629 | 0.001837943 | k_Bacteria;p_Bacteroidetes;c_Bacteroidia;o_Bacteroi dales;f_[Odoribacteraceae];g_Butyricimonas;s_ |
| 0 | 0 | 0 | k_Bacteria;p_Bacteroidetes;c_Bacteroidia;o_Bacteroidales;f_[Odoribacteraceae];g_Odoribacter;s_ |
| 0 | 6.96E-05 | 2.27E-05 | k_Bacteria;p_Bacteroidetes;c_Bacteroidia;o_Bacteroidales;f_[Paraprevotellaceae];g_[Prevotella];s_ |
| 0.000879024 | 0.001206945 | 0.000839554 | k_Bacteria;p_Bacteroidetes;c_Sphingobacteriia;o_Sphingobacteriales;f_Sphmgobacteriaceae;g_Sphingobacterium;s_ |
| 0 | 0 | 0 | k_Bacteria;p_Cyanobacteria;c_4C0d-2;o_YS2;f_;g_;s_ |
| 0 | 0 | 0 | k_Bacteria;p_Cyanobacteria;c_Chloroplast;o_Streptophyta;f_;g_;s_ |

(continued)

| two weeks after flight | one day after flight | one day before flight | Taxon |
|---|---|---|---|
| 2.75E-05 | 0 | 0 | k_Bacteria;p_Deferribacteres;c_Deferribacteres;o_Deferribacterales;f_Deferribacteraceae;g_Mucispirillum;s_schaedleri |
| 0 | 0 | 0 | k_Bacteria;p_Firmicutes;c_Bacilli;o_Bacillales;f_Bacillaceae;g_Bacillus;s_ |
| 0 | 2.32E-05 | 0 | k_Bacteria;p_Firmicutes;c_Bacilli;o_Bacillales;f_Paenibacillaceae;g_Paenibacillus;s_ |
| 0 | 2.32E-05 | 0 | k_Bacteria;p_Firmicutes;c_Bacilli;o_Bacillales;f_Staphylococcaceae;g_Macrococcus;s_brunensis |
| 0 | 0 | 4.54E-05 | k_Bacteria;p_Firmicutes;c_Bacilli;o_Gemellales;f_Gemellaceae;_g_;s_ |
| 0 | 0 | 0 | k_Bacteria;p_Firmicutes;c_Bacilli;o_Lactobacillales;f_;g_;s_ |
| 0 | 0 | 0 | k_Bacteria;p_Firmicutes;c_Bacilli;o_Lactobacillales;f_Aerococcaceae;g_Aerococcus;s_ |
| 0 | 0 | 0 | k_Bacteria;p_Firmicutes;c_Bacilli;o_Lactobacillales;f_Aerococcaceae;g_Facklamia;s_ |
| 0 | 0 | 0 | k_Bacteria;p_Firmicutes;c_Bacilli;o_Lactobacillales;f_Enterococcaceae;g_Enterococcus;s_ |
| 0.004532469 | 0.004618884 | 0.005128089 | k_Bacteria;p_Firmicutes;c_Bacilli;o_Lactobacillales;f_Lactobacillaceae;g_;s_ |
| 0 | 0 | 0 | k_Bacteria;p_Firmicutes;c_Bacilli;o_Lactobacillales;f_Lactobacillaceae;g_Lactobacillus;s_ |
| 0.000741677 | 0.000765946 | 0.000726101 | k_Bacteria;p_Firmicutes;c_Bacilli;o_Lactobacillales;f_Lactobacillaceae;g_Lactobacillus;s_iners |
| 0 | 0 | 0 | k_Bacteria;p_Firmicutes;c_Bacilli;o_Lactobacillales;f_Lactobacillaceae;g_Lactobacillus;s_reuteri |
| 0 | 0 | 0 | k_Bacteria;p_Firmicutes;c_Bacilli;o_Lactobacillales;f_Lactobacillaceae;g_Lactobacillus;s_zeae |
| 0 | 0 | 0 | k_Bacteria;p_Firmicutes;c_Bacilli;o_Lactobacillales;f_Leuconostocaceae;g_;s_ |
| 0 | 0 | 0 | k_Bacteria;p_Firmicutes;c_Bacilli;o_Lactobacillales;f_Leuconostocaceae;g_Leuconostoc;s_ |
| 0 | 0 | 0 | k_Bacteria;p_Firmicutes;c_Bacilli;o_Lactobacillales;f_Leuconostocaceae;g_Weissella;s_cibaria |
| 0.000247226 | 9.28E-05 | 4.54E-05 | k_Bacteria;p_Firmicutes;c_Bacilli;o Lactobacillales;f_Streptococcaceae;g_Lactococcus;s_ |
| 0 | 0 | 0 | k_Bacteria;p_Firmicutes;c_Bacilli;o_Lactobacillales;f_Streptococcaceae;g_Streptococcus;s_ |
| 0 | 0 | 0 | k_Bacteria;p_Firmicutes;c_Bacilli;o_Lactobacillales;f_Streptococcaceae;g_Streptococcus;s_infantis |
| 0.049417646 | 0.103774023 | 0.045335935 | k_Bacteria;p_Firmicutes;c_Bacilli;o_Turicibacterales;f_Turicibacteraceae;g_Turicibacter;s_ |
| 0 | 4.64E-05 | 0 | k_Bacteria;p_Firmicutes;c_Clostridia;o_Clostridiales;f_;g_;s_ |

(continued)

| two weeks after flight | one day after flight | one day before flight | Taxon |
|---|---|---|---|
| 0.001181189 | 0.001717575 | 0.000521885 | k_Bacteria;p_Firmicutes;c_Clostridia;o_Clostridiales;f_Christensenellaceae;g_Christensenella;s_ |
| 0 | 0 | 0 | k_Bacteria;p_Firmicutes;c_Clostridia;o_Clostridiales;f_Clostridiaceae;g_;s_ |
| 5.49E-05 | 4.64E-05 | 0 | k_Bacteria;p_Firmicutes;c_Clostridia;o_Clostridiales;f_Clostridiaceae;g_02d06;s_ |
| 0.030655972 | 0.231733358 | 0.028885208 | k_Bacteria;p_Firmicutes;c_Clostridia;o_Clostridiales;f_Clostridiaceae;g_Candidatus_Arthromitus;s_ |
| 2.75E-05 | 2.32E-05 | 2.27E-05 | k_Bacteria;p_Firmicutes;c_Clostridia;o_Clostridials;f_Clostridiaceae;g_Clostridium;s_ |
| 0 | 0 | 0 | k_Bacteria;p_Firmicutes;c_Clostridia;o_Clostridiales;f_Clostridiaceae;g_Clostridium;s_hiranonis |
| 0.001758049 | 0.001206945 | 0.000953008 | k_Bacteria;p_Firmicutes;c_Clostridia;o_Clostridiales;f_Clostridiaceae;g_Clostridium;s_perfringens |
| 2.75E-05 | 2.32E-05 | 6.81E-05 | k_Bacteria;p_Firmicutes;c_Clostridia;o_Clostridiales;f_Clostridiaceae;g_SMB53;s_ |
| 0.180199978 | 0.168183084 | 0.156951283 | k_Bacteria;p_Firmicutes;c_Clostridia;o_Clostridiales;f_Dehalobacteriaceae;g_Dehalobacterium;s_ |
| 0.004917042 | 0.001160524 | 0.027614531 | k_Bacteria;p_Firmicutes;c_Clostridia;o_Clostridiales;f_Lachnospiraceae;g_;s_ |
| 0.011647072 | 0.006638195 | 0.027614531 | k_Bacteria;p_Firmicutes;c_Clostridia;o_Clostridiales;f_Lachnospiraceae;g_Anaerostipes;s_ |
| 0 | 0 | 0 | k_Bacteria;p_Firmicutes;c_Clostridia;o_Clostridiales;f_Lachnospiraceae;g_Blautia;s_ |
| 2.75E-05 | 2.32E-05 | 2.27E-05 | k_Bacteria;p_Firmicutes;c_Clostridia;o_Clostridiales;f_Lachnospiraceae;g_Blautia;s_producta |
| 0.000164817 | 0.000278526 | 0.000113453 | k_Bacteria;p_Firmicutes;c_Clostridia;o_Clostridiales;f_Lachnospiraceae;g_Butyrivibrio;s_ |
| 0.041698714 | 0.110110482 | 0.039436364 | k_Bacteria;p_Firmicutes;c_Clostridia;o_Clostridiales;f_Lachnospiraceae;g_Clostridium;s_citroniae |
| 0 | 0 | 0 | k_Bacteria;p_Firmicutes;c_Clostridia;o_Clostridiales;f_Lachnospiraceae;g_Coprococcus;s_ |
| 0.000247226 | 4.64E-05 | 0.000136144 | k_Bacteria;p_Firmicutes;c_Clostridia;o_Clostridiales;f_Lachnospiraceae;g_Coprococcus;s_catus |
| 0.014036919 | 0.027388358 | 0.01173107 | k_Bacteria;p_Firmicutes;c_Clostridia;o_Clostridiales;f_Lachnospiraceae;g_Coprococcus;s_eutactus |
| 0 | 0 | 4.54E-05 | k_Bacteria;p_Firmicutes;c_Clostridia;o_Clostridiales;f_Lachnospiraceae;g_Dorea;s_ |
| 0.005329085 | 0.00392257 | 0.001520274 | k_Bacteria;p_Firmicutes;c_Clostridia;o_Clostridiales;f_Lachnospiraceae;g_Dorea;s_formicigenerans |
| 0.011399846 | 0.002831678 | 0.00308593 | k_Bacteria;p_Firmicutes;c_Clostridia;o_Clostridiales;f_Lachnospiraceae;g_Lachnobacterium;s_ |
| 0.002582134 | 0.000858787 | 0.00240521 | k_Bacteria;p_Firmicutes;c_Clostridia;o_Clostridiales;f_Lachnospiraceae;g_Lachnospira;s_ |

(continued)

| two weeks after flight | one day after flight | one day before flight | Taxon |
|---|---|---|---|
| 0.011399846 | 0.010630396 | 0.006671054 | k_Bacteria;p_Firmicutes;c_Clostridia;o_Clostridiales;f_Lachnospiraceae;g_Roseburia;s_ |
| 0.044006153 | 0.056076502 | 0.117446847 | k_Bacteria;p Firmicutes;c_Clostridia;o_Clostridiales;f_Lachnospiraceae;g_[Ruminococcus];s_ |
| 0 | 0 | 0 | k_Bacteria;p_Firmicutes;c_Clostridia;o_Clostridiales;f_Lachnospiraceae;g_[Ruminococcus];s_gnavus |
| 0 | 2.32E-05 | 4.54E-05 | k_Bacteria;p_Firmicutes;c_Clostridia;o_Clostridiales;f_Peptococcaceae;g_;s_ |
| 0.001236128 | 0.000394578 | 0.000158835 | k_Bacteria;p_Firmicutes;c_Clostridia;o_Clostridiales;f_Peptococcaceae;g_rc4-4;s_ |
| 0 | 0 | 0 | k_Bacteria;p_Firmicutes;c_Clostridia;o_Clostridiales;f_Peptostreptococcaceae;g_;s_ |
| 0.099192396 | 0.028293566 | 0.049216038 | k_Bacteria;p_Firmicutes;c_Clostridia;o_Clostridiales;f_Peptostreptococcaceae;g_Filifactor;s_ |
| 0 | 0 | 0 | k_Bacteria;p_Firmicutes;c_Clostridia;o_Clostridiales;f_Ruminococcaceae;g_;s_ |
| 0.002115152 | 6.96E-05 | 0.00102108 | k_Bacteria;p_Firmicutes;c_Clostridia;o_Clostridiales;f_Ruminococcaceae;g_Faecalibacterium;s_ |
| 0.000109878 | 9.28E-05 | 0.000499194 | k_Bacteria;p_Firmicutes;c_Clostridia;o_Clostridiales;f_Ruminococcaceae;g_Faecalibacterium;s_prausnitzii |
| 0.013734754 | 0.001439049 | 0.010664609 | k_Bacteria;p_Firmicutes;c_Clostridia;o_Clostridiales;f_Ruminococcaceae;g_Oscillospira;s_ |
| 0.000164817 | 0 | 0.000113453 | k_Bacteria;p_Firmicutes;c_Clostridia;o_Clostridiales;f_Ruminococcaceae;g_Ruminococcus;s_ |
| 0 | 4.64E-05 | 0 | k_Bacteria;p_Firmicutes;c_Clostridia;o_Clostridiales;f_Veillonellaceae;g_Dialister;s_ |
| 0.005823536 | 0.061647015 | 0.008327472 | k_Bacteria;p_Firmicutes;c_Clostridia;o_Clostridiales;f_Veillonellaceae;g_Megasphaera;s_ |
| 2.75E-05 | 0.000278526 | 0 | k_Bacteria;p_Firmicutes;c_Clostridia;o_Clostridiales;f_Veillonellaceae;g_Phascolarctobacterium;s_ |
| 0.000192287 | 0.000371368 | 6.81E-05 | k_Bacteria;p_Firmicutes;c_Clostridia;o_Clostridiales;f_Veillonellaceae;g_Veillonella;s_dispar |
| 0.000274695 | 6.96E-05 | 0.000181525 | k_Bacteria;p_Firmicutes;c_Clostridia;o_Clostridiales;f_Veillonellaceae;g_Veillonella;s_parvula |
| 0 | 0 | 0 | k_Bacteria;p_Firmicutes;c_Clostridia;o_Clostridiales;f_[Mogibacteriaceae];g_;s_ |
| 0 | 0 | 0 | k_Bacteria;p_Firmicutes;c_Clostridia;o_Clostridiales;f_[Tissierellaceae];g_Peptoniphilus;s_ |
| 0.005933414 | 0.000603472 | 0.003721268 | k_Bacteria;p_Finnicutes;c_Clostridia;o_SHA-98;f_;g_;s_ |
| 8.24E-05 | 0 | 0 | k_Bacteria;p_Firmicutes;c_Erysipelotrichi;o_Erysipelotrichales;f_Erysipelotrichaceae;g_;s_ |
| 0 | 0 | 0 | k_Bacteria;p_Firmicutes;c_Erysipelotrichi;o_Erysipelotrichales;f_Erysipelotrichaceae;g_Allobaculum;s_ |

(continued)

| two weeks after flight | one day after flight | one day before flight | Taxon |
|---|---|---|---|
| 0 | 0 | 0 | k_Bacteria;p_Firmicutes;c_Erysipelotrichi;o_Erysipelotrichales; f_Erysipelotrichaceae;g_Bulleidia;s_moorei |
| 0 | 0 | 0 | k_Bacteria;p_Firmicutes;c_Erysipelotrichi;o_Erysipelotrichales; f_Erysipelotrichaceae;g_Bulleidia;s_p-1630-c5 |
| 8.24E-05 | 0 | 2.27E-05 | k_Bacteria;p_Firmicutes;c_Erysipelotrichi;o_Erysipelotrichales; f_Erysipelotrichaceae;g_Coprobacillus;s_ |
| 0 | 0 | 0 | k_Bacteria;p_Firmicutes;c_Erysipelotrichi;o_Erysipelotrichales; f_Erysipelotrichaceae;g_[Eubacterium];s_biforme |
| 0 | 0 | 0 | k_Bacteria;p_Firmicutes;c_Erysipelotrichi;o_Erysipelotrichales; f_Erysipelotrichaceae;g_[Eubacteriutn];s_cyl indroides |
| 0.000109878 | 0.000116052 | 0.000204216 | k_Bacteria;p_Fusobacteria;c_Fusobacteriia;o_Fusobacteriales; f_Fusobacteriaceae;g_Fusobacteriutn;s_ |
| 8.24E-05 | 9.28E-05 | 0.000136144 | k_Bacteria;p_Proteobacteria;c_Alphaproteobacteria;o_ Caulobacterales;f_Caulobacteraceae;g_;s_ |
| 0 | 0 | 2.27E-05 | k_Bacteria;p_Proteobacteria;c_Alphaproteobacteria;o_RF32;f_; g_;s_ |
| 0 | 0 | 0 | k_Bacteria;p_Proteobacteria;c_Alphaproteobacteria;o_ Rhizobiales;f_Brucellaceae;g_Ochrobactrum;s_ |
| 2.75E-05 | 0 | 0 | k_Bacteria;p_Proteobacteria;c_Alphaproteobacteria;o_ Rhizobiales;f_Rhizobiaceae;g_;s_ |
| 0 | 2.32E-05 | 2.27E-05 | k_Bacteria;p_Proteobacteria;c_Alphaproteobacteria;o_ Rhizobiales;f_Rhizobiaceae;g_Agrobacteriutn;s_ |
| 2.75E-05 | 6.96E-05 | 0 | k_Bacteria;p_Proteobacteria;c_Alphaproteobacteria;o_ Rhodobacterales;f_Rhodobacteraceae;g_;s_ |
| 2.75E-05 | 0.002297837 | 4.54E-05 | k_Bacteria;p_Proteobacteria;c_Betaproteobacteria;o_ Burkholderiales;f_Alcaligenaceae;g_Sutterella;s_ |
| 0 | 6.96E-05 | 0 | k_Bacteria;p_Proteobacteria;c_Betaproteobacteria;o_ Burkholderiales;f_Comamonadaceae;g_Comamonas;s_ |
| 2.75E-05 | 0 | 2.27E-05 | k_Bacteria;p_Proteobacteria;c_Betaproteobacteria;o_ Burkholderiales;f_Oxalobacteraceae;g_Oxalobacter;s_ formigenes |
| 0 | 0 | 0 | k_Bacteria;p_Proteobacteria;c_Betaproteobacteria;o_ Burkholderiales;f_Oxalobacteraceae;g_Ralstonia;s_ |
| 0 | 0 | 0 | k_Bacteria;p_Proteobacteria;c_Betaproteobacteria;o_ Methylophilales;f_Methylophilaceae;g_;s_ |
| 0 | 0 | 0 | k_Bacteria;p_Proteobacteria;c_Betaproteobacteria;o_ Neisseriales;f_Neisseriaceae;g_Neisseria;s_subflava |
| 0 | 0 | 0 | k_Bacteria;p_Proteobacteria;c_Deltaproteobacteria;o_ Desulfovibrionales;f_Desulfovibrionaceae;g_;s_ |
| 0 | 0 | 0 | k_Bacteria;p_Proteobacteria;c_Deltaproteobacteria;o_ Desulfovibrionales;f_Desulfovibrionaceae;g_Bilophila;s |
| 0 | 0 | 0 | k_Bacteria;p_Proteobacteria;c_Epsilonproteobacteria;o_ Campylobacterales;f_Campylobacteraceae;g_Campyl obacter; s_ |

(continued)

| two weeks after flight | one day after flight | one day before flight | Taxon |
|---|---|---|---|
| 0 | 0 | 0 | k_Bacteria;p_Proteobacteria;c_Epsilonproteobacteria;o_Campylobacterales;f_Helicobacteraceae;g_Helicobacter;s_apodemus |
| 2.75E-05 | 0 | 0 | k_Bacteria;p_Proteobacteria;c_Gammaproteobacteria;o_Enterobacteriales;f_Enterobacteriaceae;g_;s_ |
| 0.000109878 | 0 | 2.27E-05 | k_Bacteria;p_Proteobacteria;c_Gammaproteobacteria;o_Pasteurellales;f_Pasteurellaceae;g_Haemophilus;s_parainfluenzae |
| 0 | 6.96E-05 | 0 | k_Bacteria;p_Proteobacteria;c_Gammaproteobacteria;o_Pseudomonadales;f_Pseudomonadaceae;g_;s_ |
| 0.000109878 | 4.64E-05 | 6.81E-05 | k_Bacteria;p_TM7;c_TM7-3;o_;f_;g_;s_ |
| 0 | 0 | 0 | k_Bacteria;p_Tenericutes;c_Mollicutes;o_Anaeroplasmatales;f_Anaeroplasmataceae;g_Anaeroplasma;s_ |
| k Bacteria;p_Tenericutes;c_Mollicutes;o_RF39;f_;g_;s_ | | | |

## *Discussion*

In this example, the present inventors describe that the mammalian gut microbiota displays diurnal oscillations which are governed by food consumption rhythmicity. If rhythmic feeding times are distorted, as in the case of genetic clock deficiency or time shift-induced jet lag, then microbiota oscillations are impaired **(Figure 14)**. Chronic circadian misalignment in mice and time shift-induced jet lag in humans results in dysbiosis and transmissible metabolic consequences including obesity and glucose intolerance. These observations provide the first example of how a symbiotic community may synchronize its interdependent physiologic activities to the geophysical clock, and how this promotes homeostasis of the meta-organism.

Previous studies looking at temporal fluctuations in the microbiota have considered longer time frames, and found a remarkable stability of individual microbial compositions over time (Faith et al., 2013; Lozupone et al., 2012). Here, the present inventors performed the longitudinal microbiota study with a finer temporal resolution and found an hour-scale fluctuation with a diurnal rhythm. Notably, the analysis here focuses on the diurnal variations in microbial community composition and metagenomic pathways. Since molecular components of bacterial circadian clocks have also been described to function on the transcriptional and post-transcriptional level (Lenz and Sogaard-Andersen, 2011), it is possible that some members of the commensal microbiota harbor yet another level of time-dependent activity control, which, in addition to the patterns in relative abundance, might regulate bacterial activity in a rhythmic manner.

These results have several implications. First, they suggest that the metabolic imbalances associated with chronic disturbances of host circadian rhythms, such as the ones found in shift workers and during jet lag, have a communicable component that depends on the composition of the microbiota and its effect on host metabolism. The highly multifactorial morbidities associated with disruptions of host circadian rhythms are emerging diseases of the modem life style, and the underlying etiology is poorly understood. The present study identifies alterations in intestinal microbial communities as an additional driving force of such disease manifestations and implies that targeted probiotic or antimicrobial therapy may be tested as potential new preventive or therapeutic approaches in susceptible populations. In addition, the results yield new insight into earlier studies on mice with deficiencies in the circadian clock (Karatsoreos et al., 2011; Rudic et al., 2004; Turek et al., 2005), as some of the discovered phenotypes might not be mediated solely by the genetic deficiency, but may additionally be influenced by changes in the characteristics of the microbiota and downstream metabolic and inflammatory consequences. The results presented here may thus prompt future studies to determine the impact of circadian misalignment on factors shaping the microbiota, including immune and metabolic pathways of the host, eating patterns, stress hormone levels, and bowel movement.

Second, the present study reveals that, in addition to the type of diet being a modulator of microbiota composition, the timing of food intake plays a critical role in shaping intestinal microbial ecology. When food intake is rhythmic, it was found that up to 15% of commensal bacterial taxonomic units (and a much higher percentage of abundance) fluctuate over the course of a day. In host peripheral tissues such as the liver, a similar proportion of all transcripts oscillate in a rhythmic manner (Akhtar et al., 2002; McCarthy et al., 2007; Panda et al., 2002; Storch et al., 2002; Vollmers et al.,

2009). Analogous to peripheral clocks, the microbiota rhythms are influenced by the host clock and perform critical functions in the adaptation of metabolic processes to the diurnal fluctuations in the environment. Indeed, recent work has shown that cues from the microbiota play an important role in the generation of circadian rhythms in intestinal epithelial cells (Mukherji et al., 2013). Together, this recent work and the present study suggest an emerging new paradigm whereby a feedback loop between diurnal oscillations of the host and the microbiota with mutual cross-regulation of interdependent functions.

The present observation that food rhythmicity directs microbiota oscillations might also be noteworthy in another regard. It is a well-established concept that, in addition to being synchronized to the light-sensitive central oscillator, peripheral clocks are entrained by feeding rhythms. Based on the well-documented role of the microbiota as a modulator of host gene expression, these results raise the possibility that the microbiota might be involved in this process of food entrainment and might suggest an additional mechanistic explanation for earlier observations of the beneficial metabolic effects associated with timed feeding (Adamovich et al., 2014; Damiola et al., 2000; Vollmers et al., 2009). In such a scenario, rhythmic food intake may govern microbiota oscillations, which in turn causes rhythmic induction of host gene expression. The impact of the microbiota and its oscillations on host circadian behavior and gene expression thus presents an exciting field of future research.

In addition, the diurnal fluctuations in intestinal microbial ecology discovered here should be taken into account when interpreting studies focusing on human and animal microbiota composition. For instance, based on the present results, it might be advisable that human subjects involved in microbiota studies provide their samples at a standardized time of the day in order to exclude the effect of diurnal variations on the interpretation of diet or treatment modalities. The present study reveals that dysbiosis has a temporal dimension, and that static microbiota comparisons might not be fully conclusive, unless samples were taken in a controlled manner with respect to this important additional variable. Short-term rhythmic oscillations in the microbiota, such as the ones described in this study, may be exaggerated or disrupted under various disease conditions, and it will be interesting to determine the impact of such "temporal dysbiosis" on microbiota-mediated diseases with different manifestations or varying degrees of severity at different phases of the day.

Finally, the network of co-dependent diurnal rhythms, which the host and its indigenous microbiota have evolved might confer several biological advantages to the meta-organism. A dynamic microbiota composition may be able to meet the challenges imposed by diurnal fluctuations in the environment better than a temporally static composition. As demonstrated in this study, food intake by the host undergoes circadian fluctuations, which evoke temporal changes in the bacterial species involved in nutrient metabolism. Thus, oscillations in components of the microbiota might anticipate these temporal variations in nutrient availability. The metagenomic analysis suggests that certain categories of bacterial functions feature temporal predilections of the course of a day (Figure 14). It was found that pathways involved in growth and energy metabolism (such as nucleic acid repair, nucleotide metabolism, carbohydrate and amino acid metabolism) are anti-phasic to motility and detoxification pathways (including flagellar assembly, chemotaxis, and xenobiotics degradation). Since the taxonomic analysis indicates that microbiota oscillations are following rhythmic food intake, such metagenomic fluctuations might be the result of rhythmic niche occupation by specialists which are responsive to phases of food intake/starvation. Moreover, the microbiota provides colonization resistance against foreign microbial elements including enteric pathogens (Stecher and Hardt, 2011) that are potentially introduced by food consumption during waking hours. As such, the introduction of foreign microbial elements into the intestinal microbiota underlies daily fluctuations, generating the need for diurnal rhythmicity of niche occupation by the commensal microbiota. Unraveling the roles and regulators of diurnal microbiota oscillations may add an important facet to our quest for molecular elucidation of the principles of symbiotic co-existence of host with its microbial milieu in health and disease, and modulation of microbiota rhythmicity may consequently be exploited therapeutically.

*References for Example 1*

Adamovich, Y., Rousso-Noori, L., Zwighaft, Z., Neufeld-Cohen, A., Golik, M., Kraut-Cohen, J., Wang, M., Han, X., and Asher, G. (2014). Circadian clocks and feeding time regulate the oscillations and levels of hepatic triglycerides. Cell metabolism 19, 319-330.

Akhtar, R.A., Reddy, A.B., Maywood, E.S., Clayton, J.D., King, V.M., Smith, A.G., Gant, T.W., Hastings, M.H., and Kyriacou, C.P. (2002). Circadian cycling of the mouse liver transcriptome, as revealed by cDNA microarray, is driven by the suprachiasmatic nucleus. Current biology : CB 12, 540-550.

Archer, S.N., Laing, E.E., Moller-Levet, C.S., van der Veen, D.R., Bucca, G., Lazar, A.S., Santhi, N., Slak, A., Kabiljo, R., von Schantz, M., et al. (2014). From the Cover: Mistimed sleep disrupts circadian regulation of the human transcriptome. Proceedings of the National Academy of Sciences of the United States of America 111, E682-691.

Asher, G., Reinke, H., Altmeyer, M., Gutierrez-Arcelus, M., Hottiger, M.O., and Schibler, U. (2010). Poly(ADP-ribose) polymerase 1 participates in the phase entrainment of circadian clocks to feeding. Cell 142, 943-953.

Bass, J. (2012). Circadian topology of metabolism. Nature 491, 348-356.

Buxton, O.M., Cain, S.W., O'Connor, S.P., Porter, J.H., Duffy, J.F., Wang, W., Czeisler, C.A., and Shea, S.A. (2012). Adverse metabolic consequences in humans of prolonged sleep restriction combined with circadian disruption. Science translational medicine 4, 129ra143.

Caporaso, J.G., Kuczynski, J., Stombaugh, J., Bittinger, K., Bushman, F.D., Costello, E.K., Fierer, N., Pena, A.G., Goodrich, J.K., Gordon, J.I., et al. (2010). QIIME allows analysis of high-throughput community sequencing data. Nature methods 7, 335-336.

Clemente, J.C., Ursell, L.K., Parfrey, L.W., and Knight, R. (2012). The impact of the gut microbiota on human health: an integrative view. Cell 148, 1258-1270.

Damiola, F., Le Minh, N., Preitner, N., Kornmann, B., Fleury-Olela, F., and Schibler, U. (2000). Restricted feeding uncouples circadian oscillators in peripheral tissues from the central pacemaker in the suprachiasmatic nucleus. Genes & development 14, 2950-2961.

Dibner, C., Schibler, U., and Albrecht, U. (2010). The mammalian circadian timing system: organization and coordination of central and peripheral clocks. Annual review of physiology 72, 517-549.

Edgar, R.S., Green, E.W., Zhao, Y., van Ooijen, G., Olmedo, M., Qin, X., Xu, Y., Pan, M., Valekunja, U.K., Feeney, K.A., et al. (2012). Peroxiredoxins are conserved markers of circadian rhythms. Nature 485, 459-464.

Elinav, E., Strowig, T., Kau, A.L., Henao-Mejia, J., Thaiss, C.A., Booth, C.J., Peaper, D.R., Bertin, J., Eisenbarth, S.C., Gordon, J.I., et al. (2011). NLRP6 inflammasome regulates colonic microbial ecology and risk for colitis. Cell 145, 745-757.

Fagarasan, S., Muramatsu, M., Suzuki, K., Nagaoka, H., Hiai, H., and Honjo, T. (2002). Critical roles of activation-induced cytidine deaminase in the homeostasis of gut flora. Science 298, 1424-1427.

Faith, J.J., Guruge, J.L., Charbonneau, M., Subramanian, S., Seedorf, H., Goodman, A.L., Clemente, J.C., Knight, R., Heath, A.C., Leibel, R.L., et al. (2013). The long-term stability of the human gut microbiota. Science 341, 1237439.

Fonken, L.K., Workman, J.L., Walton, J.C., Weil, Z.M., Morris, J.S., Haim, A., and Nelson, R.J. (2010). Light at night increases body mass by shifting the time of food intake. Proceedings of the National Academy of Sciences of the United States of America 107, 18664-18669.

Gerhart-Hines, Z., Feng, D., Emmett, M.J., Everett, L.J., Loro, E., Briggs, E.R., Bugge, A., Hou, C., Ferrara, C., Seale, P., et al. (2013). The nuclear receptor Rev-erbalpha controls circadian thermogenic plasticity. Nature 503, 410-413.

Gordon, J.I. (2012). Honor thy gut symbionts redux. Science 336, 1251-1253. Henao-Mejia, J., Elinav, E., Jin, C., Hao, L., Mehal, W.Z., Strowig, T., Thaiss, C.A., Kau, A.L., Eisenbarth, S.C., Jurczak, M.J., et al. (2012). Inflammasome-mediated dysbiosis regulates progression of NAFLD and obesity. Nature 482, 179-185.

Hogenesch, J.B., and Ueda, H.R. (2011). Understanding systems-level properties: timely stories from the study of clocks. Nature reviews Genetics 12, 407-416.

Hoogerwerf, W.A., Hellmich, H.L., Cornelissen, G., Halberg, F., Shahinian, V.B., Bostwick, J., Savidge, T.C., and Cassone, V.M. (2007). Clock gene expression in the murine gastrointestinal tract: endogenous rhythmicity and effects of a feeding regimen. Gastroenterology 133, 1250-1260.

Hooper, L.V., Littman, D.R., and Macpherson, A.J. (2012). Interactions between the microbiota and the immune system. Science 336, 1268-1273.

Hsiao, E.Y., McBride, S.W., Hsien, S., Sharon, G., Hyde, E.R., McCue, T., Codelli, J.A., Chow, J., Reisman, S.E., Petrosino, J.F., et al. (2013). Microbiota modulate behavioral and physiological abnormalities associated with neurodevelopmental disorders. Cell 155, 1451-1463.

Huang, W., Ramsey, K.M., Marcheva, B., and Bass, J. (2011). Circadian rhythms, sleep, and metabolism. The Journal of clinical investigation 121, 2133-2141.

Hughes, M.E., Hogenesch, J.B., and Kornacker, K. (2010). JTK_CYCLE: an efficient nonparametric algorithm for detecting rhythmic components in genome-scale data sets. Journal of biological rhythms 25, 372-380.

Human Microbiome Project, C. (2012). Structure, function and diversity of the healthy human microbiome. Nature 486, 207-214.

Johnson, C.H., Egli, M., and Stewart, P.L. (2008). Structural insights into a circadian oscillator. Science 322, 697-701.

Johnson, C.H., Stewart, P.L., and Egli, M. (2011). The cyanobacterial circadian system: from biophysics to bioevolution. Annual review of biophysics 40, 143-167.

Kaasik, K., Kivimae, S., Allen, J.J., Chalkley, R.J., Huang, Y., Baer, K., Kissel, H., Burlingame, A.L., Shokat, K.M., Ptacek, L.J., et al. (2013). Glucose sensor O-GlcNAcylation coordinates with phosphorylation to regulate circadian clock. Cell metabolism 17, 291-302.

Kanehisa, M., and Goto, S. (2000). KEGG: kyoto encyclopedia of genes and genomes. Nucleic acids research 28, 27-30.

Kanehisa, M., Goto, S., Sato, Y., Kawashima, M., Furumichi, M., and Tanabe, M. (2014). Data, information, knowledge and principle: back to metabolism in KEGG. Nucleic acids research 42, D199-D205.

Karatsoreos, I.N., Bhagat, S., Bloss, E.B., Morrison, J.H., and McEwen, B.S. (2011). Disruption of circadian clocks has ramifications for metabolism, brain, and behavior. Proceedings of the National Academy of Sciences of the United States of America 108, 1657-1662.

Keller, M., Mazuch, J., Abraham, U., Eom, G.D., Herzog, E.D., Volk, H.D., Kramer, A., and Maier, B. (2009). A circadian clock in macrophages controls inflammatory immune responses. Proceedings of the National Academy of Sciences of the United States of America 106, 21407-21412.

Lenz, P., and Sogaard-Andersen, L. (2011). Temporal and spatial oscillations in bacteria. Nature reviews Microbiology 9, 565-577.

Ley, R.E., Turnbaugh, P.J., Klein, S., and Gordon, J.I. (2006). Microbial ecology: human gut microbes associated with obesity. Nature 444, 1022-1023.

Lozupone, C.A., Stombaugh, J.I., Gordon, J.I., Jansson, J.K., and Knight, R. (2012). Diversity, stability and resilience of the human gut microbiota. Nature 489, 220-230.

McCarthy, J.J., Andrews, J.L., McDearmon, E.L., Campbell, K.S., Barber, B.K., Miller, B.H., Walker, J.R., Hogenesch, J.B., Takahashi, J.S., and Esser, K.A. (2007). Identification of the circadian transcriptome in adult mouse skeletal muscle. Physiological genomics 31, 86-95.

Mohawk, J.A., Green, C.B., and Takahashi, J.S. (2012). Central and peripheral circadian clocks in mammals. Annual review of neuroscience 35, 445-462.

Mukherji, A., Kobiita, A., Ye, T., and Chambon, P. (2013). Homeostasis in intestinal epithelium is orchestrated by the circadian clock and microbiota cues transduced by TLRs. Cell 153, 812-827.

Nguyen, K.D., Fentress, S.J., Qiu, Y., Yun, K., Cox, J.S., and Chawla, A. (2013). Circadian gene Bmal1 regulates diurnal oscillations of Ly6C(hi) inflammatory monocytes. Science 341, 1483-1488.

Panda, S., Antoch, M.P., Miller, B.H., Su, A.I., Schook, A.B., Straume, M., Schultz, P.G., Kay, S.A., Takahashi, J.S., and Hogenesch, J.B. (2002). Coordinated transcription of key pathways in the mouse by the circadian clock. Cell 109, 307-320.

Qin, J., Li, R., Raes, J., Arumugam, M., Burgdorf, K.S., Manichanh, C., Nielsen, T., Pons, N., Levenez, F., and Yamada, T. (2010). A human gut microbial gene catalogue established by metagenomic sequencing. Nature 464, 59-65.

Rakoff-Nahoum, S., Paglino, J., Eslami-Varzaneh, F., Edberg, S., and Medzhitov, R. (2004). Recognition of commensal microflora by toll-like receptors is required for intestinal homeostasis. Cell 118, 229-241.

Ridaura, V.K., Faith, J.J., Rey, F.E., Cheng, J., Duncan, A.E., Kau, A.L., Griffin, N.W., Lombard, V., Henrissat, B., Bain, J.R., et al. (2013). Gut microbiota from twins discordant for obesity modulate metabolism in mice. Science 341, 1241214.

Rudic, R.D., McNamara, P., Curtis, A.M., Boston, R.C., Panda, S., Hogenesch, J.B., and Fitzgerald, G.A. (2004). BMAL1 and CLOCK, two essential components of the circadian clock, are involved in glucose homeostasis. PLoS biology 2, e377.

Rust, M.J., Markson, J.S., Lane, W.S., Fisher, D.S., and O'Shea, E.K. (2007). Ordered phosphorylation governs oscillation of a three-protein circadian clock. Science 318, 809-812.

Scheer, F.A., Hilton, M.F., Mantzoros, C.S., and Shea, S.A. (2009). Adverse metabolic and cardiovascular consequences of circadian misalignment. Proceedings of the National Academy of Sciences of the United States of America 106, 4453-4458.

Silver, A.C., Arjona, A., Walker, W.E., and Fikrig, E. (2012). The circadian clock controls toll-like receptor 9-mediated innate and adaptive immunity. Immunity 36, 251-261.

So, A.Y., Bemal, T.U., Pillsbury, M.L., Yamamoto, K.R., and Feldman, B.J. (2009). Glucocorticoid regulation of the circadian clock modulates glucose homeostasis. Proceedings of the National Academy of Sciences of the United States of America 106, 17582-17587.

Sommer, F., and Backhed, F. (2013). The gut microbiota-masters of host development and physiology. Nature reviews Microbiology 11, 227-238.

Stecher, B., and Hardt, W.D. (2011). Mechanisms controlling pathogen colonization of the gut. Current opinion in microbiology 14, 82-91.

Stokkan, K.A., Yamazaki, S., Tei, H., Sakaki, Y., and Menaker, M. (2001). Entrainment of the circadian clock in the liver by feeding. Science 291, 490-493.

Storch, K.F., Lipan, O., Leykin, I., Viswanathan, N., Davis, F.C., Wong, W.H., and Weitz, C.J. (2002). Extensive and divergent circadian gene expression in liver and heart. Nature 417, 78-83.

Suwazono, Y., Dochi, M., Sakata, K., Okubo, Y., Oishi, M., Tanaka, K., Kobayashi, E., Kido, T., and Nogawa, K. (2008). A longitudinal study on the effect of shift work on weight gain in male Japanese workers. Obesity 16, 1887-1893.

Turek, F.W., Joshu, C., Kohsaka, A., Lin, E., Ivanova, G., McDearmon, E., Laposky, A., Losee-Olson, S., Easton, A., Jensen, D.R., et al. (2005). Obesity and metabolic syndrome in circadian Clock mutant mice. Science 308, 1043-1045.

Vollmers, C., Gill, S., DiTacchio, L., Pulivarthy, S.R., Le, H.D., and Panda, S. (2009). Time of feeding and the intrinsic circadian clock drive rhythms in hepatic gene expression. Proceedings of the National Academy of Sciences of the United States of America 106, 21453-21458.

Yamaguchi, Y., Suzuki, T., Mizoro, Y., Kori, H., Okada, K., Chen, Y., Fustin, J.M., Yamazaki, F., Mizuguchi, N., Zhang, J., et al. (2013). Mice genetically deficient in vasopressin V1a and V1b receptors are resistant to jet lag. Science 342, 85-90.

Yu, X., Rollins, D., Ruhn, K.A., Stubblefield, J.J., Green, C.B., Kashiwada, M., Rothman, P.B., Takahashi, J.S., and Hooper, L.V. (2013). TH17 cell differentiation is regulated by the circadian clock. Science 342, 727-730.

## EXAMPLE 2

### *Artificial sweeteners induce glucose intolerance by altering the microbiota*

### MATERIALS AND METHODS

*Mice* - C57B1/6 WT adult male mice were randomly assigned (without blinding) to treatment groups and were given commercial artificial sweeteners (saccharin-, sucralose- or aspartame-based) or pure saccharin (Sigma Aldrich) in drinking water and fed a high-fat (HFD D12492, 60% Kcal from fat, Research Diets) or standard polysaccharide normal chow (NC) diet (Harlan-Teklad). Compared groups were always fed from the same batch of diet. For antibiotic treatment, mice were given a combination of ciprofloxacin ($0.2gl^{-1}$) and metronidazole ($1gl^{-1}$) or vancomycin ($0.5gl^{-1}$) in their drinking water. All antibiotics were obtained from Sigma Aldrich. Adult male outbred Swiss-Webster mice (a widely used mouse strain in germ-free experiments) served as recipients for fecal transplants and were housed in sterile isolators (Park Biosciences). For fecal transplantation experiments, 200mg of stool (from mouse pellets or human swabs) was resuspended in 5ml of PBS under anaerobic conditions, vortexed for 3 minutes and allowed to settle by gravity for 2 minutes. Recipient mice were gavaged with $200\mu l$ of the supernatant and maintained on standard NC diet and water throughout the experiment.

*Artificial and caloric sweeteners* - The following commercially available NAS were dissolved in mice drinking water to obtain a 10% solution: Sucrazit (5% saccharin, 95% glucose), Sucralite (5% Sucralose), Sweet'n Low Gold (4% Aspartame). 10% glucose (J.T. Baker) and 10% sucrose (Sigma Aldrich) solutions were used for controls. The administered doses of 10% commercial NAS dissolved in water were well below their reported toxic dose ($6.3gkg^{-1}$ [51], $16gkg^{-1}$ [52], and $4gkg^{-1}$ [53], for saccharin, sucralose and aspartame, respectively). For experiments conducted with pure saccharin (Sigma Aldrich) a $0.1$ $mgml^{-1}$ solution was used in order to meet with FDA defined ADI for saccharin in humans ($5$ $mgkg^{-1}$), according to the following calculation:

$$\frac{ADI\ 5mgkg^{-1}day^{-1}\ X\ Average\ mouse\ weight\ 0.03kg}{Average\ daily\ liquid\ intake\ 2ml} = 0.075mgml^{-1} \to 0.1\ mgml^{-1}$$

*Glucose and insulin tolerance tests* - Mice were fasted for 6 hours during the light phase, with free access to water. In all groups of mice where the drinking regime was other than water, it was substituted for water for the period of the fasting and glucose or insulin tolerance test. Blood from the tail vein was used to measure glucose levels using a glucometer (Bayer) immediately before and 15, 30, 60, 90 and 120 minutes after oral feeding with 40mg glucose (J.T. Baker) or intra-peritoneal injection with $0.1Ukg^{-1}$ Insulin (Biological Industries). Plasma fasting insulin levels were measured in sera collected immediately before the start of GTT using ELISA (Ultra Sensitive Mouse Insulin ELISA Kit, Crystal Chem).

*Metabolic studies* - Food and drink intake and energy expenditure were measured using the PhenoMaster system (TSE-Systems, Bad Homburg, Germany), which consists of a combination of sensitive feeding sensors for automated measurement and a photobeam-based activity monitoring system detects and records ambulatory movements, including rearing and climbing, in each cage. All parameters were measured continuously and simultaneously. Mice were trained singly-housed in identical cages prior to data acquisition. To calculate total caloric intake, the following values were used: Chow $3kcalg^{-1}$, sucrose $0.3938kcalml^{-1}$, glucose $0.4kcalml^{-1}$, saccharin $0.38kcalml^{-1}$, sucralose $0.392kcalml^{-1}$ and aspartame $0.38kcalml^{-1}$.

*In vitro anaerobic culturing* - pooled fecal matter from naive adult WT C57B1/6 male mice was resuspended in 5 ml PBS in an anaerobic chamber (Coy Laboratory Products, 75% $N_2$, 20% $CO_2$, 5% $H_2$), vortexed for 3 minutes and allowed to settle by gravity for 2 minutes. 500 ml of the supernatant were added to a tube containing Chopped Meat Carbohydrate Broth, PR II (BD) and 500 ml of a $5mgml^{-1}$ saccharin solution or an equal volume of PBS. Every 3 days, 500 ml of culture were diluted to fresh medium containing saccharin or PBS. After 9 days, cultures were used for inoculation of germ-free mice.

*Taxonomic microbiota analysis* - Frozen fecal samples were processed for DNA isolation using the MoBio PowerSoil kit according to the manufacturer's instructions. Ing of the purified fecal DNA was used for PCR amplification and sequencing of the bacterial 16S rRNA gene. ~365bp Amplicons spanning the variable region 2 (V2) of the 16S rRNA gene were generated by using the following barcoded primers: Fwd 5'-AGAGTTTGATCCTGGCTCAG-3' (SEQ ID NO: 3), Rev 5'-TGCTGCCTCCCGTAGGAGT-3' (SEQ ID NO: 4). The reactions were subsequently pooled in an equimolar ratio, purified (PCR clean kit, Promega), and used for Illumina MiSeq sequencing to a depth of at least 18000 reads per sample (mean reads per sample 139148±5264 (SEM)). Reads were then processed using the QIIME (Quantitative Insights Into Microbial Ecology) analysis pipeline as described, version 1.8. Paired-end joined sequences were grouped into operational taxonomic units (OTUs) using the UCLUST algorithm and the greengenes database. Sequences with distance-based similarity of 97% or greater over median sequence length of 353bp were assigned to the same OTU. Samples were grouped according to the treatment. Analysis was performed at each taxonomical level (Phylum-genus + OTU level) separately. For each taxon, G test was performed between the different groups. P-values were FDR corrected for multiple hypothesis testing.

*Shotgun pyrosequencing and sequence mapping* - Was performed as previously described[57], with the following modifications: lug of DNA was sheared using the Covaris 5200 system (Covaris,Inc., Woburn, MA, USA), followed by end repair, ligation to adapters, an 8 cycle PCR amplification (Kappa HiFi) and sequenced using an Illumina HiSeq to a minimal depth of 11773345 reads per sample (mean reads per sample 20296086±637379 (SEM), read length 51 bp). Illumina sequence reads were mapped to the human microbiome reference genome database of the Human Microbiome Project [www.hmpdacc.org/HMREFG/[32]], and to a gut microbial gene catalogue[33] using GEM mapper[58] with the following parameters:

-m 3 -s 0 -q offset-33 -gem-quality-threshold 26

Microbial species abundance was measured as the fraction of reads that mapped to a single species in the database. An EM algorithm adapted from Pathoscope[59] was employed to determine the correct assignment of reads that mapped to more than one species. We considered only species for which at least 10% of the genome was covered (each coverage bin was 10,000-bp long) in at least one of the growth conditions (saccharin, water, or glucose). Reads mapped to the gut microbial gene catalogue were assigned a KEGG[34,35] ID according to the mapping available with the catalogue. Genes were subsequently mapped to KEGG pathways, and only pathways whose gene coverage was above 0.2 were included. To calculate the contribution of different bacteria to the overrepresentation of glycan degradation pathways, reads that were mapped to genes in the gut microbial gene catalogue that belong to glycan degrading pathways were extracted and re-mapped the HMP reference genome database, seeking germs that had the highest contribution.

*Short chain fatty acid quantification* - to determine the level of free fatty acids analytic HPLC (Agilent 1260) was performed as described previously[60]. In brief, standard solutions of Acetate, Butyrate and Propionate (all from Sigma-Aldrich) were prepared at various concentrations (0.01-0.2 M). These solutions were analyzed using HPLC, successive with QTOF-Mass Spec with a step-gradient of solvent solution from 0% to 60% of $CH_3CN$ with 0.1% formic acid to obtain calibration curve for each fatty acid. Fecal Media samples were dissolved with 0.1% formic acid analyzed in similar manner to measure the total concentration of all three free fatty acids.

### *Analysis of the relationship between NAS consumption and clinical parameters in humans:*

The trial was reported to clinical trials, identifier NCT01892956. The study did not necessitate or involve randomization. For each individual in the clinical nutritional study, after signing an informed consent, the parameters collected include BMI, body circumferences, fasting glucose levels, general questionnaire, complete blood counts and general chemistry parameters, a validated long-term food frequency questionnaire[44,61,62].

Long-term NAS consumption was quantified directly from answers to an explicit question regarding artificial sweeteners that participants filled out in their food frequency questionnaire. Spearman correlation was then used to examine the relationship between NAS consumption and each of the above parameters, and FDR corrected for the multiple hypotheses tests performed.

*Statistics* - The following statistical analyses were used: in GTT, a two-way ANOVA and Bonferroni post-hoc analysis were used to compare between groups in different time-points, and one-way ANOVA and Tukey post hoc analysis or unpaired two-sided Student *t-test* were used to compare between AUC of multiple or two groups, respectively. Bartlett's or F-test for equal variance were employed and no significant difference was observed between variances of the compared groups. For comparison of taxonomic data, a G-test was used and P-values were FDR corrected for multiple hypothesis testing. In metagenomics and clinical and taxonomic data from humans, Pearson and Spearman were used for correlation tests, and Mann-Whitney U was used to compare clinical parameters between groups. p<0.05 was considered significant in all analyses (* denotes p<0.05, **, p<0.01, ***, p<0.001). In all relevant panels, symbols or horizontal lines represent the mean, and error bars S.E.M. For mouse experiments, cohort sizes match common practice of the described experiments. For human experiments, sample size was chosen to validate statistical analyses.

No mice or data points were excluded from analyses. In the human studies, all humans older than 18 years of age who enrolled were included. Exclusion criteria included pregnancy.

## RESULTS

### Chronic consumption of non-caloric artificial sweeteners exacerbates glucose intolerance

To determine the effects of NAS on glucose homeostasis, we added commercial formulations of saccharin, sucralose or aspartame to the drinking water of lean 10-week-old C57B1/6 mice (Figure 15A). Since all three commercial NAS comprise ~5% sweetener and ~95% glucose, we used as controls mice drinking only water or water supplemented with either glucose or sucrose. Notably, at week 11, the three mouse groups that consumed water, glucose, and sucrose featured comparable glucose tolerance curves, whereas all three NAS-consuming mouse groups developed marked glucose intolerance ($p < 0.001$, Figure 16A-B).

As saccharin exerted the most pronounced effect, we further studied its role as a prototypical artificial sweetener. To corroborate the findings in the obesity setup, we fed C57B1/6 mice a high-fat diet (HFD, 60% Kcal from fat) while consuming either commercial saccharin or pure glucose as a control (Figure 15B). As in the lean state, mice fed HFD and commercial saccharin developed glucose intolerance, as compared to the control mouse group ($p < 0.03$, Figure 16C and 17A). To examine the effects of pure saccharin on glucose intolerance, we followed a cohort of 10-week old C57B1/6 mice fed on HFD and nourished by 0. 1mgml$^{-1}$ of pure saccharin added to their drinking water (Figure 15C). This dose corresponds to the FDA acceptable daily intake (ADI) in humans (5mgkg$^{-1}$, adjusted to mouse weights, see methods). As with commercial saccharin, this lower dose of pure saccharin was associated with impaired glucose tolerance ($p < 0.0002$, Figure 16D and 17B) starting as early as 5 weeks after HFD initiation. Similarly, HFD-fed outbred Swiss Webster mice nourished with or without 0.1mgml$^{-1}$ of pure saccharin (Figure 15D) showed significant glucose intolerance after 5 weeks of saccharin exposure as compared to controls ($p < 0.03$, Figure 17C-D).

Metabolic profiling of NC- or HFD-fed mice in metabolic cages, including liquids and chow consumption, oxygen consumption, walking distance and energy expenditure, showed similar measures between NAS- and control-drinking mice (Figures 18 and 19). Fasting serum insulin levels and insulin tolerance were also similar in all mouse groups consuming NAS or caloric sweeteners, in both the normal chow (NC) and HFD settings (Figure 20). Taken together, these results suggest that NAS promotes metabolic derangements in a range of formulations, doses, mouse strains, and diets paralleling human conditions, both in the lean and the obese state.

### NAS-induced glucose intolerance is mediated by the gut microbiota

Since diet modulates the gut microbiota[15], and microbiota alterations exert profound effects on host physiology and metabolism, the present inventors tested whether the microbiota may regulate the observed NAS effects. To this end, they treated mouse groups consuming commercial or pure NAS in the lean and HFD states (Figures 15A, C) with a Gram-negative-targeting broad-spectrum antibiotics regimen (designated 'Antibiotics A') of ciprofloxacin (0.2gl$^{-1}$) and metronidazole (1gl$^{-1}$), while maintaining mice on their diet and sweetener supplementation regimens. Notably, after 4 weeks of antibiotic treatment, differences in glucose intolerance between NAS-drinking mice and controls were abolished both in the lean (Figures 16A-B) and the obese (Figure 16D and Figure 17B) states. Similar effects were observed with the Gram-positive-targeting antibiotic vancomycin ('Antibiotics B', 0.5gl$^{-1}$, Figures 15A-B). These results suggest that NAS-induced glucose intolerance is mediated through alterations to the commensal microbiota, with contributions from diverse bacterial taxa.

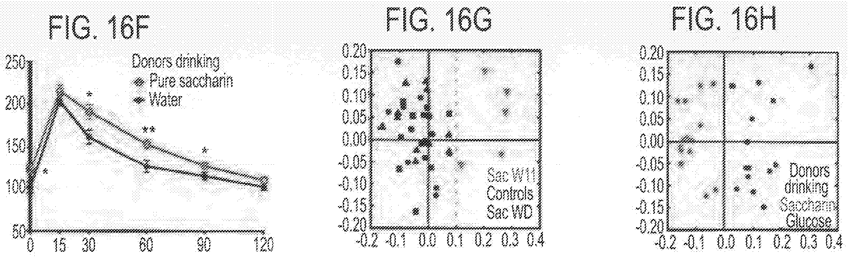

To test whether the microbiota role is causal, fecal transplantation experiments were performed, by transferring the microbiota configuration from mice on NC diet drinking commercial saccharin or glucose (control) into NC-consuming germ-free mice (Figure 15E). Notably, recipients of commercial saccharin-related microbiota exhibited impaired glucose tolerance as compared to control (glucose) microbiota recipients, determined 6 days following transfer ($p < 0.03$, Figure 16E and 17E). Transferring the microbiota composition of HFD-consuming mice drinking water or pure saccharin replicated the glucose intolerance phenotype ($p < 0.004$, Figure 16F and 17F). Together, these results establish that the metabolic derangements induced by NAS consumption are mediated by the intestinal microbiota.

### NAS consumption mediates distinct compositional and functional alterations to the microbiota

The present inventors next examined the fecal microbiota composition of the various mouse groups by sequencing their 16S rRNA gene. Mice drinking saccharin had distinct microbiota composition that clustered separately from both their starting microbiome configurations and from all control groups at week 11 (Figure 16G). Likewise, microbiota in GF recipients of stools from saccharin-consuming donor mice clustered separately from that of GF recipients of glucose-drinking donor stools (Figure 16H). Compared to all control groups, the microbiota of saccharin-consuming mice displayed considerable dysbiosis, with >40 operational taxonomic units (OTUs) significantly altered in abundance (FDR corrected p-value < 0.05 for each OTU, Figure 21). Many of the taxa that increased in relative abundance belonged to the *Bacteroides* genus and Clostridiales order, with other members of the Clostridiales order comprising the majority of under-represented taxa, along with *Lactobacillus reuteri,* and were mirrored in GF recipients of stools from saccharin-consuming donors (Figure 21, right column). Likewise, dysbiosis was observed in mice consuming pure saccharin and HFD. Together, these results demonstrate that saccharin consumption in various formulations, doses, and diets induces dysbiosis with overall similar configurations.

To study the functional consequences of NAS consumption, shotgun metagenomic sequencing of fecal samples was performed from before and after 11 weeks of commercial saccharin consumption, as compared to control mice consuming either glucose or water. To compare relative species abundance, sequencing reads were mapped to the human microbiome reference genome database[16]. In agreement with the 16S rRNA analysis, saccharin treatment induced the largest changes in microbial relative species abundance (Figure 22A, Table 1, herein below; F-test p-value<$10^{-10}$). These changes are unlikely to be an artifact of horizontal gene transfer or poorly covered genomes, since changes in relative abundance were observed across much of the length of the bacterial genomes, as exemplified by one overrepresented (*B. vulgatus,* Figure 23A) and one underrepresented species (*A. muciniphila,* Figure 23B).

*Table 1*

| Fold change (week 11/week 0) | | | Symbol | Number |
|---|---|---|---|---|
| Glucose | Water | Saccharin | | |
| 2.83 | 0.46 | 141.18 | BACT_1378 | 1375 |
| 1.04 | 2.32 | 12.66 | BACT_196 | 395 |
| 2.61 | 0.94 | 12.01 | BACt_1324 | 1264 |
| 1.54 | 2.68 | 11.92 | BACT_3004 | 5797 |
| 1.23 | 1.93 | 10.39 | BACT_915 | 1315 |
| 1.44 | 0.68 | 9.91 | BACT_409 | 845 |
| 1.58 | 0.88 | 9.41 | BACT_70 | 863 |
| 1.15 | 1.74 | 9.24 | BACt_1348 | 1311 |
| 5.63 | | 8.79 | BACT_185 | 633 |
| 1.20 | 3.15 | 6.73 | BACT_65 | 918 |
| 1.55 | 0.16 | 3.71 | BACT_511 | 591 |
| 0.96 | 1.32 | 3.38 | BACT_769 | 610 |
| 1.16 | 2.50 | 3.20 | BACT_1321 | 1255 |
| 1.02 | 2.71 | 3.17 | BACT_632 | 871 |
| 0.62 | 0.08 | 2.97 | BACT_496 | 765 |
| 1.13 | 0.95 | 2.90 | BACT_449 | 772 |
| 1.26 | 4.58 | 2.71 | BACT_3092 | 5793 |
| 1.32 | 4.52 | 2.65 | BACT_1496 | 1321 |
| 1.86 | 1.38 | 2.51 | BACT_850 | 394 |
| 1.75 | 1.44 | 2.16 | BACT_1442 | 1400 |
| 1.05 | 2.28 | 2.13 | BACT_371 | 960 |
| 1.66 | 1.55 | 1.92 | BACT_189 | 636 |
| 0.94 | 1.72 | 1.89 | BACT_3007 | 5798 |
| 1.47 | 1.27 | 1.88 | BACT_722 | 377 |
| 1.17 | 2.27 | 1.25 | BACT_413 | 847 |
| 16.24 | 2.48 | 1.16 | BACT_4040 | 5769 |

(continued)

| Fold change (week 11/week 0) | | | Symbol | Number |
|---|---|---|---|---|
| Glucose | Water | Saccharin | | |
| 1.17 | 2.06 | 1.10 | BACT_913 | 904 |
| 1.27 | 1.29 | 1.08 | BACT_1043 | 948 |
| 1.44 | 1.12 | 1.03 | BACT_172 | 768 |
| 1.56 | 0.67 | 1.01 | BACT_3005 | 5799 |
| 1.49 | 0.99 | 0.99 | BACT_3062 | 5800 |
| 1.50 | 1.41 | 0.91 | BACT_1322 | 1396 |
| 0.88 | 0.33 | 0.77 | BACT_852 | 766 |
| 0.79 | 0.27 | 0.75 | BACT_1001 | 933 |
| 0.88 | 0.33 | 0.71 | BACT_60 | 752 |
| 0.92 | 0.41 | 0.70 | BACT_572 | 963 |
| 1.33 | 0.12 | 0.70 | BACT_586 | 849 |
| 1.41 | 0.85 | 0.67 | BACT_1314 | 1399 |
| 1.00 | 0.31 | 0.66 | BACT_392 | 915 |
| 1.24 | 0.96 | 0.65 | BACT_9 | 996 |
| 0.73 | 0.49 | 0.62 | BACT_551 | 907 |
| 1.24 | 0.65 | 0.56 | BACT_171 | 748 |
| 0.94 | 0.35 | 0.55 | BACT_260 | 1443 |
| 1.13 | 0.27 | 0.55 | BACT_258 | 620 |
| 1.30 | 2.71 | 0.53 | BACT_393 | 932 |
| 0.98 | 0.29 | 0.53 | BACT_1025 | 751 |
| 0.98 | 0.34 | 0.53 | BACT_931 | 1088 |
| 1.36 | 0.33 | 0.53 | EUKY_141 | 1875 |
| 0.95 | 0.27 | 0.51 | BACT_194 | 846 |
| 1.16 | 0.51 | 0.50 | BACT_300 | 769 |
| 1.03 | 0.24 | 0.50 | BACT_38 | 780 |
| 1.60 | 0.30 | 0.49 | EUKY_222 | 1954 |
| 0.93 | 0.36 | 0.48 | BACT_170 | 883 |
| 1.09 | 1.62 | 0.46 | BACT_1315 | 1395 |
| 1.38 | 0.34 | 0.45 | EUKY_22 | 1758 |
| 0.91 | 0.37 | 0.45 | BACT_85 | 934 |
| 1.35 | 0.27 | 0.45 | BACT_397 | 914 |
| 1.18 | 0.45 | 0.44 | BACT_225 | 953 |
| 1.01 | 2.48 | 0.42 | BACT_142 | 843 |
| 1.14 | 2.25 | 0.40 | BACT_181 | 767 |
| 1.15 | 1.16 | 0.40 | BACT_195 | 523 |
| 0.86 | 0.32 | 0.36 | BACT_192 | 995 |
| 0.99 | 1.62 | 0.36 | BACT_22 | 639 |

(continued)

| Fold change (week 11/week 0) | | | Symbol | Number |
|---|---|---|---|---|
| Glucose | Water | Saccharin | | |
| 0.79 | 0.44 | 0.34 | BACT_1131 | 942 |
| 1.23 | 0.33 | 0.34 | BACT_855 | 835 |
| 1.34 | 0.31 | 0.34 | BACT_374 | 805 |
| 1.16 | 2.24 | 0.34 | BACT_1311 | 1259 |
| 0.87 | 1.82 | 0.33 | BACT_420 | 922 |
| 0.78 | 0.45 | 0.30 | BACT_1569 | 1462 |
| 1.13 | 0.33 | 0.29 | BACT_424 | 906 |
| 1.53 | 1.14 | 0.28 | BACT_178 | 921 |
| 0.73 | 0.32 | 0.24 | BACT_499 | 35 |
| 0.82 | 2.15 | 0.23 | BACT_1457 | 1337 |
| 0.75 | 0.41 | 0.22 | BACT_539 | 117 |
| 1.35 | 1.68 | 0.22 | BACT_418 | 749 |
| 0.62 | 0.26 | 0.21 | BACT_1555 | 1469 |
| 0.79 | 0.80 | 0.17 | BACT_1501 | 1331 |
| 0.69 | 0.29 | 0.16 | BACT_843 | 1318 |
| 0.66 | 0.32 | 0.16 | BACT_627 | 886 |
| 0.67 | 0.30 | 0.15 | BACT_419 | 920 |
| 0.74 | 2.08 | 0.15 | BACT_890 | 163 |
| 0.66 | 0.31 | 0.14 | BACT_535 | 940 |
| 1.39 | 0.54 | 0.12 | BACT_1596 | 1502 |
| 1.55 | 0.45 | 0.12 | BACT_1656 | 1503 |
| 1.54 | 0.44 | 0.11 | BACT_3058 | 5816 |
| 1.58 | 0.39 | 0.07 | BACT_1526 | 1227 |
| 0.28 | 0.11 | 0.04 | BACT_4042 | 5770 |
| 1.90 | 0.34 | 0.03 | BACT_3085 | 5842 |
| 0.72 | 0.19 | 0.02 | BACT_3024 | 5841 |
| 2.13 | 0.38 | 0.02 | VIRL3522 | 3522 |
| 0.15 | 1.50 | 0.02 | BACT_49 | 420 |
| 1.19 | 0.30 | 0.01 | BACT_3045 | 5818 |
| 1.55 | 0.57 | 0.00 | BACT_463 | 719 |
| 1.06 | 0.29 | 0.00 | BACT_686 | 1007 |
| 1.13 | 0.28 | 0.00 | BACT_1427 | 1266 |
| 2.25 | 0.31 | 0.00 | BACT_1083 | 1008 |
| 1.00 | 0.05 | 0.00 | BACT_3056 | 5836 |
| 1.55 | 0.58 | 0.00 | BACT_681 | 125 |
| 1.13 | 0.27 | 0.00 | BACT_1595 | 1533 |
| 1.62 | 0.58 | 0.00 | BACT_3060 | 5821 |

The present inventors next mapped the metagenomic reads to a gut microbial gene catalogue, evenly dividing reads mapping to more than one gene, and then grouping genes into KEGG pathways. Examining pathways with gene coverage above 0.2 (115 pathways), changes in pathway abundance were inversely correlated between commercial saccharin- and glucose-consuming mice (R=-0.45, p<10[-6], Figure 22B). Since commercial saccharin consists of 95% glucose, these results suggest that saccharin greatly affects microbiota function. Notably, pathways overrepresented in saccharin-consuming mice include a strong increase in glycan degradation pathways (Figures 22C-D), in which glycans are fermented to form various compounds including short-chain fatty acids (SCFAs)[17]. These pathways mark enhanced energy harvest and their enrichment was previously associated with obesity in mice[11] and humans[18], with SCFA possibly serving as precursors and/or signaling molecules for de-novo glucose and lipid synthesis by the host[19]. To identify the underlying bacteria, the present inventors annotated every read that mapped to glycan degradation pathways by its originating bacteria. Much of the increase in these pathways is attributable to reads originating from 5 Gram-negative and positive species, of which two belong to the *Bacteroides* genus (Figure 22E), consistent with the sharp increase in the abundance of this genus in saccharin-consuming mice observed in the 16S rRNA analysis (Figure 21). Consequently, levels of the SCFA propionate and acetate measured in stool were markedly higher in commercial saccharin-consuming mice as compared to control glucose consuming mice (Figures 22F-G), reflective of the differential effects mediated by chronic glucose consumption with and without NAS exposure. Butyrate levels were similar between the groups (data not shown).

In addition to glycan degradation, and in-line with previous studies on humans with T2DM[13,20], other pathways were enriched in microbiomes of saccharin consuming mice, including starch and sucrose metabolism, fructose and mannose metabolism, and folate-, glycerolipid- and fatty acid biosynthesis (Table 2, herein below), while glucose transport pathways were underrepresented in saccharin-consuming mice (Figure 23C). Mice consuming HFD and pure saccharin featured multiple enriched pathways (Figure 23D), including ascorbate and aldarate metabolism (previously reported to be enriched in leptin-receptor deficient diabetic mice[21]), lipopolysaccharide biosynthesis (linked to metabolic endotoxemia[22]) and bacterial chemotaxis (previously reported to be enriched in obese mice[11]).

*Table 2*

| Log fold change | | | | Pathway name |
|---|---|---|---|---|
| **In vitro** | **In vivo (week 11 / week 0)** | | | |
| **PBS vs. SAC** | **Saccharin** | **Glucose** | **Water** | |
| -0.01 | 0.36 | 0.09 | 0.07 | Lipoic acid metabolism |
| 0.03 | 0.36 | -0.04 | 0.01 | Other glycan degradation |
| 0.03 | 0.34 | -0.01 | 0.04 | Glycosaminoglycan degradation |
| 0.04 | 0.34 | -0.03 | 0.01 | Sphingolipid metabolism |
| 0.05 | 0.33 | 0.03 | 0.00 | Glycosphingolipid biosynthesis - globo series |
| 0.02 | 0.31 | -0.05 | 0.05 | Pentose and glucuronate interconversions |
| 0.03 | 0.30 | 0.06 | -0.03 | Bacterial chemotaxis |
| 0.01 | 0.30 | 0.04 | -0.02 | Phenylalanine metabolism |
| -0.01 | 0.30 | 0.09 | -0.08 | Phosphonate and phosphinate metabolism |
| -0.02 | 0.29 | 0.03 | 0.09 | Caprolactam degradation |
| 0.02 | 0.29 | 0.04 | 0.02 | Novobiocin biosynthesis |
| 0.02 | 0.27 | 0.06 | -0.01 | Tropane, piperidine and pyridine alkaloid biosynthesis |
| 0.03 | 0.27 | -0.13 | 0.10 | Ascorbate and aldarate metabolism |
| 0.01 | 0.24 | -0.05 | 0.06 | Phenylalanine, tyrosine and tryptophan biosynthesis |
| 0.02 | 0.24 | 0.06 | -0.03 | Galactose metabolism |
| 0.01 | 0.23 | 0.01 | 0.02 | Porphyrin and chlorophyll metabolism |
| 0.01 | 0.21 | -0.09 | 0.07 | Lipopolysaccharide biosynthesis |
| -0.01 | 0.19 | 0.12 | -0.04 | Sulfur metabolism |
| 0.01 | 0.19 | 0.00 | 0.00 | Starch and sucrose metabolism |

(continued)

| Log fold change | | | | Pathway name |
|---|---|---|---|---|
| In vitro | In vivo (week 11 / week 0) | | | |
| PBS vs. SAC | Saccharin | Glucose | Water | |
| 0.02 | 0.18 | -0.07 | 0.04 | Cyanoamino acid metabolism |
| 0.00 | 0.18 | 0.03 | 0.00 | Fructose and mannose metabolism |
| 0.00 | 0.16 | -0.07 | 0.08 | Vitamin B6 metabolism |
| -0.02 | 0.16 | -0.19 | 0.06 | Biosynthesis of siderophore group nonribosomal peptides |
| -0.03 | 0.14 | 0.06 | -0.03 | Lysine degradation |
| -0.01 | 0.14 | -0.02 | 0.08 | beta-Alanine metabolism |
| 0.00 | 0.14 | -0.02 | 0.00 | Taurine and hypotaurine metabolism |
| -0.01 | 0.13 | -0.08 | 0.04 | Biotin metabolism |
| 0.01 | 0.13 | 0.03 | -0.01 | Glyoxylate and dicarboxylate metabolism |
| 0.00 | 0.13 | -0.08 | 0.04 | Folate biosynthesis |
| 0.00 | 0.13 | 0.03 | -0.02 | Glycine, serine and threonine metabolism |
| 0.01 | 0.13 | -0.08 | 0.05 | Histidine metabolism |
| -0.03 | 0.12 | 0.40 | -0.17 | Glutathione metabolism |
| -0.05 | 0.12 | -0.03 | 0.10 | Atrazine degradation |
| 0.00 | 0.11 | 0.19 | -0.07 | Glycerolipid metabolism |
| 0.01 | 0.11 | 0.03 | 0.02 | C5-Branched dibasic acid metabolism |
| 0.01 | 0.11 | -0.01 | 0.02 | beta-Lactam resistance |
| 0.00 | 0.10 | -0.04 | 0.04 | Streptomycin biosynthesis |
| 0.01 | 0.10 | -0.04 | 0.01 | 2-Oxocarboxylic acid metabolism |
| 0.01 | 0.10 | -0.05 | 0.03 | Valine, leucine and isoleucine biosynthesis |
| 0.01 | 0.09 | 0.00 | -0.02 | Amino sugar and nucleotide sugar metabolism |
| 0.01 | 0.07 | -0.02 | 0.03 | One carbon pool by folate |
| -0.02 | 0.06 | 0.20 | -0.08 | Glycerophospholipid metabolism |
| 0.01 | 0.06 | 0.02 | 0.01 | Two-component system |
| 0.00 | 0.06 | -0.08 | 0.02 | Fatty acid metabolism |
| 0.00 | 0.06 | -0.09 | 0.05 | Valine, leucine and isoleucine degradation |
| -0.01 | 0.05 | -0.01 | 0.00 | Arginine and proline metabolism |
| -0.02 | 0.05 | -0.13 | 0.08 | Ether lipid metabolism |
| -0.01 | 0.05 | -0.07 | 0.03 | Fatty acid biosynthesis |
| 0.00 | 0.05 | 0.07 | -0.03 | Drug metabolism - other enzymes |
| -0.01 | 0.04 | 0.00 | 0.01 | Aminobenzoate degradation |
| -0.01 | 0.04 | -0.02 | 0.01 | Limonene and pinene degradation |
| 0.00 | 0.04 | -0.14 | 0.08 | Geraniol degradation |
| -0.01 | 0.04 | 0.03 | -0.03 | Terpenoid backbone biosynthesis |
| -0.02 | 0.04 | 0.00 | -0.02 | Naphthalene degradation |
| 0.00 | 0.04 | 0.00 | -0.01 | Pantothenate and CoA biosynthesis |

(continued)

| Log fold change | | | | Pathway name |
|---|---|---|---|---|
| In vitro | In vivo (week 11 / week 0) | | | |
| PBS vs. SAC | Saccharin | Glucose | Water | |
| 0.00 | 0.04 | 0.03 | 0.01 | Bisphenol degradation |
| 0.00 | 0.03 | -0.01 | -0.02 | Biosynthesis of amino acids |
| -0.01 | 0.03 | 0.09 | -0.08 | Selenocompound metabolism |
| 0.00 | 0.03 | -0.07 | 0.05 | Citrate cycle (TCA cycle) |
| 0.00 | 0.03 | 0.12 | -0.08 | Pentose phosphate pathway |
| 0.00 | 0.03 | 0.09 | -0.03 | Base excision repair |
| -0.03 | 0.03 | 0.09 | -0.07 | Ubiquinone and other terpenoid-quinone biosynthesis |
| -0.03 | 0.02 | 0.25 | -0.15 | ABC transporters |
| -0.01 | 0.02 | -0.04 | -0.01 | Alanine, aspartate and glutamate metabolism |
| -0.01 | 0.02 | 0.02 | -0.01 | Tyrosine metabolism |
| -0.01 | 0.02 | 0.05 | -0.04 | Methane metabolism |
| -0.01 | 0.02 | 0.01 | -0.03 | Propanoate metabolism |
| -0.02 | 0.01 | 0.05 | -0.04 | Peptidoglycan biosynthesis |
| -0.04 | 0.01 | 0.13 | -0.03 | Tryptophan metabolism |
| 0.00 | 0.01 | -0.04 | 0.03 | Carbon fixation pathways in prokaryotes |
| 0.00 | 0.01 | 0.06 | -0.03 | Pyruvate metabolism |
| 0.00 | 0.00 | -0.04 | 0.00 | Nitrogen metabolism |
| -0.02 | 0.00 | 0.05 | 0.03 | Penicillin and cephalosporin biosynthesis |
| -0.01 | 0.00 | 0.02 | -0.01 | Carbon metabolism |
| -0.01 | 0.00 | 0.01 | 0.00 | Butanoate metabolism |
| -0.01 | 0.00 | 0.02 | -0.01 | Benzoate degradation |
| -0.02 | -0.01 | 0.05 | -0.03 | Proximal tubule bicarbonate reclamation |
| 0.02 | -0.01 | -0.06 | 0.03 | Inositol phosphate metabolism |
| 0.00 | -0.01 | -0.05 | 0.06 | Vibrio cholerae pathogenic cycle |
| 0.01 | -0.02 | -0.04 | 0.00 | Riboflavin metabolism |
| 0.01 | -0.02 | 0.01 | -0.04 | Cysteine and methionine metabolism |
| -0.03 | -0.03 | 0.22 | -0.13 | Sulfur relay system |
| -0.02 | -0.03 | 0.00 | 0.01 | Oxidative phosphorylation |
| -0.01 | -0.03 | 0.03 | -0.01 | Mismatch repair |
| -0.02 | -0.03 | 0.04 | -0.06 | Thiamine metabolism |
| -0.01 | -0.03 | 0.00 | -0.02 | Fatty acid degradation |
| -0.03 | -0.04 | 0.01 | -0.03 | D-Glutamine and D-glutamate metabolism |
| -0.01 | -0.04 | -0.02 | 0.01 | Protein export |
| -0.01 | -0.05 | -0.01 | -0.01 | RNA degradation |
| -0.02 | -0.05 | 0.00 | -0.02 | Nicotinate and nicotinamide metabolism |
| 0.00 | -0.06 | 0.05 | -0.03 | Cell cycle - Caulobacter |

(continued)

| Log fold change | | | | Pathway name |
| --- | --- | --- | --- | --- |
| In vitro | In vivo (week 11 / week 0) | | | |
| PBS vs. SAC | Saccharin | Glucose | Water | |
| 0.06 | -0.06 | 0.31 | -0.37 | Styrene degradation |
| 0.00 | -0.06 | 0.02 | 0.00 | DNA replication |
| -0.03 | -0.07 | 0.12 | -0.02 | Biosynthesis of unsaturated fatty acids |
| 0.00 | -0.07 | 0.02 | -0.07 | Lysine biosynthesis |
| -0.03 | -0.07 | 0.10 | -0.01 | Chloroalkane and chloroalkene degradation |
| 0.00 | -0.08 | 0.00 | 0.00 | Nucleotide excision repair |
| -0.01 | -0.08 | 0.01 | -0.02 | Pyrimidine metabolism |
| -0.01 | -0.08 | 0.02 | -0.03 | Purine metabolism |
| 0.24 | -0.08 | 0.28 | -0.22 | D-Arginine and D-ornithine metabolism |
| -0.02 | -0.09 | 0.10 | -0.06 | Glycolysis / Gluconeogenesis |
| -0.01 | -0.09 | -0.03 | 0.01 | Homologous recombination |
| 0.01 | -0.10 | 0.03 | -0.03 | Bacterial secretion system |
| -0.04 | -0.10 | 0.25 | -0.11 | D-Alanine metabolism |
| 0.00 | -0.13 | -0.03 | 0.00 | Carbon fixation in photosynthetic organisms |
| -0.02 | -0.13 | 0.00 | -0.03 | Aminoacyl-tRNA biosynthesis |
| 0.07 | -0.18 | 0.06 | -0.15 | Flagellar assembly |
| -0.05 | -0.22 | 0.21 | -0.08 | Nitrotoluene degradation |
| -0.01 | -0.24 | -0.01 | -0.01 | Ribosome |
| -0.12 | -0.34 | 0.05 | -0.03 | Dioxin degradation |
| -0.09 | -0.35 | 0.42 | -0.32 | Synthesis and degradation of ketone bodies |
| -0.17 | -0.43 | 0.60 | -0.28 | Phosphotransferase system (PTS) |
| -0.15 | -0.50 | 0.36 | -0.20 | Tetracycline biosynthesis |
| -0.12 | -0.51 | 0.03 | -0.02 | Xylene degradation |
| -0.35 | -0.61 | 0.34 | 0.31 | DDT degradation |

Altogether, saccharin consumption results in distinct diet-dependent functional alterations in the microbiota, including NC-related expansion in glycan degradation contributed by several of the increased taxa, ultimately resulting in elevated stool SCFA levels, characteristic of increased microbial energy harvest[11].

*Glucose intolerance is directly mediated **by** saccharin-induced modulation of the **gut** microbiota*

To determine whether saccharin directly affects the gut microbiota, the present inventors cultured fecal matter from naïve mice under strict anaerobic conditions (75% $N_2$, 20% $CO_2$, 5% $H_2$) in the presence of saccharin (5mgml$^{-1}$) or control growth media. Cultures from day 9 of incubation were administered by gavage to germ-free mice (Figure 24A). In-vitro stool culture with saccharin induced an increase of the Bacteroidetes phyla and reduction in Firmicutes (Bacteroidetes 89% vs. 70%, Firmicutes 6% vs. 22%, Figure 24B). Transferring this *in vitro* saccharin-treated microbiota configuration into germ-free mice resulted in significantly higher glucose intolerance (p<0.002) compared with germ-free mice receiving the control culture (Figure 25A and 24C Similar to the composition of the saccharin-supplemented anaerobic culture, germ-free recipients of this cultured-configuration featured overrepresentation of members of the *Bacteroides* genus, and under-representation of several Clostridiales (Figure 25B and Table 3).

**Table 3**

| EnrPBS vs. EnrSac q-value | Saccharin Enriched D6 | PBS Enriched D6 | Observation | |
|---|---|---|---|---|
| 6.83448E-14 | 0.610984828 | 0.580639293 | k_Bacteria;p_Bacteroidetes | 2 |
| 1.63098E-58 | 0.364857511 | 0.41749352 | k_Bacteria;p_Firmicutes | 2 |
| 0 | 0.022909864 | 0.000402903 | k_Bacteria;p_Tenericutes | 2 |
| 1.79068E-13 | 0.61080743 | 0.580556608 | k_Bacteria;p_Bacteroidetes;c_Bacteroidia | 3 |
| 1.46602E-11 | 0.312188426 | 0.332556091 | k_Bacteria;p_Firmicutes;c_Bacilli | 3 |
| 4.3609E-124 | 0.052661567 | 0.084935926 | k_Bacteria;p_Firmicutes;c_Clostridia | 3 |
| 0 | 0.022909864 | 0.000402903 | k_Bacteria;p_Tenericutes;c_Mollicutes | 3 |
| 0.001583437 | 0.000127786 | 1.50337E-06 | k_Bacteria;p_Proteobacteria;c_Gammaproteobacteria | 3 |
| 2.76741E-13 | 0.61080743 | 0.580556608 | k_Bacteria;p_Bacteroidetes;c_Bacteroidia;o_Bacteroidales | 4 |
| 2.99864E-11 | 0.311563024 | 0.331790875 | k_Bacteria;p_Firmicutes;c_Bacilli;o_Lactobacillales | 4 |
| 6.5421E-124 | 0.052660064 | 0.084935926 | k_Bacteria;p_Firmicutes;c_Clostridia;o_Clostridiales | 4 |
| 0 | 0.022909864 | 0.00039689 | k_Bacteria;p_Tenericutes;c_Mollicutes;o_Anaeroplasmatales | 4 |
| 0.002877755 | 0.00012478 | 1.50337E-06 | k_Bacteria;p_Proteobacteria;c_Gammaproteobacteria;o_Enterobacteriales | 4 |
| 5.05927E-88 | 0.467037097 | 0.398576609 | k_Bacteria;p_Bacteroidetes;c_Bacteroidia;o_Bacteroidales;f_Bacteroidaceae | 5 |
| 1.3487E-08 | 0.308681063 | 0.326019436 | k_Bacteria;p_Firmicutes;c_Bacilli;o_Lactobacillales;f_Lactobacillaceae | 5 |
| 1.58777E-67 | 0.137898168 | 0.173774603 | k_Bacteria;p_Bacteroidetes;c_Bacteroidia;o_Bacteroidales;f_Porphyromonadaceae | 5 |
| 9.07031E-27 | 0.042752852 | 0.055245861 | k Bacteria;p Firmicutes;c_Clostridia;o_Clostridiale s;f_ | 5 |
| 0 | 0.022909864 | 0.00039689 | k_Bacteria;p_Tenericutes;c_Mollicutes;o_Anaeroplasmatales;f_Anaeroplasmataceae | 5 |
| 1.6639E-144 | 0.005131004 | 0.01950924 | k_Bacteria;p_Firmicutes;c_Clostridia;o_Clostridiales;f_Clostridiaceae | 5 |
| 7.76553E-06 | 0.004319184 | 0.006096168 | k_Bacteria;p_Bacteroidetes;c_Bacteroidia;o_Bacteroidales;f_[Odoribacteraceae] | 5 |
| 1.10131E-12 | 0.003364543 | 0.005948837 | k_Bacteria;p_Firmicutes;c_Clostridia;o_Clostridiale s;f_Ruminococcaceae | 5 |
| 3.7939E-30 | 0.000977191 | 0.003838105 | k_Bacteria;p_Firmicutes;c_Bacilli;o_Lactobacillales;f_Streptococcaceae | 5 |
| 4.47696E-27 | 0.000838881 | 0.003361537 | k_Bacteria;p_Firmicutes;c_Clostridia;o_Clostridiales;f_Dehalobacteriaceae | 5 |
| 0.002846977 | 0.00012478 | 1.50337E-06 | k_Bacteria;p_Proteobacteria;c_Gammaproteobacteria;o_Enterobacteriales;f_Enterobacteriaceae | 5 |
| 0.009006289 | 7.06584E-05 | 0.0002676 | k_Bacteria;p_Bacteroidetes;c_Bacteroidia;o_Bacteroidales;f_S24-7 | 5 |

(continued)

| EnrPBS vs. EnrSac q-value | Saccharin Enriched D6 | PBS Enriched D6 | Observation | |
|---|---|---|---|---|
| 6.70011E-88 | 0.467037097 | 0.398576609 | k_Bacteria;p_Bacteroidetes;c_Bacteroidia;o_Bacteroidales;f_Bacteroidaceae;g_Bacteroides | 6 |
| 1.60751E-08 | 0.308681063 | 0.326019436 | k_Bacteria;p_Firmicutes;c_Bacilli;o_Lactobacillales;f_Lactobacillaceae;g_Lactobacillus | 6 |
| 1.68217E-67 | 0.137898168 | 0.173774603 | k_Bacteria;p_Bacteroidetes;c_Bacteroidia;o_Bacteroidales;f_Porphyromonadaceae;g_Parabacteroides | 6 |
| 1.05105E-26 | 0.042752852 | 0.055245861 | k_Bacteria;p_Firmicutes;c_Clostridia;o_Clostridiales;f_;g_ | 6 |
| 0 | 0.022909864 | 0.00039689 | k_Bacteria;p_Tenericutes;c_Mollicutes;o_Anaeroplasmatales;f_Anaeroplasmataceae;g_Anaeroplasma | 6 |
| 3.6778E-92 | 0.005046815 | 0.015648584 | k_Bacteria;p_Firmicutes;c_Clostridia;o_Clostridiales;f_Clostridiaceae;g_Candidatus Arthromitus | 6 |
| 9.34916E-06 | 0.004319184 | 0.006096168 | k_Bacteria;p_Bacteroidetes;c_Bacteroidia;o_Bacteroidales;f_[Odoribacteraceae];g_Odoribacter | 6 |
| 2.86812E-12 | 0.003111977 | 0.005569988 | k_Bacteria;p_Firmicutes;c_Clostridia;o_Clostridiales;f_Ruminococcaceae;g_ | 6 |
| 9.11656E-32 | 0.000850908 | 0.003696788 | k_Bacteria;p_Firmicutes;c_Bacilli;o_Lactobacillales;f_Streptococcaceae;g_Lactococcus | 6 |
| 5.08196E-27 | 0.000838881 | 0.003361537 | k_Bacteria;p_Firmicutes;c_Clostridia;o_Clostridiales;f_Dehalobacteriaceae;g_Dehalobacterium | 6 |
| 0.003456128 | 0.00012478 | 1.50337E-06 | k_Bacteria;p_Proteobacteria;c_Gammaproteobacteria;o_Enterobacteriales;f_Enterobacteriaceae;g | 6 |
| 0.023047817 | 7.81753E-05 | 0.00025858 | _k_Bacteria;p_Firmicutes;c_Clostridia;o_Clostridiales;f_Lachnospiraceae;g_[Ruminococcus] | 6 |
| 0.011009767 | 7.06584E-05 | 0.0002676 | k_Bacteria;p_Bacteroidetes;c_Bacteroidia;o_Bacteroidales;f_S24-7;g_ | 6 |
| 3.98858E-79 | 4.20944E-05 | 0.003809541 | k_Bacteria;p_Firmicutes;c_Clostridia;o_Clostridiale s;f_Clostridiaceae;g_Clostridium | 6 |

Shotgun metagenomic sequencing analysis revealed that in-vitro saccharin treatment induced similar functional alterations to those found in mice consuming commercial saccharin (FIG. 21C, p<10$^{-4}$), with glycan degradation pathways being highly enriched in both settings. Other pathways highly enriched in both settings included those involved in sphingolipid metabolism, previously shown to be overrepresented in microbiomes of non-obese diabetic mice[23], and common under-represented pathways included glucose transport (Figure. 25C and FIG. 23C, right column).

Collectively, these results demonstrate that saccharin directly modulates the composition and function of the microbiome and induces dysbiosis, accounting for the downstream glucose intolerance phenotype in the mammalian host.

### NAS consumption in humans is associated with impaired glucose tolerance

To study the effect of NAS in humans, the relationship between long-term NAS consumption (based on a validated food frequency questionnaire, see methods) and various clinical parameters in data collected from 381 non-diabetic individuals (44% males and 56% females; Age 43.3±13.2) in an ongoing clinical nutritional study was compared. Significant positive correlations between NAS consumption and several metabolic syndrome-related clinical parameters were found (Table 4, herein below), including increased weight & waist-hip ratio (measures of central obesity); higher fasting blood glucose, glycosylated hemoglobin (HbA1C%) & glucose tolerance test (GTT, measures of impaired glucose tolerance), and elevated serum alanine aminotransferase (ALT, measure of hepatic damage that is likely to be secondary, in this context, to non-alcoholic fatty liver disease). Moreover, the levels of glycosylated hemoglobin (HbA1C%), indicative of glucose concentration over the previous 3 months, were significantly increased when comparing a subgroup of high NAS consumers (40 individuals) to non-NAS consumers (236 individuals, Figure 26A, ranksum p<0.002). This increase remained significant when corrected to BMI levels (ranksum p<0.015). In this cohort, the 16S rRNA in 172 randomly selected individuals was characterized. Notably, statistically significant positive correlations were found between multiple taxonomic entities and NAS consumption, including the *Enterobacteriaceae* family (Pearson r=0.36, FDR corrected $p<10^{-6}$), the Deltaproteobacteria class (Pearson r=0.33, FDR corrected $p<10^{-5}$) and the Actinobacteria phylum (Pearson r=0.27, FDR corrected p<0.0003, Table 5). Importantly, statistically significant correlations between OTU abundances and BMI were not detected, suggesting that the above correlations are not due to the distinct BMI of NAS consumers.

*Table 4*

| Spearman correlation to NAS consumption | | | |
|---|---|---|---|
| q | p | R | |
| 7.0E-07 | 5.0E-08 | 0.29 | BPSys |
| 7.0E-07 | 1.0E-07 | 0.28 | BMI |
| 4.0E-06 | 9.0E-07 | 0.26 | Bpdia |
| 4.0E-04 | 1.0E-04 | 0.21 | Waist-hip Ratio |
| 4.0E-04 | 1.0E-04 | 0.20 | HbA1c% |
| 9.0E-04 | 3.0E-04 | 0.19 | Fasting glucose |
| 3.0E-03 | 1.0E-03 | 0.17 | Weight |
| 2.0E-02 | 1.0E-02 | 0.13 | GTT |
| 2.0E-02 | 1.0E-02 | 0.13 | ALT |

*Table 5*

| Pearson | | | | OTU / Taxonomic Rank |
|---|---|---|---|---|
| q | p | R | Taxonomy | |
| 0.005015622 | 0.000295037 | 0.270439116 | k_Bacteria;p_Actinobacteria | 2 |
| 0.000253377 | 7.91803E-06 | 0.330530957 | k_Bacteria;p_Proteobacteria;c_ Deltaproteobacteria | 3 |
| 0.005014662 | 0.000313416 | 0.269312074 | k_Bacteria;p_Actinobacteria;c_ Coriobacteriia | 3 |
| 0.391360535 | 0.03669005 | 0.15806695 | k_Bacteria;p_Proteobacteria;c_ Epsilonproteobacteria | 3 |
| 4.09983E-05 | 7.48663E-07 | 0.363767572 | k_Bacteria;p_Proteobacteria;c_ Gammaproteobacteria;o_Enterobacteriales | 4 |
| 4.09983E-05 | 1.46422E-06 | 0.354694435 | k_Bacteria;p_Proteobacteria;c_ Alphaproteobacteria;o_Rhodospirillales | 4 |
| 0.000147803 | 7.91803E-06 | 0.330530957 | k_Bacteria;p_Proteobacteria;c_ Deltaproteobacteria;o_Desulfovibrionales | 4 |
| 0.004387829 | 0.000313416 | 0.269312074 | k_Bacteria;p_Actinobacteria;c_ Coriobacteriia;o_Coriobacteriales | 4 |

(continued)

| Pearson | | | Taxonomy | OTU / Taxonomic Rank |
|---|---|---|---|---|
| q | p | R | | |
| 0.410928562 | 0.03669005 | 0.15806695 | k_Bacteria;p_Proteobacteiria;c_ Epsilonproteobacteiria;o_ Campylobacterales | 4 |
| 0.457480662 | 0.049015785 | 0.149037116 | k_Bacteria;p_Firmicutes;c_Clostridia;o_ SHA-98 | 4 |
| 7.10149E-05 | 7.48663E-07 | 0.363767572 | k_Bacteria;p_Proteobacteria;c_ Gammaproteobacteria;o_ Enterobacteriales;f_Enterobacteriaceae | 5 |
| 7.10149E-05 | 1.46422E-06 | 0.354694435 | k_Bacteria;p_Proteobacteria;c_ Alphaproteobacteria;o_Rhodos pirillales;f_ Acetobacteraceae | 5 |
| 0.000256016 | 7.91803E-06 | 0.330530957 | k_Bacteria;p_Proteobacteria;c_ Deltaproteobacteria;o_Desulfovibrionales; f_Desulfovibrionaceae | 5 |
| 0.007600347 | 0.000313416 | 0.269312074 | k_Bacteria;p_Actinobacteria;c_ Coriobacteriia;o_Coriobacteriales;f_ Coriobacteriaceae | 5 |
| 0.320157986 | 0.016502989 | 0.181038522 | k_Bacteria;p_Firmicutes;c_Clostiridia;o_ Clostridiales;f_Christensenellaceae | 5 |
| 0.429306964 | 0.02655507 | 0.167677541 | k_Bacteria;p_Firmicutes;c_Clostridia;o_ Clostridiales;f_[Mogibacteriaceae] | 5 |
| 0.450076086 | 0.033960575 | 0.160407785 | k_Bacteria;p_Proteobacteria;c_ Epsitonproteobacteria;o_Camp ylobacterales;f_Campylobacteraceae | 5 |
| 0.450076086 | 0.037119677 | 0.157712006 | k_Bacteria;p_TM7;c_TM7-3;o_CW040;f_ | 5 |
| 0.52828124 | 0.049015785 | 0.149037116 | k_Bacteria;p_Firmicutes;c_Clostridia;o_ SHA-98;f_ | 5 |
| 9.66388E-05 | 7.48663E-07 | 0.363767572 | k_Bacteria;p_Proteobacteria;c_ Gammaproteobacteria;o_ Enterobacteriales;f_Enterobacteriaceae;g_ | 6 |
| 9.66388E-05 | 1.46422E-06 | 0.354694435 | k Bacteria;p_Proteobacteria;c_ Alphaproteobacteria;o_Rhodospirillales;f_ Acetobacteraceae;g_ | 6 |
| 9.66388E-05 | 1.46422E-06 | 0.354694435 | k_Bacteria;p_Proteobacteria;c_ Alphaproteobacteria;o_Rhodospirillales;f_ Acetobacteraceae;g Acidocella | 6 |
| 0.001126756 | 2.27627E-05 | 0.314304476 | k_Bacteria;p_Proteobacteria;c_ Deltaproteobacteria;o_Desulfovibrionales; f_Desulfovibrionaceae;g_Desulfovibrio | 6 |
| 0.01240968 | 0.000313376 | 0.269314495 | k_Bacteria;p_Actinobacteria;c_ Coriobacteriia;o_Coriobacteriales;f_ Coriobacteriaceae;g_ | 6 |
| 0.084931852 | 0.002573692 | 0.226531334 | k_Bacteria;p_Proteobacteria;c_ Betaproteobacteria;o_Burkholderiales;f_ Oxalobacteraceae;g_ | 6 |

(continued)

| Pearson | | | Taxonomy | OTU / Taxonomic Rank |
|---|---|---|---|---|
| q | p | R | | |
| 0.151044118 | 0.005339944 | 0.209740583 | k_Bacteria;p_Proteobacteria;c_ Deltaproteobacteria;o_Desulfovibrionales; f_Desulfovibrionaceae;g_ | 6 |
| 0.301018986 | 0.012162383 | 0.189182409 | k_Bacteria;p_Firmicutes;c_Clostridia;o_ Clostridiales;f_Christensenellaceae;g_ Christensenella | 6 |
| 0.430670623 | 0.019575937 | 0.176340986 | k_Bacteria;p_Firmicutes;c_Bacilli;o_ Lactobacillales;f_Streptococcaceae;g_ Lactococcus | 6 |
| 0.477991258 | 0.024632175 | 0.169847578 | k_Bacteria;p_Firmicutes;c_Clostiridia;o_ Clostridiales;f_Christensenettaceae;g_ | 6 |
| 0.477991258 | 0.02655507 | 0.167677541 | k_Bacteria;p_Firmicutes;c_Clostridia;o_ Clostridiales;f_[Mogibacteriaceae];g_ | 6 |
| 0.557356756 | 0.034002746 | 0.160370426 | k_Bacteria;p_Proteobacteria;c_ Epsilonproteobacteria;o_ Campylobacterales;f_ Campylobacteraceae;g_Campylobacter | 6 |
| 0.557356756 | 0.037119677 | 0.157712006 | k_Bacteria;p_TM7;c_TM7-3;o_CW040;f_; g_ | 6 |
| 0.557356756 | 0.039409064 | 0.155877014 | k_Bacteria;p_Bacteroidetes;c_Bacteroidia; o_Bacteroidales;f_Rikenellaceae;Other | 6 |
| 0.647008364 | 0.049015785 | 0.149037116 | k_Bacteria;p_Firmicutes;c_Clostridia;o_ SHA-98;f_;g | 6 |
| 7.40013E-06 | 2.2057E-09 | 0.432812556 | p_Firmicutes c_Clostridia o_Clostridiales f_ Lachnospiraceae g_s_ | 177702 |
| 1.09643E-05 | 6.53609E-09 | 0.421074192 | p_Firmicutes c_Clostridia o_Clostridiales f_ Ruminococcaeg_Oscillospiras_ | 306315 |
| 0.000420187 | 6.75487E-07 | 0.365134424 | p_Proteobacteria c_Gammaproteobacteria o_Enterobacteriales f_Enterobacteriaceae g_s_ | 4448331 |
| 0.000420187 | 7.13259E-07 | 0.364412146 | p_Firmicutes c_Clostridia o_Clostridiales f_ [Mogibacteriaceae]g_s_ | 214036 |
| 0.000420187 | 1.17315E-06 | 0.35772335 | p_Bacteroidetes c_Bacteroidia o_ Bacteroidales f_Rikenellaceae g_s_ | 512253 |
| 0.000420187 | 1.46422E-06 | 0.354694435 | p_Proteobacteria c_Alphaproteobacteria o_ Rhodospirillales f_Acetobacteraceae g_ Acidocella s_ | 53994 |
| 0.000420187 | 1.46422E-06 | 0.354694435 | p_Firmicutes c_Clostridia o_Clostridiales f_ Clostridiaceae g_Clostridium s_ | 181849 |
| 0.000420187 | 1.46422E-06 | 0.354694435 | p_Firmicutes c_Clostridia o_Clostridiales f_ Lachnospiraceae | 184021 |
| 0.000420187 | 1.46422E-06 | 0.354694435 | p_Bacteroidetes c_Bacteroidia o_ Bacteroidales f_[Odoribacteraceae]g_ Odoribacter s_ | 191251 |

(continued)

| Pearson | | | Taxonomy | OTU / Taxonomic Rank |
|---|---|---|---|---|
| q | p | R | | |
| 0.000420187 | 1.46422E-06 | 0.354694435 | p_Firmicutes c_Clostridia o_Clostridiales f_ g_ s_ | 324015 |
| 0.000420187 | 1.46422E-06 | 0.354694435 | p_Firmicutes c_Clostridia o_Clostridiales f_ Lachnospiraceae g_Epulopiscium s_ | 3654345 |
| 0.000420187 | 1.50291E-06 | 0.354336023 | p_Firmicutes c_Clostridia o_Clostridiales f_ Clostridiaceae | 336228 |
| 0.000715938 | 2.91704E-06 | 0.345069308 | p_Bacteroidetes c_ Bacteroidia o_ Bacteroidales f_[Odoribacteraceae] g_ Odoribacter s_ | 4444277 |
| 0.000715938 | 2.99029E-06 | 0.344716975 | p_Firmicutes c_Clostridia o_Clostridiales f_ Lachnospiraceae | 198031 |
| 0.000715938 | 3.20092E-06 | 0.343747738 | p_Firmicutes c_Clostridia o_Clostridiales f_ Ruminococcaceae g_ s | 343709 |
| 0.000757454 | 3.6123E-06 | 0.342018044 | p_Firmicutes c_Clostridia o_Clostridiales f_ Ruminococcaceae g_ s_ | 365688 |
| 0.001135849 | 6.40224E-06 | 0.333686942 | p_Firmicutes c_Clostridia o_Clostridiales f_ Lachnospiraceae g_ s_ | 195774 |
| 0.001135849 | 6.49116E-06 | 0.333483137 | p_Firmicutes c_Clostridia o_Clostridiales f_ Ruminococcaceae g_ s_ | 176337 |
| 0.001135849 | 6.54585E-06 | 0.333359087 | p_Firmicutes c_Clostridia o_Clostridiales f_ Ruminococcaceae g_Oscillospira s_ | 192945 |
| 0.001135849 | 6.77108E-06 | 0.332858399 | p_Firmicutes c_Clostridia o_Clostridiales f_ Ruminococcaceae g_ s_ | 510524 |
| 0.001395442 | 8.73451E-06 | 0.329061519 | p_Firmicutes c_Clostridia o_Clostridiales f_ Lachnospiraceae g_ s_ | 157611 |
| 0.001410342 | 9.24814E-06 | 0.32820245 | p_Firmicutes c_Clostridia o_Clostridiales f_ Ruminococcaceae g_Ruminococcus s_ | 174924 |
| 0.001926803 | 1.32091E-05 | 0.32278348 | p_Proteobacteria c_Alphaproteobacteria o_ RF32f_g_s_ | 370077 |
| 0.002960899 | 2.11808E-05 | 0.315441536 | p_Proteobacteria c_Deltaproteobacteria o_ Desulfovibrionales f_Desulfovibrionaceae g_Desulfovibrio s_ | 4453773 |
| 0.005544729 | 4.13169E-05 | 0.304710904 | p_Firmicutes c_Clostridia o_Clostridiales f_ Lachnospiraceae g_ s_ | 158742 |
| 0.006057101 | 4.73382E-05 | 0.30247412 | p_Firmicutes c_Clostridia o_Clostridiales f_ Ruminococcaceae g_ s_ | 359788 |
| 0.006057101 | 4.87457E-05 | 0.301990001 | p_Firmicutes c_Clostridia o_Clostridiales f_ Ruminococcaceae g_Ruminococcus s_ | 186133 |
| 0.006727873 | 5.69388E-05 | 0.299408561 | p_Actinobacteria c_Coriobacteriia o_ Coriobacteriales f_Coriobacteriaceae g_s_ | 2423305 |
| 0.006727873 | 5.99745E-05 | 0.298540032 | p_Firmicutes c_Clostridia o_Clostridiales | 195716 |
| 0.006727873 | 6.01598E-05 | 0.298488361 | p_Firmicutes c_Clostridia o_Clostridiales f_ Lachnospiraceae g_Roseburia s_ | 4450010 |

(continued)

| Pearson | | | Taxonomy | OTU / Taxonomic Rank |
|---|---|---|---|---|
| q | p | R | | |
| 0.007290794 | 6.73665E-05 | 0.296586508 | p_Firmicutes c_Clostridia o_Clostridiales f_Lachnospiraceae g_Anaerostipes s_ | 205765 |
| 0.007724875 | 7.48916E-05 | 0.294794375 | p_Firmicutes c_Clostridia o_Clostridiales f_Clostridiaceae g_s | 4387453 |
| 0.007724875 | 7.59824E-05 | 0.294548753 | p_Firmicutes c_Clostridia o_Clostridiales f_Lachnospiraceae | 176967 |
| 0.00853502 | 8.6495E-05 | 0.292337437 | p_Firmicutes c_Clostridia o_Clostridiales f_Ruminococcaceae g_s | 296821 |
| 0.008746661 | 9.12468E-05 | 0.291419493 | p_Firmicutes c_Clostridia o_Clostridiales f_Christensenellaceae g_s_ | 175037 |
| 0.008869928 | 9.51766E-05 | 0.29069357 | p_Firmicutes c_Clostridia o_Clostridiales f_Lachnospiraceae g_s | 157470 |
| 0.01104651 | 0.000121824 | 0.286404092 | p_Firmicutes c_Clostridia o_Clostridiales f_Lachnospiraceae g_s_ | 198102 |
| 0.013500854 | 0.000157842 | 0.281827961 | p_Firmicutes c_Clostridia o_Clostridiales f_Christensenellaceae g_Christensenella s_ | 1146771 |
| 0.013500854 | 0.00016284 | 0.281271882 | p_Firmicutes c_Clostridia o_Clostridiales f_Lachnospiraceae g_s_ | 183954 |
| 0.013500854 | 0.00016284 | 0.281271882 | p_Firmicutes c_Clostridia o_Clostridiales f_Lachnospiraceae g_s_ | 198450 |
| 0.013500854 | 0.000164988 | 0.28103778 | p_Firmicutes c_Clostridia o_Clostridiales f_Lachnospiraceae | 4425669 |
| 0.021869679 | 0.000273778 | 0.271827001 | p_Firmicutes c_Clostridia o_Clostridiales f_Lachnospiraceae g_s_ | 181756 |
| 0.025157435 | 0.000322435 | 0.268781312 | p_Firmicutes c_Clostridia o_Clostridiales f_Lachnospiraceae g_Anaerostipes s_ | 359020 |
| 0.027619067 | 0.000362217 | 0.266593219 | p_Firmicutes c_Clostridia o_Clostridiales f_Lachnospiraceae g_Coprococcus s_ | 197003 |
| 0.02876465 | 0.000385815 | 0.265398422 | p_Firmicutes c_Clostridia o_Clostridiales | 194610 |
| 0.028929779 | 0.000396653 | 0.264872223 | p_Firmicutes c_Clostridia o_Clostridiales f_Ruminococcaceae g_s_ | 688800 |
| 0.031091126 | 0.000435554 | 0.263087188 | p_Firmicutes c_Clostridia o_Clostridiales f_Clostridiaceae g_s_ | 192240 |
| 0.031196487 | 0.000446328 | 0.262618899 | p_Firmicutes c_Clostridia o_Clostridiales f_Ruminococcaceae g_Ruminococcus s_ | 177403 |
| 0.032243461 | 0.000470918 | 0.261588167 | p_Firmicutes c_Bacilli o_Lactobacillales f_Streptococcaceae g_Streptococcus s_ | 981894 |
| 0.034295897 | 0.000511116 | 0.260005764 | p_Firmicutes c_Bacilli o_Lactobacillales f_Streptococcaceae g_Lactococcus s_ | 288680 |
| 0.037292206 | 0.000566886 | 0.257991003 | p_Firmicutes c_Clostridia o_Clostridiales f_Ruminococcaceae g_s_ | 2066056 |
| 0.038157928 | 0.000595616 | 0.257023553 | p_Firmicutes c_Clostridia o_Clostridiales f_Lachnospiraceae g_s_ | 189271 |

(continued)

| Pearson | | | Taxonomy | OTU / Taxonomic Rank |
|---|---|---|---|---|
| q | p | R | | |
| 0.038157928 | 0.000614166 | 0.256421499 | p_Firmicutes c_Clostridia o_Clostridiales f_Clostridiaceae g_s_ | 174688 |
| 0.038157928 | 0.000614166 | 0.256421499 | p_Firmicutes c_Clostridia o_Clostridiales | 181759 |
| 0.038879206 | 0.000637364 | 0.255691802 | p_Firmicutes c_Clostridia o_Clostridiales f_Lachnospiraceae g_s_ | 192857 |
| 0.04115973 | 0.000687018 | 0.25420883 | p_Firmicutes c_Clostridia o_Clostridiales f_Lachnospiraceae g_s_ | 194053 |
| 0.044348114 | 0.000753455 | 0.252372017 | p_Firmicutes c_Clostridia o_Clostridiales f_[Mogibacteriaceae] g_s_ | 790466 |
| 0.045388164 | 0.000790456 | 0.251412748 | p_Firmicutes c_Clostridia o_Clostridiales f_Lachnospiraceae | 174008 |
| 0.045388164 | 0.000798182 | 0.251217676 | p_Firmicutes c_Clostridia o_Clostridiales f_Lachnospiraceae | 190226 |
| 0.04843324 | 0.000866168 | 0.249572259 | p_Firmicutes c_Clostridia o_Clostridiales f_Lachnospiraceae | 312586 |
| 0.048860657 | 0.000888376 | 0.24906047 | p_Firmicutes c_Clostridia o_Clostridiales f_Ruminococcaceae g_s_ | 158331 |
| 0.054341176 | 0.001005912 | 0.246533111 | p_Firmicutes c_Clostridia o_Clostridiales f_Lachnospiraceae g_Roseburia s_ | 4333509 |
| 0.054341176 | 0.001024819 | 0.246152126 | p_Firmicutes c_Clostridia o_Clostridiales f_Ruminococcaceae g_s_ | 175232 |
| 0.054341176 | 0.001047172 | 0.245709927 | p_Firmicutes c_Clostridia o_Clostridiales f_Lachnospiraceae g_Blautia s_ | 197208 |
| 0.054341176 | 0.00105281 | 0.245599779 | p_Firmicutes c_Clostridia o_Clostridiales f_Ruminococcaceae g_s | 561607 |
| 0.056376432 | 0.001109045 | 0.244529635 | p_Tenericutes c_RF3 o_ML615J-28f_g_s_ | 360268 |
| 0.075527934 | 0.001525068 | 0.237875353 | p_Firmicutes c_Clostridia o_Clostridiales f_Lachnospiraceae g_Anaerostipes s_ | 198478 |
| 0.075527934 | 0.00153082 | 0.237795632 | p_Firmicutes c_Clostridia o_Clostridiales f_Lachnospiraceae g_s_ | 258148 |
| 0.079635136 | 0.001663841 | 0.236024074 | p_Firmicutes c_Clostridia o_Clostridiales f_Lachnospiraceae | 179911 |
| 0.079635136 | 0.001702595 | 0.235532264 | p_Firmicutes c_Clostridia o_Clostridiales | 349482 |
| 0.079635136 | 0.001708383 | 0.235459686 | p_Firmicutes c Bacilli o_Lactobacillales f_Streptococcaceae g_Lactococcus s_ | 258548 |
| 0.079635136 | 0.00170901 | 0.235451837 | p_Firmicutes c_Clostridia o_Clostridiales f_Lachnospiraceae | 4375894 |
| 0.083255909 | 0.001821244 | 0.234087554 | p_Firmicutes c_Bacilli o_Lactobacillales f_Streptococcaceae g_Streptococcus s_ | 871442 |
| 0.083255909 | 0.0018365 | 0.233908061 | p_Firmicutes c_Clostridia o_Clostridiales f_Lachnospiraceae g_s | 198243 |

(continued)

| Pearson | | | Taxonomy | OTU / Taxonomic Rank |
|---|---|---|---|---|
| q | p | R | | |
| 0.083255909 | 0.00186116 | 0.233620763 | p_Firmicutes c_Clostridia o_Clostridiales f_Ruminococcaceae g_s | 176617 |
| 0.086377464 | 0.001956688 | 0.232539629 | p_Firmicutes c_Clostridia o_Clostridiales f_g_s_ | 389371 |
| 0.096302177 | 0.002390704 | 0.228163016 | p_Firmicutes c_Clostridia o_Clostridiales f_Lachnospiraceae g_Blautia s_ | 180629 |
| 0.096302177 | 0.002573692 | 0.226531334 | p_Firmicutes c_Bacilli o_Gemellales f_Gemellaceae g_s_ | 14920 |
| 0.096302177 | 0.002573692 | 0.226531334 | p_Proteobacteria c_Betaproteobacteria o_Burkholderiales f_Oxalobacteraceae g_s_ | 341939 |
| 0.096302177 | 0.002573692 | 0.226531334 | p_Firmicutes c_Clostridia o_Clostridiales f_Lachnospiraceae g_s_ | 364048 |
| 0.096302177 | 0.002573692 | 0.226531334 | p_Firmicutes c_Clostridia o_Clostridiales f_[Tissierellaceae] g_Peptoniphilus s_ | 521795 |
| 0.096302177 | 0.002573692 | 0.226531334 | p_Firmicutes c_Clostridia o_Clostridiales f_[Tissierellaceae] g_Anaerococcus s_ | 898755 |
| 0.096302177 | 0.002573692 | 0.226531334 | p_Firmicutes c_Bacilli o_Lactobacillales f_Streptococcaceae g_Streptococcus s_ | 1000872 |
| 0.096302177 | 0.002573692 | 0.226531334 | p_Firmicutes c_Bacilli o_Lactobacillales f_Streptococcaceae g_Streptococcus s_ | 1059655 |
| 0.096302177 | 0.002573692 | 0.226531334 | p_Firmicutes c_Bacilli o_Lactobacillales f_Streptococcaceae g_Streptococcus s_ | 4346722 |
| 0.096302177 | 0.002573692 | 0.226531334 | p_Proteobacteria c_Gammaproteobacteria o_Pasteurellales f_Pasteurellaceae g_Haemophilus s_parainfluenzae | 4383918 |
| 0.096302177 | 0.002573692 | 0.226531334 | p_Bacteroidetes c_Bacteroidia o_Bacteroidales f_Prevotellaceae g_Prevotella s_copri | 4406684 |
| 0.096302177 | 0.002573692 | 0.226531334 | p_Firmicutes c_Bacilli o_Lactobacillales f_Leuconostocaceae g_Weissella s_ | 4440018 |
| 0.096302177 | 0.002577614 | 0.226497533 | p Bacteroidetes c_Bacteroidia o_Bacteroidales f_Bacteroidaceae g_Bacteroides s_fragilis | 3550159 |
| 0.096302177 | 0.002583367 | 0.226448028 | p_Firmicutes c_Clostridia o_Clostridiales f_Ruminococcaceae g_s_ | 4333654 |
| 0.099568808 | 0.002718801 | 0.225310567 | p_Firmicutes c_Clostridia o_Clostridiales f_Lachnospiraceae g_s_ | 180999 |
| 0.099568808 | 0.002775855 | 0.224846693 | p_Firmicutes c_Clostridia o_Clostridiales f_Lachnospiraceae g_Dorea s_ | 187035 |
| 0.099568808 | 0.002831695 | 0.224400974 | p_Firmicutes c_Clostridia o_Clostridiales f_Ruminococcaceae g_Ruminococcus s_ | 180509 |
| 0.099568808 | 0.002859886 | 0.224178968 | p_Firmicutes c_Clostridia o_Clostridiales f_g_s_ | 534874 |

(continued)

| Pearson | | | Taxonomy | OTU / Taxonomic Rank |
|---|---|---|---|---|
| q | p | R | | |
| 0.099568808 | 0.002867967 | 0.224115693 | p_Bacteroidetes c_Bacteroidia o_ Bacteroidales f_Porphyromonadaceae g_ Parabacteroides s_gordonii | 150412 |
| 0.099568808 | 0.002876843 | 0.224046381 | p_Firmicutes c_Clostridia o_Clostridiales f_ Ruminococcaceae g_s_ | 409786 |
| 0.099568808 | 0.002900718 | 0.223860903 | p_Firmicutes c_Clostridia o_Clostridiales f_ Lachnospiraceae g_Dorea s_ | 185961 |
| 0.099568808 | 0.002908418 | 0.223801372 | p_Firmicutes c_Clostridia o_Clostridiales f_ Lachnospiraceae | 195086 |
| 0.100252368 | 0.002958267 | 0.223419422 | p_Firmicutes c_Clostridia o_Clostridiales f_ Ruminococcaceae g_s_ | 178675 |
| 0.102033039 | 0.003065083 | 0.222620195 | p_Firmicutes c_Clostridia o_Clostridiales f_ Ruminococcaceae g_s_ | 194659 |
| 0.102033039 | 0.003085406 | 0.222470987 | p_Firmicutes c_Clostridia o_Clostridiales f_ Lachnospiraceae | 184120 |
| 0.102033039 | 0.003102048 | 0.222349469 | p_Firmicutes c_Clostridia o_Clostridiales f_ Lachnospiraceae g_Roseburia s_ | 189411 |
| 0.11369529 | 0.003490496 | 0.219668267 | p_Firmicutes c_Clostridia o_Clostridiales f_ g_s_ | 174516 |
| 0.118313702 | 0.003667548 | 0.218534595 | p_Firmicutes c_Clostridia o_Clostridiales f_ Lachnospiraceae g_Roseburia s_ | 178751 |
| 0.124517454 | 0.00389697 | 0.217136815 | p_Firmicutes c_Clostridia o_Clostridiales f_ Lachnospiraceae | 191658 |
| 0.125196388 | 0.003955534 | 0.216791895 | p_Bacteroidetes c_Bacteroidia o_ Bacteroidales f_[Barnesiellaceae] g_s_ | 180133 |
| 0.126581838 | 0.004037036 | 0.216319454 | p_Bacteroidetes c_Bacteroidia o_ Bacteroidales f_Bacteroidaceae g_ Bacteroides s_ | 581814 |
| 0.130210026 | 0.00419156 | 0.215446822 | p_Proteobacteria c_Deltaproteobacteria o_ Desulfovibrionales f_Desulfovibrionaceae g_s_ | 295094 |
| 0.139730206 | 0.00453967 | 0.213582345 | p_Firmicutes c_Clostridia o_Clostridiales f_ Lachnospiraceae g_Coprococcus s_ | 183057 |
| 0.14185431 | 0.004650961 | 0.213013337 | p_Firmicutes c_Clostridia o_Clostridiales f_ Ruminococcaceae g_s_ | 182674 |
| 0.143053688 | 0.004732924 | 0.212602041 | p_Firmicutes c_Clostridia_o_Clostridiales f_ Lachnospiraceae g_s_ | 358104 |
| 0.148135382 | 0.004971766 | 0.211438941 | p_Firmicutes c_Clostridia o_Clostridiales f_ Lachnospiraceae g_s_ | 180190 |
| 0.148135382 | 0.004995013 | 0.211328426 | p_Firmicutes c_Clostridia o_Clostridiales f_ Ruminococcaceae g_s_ | 315589 |
| 0.148135382 | 0.005033512 | 0.211146412 | p_Firmicutes c_Bacilli o_Lactobacillales f_ Streptococcaceae g_Streptococcus s_ | 1082539 |

(continued)

| Pearson | | | Taxonomy | OTU / Taxonomic Rank |
|---|---|---|---|---|
| q | p | R | | |
| 0.149076862 | 0.005109937 | 0.210788763 | p_Firmicutes c_Clostridia o_Clostridiales | 367312 |
| 0.150103098 | 0.005189854 | 0.210419864 | p_Firmicutes c_Clostridia o_Clostridiales f_Lachnospiraceae g_Anaerostipes s_ | 197529 |
| 0.1515203 | 0.005284016 | 0.209991677 | p_Firmicutes c_Clostridia o_Clostridiales f_Ruminococcaceae g_Ruminococcus | 174654 |
| 0.151824991 | 0.005339895 | 0.209740798 | p_Firmicutes c_Bacilli o_Lactobacillales f_Streptococcaceae g_Streptococcus s_ | 15564 |
| 0.154154888 | 0.005467789 | 0.209175326 | p_Firmicutes c_Clostridia o_Clostridiales f_Lachnospiraceae | 177172 |
| 0.162468389 | 0.005845895 | 0.207570159 | p_Firmicutes c_Clostridia o_Clostridiales f_g_s_ | 185140 |
| 0.162468389 | 0.005859516 | 0.207514085 | p_Firmicutes c_Bacilli o_Lactobacillales f_Streptococcaceae g_Streptococcus s_ | 4384044 |
| 0.165740782 | 0.006026938 | 0.206834199 | p_Firmicutes c_Clostridia o_Clostridiales f_Clostridiaceae g_Clostridium s_ | 69664 |
| 0.169454014 | 0.006212472 | 0.206100187 | p_Firmicutes c_Clostridia o_Clostridiales f_Lachnospiraceae | 1917420 |
| 0.170127797 | 0.006298895 | 0.20576494 | p_Firmicutes c_Clostridia o_Clostridiales f_Ruminococcaceae g_Oscillospira s_ | 196831 |
| 0.170127797 | 0.006338592 | 0.205612323 | p_Firmicutes c_Clostridia o_Clostridiales f_g_s_ | 191945 |
| 0.176909046 | 0.006643976 | 0.204465954 | p_Firmicutes c_Clostridia o_Clostridiales | 342575 |
| 0.181854498 | 0.006883911 | 0.203597766 | p_Firmicutes c_Clostridia o_Clostridiales f_Ruminococcaceae | 158836 |
| 0.190415465 | 0.007264733 | 0.202273599 | p_Firmicutes c_Clostridia o_Clostridiales f_Ruminococcaceae g_Ruminococcus s_ | 369797 |
| 0.19577861 | 0.007527702 | 0.201394814 | p_Firmicutes c_Clostridia o_Clostridiales f_Lachnospiraceae g_s_ | 688701 |
| 0.208869508 | 0.008093304 | 0.199593549 | p_Firmicutes c_Clostridia o_Clostridiales f_Ruminococcaceae g_Ruminococcus s_ | 169600 |
| 0.209533959 | 0.008181505 | 0.199322796 | p_Firmicutes c_Bacilli o_Lactobacillales f_Streptococcaceae g_Streptococcus s_ | 4321136 |
| 0.213602693 | 0.00840404 | 0.198651007 | p_Firmicutes c_Clostridia o_Clostridiales f_Lachnospiraceae g_s_ | 4448492 |
| 0.213797458 | 0.008475428 | 0.198438841 | p_Firmicutes c_Clostridia o_Clostridiales f_Ruminococcaceae g_s | 194471 |
| 0.225946532 | 0.009093004 | 0.196666721 | p_Firmicutes c_Clostridia o_Clostridiales f_Ruminococcaceae g_s_ | 194567 |
| 0.225946532 | 0.00913785 | 0.196542228 | p_Firmicutes c_Clostridia o_Clostridiales f_Lachnospiraceae | 180654 |
| 0.225946532 | 0.009263992 | 0.196194935 | p_Firmicutes c_Clostridia o_Clostridiales f_Lachnospiraceae | 180610 |

(continued)

| Pearson | | | Taxonomy | OTU / Taxonomic Rank |
|---|---|---|---|---|
| q | p | R | | |
| 0.225946532 | 0.009364911 | 0.195920082 | p_Firmicutes c_Clostridia o_Clostridiales f_[Tissierellaceae] g_WAL_1855D s_ | 992686 |
| 0.225946532 | 0.00940136 | 0.195821457 | p_Firmicutes c_Clostridia o_Clostridiales f_[Mogibacteriaceae] g_s_ | 216010 |
| 0.225946532 | 0.009403186 | 0.195816525 | p_Firmicutes c_Clostridia o_Clostridiales f_g_s_ | 520358 |
| 0.225946532 | 0.009428469 | 0.195748323 | p_Firmicutes c_Clostridia o_Clostridiales f_Lachnospiraceae g_s_ | 183969 |
| 0.226837352 | 0.009576047 | 0.195353425 | p_Firmicutes c_Clostridia o_Clostridiales f_Ruminococcaceae g_s_ | 302160 |
| 0.226837352 | 0.009600866 | 0.195287544 | p_Bacteroidetes c_Bacteroidia o_Bacteroidales f_Prevotellaceae g_Prevotella s_ | 134265 |
| 0.231616992 | 0.009893888 | 0.194520872 | p_Firmicutes c_Bacilli o_Lactobacillales f_Lactobacillaceae g_Lactobacillus s_zeae | 4480189 |
| 0.231616992 | 0.009941236 | 0.194398877 | p_Firmicutes c_Clostridia o_Clostridiales f_Lachnospiraceae | 178513 |
| 0.233822876 | 0.010105609 | 0.193979304 | p_Firmicutes c_Clostridia_o_Clostridiales f_Lachnospiraceae g_Dorea s_ | 182653 |
| 0.235982923 | 0.010326272 | 0.193425419 | p_Firmicutes c_Clostridia o_Clostridiales f_Ruminococcaceae g_s_ | 183715 |
| 0.235982923 | 0.010339639 | 0.193392201 | p_Firmicutes c_Clostridia o_Clostridiales f_Ruminococcaceae g_s_ | 366702 |
| 0.237303757 | 0.010468243 | 0.193074551 | p_Firmicutes c_Clostridia o_Clostridiales f_Lachnospiraceae g_Coprococcus s_ | 185312 |
| 0.238415436 | 0.01062496 | 0.192692082 | p_Firmicutes c_Clostridia o_Clostridiales f_Ruminococcaceae g_s_ | 233253 |
| 0.238415436 | 0.010659408 | 0.192608678 | p_Firmicutes c_Bacilli o_Lactobacillales f_Streptococcaceae g_Streptococcus s_ | 950828 |
| 0.238663073 | 0.010741617 | 0.192410597 | p_Firmicutes c_Clostridia o_Clostridiales f_Ruminococcaceae g_s_ | 187868 |
| 0.23984681 | 0.010866383 | 0.192112509 | p_Firmicutes c_Bacilli o_Lactobacillales f_Lactobacillaceae g_Lactobacillus s_ | 129798 |
| 0.247863907 | 0.011303481 | 0.191091458 | p_Bacteroidetes c_Bacteroidia o_Bacteroidales f_Rikenellaceae g_s_ | 107044 |
| 0.261448717 | 0.012000925 | 0.18953203 | p_Firmicutes c_Clostridia o_Clostridiales f_Lachnospiraceae g_s_ | 195599 |
| 0.287768124 | 0.013294802 | 0.18683879 | p_Lentisphaerae c_[Lentisphaeria] o_Victivallales f_Victivallaceae g_s_ | 4329575 |
| 0.302673859 | 0.014073658 | 0.185326483 | p_Firmicutes c_Clostridia o_Clostridiales f_Lachnospiraceae | 332846 |
| 0.323696758 | 0.015147658 | 0.183356938 | p_Firmicutes c_Clostridia o_Clostridiales f_Lachnospiraceae g_Lachnospira s_ | 4424598 |

(continued)

| Pearson | | | Taxonomy | OTU / Taxonomic Rank |
|---|---|---|---|---|
| q | p | R | | |
| 0.330868054 | 0.015581864 | 0.182595152 | p_Firmicutes c_Bacilli o_Lactobacillales f_ Streptococcaceae g_Streptococcus s_ | 4322712 |
| 0.338949108 | 0.016118761 | 0.181678396 | p_Firmicutes c_Clostridia o_Clostridiales f_ Ruminococcaceae g_s_ | 179664 |
| 0.338949108 | 0.016164488 | 0.181601552 | p_Firmicutes c_Clostridia o_Clostridiales f_ Lachnospiraceae | 177567 |
| 0.340856911 | 0.016357068 | 0.181279989 | p_Firmicutes c_Clostridia o_Clostridiales f_ Ruminococcaceae g_s_ | 850841 |
| 0.34855419 | 0.016830336 | 0.180503528 | p_Firmicutes c_Clostridia o_Clostridiales f_ Lachnospiraceae g_s_ | 181155 |
| 0.353319387 | 0.017165741 | 0.179964676 | p_Firmicutes c_Clostridia o_Clostridiales f_ Lachnospiraceae g_s_ | 175222 |
| 0.3633988 | 0.0177&a63757 | 0.179026233 | p_Firmicutes c_Clostridia o_Clostridiales f_ Ruminococcaceae g_s_ | 187924 |
| 0.369942351 | 0.018193886 | 0.178368072 | p_Firmicutes c_Clostridia o_Clostridiales f_ Lachnospiraceae | 179551 |
| 0.373173167 | 0.01848682 | 0.177 2753 | p_Firmicutes c_Clostridia o_Clostridiales f_ Lachnospiraceae | 3563387 |
| 0.373173167 | 0.018575237 | 0.177795751 | p_Bacteroidetes c_Bacteroidia o_ Bacteroidales f_Bacteroidaceae g_ Bacteroides s_caccae | 184610 |
| 0.374415745 | 0.018924062 | 0.177281099 | p_Firmicutes c_Clostridia o_Clostridiales f_ Dehalobacteriaceae g_Dehalobacterium s_ | 1056816 |
| 0.374415745 | 0.018946189 | 0.177248732 | p_Firmicutes c_Clostridia o_Clostridiales f_ g_s_ | 553842 |
| 0.374415745 | 0.018971886 | 0.177211183 | p_Firmicutes c_Clostridia o_Clostridiales f_ Lachnospiraceae | 193235 |
| 0.380595909 | 0.01939848 | 0.17659419 | p_Firmicutes c_Clostridia o_Clostridiales f_ Lachnospiraceae | 192711 |
| 0.391221451 | 0.020056659 | 0.175664921 | p_Firmicutes c_Clostridia o_Clostridiales | 185158 |
| 0.396931162 | 0.020549535 | 0.174986163 | p_Firmicutes c Clostridia o_Clostridiales f_ g_s_ | 177704 |
| 0.396931162 | 0.020660905 | 0.174834744 | p_Firmicutes c_Clostridia o_Clostridiales f_ Lachnospiraceae | 189987 |
| 0.396931162 | 0.020704308 | 0.174775923 | p_Firmicutes c_Clostridia o_Clostridiales f_ Ruminococcaceae g_s | 182129 |
| 0.397046671 | 0.020882053 | 0.174536148 | p_Firmicutes c_Clostridia o_Clostridiales f_ Dehalobacteriaceae g_Dehalobacterium s_ | 121873 |
| 0.397046671 | 0.020947023 | 0.174448945 | p_Firmicutes c_Clostridia o_Clostridiales f_ Lachnospiraceae | 187256 |
| 0.397247021 | 0.021075997 | 0.174276524 | p_Firmicutes c_Clostridia o_Clostridiales f Ruminococcaceae g_s | 578147 |

(continued)

| Pearson | | | Taxonomy | OTU / Taxonomic Rank |
|---|---|---|---|---|
| q | p | R | | |
| 0.399472241 | 0.021313124 | 0.173961882 | p_Firmicutes c_Clostridia o_Clostridiales f_ Lachnospiraceae | 185821 |
| 0.405720688 | 0.021767429 | 0.173367417 | p_Firmicutes c_Clostridia o_Clostridiales f Lachnospiraceae g_s | 183147 |
| 0.40791776 | 0.02200689 | 0.173058374 | p_Firmicutes c_Clostridia o_Clostridiales | 367889 |
| 0.408438108 | 0.022156702 | 0.172866501 | p_Firmicutes c_Clostridia o_Clostridiales f_ Ruminococcaceae | 196942 |
| 0.410374068 | 0.02238404 | 0.172577463 | p_Firmicutes c_Clostridia o_Clostridiales f Ruminococcaceae g_s | 212532 |
| 0.413966807 | 0.022703396 | 0.172175677 | p_Firmicutes c_Clostridia o_Clostridiales f Lachnospiraceae g Anaerostipes s | 188694 |
| 0.419538416 | 0.023134011 | 0.171641552 | p_Firmicutes c_Clostridia o_Clostridiales f_ Lachnospiraceae | 353791 |
| 0.425106144 | 0.023567733 | 0.171112156 | p_Firmicutes c_Clostridia o_Clostridiales f_ Ruminococcaceae g_s | 179785 |
| 0.428491822 | 0.023883151 | 0.170732399 | p_Firmicutes c_Clostridia o_Clostridiales f_ Christensenellaceae g_s_ | 184940 |
| 0.435048295 | 0.024471323 | 0.170035623 | p_Firmicutes c_Clostridia o_Clostridiales f_ Ruminococcaceae g s | 179647 |
| 0.435048295 | 0.024507937 | 0.169992727 | p_Firmicutes c_Clostridia o_Clostridiales f_ Ruminococcaceae g_Ruminococcus s_ | 192383 |
| 0.438925631 | 0.024898337 | 0.169538722 | p_Firmicutes c_Clostridia o_Clostridiales f_ [Mogibacteriaceae] g_s_ | 189936 |
| 0.438925631 | 0.024988017 | 0.169435296 | p_Firmicutes c_Clostridia o_Clostridiales f_ Ruminococcaceae g_s | 165346 |
| 0.454192137 | 0.026231528 | 0.168033032 | p_Firmicutes c_Clostridia o_Clostridiales f_ Lachnospiraceae | 194110 |
| 0.454192137 | 0.026269149 | 0.167991503 | p_Firmicutes c_Clostridia o_Clostridiales f_ Ruminococcaceae g_s | 3524537 |
| 0.454192137 | 0.026374146 | 0.16787587 | p_Firmicutes c_Clostridia o_Clostridiales | 173876 |
| 0.454192137 | 0.026398649 | 0.167848942 | p_Firmicutes c_Clostridia o_Clostridiales f_ Lachnospiraceae g_s | 4428949 |
| 0.455978219 | 0.02663837 | 0.167586616 | p_Firmicutes c_Clostridia o_Clostridiales f_ Lachnospiraceae | 197004 |
| 0.457142807 | 0.026842663 | 0.167364649 | p_Firmicutes c_Clostridia o_Clostridiales f_ Lachnospiraceae g_Coprococcus s_ | 193163 |
| 0.458537809 | 0.027061248 | 0.167128749 | p_Firmicutes c_Clostridia o_Clostridiales f_ g_s_ | 176947 |
| 0.463547189 | 0.027495049 | 0.166665372 | p_Bacteroidetes c_Bacteroidia o_ Bacteroidales f_Rikenellaceae g_s_ | 357046 |
| 0.46501124 | 0.027811613 | 0.166331155 | p_Firmicutes c_Clostridia o_Clostridiales f_ Lachnospiraceae | 176323 |

(continued)

| Pearson | | | Taxonomy | OTU / Taxonomic Rank |
|---|---|---|---|---|
| q | p | R | | |
| 0.46501124 | 0.027859094 | 0.166281308 | p_Firmicutes c_Clostridia o_Clostridiales f_Lachnospiraceae | 318865 |
| 0.47031335 | 0.028594899 | 0.165517956 | p_Firmicutes c_Clostridia o_Clostridiales f_Ruminococcaceae g_Oscillospira s_ | 250784 |
| 0.47031335 | 0.028691156 | 0.16541934 | p_Firmicutes c_Clostridia o_Clostridiales | 185765 |
| 0.47031335 | 0.028827255 | 0.165280386 | p_Firmicutes c_Clostridia o_Clostridiales f_Veillonellaceae g_Megasphaera s_ | 817140 |
| 0.47031335 | 0.028874024 | 0.165232765 | p_Firmicutes c_Clostridia o_Clostridiales f_Lachnospiraceae | 187505 |
| 0.47031335 | 0.02887766 | 0.165229065 | p_Firmicutes c_Clostridia o_Clostridiales f_Ruminococcaceae g_s_ | 177070 |
| 0.473284067 | 0.029201133 | 0.164901526 | p_Firmicutes c_Clostridia o_Clostridiales f_Ruminococcaceae g_s_ | 196787 |
| 0.474354369 | 0.029408557 | 0.164693119 | p_Firmicutes c_Clostridia o_Clostridiales f_Lachnospiraceae g_Dorea s_ | 198490 |
| 0.477917311 | 0.02984774 | 0.164255961 | p_Firmicutes c_Clostridia o_Clostridiales f_Lachnospiraceae g_s_ | 191974 |
| 0.477917311 | 0.029987642 | 0.164117854 | p_Firmicutes c_Clostridia o_Clostridiales f_Ruminococcaceae g_s_ | 363017 |
| 0.477917311 | 0.030056797 | 0.16404979 | p_Firmicutes c_Clostridia o_Clostridiales f_Lachnospiraceae g_s_ | 198270 |
| 0.481022925 | 0.030573039 | 0.163545861 | p_Firmicutes c_Clostridia o_Clostridiales f_[Tissierellaceae] g_Peptoniphilus s_ | 1064036 |
| 0.481022925 | 0.030599875 | 0.163519863 | p_TM7 c_TM7-3 o_CW040 f_g_s_ | 4365684 |
| 0.481022925 | 0.030682237 | 0.163440196 | p_Firmicutes c_Clostridia o_Clostridiales f_g_s_ | 187894 |
| 0.48202733 | 0.030889978 | 0.163240059 | p_Firmicutes c_Bacilli o_Lactobacillales f_Streptococcaceae g_Streptococcus s_ | 152823 |
| 0.486916365 | 0.031348416 | 0.162802421 | p_Bacteroidetes c_Bacteroidia o_Bacteroidales f_Bacteroidaceae g_Bacteroides s_ | 3562626 |
| 0.494806999 | 0.03200391 | 0.162186028 | p_Firmicutes c_Clostridia o_Clostridiales | 178742 |
| 0.498186715 | 0.032371 | 0.161845509 | p_Firmicutes c_Clostridia o_Clostridiales f_Veillonellaceae g_Megasphaera s_ | 264967 |
| 0.498925377 | 0.032567707 | 0.161664386 | p_Proteobacteria c_Epsilonproteobacteria o_Campylobacterales f_Campylobacteraceae g_Campylobacter s_ | 246717 |
| 0.500167278 | 0.032797854 | 0.161453649 | p_Firmicutes c_Clostridia o_Clostridiales f_Ruminococcaceae | 158553 |
| 0.508557336 | 0.033499604 | 0.160818739 | p_Bacteroidetes c_Bacteroidia o_Bacteroidales f_Bacteroidaceae g_Bacteroides s_ | 196131 |

(continued)

| Pearson | | | Taxonomy | OTU / Taxonomic Rank |
|---|---|---|---|---|
| q | p | R | | |
| 0.511346468 | 0.033835742 | 0.160518604 | p_Firmicutes c_Clostridia o_Clostridiales f_Lachnospiraceae g_s_ | 189914 |
| 0.518736623 | 0.03459983 | 0.159845646 | p_Firmicutes c_Clostridia o_Clostridiales f_Lachnospiraceae g_s | 296661 |
| 0.518736623 | 0.034653803 | 0.159798587 | p_Firmicutes c_Clostridia o_Clostridiales f_g_s_ | 177153 |
| 0.518736623 | 0.034788596 | 0.159681334 | p_Firmicutes c_Clostridia o_Clostridiales f_Lachnospiraceae g_Coprococcus s_ | 180898 |
| 0.524709474 | 0.035345556 | 0.159200886 | p_Firmicutes c_Clostridia o_Clostridiales f_Lachnospiraceae g_s | 329703 |
| 0.549741435 | 0.03719562 | 0.157649625 | p_Firmicutes c_Clostridia o_Clostridiales f_Lachnospiraceae g_s | 188099 |
| 0.555352116 | 0.037830152 | 0.15713257 | p_Firmicutes c_Bacilli o_Lactobacillales f_Streptococcaceae g_Streptococcus s_infantis | 711275 |
| 0.555352116 | 0.037906299 | 0.157071013 | p_Firmicutes c_Clostridia o_Clostridiales f_Lachnospiraceae | 196312 |
| 0.573693262 | 0.039329195 | 0.155939499 | p_Firmicutes c_Clostridia o_Clostridiales f_Lachnospiraceae g_s | 197458 |
| 0.574732455 | 0.039571743 | 0.155750071 | p_Bacteroidetes c_Bacteroidia o_Bacteroidales f_Bacteroidaceae g_Bacteroides s_ | 182874 |
| 0.584610405 | 0.040446639 | 0.155074798 | p_Firmicutes c_Clostridia o_Clostridiales | 180781 |
| 0.584610405 | 0.040600365 | 0.154957418 | p_Firmicutes c_Clostridia o_Clostridiales f_Lachnospiraceae g_s_ | 195843 |
| 0.594004262 | 0.041429805 | 0.154330453 | p_Firmicutes c_Clostridia o_Clostridiales | 179549 |
| 0.615656015 | 0.04347208 | 0.152830471 | p_Firmicutes c_Clostridia o_Clostridiales | 197864 |
| 0.615656015 | 0.043629034 | 0.152717654 | p_Firmicutes c_Clostridia o_Clostridiales f_Lachnospiraceae g_Coprococcus s_ | 290963 |
| 0.615656015 | 0.04366299 | 0.152693291 | p_Firmicutes c_Clostridia o_Clostridiales f_g_s_ | 195828 |
| 0.615656015 | 0.043673959 | 0.152685425 | p_Firmicutes c_Clostridia o_Clostridiales f_Lachnospiraceae | 4366867 |
| 0.623041699 | 0.044383596 | 0.15217998 | p_Firmicutes c_Clostridia o_Clostridiales f_Lachnospiraceae g_Blautia s_ | 194063 |
| 0.623129688 | 0.044575596 | 0.15204439 | p_Firmicutes c_Clostridia o_Clostridiales f_Lachnospiraceae | 199300 |
| 0.649317245 | 0.046642461 | 0.150614849 | p_Firmicutes c_Clostridia o_Clostridiales f_Ruminococcaceae g_s_ | 322258 |
| 0.654519765 | 0.047211262 | 0.150230731 | p_Firmicutes c_Clostridia o_Clostridiales f_Ruminococcaceae g_s | 3609545 |
| 0.655957565 | 0.047510488 | 0.150030206 | p_Firmicutes c_Clostridia o_Clostridiales f_Ruminococcaceae g_Oscillospira s_ | 146586 |

(continued)

| Pearson | | | Taxonomy | OTU / Taxonomic Rank |
|---|---|---|---|---|
| q | p | R | | |
| 0.6562387 | 0.047827827 | 0.14981869 | p_Fusobacteria c_Fusobacteriia o_ Fusobacteriales f_Fusobacteriaceae g_ Fusobacterium s_ | 257278 |
| 0.6562387 | 0.047950677 | 0.149737121 | p_Firmicutes c_Clostridia o_Clostridiales f_ Ruminococcaceae g_s_ | 338156 |
| 0.6562387 | 0.048197587 | 0.149573704 | p_Firmicutes c_Bacilli o_Lactobacillales f_ Streptococcaceae g_Streptococcus s_ | 521996 |
| 0.6562387 | 0.048313252 | 0.149497391 | p_Firmicutes c_Clostridia o_Clostridiales | 188725 |
| 0.664099917 | 0.049089949 | 0.148988848 | p_Firmicutes c_Clostridia o_Clostridiales f_ Lachnospiraceae | 177040 |

Finally, as an initial assessment of whether the relationship between human NAS consumption and blood glucose control is causative, seven healthy volunteers (5 males and 2 females, aged 28-36) who do not normally consume NAS or NAS-containing foods were followed for one week. During this week, participants consumed on days 2-7 the FDA's maximal acceptable daily intake (ADI) of commercial saccharin ($5 mgkg^{-1}$) as 3 divided daily doses equivalent to 120mg, and were monitored by continuous glucose measurements and daily GTT (Figure 27A). Notably, even in this short-term 7-day exposure period, most individuals (4 of 7) developed significantly poorer glycemic responses 5-7 days following NAS consumption (hereinafter termed 'NAS responders'), as compared to their individual glycemic response on days 1-4 (Figures. 26B-C and 27B, p<0.001). None of the 3 NAS non-responders featured improved glucose tolerance (Figure 26B, D and 27C).

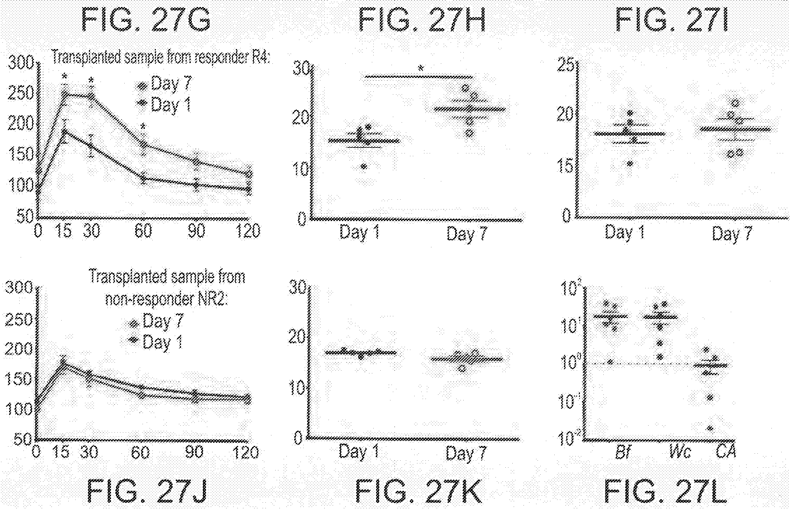

The microbiome configurations of NAS responders, as assessed by 16S rRNA analysis, clustered differently from non-responders both prior to and following NAS consumption (Figure 26E and 27D, respectively). Moreover, microbiomes from non-responders featured little changes in composition during the study week, while pronounced compositional changes were observed in NAS responders (Figure 26F and 27E). To study whether this NAS-induced dysbiosis has a causal role in generating glucose intolerance, stool from before (day 1, D1) or after (day 7, D7) NAS exposure were transferred from two NAS responders and two NAS non-responders into groups of NC-fed germ-free mice. Indeed, transfer of post-NAS exposure (D7) stool from NAS responders induced significant glucose intolerance in recipient GF mice, as compared to the response noted with D1 stool transferred from the same NAS-responding individuals (Figure 26G and 27F, P<0.004 and 27G-H, P<0.02). In contrast, D7 stools transferred into GF mice from the two NAS non-responders induced normal glucose tolerance, which was indistinguishable from that of mice transferred with D1 stools from the same 'non-responding' individuals (Figure 26H and Figures 27I-K). GF mice transplanted with 'responders' microbiome replicated some of the donor saccharin-induced dysbiosis, including 20-fold relative increase of *Bacteroides fragilis* (Bacteroidales order) and *Weissella cibaria* (Lactobacillales), and ~10-fold decrease in *Candidatus Arthromitus* (Clostridiales)(Figure 27I).

**CONCLUSION**

In summary, our results suggest that NAS consumption in both mice and humans enhances the risk of glucose intolerance and that these adverse metabolic effects are mediated by modulation of the composition and function of the microbiota. Notably, several of the bacterial taxa that changed following NAS consumption were previously associated with T2DM in humans[13,20], including over-representation of *Bacteroides* and under-representation of Clostridiales. Both gram positive and negative taxa contributed to the NAS-induced phenotype (Figures 16A-B) and were enriched for glycan degradation pathways (Figure 21), previously linked to enhanced energy harvest (Figures. 22C-D)[11,24]. This suggests that elaborate inter-species microbial cooperation may functionally orchestrate the gut ecosystem and contribute to vital community activities in diverging environmental conditions (e.g. normal chow vs. high fat dietary conditions). In addition, we show that metagenomes of saccharin-consuming mice are enriched with multiple additional pathways previously shown to associate with diabetes mellitus[23] or obesity[11] in mice and humans, including sphingolipid metabolism and lipopolysaccharide biosynthesis[25].

Our results from short and long-term human NAS consumer cohorts (Figure 26, Figure 27 & Tables 4-5) suggest that human individuals feature a personalized response to NAS, possibly stemming from differences in their microbiota composition and function. The changes noted in our studies may be further substantiated in mice consuming different human diets[26]. Artificial sweeteners were extensively introduced into our diets with the intention of reducing caloric intake and normalizing blood glucose levels without compromising the human 'sweet-tooth'. Together with other major shifts that occurred in human nutrition, this increase in NAS consumption coincides with the dramatic increase in the obesity and diabetes epidemics. Our findings suggest that NAS may have directly contributed to enhancing the exact epidemic that they themselves were intended to fight. Moreover, our results point towards the need to develop new nutritional strategies tailored to the individual while integrating personalized differences in the composition and function of the gut microbiota.

***References for Example 2***

1 Gardner, C. et al. Nonnutritive Sweeteners: Current Use and Health Perspectives A Scientific Statement from the American Heart Association and the American Diabetes Association. Diabetes care 35, 1798-1808 (2012).

2 Fitch, C. & Keim, K. S. Position of the academy of nutrition and dietetics: use of nutritive and nonnutritive sweeteners. Journal of the Academy of Nutrition and Dietetics 112, 739-758 (2012).

3 Tordoff, M. G. & Alleva, A. M. Effect of drinking soda sweetened with aspartame or high-fructose corn syrup on food intake and body weight. The American journal of clinical nutrition 51, 963-969 (1990).

4 Horwitz, D. L., McLane, M. & Kobe, P. Response to single dose of aspartame or saccharin by NIDDM patients. Diabetes Care 11, 230-234 (1988).

5 Nettleton, J. A. et al. Diet soda intake and risk of incident metabolic syndrome and type 2 diabetes in the Multi-Ethnic Study of Atherosclerosis (MESA). Diabetes Care 32, 688-694 (2009).

6 Roberts, A., Renwick, A. G., Sims, J. & Snodin, D. J. Sucralose metabolism and pharmacokinetics in man. Food and chemical toxicology : an international journal published for the British Industrial Biological Research Association 38 Suppl 2, S31-41 (2000).

7 Byard, J. L. & Goldberg, L. The metabolism of saccharin in laboratory animals. Food and cosmetics toxicology 11, 391-402 (1973).

8 Clemente, J. C., Ursell, L. K., Parfrey, L. W. & Knight, R. The impact of the gut microbiota on human health: an integrative view. Cell 148, 1258-1270, doi:10.1016/j.cell.2012.01.035 (2012).

9 Claesson, M. J. et al. Gut microbiota composition correlates with diet and health in the elderly. Nature 488, 178-184, doi:10.1038/nature11319 (2012).

10 Muegge, B. D. et al. Diet drives convergence in gut microbiome functions across mammalian phylogeny and within humans. Science 332, 970-974 (2011).

11 Turnbaugh, P. J. et al. An obesity-associated gut microbiome with increased capacity for energy harvest. Nature 444, 1027-1131 (2006).

12 Ley, R. E., Turnbaugh, P. J., Klein, S. & Gordon, J. I. Microbial ecology: human gut microbes associated with obesity. Nature 444, 1022-1023 (2006).

13 Qin, J. et al. A metagenome-wide association study of gut microbiota in type 2 diabetes. Nature 490, 55-60 (2012).

14 Henao-Mejia, J. et al. Inflammasome-mediated dysbiosis regulates progression of NAFLD and obesity. Nature 482, 179-185 (2012).

15 David, L. A. et al. Diet rapidly and reproducibly alters the human gut microbiome. Nature (2013).

16 Peterson, J. et al. The NIH human microbiome project. Genome research 19, 2317-2323 (2009).

17 Koropatkin, N. M., Cameron, E. A. & Martens, E. C. How glycan metabolism shapes the human gut microbiota. Nature reviews. Microbiology 10, 323-335, doi:10.1038/nrmicro2746 (2012).

18 Schwiertz, A. et al. Microbiota and SCFA in lean and overweight healthy subjects. Obesity (Silver Spring) 18, 190-195, doi:10.1038/oby.2009.167 (2010).

19 Bergman, E. N. Energy contributions of volatile fatty acids from the gastrointestinal tract in various species. Physiological reviews 70, 567-590 (1990).

20 Karlsson, F. H. et al. Gut metagenome in European women with normal, impaired and diabetic glucose control. Nature 498, 99-103 (2013).

21 Connor, S. C., Hansen, M. K., Corner, A., Smith, R. F. & Ryan, T. E. Integration of metabolomics and transcriptomics data to aid biomarker discovery in type 2 diabetes. Molecular bioSystems 6, 909-921, doi:10.1039/b914182k (2010).

22 Cani, P. D. et al. Changes in gut microbiota control metabolic endotoxemia-induced inflammation in high-fat diet-induced obesity and diabetes in mice. Diabetes 57, 1470-1481, doi:10.2337/db07-1403 (2008).

23 Markle, J. G. et al. Sex differences in the gut microbiome drive hormone-dependent regulation of autoimmunity. Science 339, 1084-1088, doi:10.1126/science.1233521 (2013).

24 Sonnenburg, J. L. et al. Glycan foraging in vivo by an intestine-adapted bacterial symbiont. Science 307, 1955-1959 (2005).

25 Cani, P. D. et al. Metabolic endotoxemia initiates obesity and insulin resistance. Diabetes 56, 1761-1772, doi:10.2337/db06-1491 (2007).

26 Smith, M. I. et al. Gut microbiomes of Malawian twin pairs discordant for kwashiorkor. Science 339, 548-554, doi:10.1126/science.1229000 (2013).

## EXAMPLE 3

### *Diurnal fluctuations in gut microbiota biogeography and function globally program the host circadian transcriptome*

### MATERIALS AND METHODS

*Mice:* C57Bl/6 mice were purchased from Harlan and allowed to acclimatize to the local animal facility for 2 weeks before used for experimentation. Mice were kept under strict light-dark cycles, with lights being turned on at 6am and turned off at 6pm. In all experiments, age- and gender-matched mice were used. Mice were 8-9 weeks of age at the beginning of experiments. Generally, both male and female mice were used. Stool samples, fecal content, and tissue samples were collected fresh and on the basis of individual mice. Fresh samples were collected in tubes, immediately frozen in liquid nitrogen upon collection, and stored at -80°C until RNA or DNA isolation. In food timing experiments, mice were housed under standard light-dark conditions (6am to 6pm), but had access to food only during the light or dark phase, respectively, for 2 weeks. For antibiotic treatment, mice were given a combination of vancomycin (0.5 g/l), ampicillin (1 g/l), kanamycin (1 g/1), and metronidazole (1 g/l) in their drinking water. All antibiotics were obtained from Sigma Aldrich. Antibiotics were given continuously for one week. For experiments involving genotobiotic mice, germ-free Swiss Webster mice were housed in sterile isolators. All experimental procedures were approved by the local IACUC.

RNA-seq: Tissues were preserved in RNAlater solution (Ambion) and subsequently homogenized in Trizol reagent (Invitrogen). RNA was purified according to the manufacturer's instructions. 400 ng of total RNA were used for library preparation. mRNA was captured with 12 $\mu$l of Dynabeads oligo(dT) (Life technologies), and washed according to the manufacture's guidelines. Purified messenger RNA was eluted at 70°C with 10 $\mu$l of 10 mM Tris-Cl pH 7.5. cDNA was generated from 1$\mu$l of mRNA of each sample. cDNA quantity in each sample was evaluated by qPCR for Actin B gene, and then equivalent amounts of mRNA of each sample were taken for RNAseq library construction. Library construction was performed in a 96-well plate format. First, to open secondary RNA structures and allow annealing of the RT primer, the samples were incubated at 72 °C for 3 min and immediately transferred to 4 °C. Then, RT reaction mix (10 mM DTT, 4 mM dNTP, 2.5 U/$\mu$l Superscript III RT enzyme in 50 mM Tris-HCl (pH 8.3), 75 mM KCl, 3 mM MgCl$_2$)

was added into each well of the 96-well plate and the reaction was mixed. The 96-well plate was then spun down and moved into a cycler (Eppendorf) for the following incubation: 2 min at 42 °C, 50 min at 50 °C, 5 min at 85 °C. Indexed samples with equivalent amount of cDNA were pooled and the product was purified with 1.4x volumes of SPRI beads. The library was completed and amplified through a 12-cycle PCR reaction with 0.5 $\mu$M of P5_Rd1 and P7_Rd2 primers and PCR ready mix (Kapa Biosystems). The forward primer contains the Illumina P5-ReadI sequences and the reverse primer contains the P7-Read2 sequences. The amplified pooled library was purified with 0.7x volumes of SPRI beads to remove primer leftovers. Library concentration was measured with a Qubit fluorometer (Life Technologies) and mean molecule size was determined with a 2200 TapeStation instrument (Agilent). Libraries were sequenced using an Illumina HiSeq 1500. Reads were analyzed as previously described (Lavin et al., 2014) and normalized to the number of total reads for equal coverage across samples. Only genes with more than 10 reads per sample were considered for analysis.

*Quantification of bacterial DNA:* Luminal and mucosal-adherent communities were harvested by extensive flushing of the intestinal lumen to remove non-adherent commensal bacteria. DNA was extracted from the luminal and mucosal fractions using the MoBio PowerSoil kit. DNA concentration was calculated using a standard curve of known DNA concentrations from E.coli K12. 16S qPCR using primers identifying different regions of the V6 16S gene was performed using SYBR fast mix (Kapa Biosystems). The absolute number of bacteria in the samples was then approximated as DNA amount in a sample/DNA molecule mass of bacteria.

*Scanning Electron Microscopy:* Mice were perfused with fixative containing 2% glutaraldehyde and 3% PFA in 0.1 M sodium cacodylate. Colonic samples were extensively washed from fecal matter and fixed for 1-2 hr. Samples were rinsed three times in sodium cacodylate buffer and postfixed in 1% osmium tetroxide for 1hr, stained in 1% uranyl acetate for a further hour, then rinsed, dehydrated, and dried using critical point drying. Samples were then gold-coated and viewed in an ULTRA 55 FEG (ZEISS). For image quantification, the number of bacteria on 10 randomly selected fields per sample were counted and averaged.

*Metabolomics:* Metabolomics analysis was carried out by Metabolon, Inc (Morrisville, NC). Samples were analyzed by Ultrahigh Performance Liquid Chromatography-Tandem Mass Spectroscopy (UPLC-MS/MS), and Gas Chromatography-Mass Spectroscopy (GC-MS). The LC/MS portion of the platform was based on a Waters ACQUITY ultra-performance liquid chromatography (UPLC) and a Thermo Scientific Q-Exactive high resolution/accurate mass spectrometer interfaced with a heated electrospray ionization (HESI-II) source and Orbitrap mass analyzer operated at 35,000 mass resolution. The sample extract was dried then reconstituted in acidic or basic LC-compatible solvents, each of which contained 8 or more injection standards at fixed concentrations to ensure injection and chromatographic consistency. One aliquot was analyzed using acidic positive ion optimized conditions and the other using basic negative ion optimized conditions in two independent injections using separate dedicated columns (Waters UPLC BEH C18-2.1x100 mm, 1.7 $\mu$m). Extracts reconstituted in acidic conditions were gradient eluted from a C18 column using water and methanol containing 0.1% formic acid. The basic extracts were similarly eluted from C18 using methanol and water, however with 6.5mM Ammonium Bicarbonate. The third aliquot was analyzed via negative ionization following elution from a HILIC column (Waters UPLC BEH Amide 2.1x150 mm, 1.7 $\mu$m) using a gradient consisting of water and acetonitrile with 10mM Ammonium Formate. The MS analysis alternated between MS and data-dependent MS2 scans using dynamic exclusion, and the scan range was from 80-1000 m/z. The samples destined for analysis by GC-MS were dried under vacuum for a minimum of 18 h prior to being derivatized under dried nitrogen using bistrimethyl-silyltrifluoroacetamide. Derivatized samples were separated on a 5% diphenyl / 95% dimethyl polysiloxane fused silica column (20 m x 0.18 mm ID; 0.18 um film thickness) with helium as carrier gas and a temperature ramp from 60° to 340°C in a 17.5 min period. Samples were analyzed on a Thermo-Finnigan Trace DSQ fast-scanning single-quadrupole mass spectrometer using electron impact ionization (EI) and operated at unit mass resolving power. The scan range was from 50-750 m/z. Raw data was extracted, peak-identified and QC processed. Compounds were identified by comparison to library entries of purified standards or recurrent unknown entities. Furthermore, biochemical identifications were based on three criteria: retention index within a narrow RI window of the proposed identification, accurate mass match to the library +/- 0.005 amu, and the MS/MS forward and reverse scores between the experimental data and authentic standards. The MS/MS scores are based on a comparison of the ions present in the experimental spectrum to the ions present in the library spectrum. Peaks were quantified using area-under-the-curve. For studies spanning multiple days, a data normalization step was performed to correct variation resulting from instrument inter-day tuning differences. For analysis, values were normalized in terms of raw area counts and afterwards rescaled to set the median equal to 1.

*Taxonomic Microbiota Analysis:* Adherent bacterial communities were obtained by serially washing colons from their luminal content, followed by snap-freezing of the mucosal layer in liquid nitrogen. Frozen samples were processed for DNA isolation using the MoBio PowerSoil kit according to the manufacturer's instructions. 1ng of the purified fecal DNA was used for PCR amplification and sequencing of the bacterial 16S rRNA gene. Amplicons spanning the variable region 1/2 (V1/2) of the 16S rRNA gene were generated by using the following barcoded primers: Fwd 5'-NNNNNNNNAGAGTTTGATCCTGGCTCAG-3' (SEQ ID NO: 1), Rev 5'-TGCTGCCTCCCGTAGGAGT-3' (SEQ ID NO: 2), where N represents a barcode base. The reactions were subsequently pooled in an equimolar ratio, purified (PCR clean kit, Promega), and used for Illumina MiSeq sequencing. 500 bp paired-end sequencing was employed. An in-house script was used to assembly the paired-end reads. Assembly rates of 90% were achieved in all experiments. Reads were then processed using the QIIME (Quantitative Insights Into Microbial Ecology www.qiime.org) analysis pipeline (Caporaso et al., 2010). In brief, fasta quality files and a mapping file indicating the barcode sequence corresponding to each sample were used as inputs, reads were split by samples according to the barcode, taxonomical classification was performed using the RDP-classifier, and an OTU table was created. We employed open-reference OTU mapping using the Greengenes database. No non-matching nucleotides were allowed on the total paired-end assembled read length of 359bp. Rarefaction was performed to exclude samples with insufficient reads per sample counts. Sequences sharing 97% nucleotide sequence identity in the V2 region were binned into operational taxonomic units (97% ID OTUs). For beta-diversity, unweighted unifrac measurements were plotted according to the first two principal coordinates based on 1,000 reads per sample.

*Metagenomic Sequence Mapping:* Illumina sequencing reads were mapped to a gut microbial gene catalog (Human Microbiome Jumpstart Reference Strains et al., 2010) using GEM mapper (Marco-Sola et al., 2012) with the following parameters: -m 0.08 -s 0 -q offset-33 -gem-quality-threshold 26.

*Functional Assignment:* Reads mapped to the gut microbial gene catalog were assigned a KEGG (Kanehisa and Goto, 2000) identification number, according to the gene to category mapping that accompanied the gene catalog. Genes were subsequently mapped to KEGG modules and pathways. Only genes with more than 10 assigned reads were considered. For the KEGG pathway analysis, only pathways whose gene coverage was above 0.2 were included. KEGG pathways were then tested by JTK_cycle for daily oscillations.

*Statistical Analysis:* Data are expressed as mean $\pm$ SEM. For the analysis of rhythmic oscillations and their amplitudes, the non-parametric test JTK cycle was used in R (Hughes et al., 2010), allowing 24 hr for the determination of circadian periodicity. For the detection of rhythmic metabolites, metagenomic content, and transcripts, $p<0.05$ and $q<0.2$ was considered significant. Chow-Ruskey diagrams were generated in R using the Vennerable package. Hypergeometrical testing for KEGG pathway enrichment analysis was done using DAVID (Huang da et al., 2009).

## RESULTS

### Diurnal rhythms of microbiota biogeographical localization

The intestinal microbiota undergoes rhythmic oscillations in composition and gene content, but the mechanisms by which these functional microbial oscillations impact the host remain elusive. Since commensal bacteria most strongly affecting the host are believed to be located in close proximity to the intestinal mucosal surface, the present inventors sought to study the bio-geographical aspects of microbiome diurnal rhythmicity. They therefore analyzed fluctuations in the abundance of epithelial-adherent commensal bacteria over the course of two days (Figure 28A) and analyzed rhythmicity with the commonly used non-parametric algorithm JTK cycle (Hughes et al., 2010). All mice were fed ad libitum and housed under strict 24-hour dark-light conditions, with lights being kept on for 12 hours (Zeitgeber times (ZT) 0-12). At each time point, proximal colons were extensively cleared from luminal content, and only the mucosal-adherent bacteria were isolated, followed by DNA quantification via qPCR. The numbers of bacteria colonizing the epithelial niche underwent marked diurnal changes, with commensal epithelial layer adherence being highest in the dark phase (Figure 28B, $p=3x10^{-7}$, JTK_cycle), while luminal numbers of bacteria remained constant (Figure 29A). Scanning electron microscopy (SEM) imaging confirmed daily fluctuations in the amount of commensals in tight association with the intestinal epithelium (Figures 28C, 29B, and 29C). The SEM images suggested that the taxonomic composition of adherent bacteria was not uniform at different time of the day (Figure 28C). They therefore performed 16S rDNA sequencing of epithelial-associated communities harvested at different times of the day. Indeed, the composition of adherent bacteria underwent marked diurnal fluctuations, so that the beta-diversity of epithelial-associated commensals changed rhythmically over the course of consecutive light-dark cycles (Figures 28D, 28E, and 30A-C; $p=4x10^{-13}$ for oscillations of UniFrac distances). Thus, the community composition localized to the intestinal mucosa at any time point was more similar to the one present 24 hours later than to any other time point in between. Certain commensals featured different preferential times of the day for localization in epithelial proximity (Figure 28F), including *Ruminococcus, Mucispirillum,* and *Lactobacillus* ($p<10^{-6}$ and $p<10^{-5}$, respectively, Figure 28G). Therefore, the host mucosa was exposed to diurnally fluctuating numbers and species of bacteria, in both relative (Figures 28G-28I) and absolute abundances (Figures 30D-I). Of note, peak and trough abundances of certain bacterial species differed by more than 100-fold, as exemplified by *Mucispirillum schaedleri* (Figure 30F), a commensal known to colonize the mucus layer in mice. Together, these results demonstrate that the gut microbiota undergoes oscillations in bio-geographical distribution, resulting in periodic profiles of mucosal-associated commensals characteristic for different times of the day.

### Diurnal rhythms of the intestinal metabolome

In order to determine whether diurnal rhythmicity occurs within the host-microbiome interface, in which intense and intimate communication occurs primarily through the exchange and sensing of metabolites, the temporal dynamics of the intestinal metabolome was determined by metabolite profiling in wild-type mice every 6 hours over the course of two light-dark cycles (Figure 28A). Using JTK_cycle, it was found that 18% of all detected metabolites undergo fluctuations in a 24-hour rhythm when using $p<0.05$ and $q<0.2$ as a threshold (Figure 31A). Oscillating metabolites belonged to diverse chemical groups, including carbohydrates (Figures 31B and 31C), vitamins (Figures 31D and 31E), amino acids (Figures 31F and 31G), lipids (Figure 31H), nucleotides (Figures 32A and 32B), peptides (Figures 32C and 32D), and xenobiotics (Figures 32E-32F). Highly robust diurnal fluctuations were found, for instance, in the carbohydrates xylose, glucose, and lactate ($p<0.007$, Figures 31B and 31C), the microbiota-dependent essential vitamin biotin ($p<10^{-5}$, Figure 31E), and in the amino acids proline and asparagine ($p<0.0004$, Figures 31F and 31G). These results indicate that the host-microbiota interface is characterized by multi-faceted rhythmicity composed of compositional (Thaiss et al., 2014b), metagenomic (Figure 32I), and bio-geographical (Figures 28A-I) microbiome rhythmicity, host transcriptome rhythmicity as quantified by periodic RNA-seq (Figure 31J), and metabolomics rhythmicity (Figure 31K), integrating the diurnal patterns of the host, its microbiome and dietary input. In some cases, a direct link could be recognized between the different components of this multi-layered rhythmic environment. For example, rhythmic abundance of the microbial genes encoding for the final two enzymatic steps in biotin biosynthesis, *bioD* and *bioB*, accompanied phasic rhythmic luminal abundance of biotin (Figures 32G and 32H).

### The microbiota programs colonic transcriptome oscillations

To globally determine the regulatory roles that the microbiota plays on oscillatory processes of the host, mice were administered with broad-spectrum antibiotics the impact of microbiota depletion on meta-organismal diurnal oscillations was determined (Figure 33A). Expectedly, antibiotic treatment drastically reduced the numbers of epithelial-adherent bacteria and ablated any rhythmicity in the remaining community, as determined by quantitative PCR and SEM (Figures 33B, 33C, 34A, and 34B). As a result, the rhythmic taxonomic composition of epithelial-associated commensals was abrogated by antibiotic treatment (Figures 3D and 34C-34E). For instance, *Lactobacillus reuteri* lost its characteristic relative abundance peak and trough phases in mice drinking antibiotics (Figure 34E). To determine the effects of microbial depletion and loss of oscillations on the host, comparative RNA-sequencing of colonic tissue from control and antibiotics-treated mice was performed every 6 hours over the course of two light-dark cycles, using 5 mice at each ZT in each group (Figure 33A). Rhythmicity in transcript profiles was evaluated using JTK cycle with a threshold of $p<0.05$ and $q<0.2$. Both groups featured comparable numbers of oscillating host transcripts (Figure 35A), indicating that the global intestinal transcriptional circadian rhythmicity was independent of the presence of intestinal microbiota. Nonetheless, the identity of the oscillating gene program was markedly different in antibiotics-treated mice as compared to controls (Figure 33F). Overall, 2102 out of a total of 3133 genes oscillating in colonized mice lost their rhythmicity in antibiotics-treated mice. In contrast, 1194 genes featured *de-novo* oscillations antibiotics-treated mice, while only 1031 rhythmic transcripts were shared between both groups (Figures 33F-3I). Among the shared transcripts were genes involved in the core circadian clock and intestinal housekeeping functions, suggesting that the function of the host peripheral clock machinery was not intrinsically dependent on the presence of an intact microbiota (Figures 33J, 35B). Transcripts that lost their oscillations in the absence of the microbiota mainly belonged to nucleotide metabolism and cell cycle pathways, as well as host metabolic pathways activated downstream of microbiota metabolites, such as pathways metabolizing short-chain fatty acids and mucus production pathways (Figure 33K). Microbiome-dependent host oscillatory transcripts also included genes involved in immune receptor signaling (Figures 33K and 35C), in line with an earlier report on microbial control of rhythmic TLR signaling in intestinal epithelial cells (Mukherji et al., 2013). Most remarkable, however, were the functionalities that gained rhythmicity upon microbiota depletion, which included major metabolic pathways like the TCA cycle and oxidative phosphorylation (Figure 33L), as exemplified by members of the cytochrome c oxidase complex (Figure 35D). As a result, the global temporal coordination of colonic metabolic activity differed between microbiota-depleted and control mice, with rhythmic functions peaking at different times of the day (Figure 35E). To rule out potential confounding effects of antibiotic treatment per se, these results were confirmed in germ-free mice that are housed under sterile conditions. Indeed, similar to antibiotics-treated mice, germ-free mice featured massive alterations in their cyclic transcriptome, while the total numbers of cyclic elements was comparable to controls (Figures 35F-35I). As a result, KEGG functionalities lost and gained rhythmicity, similar to antibiotics-treated mice (data not shown). Together, these data indicate that the microbiota reprograms the rhythmic host transcriptome to alter the temporal sequence of metabolic and immune programs, many of which are directly relevant to its own symbiotic niche function.

### Coordinated meta-organismal oscillations are driven by feeding times

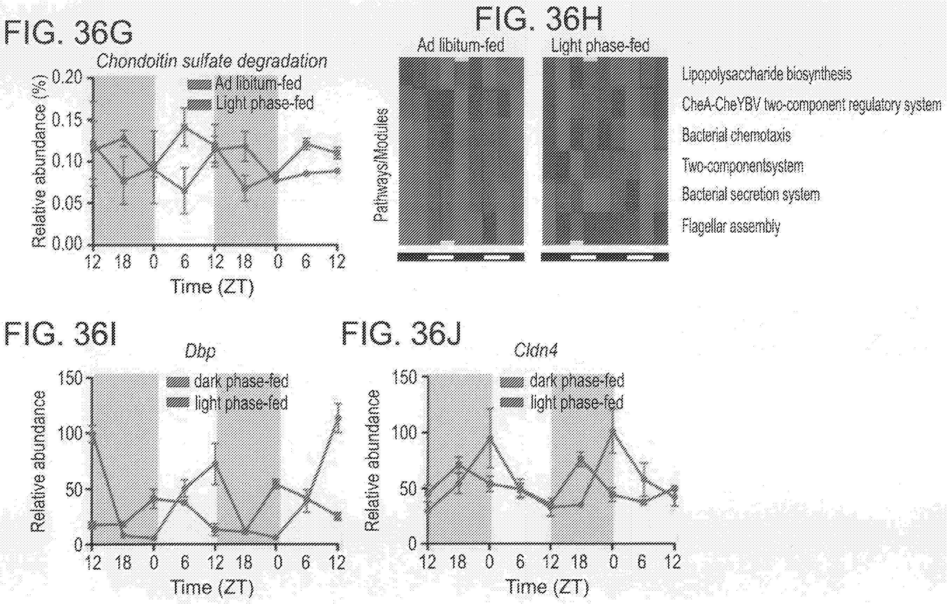

To determine whether rhythmic food intake controls the rhythmic activity of commensals, timed feeding experiments were performed. In these, mice (which preferentially consume food during the dark phase, Figure 36A), were either fed ad libitum, only during the dark phase, or only during the light phase (Figures 37A, 36B, and 36C). Shotgun metagenomic sequencing, bio-geographical assessment, and host transcriptome quantification were performed on mice of different scheduled feeding groups (Figure 37A). While the overall microbiome rhythmicity was not affected by altered feeding times (Figure 37B), reversed feeding times phase-shifted the oscillations in gene abundance (Figures 37C and 37D), as exemplified by the bacterial galactoseamine sulfatase (Figure 36D). As a consequence, rhythmic metagenomic modules and pathways followed times of food intake and featured reverse-phase acro- and bathyphase between *ad libitum*-fed and light phase-fed mice (Figures 36E and 36F). Functional elements tightly controlled by the timing of feeding included bacterial mucus degradation (Figures 37E, 37F, and 36G) and motility (Figure 36H), potentially explaining how feeding times determine the rhythm of microbiota translocation and mucus metabolism and thereby regulate diurnal fluctuations in epithelial bio-geographical attachment. Indeed, reversed feeding times also inverted the pattern of rhythmic epithelial adherence (Figure 37G), as measured by qPCR of attached commensals over the course of a day.

Rhythmic feeding also reprogrammed the colonic transcriptome, as has been previously noted for the liver (Vollmers et al., 2009), while keeping a constant total number of oscillating elements (Figures 37H and 37I). In synchronization to phase shifts in the microbiome, the rhythmic transcriptome of the colon also followed feeding times (Figures 37J-37L, 36I, and 36J). Together, these results suggest that rhythmic nutrient availability directs functional diurnal oscillations in microbiota activity and bio-geographical localization, simultaneous to its control of the rhythmic activity of multiple host pathways related to host-microbiota mutualism.

**Feeding times, the microbiota, and host genetics jointly orchestrate the cyclic transcriptome**

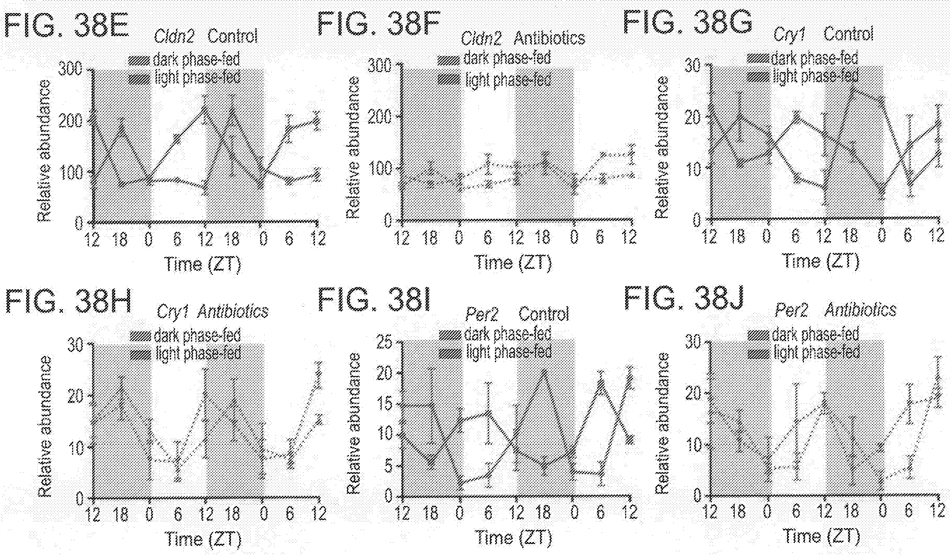

Given the finding that both the microbiota and feeding times are able to program colonic transcriptome oscillations, the present inventors next addressed the interplay between both types of environmental influences on host circadian function. To determine the resemblance and inter-dependency between feeding rhythmicity disruption-induced and microbiome-depletion induced host transcriptome reprogramming, a quadruple comparison of unique and shared oscillating transcripts between mice on timed feeding, both with and without additional antibiotic treatment was performed (Figure 37A). Generally, the numbers of oscillating transcripts were stable across all conditions (data not shown). To identify shared and uniquely cycling elements in each group, a 4-order Chow-Ruskey comparison was used (Figure 38A), which revealed several features. First, both timed feeding and microbiota depletion created independent patterns of unique and shared host transcripts (Figure 38A). 300 transcripts were only microbiota-dependent, as they cycled regardless of feeding condition (with a phase shift between inverse feeding times) but lost oscillations upon antibiotic treatment (Figure 38B). These genes included members of immune pathways, such as the interferon-γ receptor (Figures 38C and 38D), as well as genes involved in barrier function, such as claudin-2 (Figures 38E and 38F). Second, only a minority of transcripts oscillated across all conditions. In fact, only 44 transcripts that showed significant oscillations according to the JTK cycle algorithm were shared between all conditions (Figure 38A). KEGG pathway analysis of these transcripts revealed that this group was enriched for members of the core molecular clock (Figures 38A, and 38G-38J), indicating that the molecular clock itself maintains robust oscillations regardless of feeding and microbiome availability conditions, while the majority of the downstream targets only oscillate in the appropriate feeding and colonization conditions. Third, the number of genes uniquely cycling in light phase-fed, antibiotics-treated mice was about 50% lower than that of all other groups, indicating that the microbiota has a role in the reprogramming of the cycling transcriptome in response to feeding time reversal. In support of this notion, the canonical clock members oscillating in all four groups were phase-shifted between dark phase- and light phase-fed mice, but this phase shift was lost when mice were also treated with antibiotics. For instance, the phase shift in *Cry1* oscillations between dark phase- and light phase-fed groups was largely attenuated when the microbiota in both groups was disrupted by antibiotic treatment (Figures 38G and 38H). The same phenomenon was observed with other clock genes (Figures 38I and 38J). These results reveal an unexpectedly large impact of microbiota activity on the maintenance of rhythmic transcription in the gut, including both identify and phase distribution of cycling elements.

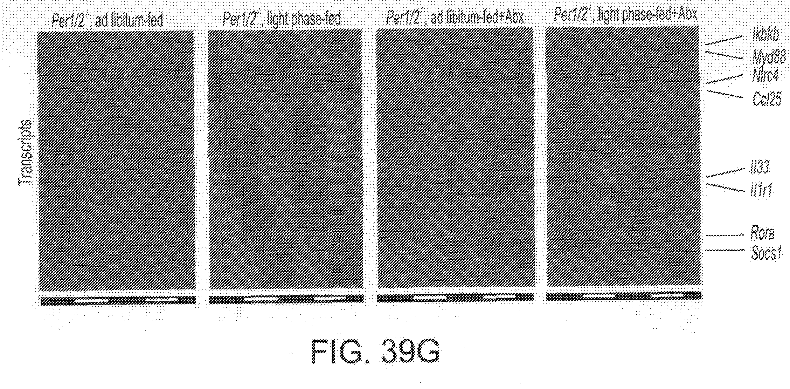

The present results indicate that the presence of the microbiota is critical for the maintenance of colonic transcriptome oscillations. To understand whether the necessity for microbiota in driving host circadian activity stems from only its presence or from its diurnally fluctuating activity, the present inventors took advantage of the finding that compositional microbial oscillations cease to be present in the absence of a functional host circadian clock, but can be restored by rhythmic feeding (Thaiss et al., 2014b). They thus performed a scheduled feeding experiment on *Per1/2*-deficient mice, which lack essential components of the molecular clock (Figure 39A), and tested for metagenomic and colonic transcriptome oscillations in these mice in *ad-libitum* and timed-feeding conditions. While ad libitum-fed *Per1/2$^{-/-}$* mice lacked oscillatory activity in their metagenome, diurnal rhythms of the microbiome were induced by timed feeding (Figures 39B-39D), as exeplified by the bacterial hydrogenase hyoA (Figure 39E). Accordingly, functional modules of the microbiome gained temporal organization in a 24-hour rhythm in *Per1/2*-deficient mice upon scheduled feeding (Figure 39F). In line with previous findings in liver samples (Vollmers et al., 2009), timed feeding also restored oscillations in several hundred colonic transcripts in *Per1/2$^{-/-}$* mice (Figure 39G). Remarkably, however, the regain of diurnal activity in these transcripts depended on an intact microbiota, since antibiotic treatment of timed-fed *Per1/2$^{-/-}$* mice abrogated the restoration of these host oscillations (Figure 39G). Transcripts whose feeding-induced rhythmicity induction depended on the microbiota included multiple genes involved in the mucosal immune response, including members of the IL-1 family of cytokines and their signaling pathway (*Il33, Il1r1, Myd88, Socs1*). These data indicate that microbiota oscillations are necessary for the maintenance of transcript oscillations in a large number of colonic genes.

### The microbiota programs the hepatic clock

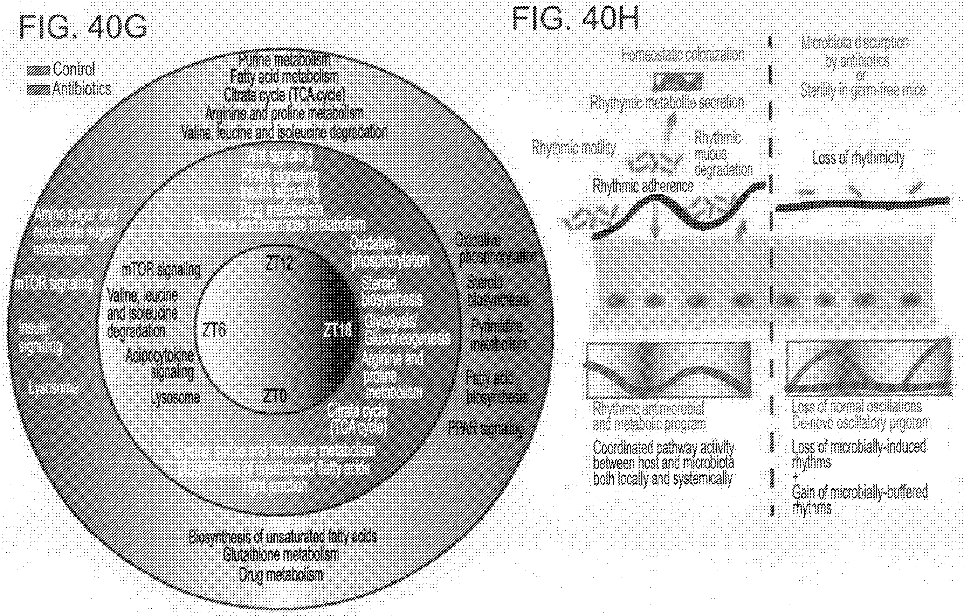

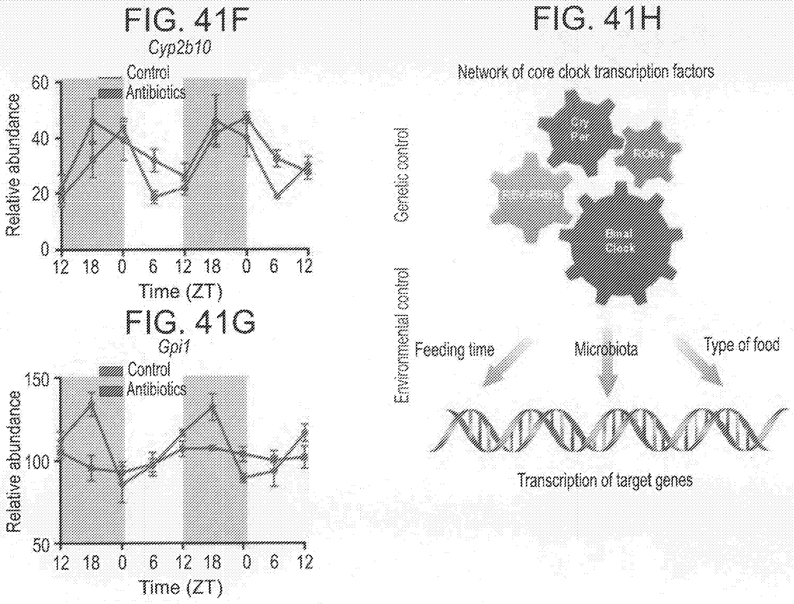

Finally, we sought to determine whether the effects mediated by the diurnally oscillating gut microbiome on the intestinal oscillating transcriptome program could be systemically transmitted. Such distant microbiome-induced regulatory effects on the host circadian rhythmicity would be of potential pathophysiological relevance. We chose to focus on the effects of microbiome diurnal activity on liver circadian rhythmicity, as microbiome impacts on hepatic physiology and liver disease have been documented in multiple recent studies, while many of the mechanisms for such distal effects remain elusive (Henao-Mejia et al., 2012; Qin et al., 2014). To assess whether hepatic transcriptome reprogramming is impacted by microbiota disruption, mice were treated for one week with broad spectrum antibiotic treatment, the hepatic rhythmic transcription of naive or antibiotics-treated mice was profiled using JTK cycle, with $p<0.05$ and $q<0.2$ as threshold (Figure 33A). Similar to the colon, antibiotics-mediated microbiota disruption reprogrammed liver transcriptome oscillations, while the total number of rhythmic elements remaining comparable to that of controls (Figures 40A and 41A). As in the gut, the canonical clock components maintained their rhythms (Figures 40B and 41B-D), and likewise hepatic housekeeping, metabolic, and detoxification functions continued cycling upon antibiotics exposure (Figures 41B, 41E, and 41F). 629 out of a total of 1796 transcripts lost oscillatory profiles (Figure 40C), including genes involved in oxidative phosphorylation and other catabolic pathways (Figure 40D). For instance, the gene encoding glucose phosphate isomerase-1 lost detectable rhythmicity after one week of antibiotic treatment (Figure 41G). In contrast, 1825 transcripts developed *de-novo* rhythmicity (Figure 40E), many of which are involved in fatty acid, nucleic acid, and amino acid metabolism (Figure 40F). As a result of these massive alterations in the cycling transcriptome, the normal temporal sequence of hepatic metabolic activity was partially ablated and replaced by *de-novo* oscillatory programs with peak activities at markedly different times of the day than in non-antibiotic treated conditions (Figure 40G). For instance, pathways involved in metabolism of amino acids, insulin signaling, and PPAR signaling featured phase-shifted activity peaks (Figure 40G). Thus, unexpectedly, the microbiota was found to be critical for maintaining the homeostatic daily succession of metabolic activity, both in the local intestinal environment and systemically.

In this example, diurnal rhythmicity is identified as an essential component in the regulation of host-microbiota symbiosis. Two new elements of microbiota oscillatory activity have been identified that provide a mechanistic explanation for its functional importance: oscillations in biogeographical localization and metabolite secretion patterns. Rhythmically coordinated functions such as bacterial motility and mucus degradation establish a temporal pattern of mucosal adherence of defined microbiota members, inducing a homeostatic state in with the host is periodically exposed to different bacterial numbers, species, functions, and products at different times of the day. In response, the host exerts a rhythmic metabolic and immune program in synchrony to corresponding microbial activity (Figure 40H). Once homeostatic colonization is abrogated, as in the case of antibiotic treatment, microbial rhythmicity is lost at all of its layers, including composition and mucosal adherence, which results in uncoupling of the corresponding host rhythmicity. Surprisingly, an integral part of this microbiota-host circadian uncoupling involves massive *de-novo* genesis of host transcriptional oscillations in both intestine and liver, potentially to meet the needs imposed by rhythmic food intake that is normally buffered by diurnal microbiota activity (Figure 40H). Loss of the symbiotic microbial partner or of its normal cycling behavior perturbs the temporal transcriptome orchestration of the mammalian host, even at locations far from the microbial colonization site.

These findings have numerous important implications. First, exchange of metabolites is increasingly recognized as a major means of communication between the microbiota and the host, thereby creating a meta-organism-wide network of metabolic and immune cross-talk and interdependency. This is exemplified by metabolite-induced regulation of host physiology and molecular recognition of microbial presence by receptors of the innate immune system. All such functions critically depend on the biogeographical localization of the microbiota, and thus the regulatory mechanisms that underlie microbiota stratification in the intestinal ecosystem may hold the key for understanding how microbial colonization within the host is established and maintained. The present findings demonstrate that the time of day dramatically influences all three parameters: a given time of the day features unique profiles of metabolome, microbial composition, and mucosal adherence, resulting in a time-specific host transcriptional program highlighting distinct immune and metabolic functions at different diurnal periods. Microbial-derived molecules may also shape systemic circadian metabolomic patterns, which have been recently found to harbor rhythmic behavior, by diurnally contributing essential microbial-produced or -modulated compounds like essential amino acids and vitamins.

Consequently, the present results suggest that host-microbiome interactions in the steady state, currently regarded as static, may in fact be viewed as a constantly altering yet tightly coupled and highly regulated state of 'fluctuating homeostasis'. Moreover, it may be suggested that these diurnal functional and compositional microbial properties are also important in determining the host response to loss of microbiota homeostasis (such as during exposure to antibiotics). Thus, the present results suggest that the kinetics of microbiota function needs to be taken into consideration when interpreting the downstream effects of microbiota-modulating dietary and medical interventions.

Second, the identification of coordinated diurnal rhythmicity between corresponding host and microbial metabolic activity adds an unexpected facet to our understanding of host-microbial co-evolution. The microbial induction of host transcript oscillations might be functionally beneficial for the meta-organismal ecosystem in at least two ways. On the one hand, by synchronizing its metabolic activity to diurnal fluctuations of the microbiome, the host may optimize the uptake and processing of essential microbiota-derived compounds, such as nutrients and vitamins. On the other hand, coordinated meta-organismal metabolic and immune activity may be ideally suited to meet the fluctuations imposed on the ecosystem by the introduction of nutrients, noxious xenobiotics, and pathogens. Furthermore, the hour-scale changes in colonization conditions along the colonic mucosa create a dynamic niche for commensals and might support long-term community stability by short-term oscillations around a stable colonization state. As such, the concerted meta-organismal activity identified in this study may provide an example for active niche construction by the microbiota, which occurs periodically over the course of 24 hours.

Furthermore, this study provides support for the notion that the circadian program of transcriptome oscillations in peripheral clocks is not independent of environmental signals, and provides a new perspective on the integration of these signals. Previous studies have indicated that both the timing of food intake and the type of diet determine circadian programming of the peripheral transcriptome. It has now been found that the microbiota plays an equally important role in the orchestration of transcriptome oscillations, by inducing and suppressing transcript cycling, both jointly and independently of feeding times (Figure 38A). Furthermore, timed feeding has been shown to restore transcriptome oscillations in mice lacking a functional molecular clock. It has been determined that the microbiota contributes to this effect, and that this feeding-dependent regain of oscillations at least partly depends on microbiome oscillations. Thus, the present discovery of microbial programming of host transcript oscillations may potentially link many of the so far described instances of peripheral transcriptome reprogramming by food, providing an integrated picture by which the dietary impact on diurnal activity of the meta-organism may be indirectly exerted through modulation of microbiota composition and function. Of note, it was found that only a small number of host transcripts oscillate independently of dietary and microbial influence (Figure 5A), the majority of which belongs to the core members of the circadian clock. Thus, the present findings suggest a model by which the molecular clock undergoes self-sustained rhythmicity, while the downstream induction of rhythmicity in large portions of the transcriptome may depend on the proper integration of environmental signals (Figure 41H).

Finally, the present study provides new insight into the multifaceted effects of antibiotic usage on mammalian physiology. Frequent disruption of the microbiome by massive antibiotic exposure has become a hallmark of both modern human medical practice and industrialized food preparation. In addition to the well explained direct deleterious effect on microbiome diversity and susceptibility to pathogenic infection, such exposure is also associated with a variety of adverse metabolic derangements (Ayres et al., 2012; Cox et al., 2014; Ng et al., 2013; Zeissig and Blumberg, 2014), yet these indirect systemic antibiotic effects remain poorly understood. It is found that antibiotics-mediated disruption of multiple levels of microbiota diurnal rhythmicity is associated with abrogation of the normal temporal sequence of both colonic and hepatic host metabolic activity over the course of a day, generating a temporal desynchronization compared to the homeostatic daily activity profile. This finding implies that the effects of antibiotics on host physiology far exceed those exerted directly on the microbiome (such as emergence of drug resistant and opportunistic infections) as well as direct antibiotic-mediated adverse effects. Rather, antibiotic-induced dysbiosis uncouples the microbial and host coordinated rhythmicity, resulting in a massive loss and gain of host transcriptional activity. This misalignment of pathway activity, noted in both gut and liver, may result in altered functions that range from impaired or untimely hepatic detoxification, catabolism, and synthetic function, to altered immunity, potentially leading to long-term consequences such as the association between childhood antibiotic treatment and susceptibility to obesity (Cox et al., 2014).

**References**

Arpaia, N., Campbell, C., Fan, X., Dikiy, S., van der Veeken, J., deRoos, P., Liu, H., Cross, J.R., Pfeffer, K., Coffer, P.J., et al. (2013). Metabolites produced by commensal bacteria promote peripheral regulatory T-cell generation. Nature 504, 451-455.

Asher, G., and Sassone-Corsi, P. (2015). Time for Food: The Intimate Interplay between Nutrition, Metabolism, and the Circadian Clock. Cell 161, 84-92.

Ayres, J.S., Trinidad, N.J., and Vance, R.E. (2012). Lethal inflammasome activation by a multidrug-resistant pathobiont upon antibiotic disruption of the microbiota. Nature medicine 18, 799-806.

Bass, J. (2012). Circadian topology of metabolism. Nature 491, 348-356.

Cox, L.M., Yamanishi, S., Sohn, J., Alekseyenko, A.V., Leung, J.M., Cho, I., Kim, S.G., Li, H., Gao, Z., Mahana, D., et al. (2014). Altering the intestinal microbiota during a critical developmental window has lasting metabolic consequences. Cell 158, 705-721.

Damiola, F., Le Minh, N., Preitner, N., Kornmann, B., Fleury-Olela, F., and Schibler, U. (2000). Restricted feeding uncouples circadian oscillators in peripheral tissues from the central pacemaker in the suprachiasmatic nucleus. Genes & development 14, 2950-2961.

De Vadder, F., Kovatcheva-Datchary, P., Goncalves, D., Vinera, J., Zitoun, C., Duchampt, A., Backhed, F., and Mithieux, G. (2014). Microbiota-generated metabolites promote metabolic benefits via gut-brain neural circuits. Cell 156, 84-96.

Dibner, C., Schibler, U., and Albrecht, U. (2010). The mammalian circadian timing system: organization and coordination of central and peripheral clocks. Annual review of physiology 72, 517-549.

Eckel-Mahan, K.L., Patel, V.R., de Mateo, S., Orozco-Solis, R., Ceglia, N.J., Sahar, S., Dilag-Penilla, S.A., Dyar, K.A., Baldi, P., and Sassone-Corsi, P. (2013). Reprogramming of the circadian clock by nutritional challenge. Cell 155, 1464-1478.

Eckel-Mahan, K.L., Patel, V.R., Mohney, R.P., Vignola, K.S., Baldi, P., and Sassone-Corsi, P. (2012). Coordination of the transcriptome and metabolome by the circadian clock. Proceedings of the National Academy of Sciences of the United States of America 109, 5541-5546.

Fung, T.C., Artis, D., and Sonnenberg, G.F. (2014). Anatomical localization of commensal bacteria in immune cell homeostasis and disease. Immunological reviews 260, 35-49.

Henao-Mejia, J., Elinav, E., Jin, C., Hao, L., Mehal, W.Z., Strowig, T., Thaiss, C.A., Kau, A.L., Eisenbarth, S.C., Jurczak, M.J., et al. (2012). Inflammasome-mediated dysbiosis regulates progression of NAFLD and obesity. Nature 482, 179-185.

Hsiao, E.Y., McBride, S.W., Hsien, S., Sharon, G., Hyde, E.R., McCue, T., Codelli, J.A., Chow, J., Reisman, S.E., Petrosino, J.F., et al. (2013). Microbiota modulate behavioral and physiological abnormalities associated with neurodevelopmental disorders. Cell 155, 1451-1463.

Huang da, W., Sherman, B.T., and Lempicki, R.A. (2009). Systematic and integrative analysis of large gene lists using DAVID bioinformatics resources. Nature protocols 4, 44-57.

Hughes, M.E., Hogenesch, J.B., and Kornacker, K. (2010). JTK_CYCLE: an efficient nonparametric algorithm for detecting rhythmic components in genome-scale data sets. Journal of biological rhythms 25, 372-380.

Lavin, Y., Winter, D., Blecher-Gonen, R., David, E., Keren-Shaul, H., Merad, M., Jung, S., and Amit, I. (2014). Tissue-resident macrophage enhancer landscapes are shaped by the local microenvironment. Cell 159, 1312-1326.

Lopez, C.A., Kingsbury, D.D., Velazquez, E.M., and Baumler, A.J. (2014). Collateral damage: microbiota-derived metabolites and immune function in the antibiotic era. Cell host & microbe 16, 156-163.

Minami, Y., Kasukawa, T., Kakazu, Y., Iigo, M., Sugimoto, M., Ikeda, S., Yasui, A., van der Horst, G.T., Soga, T., and Ueda, H.R. (2009). Measurement of internal body time by blood metabolomics. Proceedings of the National Academy of Sciences of the United States of America 106, 9890-9895.

Mukherji, A., Kobiita, A., Ye, T., and Chambon, P. (2013). Homeostasis in intestinal epithelium is orchestrated by the circadian clock and microbiota cues transduced by TLRs. Cell 153, 812-827.

Ng, K.M., Ferreyra, J.A., Higginbottom, S.K., Lynch, J.B., Kashyap, P.C., Gopinath, S., Naidu, N., Choudhury, B., Weimer, B.C., Monack, D.M., et al. (2013). Microbiota-liberated host sugars facilitate post-antibiotic expansion of enteric pathogens. Nature 502, 96-99.

Qin, N., Yang, F., Li, A., Prifti, E., Chen, Y., Shao, L., Guo, J., Le Chatelier, E., Yao, J., Wu, L., et al. (2014). Alterations of the human gut microbiome in liver cirrhosis. Nature 513, 59-64.

Robertson, B.R., O'Rourke, J.L., Neilan, B.A., Vandamme, P., On, S.L., Fox, J.G., and Lee, A. (2005). Mucispirillum schaedleri gen. nov., sp. nov., a spiral-shaped bacterium colonizing the mucus layer of the gastrointestinal tract of laboratory rodents. International journal of systematic and evolutionary microbiology 55, 1199-1204.

Shapiro, H., Thaiss, C.A., Levy, M., and Elinav, E. (2014). The cross talk between microbiota and the immune system: metabolites take center stage. Current opinion in immunology 30, 54-62.

Sharon, G., Garg, N., Debelius, J., Knight, R., Dorrestein, P.C., and Mazmanian, S.K. (2014). Specialized metabolites from the microbiome in health and disease. Cell metabolism 20, 719-730.

Smith, P.M., Howitt, M.R., Panikov, N., Michaud, M., Gallini, C.A., Bohlooly, Y.M., Glickman, J.N., and Garrett, W.S. (2013). The microbial metabolites, short-chain fatty acids, regulate colonic Treg cell homeostasis. Science 341, 569-573.

Thaiss, C.A., and Elinav, E. (2014). Exploring new horizons in microbiome research. Cell host & microbe 15, 662-667.

Thaiss, C.A., Levy, M., Suez, J., and Elinav, E. (2014a). The interplay between the innate immune system and the microbiota. Current opinion in immunology 26, 41-48. Thaiss, C.A., Zeevi, D., Levy, M., Zilberman-Schapira, G., Suez, J., Tengeler, A.C., Abramson, L., Katz, M.N., Korem, T., Zmora, N., et al. (2014b). Transkingdom control of microbiota diurnal oscillations promotes metabolic homeostasis. Cell 159, 514-529. Voigt, R.M., Forsyth, C.B.,

Green, S.J., Mutlu, E., Engen, P., Vitaterna, M.H., Turek, F.W., and Keshavarzian, A. (2014). Circadian disorganization alters intestinal microbiota. PloS one 9, e97500.

Vollmers, C., Gill, S., DiTacchio, L., Pulivarthy, S.R., Le, H.D., and Panda, S. (2009). Time of feeding and the intrinsic circadian clock drive rhythms in hepatic gene expression. Proceedings of the National Academy of Sciences of the United States of America 106, 21453-21458.

Zarrinpar, A., Chaix, A., Yooseph, S., and Panda, S. (2014). Diet and feeding pattern affect the diurnal dynamics of the gut microbiome. Cell metabolism 20, 1006-1017. Zeissig, S., and Blumberg, R.S. (2014). Life at the beginning: perturbation of the microbiota by antibiotics in early life and its role in health and disease. Nature immunology 15, 307-310.

## EXAMPLE 4

### *Effect of timing of food intake on microbiome*

## MATERIALS AND METHODS

16 impaired glycemic response and healthy participants engaged in a three week experiment of diet intervention. The first week was a profiling week, from which two personalized test diets were computed: (1) one full week of a personalized diet predicted to have "good" (low) postprandial blood glucose responses; and (2) one full week of a personalized diet predicted to have "bad" (high) postprandial blood glucose responses. The present inventors evaluated whether indeed the personalized diet of the "good" week elicited lower blood glucose responses as compared to the personalized diet given on the "bad" week.

Before the experiment, a dietitian planned a personal tailored diet for 6 days as follows: each participant decided how many meals and calories he or she eats in a day. All meals in the 6 days were different and in every day the same number of meals and calories were consumed with a gap of at least 3 hours between meals. The content of the meals was decided by the participant to match their taste and regular diet. For example, a participant may choose to eat 5 meal categories a day as following: a 300 calorie breakfast, 200 calorie brunch, 500 calorie launch, 200 calorie snack and 800 calorie dinner. The participant decides on 6 different options for each meal category (5 meal categories in the example: breakfasts, brunch, launch, snack and dinner) with the help of the dietitian to ensure that all breakfasts are isocaloric with a maximum deviation of 10%.

The experiment began with taking a blood sample and anthropometric measurements from the participant, connecting the participant to a continuous glucose monitor and starting the 6 day diet, while logging all eaten meals during the time of the study. On the 7th day of the experiment, the participant performed a standard (50g) oral glucose tolerance test after which he ate normally throughout that day. The first week which is referred to as the "mix week" exposed the participant to a variety of foods which afterwards determined which meals were relatively "good" and "bad" i.e. which meals resulted in low and high glucose response respectively. The glucose blood levels were monitored using a continuous glucose monitor (Medtronic iPro2) with a high 5 minute temporal resolution. The glucose rise and glucose incremental area under the curve (AUC) was measured for each meal. The meals from low to high response were selected where the best and worst two meals of every meal category were selected and marked as good meals and bad meals.

After the good and bad meals were selected, the participants continued with the additional two weeks of the experiment, which were the test weeks. The "good week" comprised only of good meals and "bad week" comprised only of meals predicted to elicit "bad" (high) blood glucose responses. A week comprised 6 days of diet and one day of 50 grams glucose tolerance test as described above. The order of the weeks was randomly chosen and neither participant nor dietitian were exposed to the order of the weeks. After three weeks, the glucose level between weeks was compared.

To date, 16 individuals completed the experiment out of which 10 had an impaired glycemic response and 6 were healthy.

## RESULTS

"Good" and 'bad" meals were correctly categorized: It was found that the vast majority of the meals tested in the two test weeks showed a glucose response in accord with the predictions (low / high).

A significant improvement in the average AUC following a meal in the "good" week compared to the "bad" week was observed. This result holds for both healthy and impaired glucose tolerance individuals where in the latter group the differences between the "good" and "bad" week were greater (Figure 42).

The results also showed that blood glucose responses following meals show a diurnal pattern (Figure 43). When fitting a line though average AUC responses of all meals in a category it can be seen that breakfast AUC trend is relatively low followed by lunch and dinner with the highest trend of AUC. This trend remains after normalizing either by carbohydrates or calories in a meal (Figures 44A-B).

## Claims

1. A method of determining tolerance to a non-caloric substance in a healthy subject, comprising:

   (a) determining a signature of a gut microbiome in a sample of the healthy subject who has been subjected to the non-caloric substance;
   (b) comparing said signature of said gut microbiome of the healthy subject to at least one reference signature of a pathological gut microbiome, wherein when said signature of the gut microbiome of the healthy subject is statistically significantly similar to said reference signature of said pathological gut microbiome, it is indicative that the healthy subject is intolerant to the non-caloric substance; and
   (c) providing a recommendation about the amount of said non-caloric substance that can be tolerated by the healthy subject.

2. The method of claim 1, further comprising comparing said signature of said gut microbiome of the healthy subject to at least one non-pathological reference signature, wherein when said signature of said gut microbiome of the healthy subject is statistically significantly different to said at least one non-pathological reference signature, it is indicative that the healthy subject is intolerant to the non-caloric substance.

3. The method of claim 1, wherein said pathological gut microbiome is derived from a healthy subject who is intolerant to the non-caloric substance.

4. The method of claim 3, wherein said non-pathological gut microbiome is derived from a healthy subject who is tolerant to the non-caloric substance.

5. The method of claim 1, wherein the healthy subject has been subjected to the non-caloric substance at least one month prior to said determining.

6. The method of claim 1, wherein the healthy subject has been subjected to the non-caloric substance at least once a day.

7. The method of claim 1, wherein the healthy subject has been subjected to the non-caloric substance for at least one week.

## Patentansprüche

1. Verfahren zum Bestimmen der Toleranz gegenüber einer nichtkalorischen Substanz bei einem gesunden Subjekt, umfassend:

   (a) Bestimmen einer Signatur eines Darmmikrobioms in einer Probe des gesunden Subjekts, das der nichtkalorischen Substanz ausgesetzt wurde;

(b) Vergleichen der Signatur des Darmmikrobioms des gesunden Subjekts mit mindestens einer Referenzsignatur eines pathologischen Darmmikrobioms, wobei, wenn die Signatur des Darmmikrobioms des gesunden Subjekts statistisch signifikant ähnlich der Referenzsignatur des pathologischen Darmmikrobioms ist, es indikativ ist, dass das gesunde Subjekt intolerant gegenüber der nichtkalorischen Substanz ist; und

(c) Bereitstellen einer Empfehlung über die Menge der nichtkalorischen Substanz, die vom gesunden Subjekt toleriert werden kann.

2. Verfahren nach Anspruch 1, weiter umfassend das Vergleichen der Signatur des Darmmikrobioms des gesunden Subjekts mit mindestens einer nichtpathologischen Referenzsignatur, wobei, wenn sich die Signatur des Darmmikrobioms des gesunden Subjekts statistisch signifikant von der mindestens einen nichtpathologischen Referenzsignatur unterscheidet, es indikativ ist, dass das gesunde Subjekt intolerant gegenüber der nichtkalorischen Substanz ist.

3. Verfahren nach Anspruch 1, wobei das pathologische Darmmikrobiom von einem gesunden Subjekt stammt, das intolerant gegenüber der nichtkalorischen Substanz ist.

4. Verfahren nach Anspruch 3, wobei das nichtpathologische Darmmikrobiom von einem gesunden Subjekt stammt, das tolerant gegenüber der nichtkalorischen Substanz ist.

5. Verfahren nach Anspruch 1, wobei das gesunde Subjekt der nichtkalorischen Substanz mindestens einen Monat vor dem Bestimmen ausgesetzt wurde.

6. Verfahren nach Anspruch 1, wobei das gesunde Subjekt der nichtkalorischen Substanz mindestens einmal pro Tag ausgesetzt wurde.

7. Verfahren nach Anspruch 1, wobei das gesunde Subjekt der nichtkalorischen Substanz für mindestens eine Woche ausgesetzt wurde.


**Revendications**

1. Procédé de détermination de la tolérance à une substance non calorique chez un sujet en bonne santé, comprenant :

(a) la détermination d'une signature d'un microbiome intestinal dans un échantillon du sujet en bonne santé qui a été soumis à la substance non calorique ;
(b) la comparaison de ladite signature dudit microbiome intestinal du sujet en bonne santé avec au moins une signature de référence d'un microbiome intestinal pathologique, dans laquelle, lorsque ladite signature du microbiome intestinal du sujet en bonne santé est statistiquement significativement similaire à ladite signature de référence dudit microbiome intestinal pathologique, cela indique que le sujet en bonne santé est intolérant à la substance non calorique ; et
(c) la fourniture d'une recommandation concernant la quantité de ladite substance non calorique qui peut être tolérée par le sujet en bonne santé.

2. Procédé selon la revendication 1, comprenant en outre la comparaison de ladite signature dudit microbiome intestinal du sujet en bonne santé avec au moins une signature de référence non pathologique, dans lequel, lorsque ladite signature dudit microbiome intestinal du sujet en bonne santé est statistiquement significativement différente de ladite au moins une signature de référence non pathologique, cela indique que le sujet en bonne santé est intolérant à la substance non calorique.

3. Procédé selon la revendication 1, dans lequel ledit microbiome intestinal pathologique provient d'un sujet en bonne santé qui est intolérant à la substance non calorique.

4. Procédé selon la revendication 3, dans lequel ledit microbiome intestinal non pathologique provient d'un sujet en bonne santé, lequel est tolérant à la substance non calorique.

5. Procédé selon la revendication 1, dans lequel le sujet en bonne santé a été soumis à la substance non calorique au moins un mois avant ladite détermination.

**6.** Procédé selon la revendication 1, dans lequel le sujet en bonne santé a été soumis à la substance non calorique au moins une fois par jour.

**7.** Procédé selon la revendication 1, dans lequel le sujet en bonne santé a été soumis à la substance non calorique pendant au moins une semaine.

FIG. 1A

Wild-type mice, food ad libitum

ZT12  ZT18  ZT0  ZT6  ZT12  ZT18  ZT0  ZT6  ZT12

6pm    12am   6am    12pm   6pm    12am   6am    12pm   6pm
"dusk"  "dark"  "dawn"  "light"  "dusk"  "dark"  "dawn"  "light"  "dusk"

16S rDNA sequencing
Shotgun metagenomic sequencing

FIG. 1B

Dehalobacterium spp.
Lactobacillus reuteri

Amplitude (rel. abundance)

FIG. 1C

Bacterial genera (relative abundance)

non-rhythmic

rhythmic

ZT:12 18 0  6 12 18  0  6 12

FIG. 1D

Lactobacillaceae
Odoribacteriaceae
Streptococcaceae
Lachnospiraceae
Anaeroplasmataceae
Dehalobacteriaceae

Taxonomic units

ZT:12  0  12  0  12

FIG. 1E

Lactobacillus reuteri

12  18  0  6  12  18  0  6  12
Time (ZT)

FIG. 1F

FIG. 1G

FIG. 1H

FIG. 1I

FIG. 1J

FIG. 1K

FIG. 2A

FIG. 2B

FIG. 2C

FIG. 2D

FIG. 2E
Pyrimidine metabolism

FIG. 2F
Alanine, aspartate and glutamate metabolism

FIG. 2G
Glycosaminoglycan degradation

FIG. 2H

FIG. 3A

FIG. 3B

FIG. 3C

FIG. 3D

# FIG. 3E

# FIG. 3F

Vitamin metabolism

# FIG. 3G

Nucleotide metabolism

# FIG. 3H

Two-component and secretion systems

FIG. 4A  *DNA repair*

FIG. 4B  *Cell wall synthesis*

FIG. 4C  *Motility*

FIG. 4D

FIG. 4E

FIG. 4F

EP 3 058 085 B1

FIG. 4G

FIG. 4H

FIG. 4I

FIG. 4J

FIG. 4K

FIG. 4L

FIG. 5A

FIG. 5B

FIG. 5C Bacteroides acidifaciens

FIG. 5D Lactobacillus reuteri

FIG. 5E Peptococcaceae

FIG. 5F Candidatus Arthromitus

FIG. 5G

FIG. 5H

FIG. 5I

FIG. 5J

FIG. 5K

FIG. 5L

FIG. 6A Wild-type, dark phase-fed

FIG. 6B Wild-type, light phase-fed

FIG. 6C

FIG. 6D

FIG. 6E

FIG. 6F
Per1/2-/- into germ-free

FIG. 6G
Per1/2-/- into germ-free

FIG. 6H
WT into germ-free

FIG. 7A
Induction of jet lag:

8 hours "time difference" — every three days

FIG. 7B

# FIG. 7C

Wild-type     Jet lag

Taxonomic units cycling in wild-type

ZT:12   0   12   0   12   0   12   0   12

# FIG. 7D

p-value

Control
Jet lag

Amplitude (rel. abundance)

# FIG. 7E

*Ruminococcaceae*

Relative abundance (%)

Control
Jet lag

Time (ZT)

# FIG. 7F

PC2 (4.52%)

Control
Jet lag
(4 weeks)

PC1 (5.19%)

# FIG. 7G

PC2 (24.04%)

Control
Jet lag
(4 months)

PC1 (47.09%)

# FIG. 7H

depleted

Christensenellaceae
Dorea
Aneaeroplasmatales
Anaeroplasmataceae
Anaeroplasma
Lactobacillus
Lactococcus
RF32
Alphaproteobacteria
Proteobacteria
Ruminococus
Lactobacillus ruminis
Phascolarctobacterium
AF12
Rikenellaceae
Paraprevotellaceae
Fusobacteria
Fusobacteriales

enriched

FIG. 8A  FIG. 8B  FIG. 8C  FIG. 8D  FIG. 8E  FIG. 8F

FIG. 9A

FIG. 9B

FIG. 9C

FIG. 9D

FIG. 9E

FIG. 9F

EP 3 058 085 B1

FIG. 9G

FIG. 9H

FIG. 9I

Control HFD  Jet lag HFD    Control into GF  Jet lag into GF

FIG. 9J

FIG. 9K

EP 3 058 085 B1

FIG. 10A
Control, high-fat-diet

FIG. 10B
Control, high-fat-diet, Abx

FIG. 10E

FIG. 10C
Jet lag, high-fat-diet

FIG. 10D
Jet lag, high-fat-diet, Abx

FIG. 10F

EP 3 058 085 B1

FIG. 10G

FIG. 10H
*Ruminococcaceae*

FIG. 10I
*Streptococcaceae*

FIG. 10J

FIG. 10K
*Clostridiaceae*

FIG. 10L
*Lachnospiraceae*

FIG. 11A

Subject 1:

Subject 2:

16S rDNA sequencing
Shotgun metagenomic sequencing

Oscillating taxonomic units

ZT: 0 12 0 12 0 12 0 12 0 12

FIG. 11D

FIG. 11B

*Subject 1:*

p-value

Amplitude (rel. abundance)

FIG. 11C

*Subject 2:*

p-value

Amplitude (rel. abundance)

FIG. 11E

*Parabacterides*

Relative abundance (%)

Time (ZT)

126

FIG. 11F

FIG. 11G

FIG. 11I

FIG. 11H

EP 3 058 085 B1

FIG. 12A

*Lachnospira*

FIG. 12B

*Bulleida*

FIG. 12C

FIG. 12D

*Dioxin degradation*

FIG. 13A

FIG. 13B

FIG. 13C

FIG. 13D

FIG. 13E

Before jet lag into GF    During jet lag into GF    After jet lag into GF

FIG. 13F

FIG. 14A

Cell growth

Dehalobacterium

DNA repair
Energy metabolism

0    *Lactobacillus*

18    6    Detoxification
Motility
Envionmental sensing

12

*Lachnospira*

FIG. 14B

Wild-type

Resting    Activity    Resting    Activity

Energy metabolism
Cell growth
DNA repair
Detoxification
Motility

Clock-deficient
or jet lagged

Irregular resting/activity

Activity    Resting    Activity    Resting

FIG. 15A

FIG. 15B

FIG. 15C

FIG. 15D

FIG. 15E

FIG. 16A

FIG. 16B

FIG. 16C

FIG. 16D

FIG. 16E

FIG. 16F

FIG. 16G

FIG. 16H

EP 3 058 085 B1

FIG. 17A  FIG. 17B  FIG. 17C  FIG. 17D  FIG. 17E  FIG. 17F

FIG. 18A

FIG. 18B

FIG. 18C

FIG. 18D

FIG. 18E

FIG. 18F

FIG. 18G    FIG. 18H    FIG. 18I

FIG. 18J    FIG. 18K    FIG. 18L

EP 3 058 085 B1

FIG. 19A

FIG. 19B

FIG. 19C

FIG. 19D

FIG. 19E

FIG. 19F

EP 3 058 085 B1

FIG. 19G    FIG. 19H    FIG. 19I

FIG. 20A    FIG. 20B    FIG. 20C

level identified

-1.2  0  1.2
Fold change

| 162684 | *Bacteroides uniformis [S]* |
| 520358 | *Clostridiales [O]* |
| 176071 | *Bacteroides [G]* |
| 262409 | *Clostridiales [O]* |
| 178064 | *Bacteroides [G]* |
| 234054 | *Clostridiales [O]* |
| 196219 | *Parabacteroides [G]* |
| 51677 | *Clostridiales [O]* |
| 422727 | *Clostridiales [O]* |
| 392918 | *Ruminococcaceae [F]* |
| 190649 | *Ruminococcaceae [F]* |
| 190473 | *Clostridiales [O]* |
| 817550 | *Staphylococcus [G]* |
| 259372 | *Lactobacillus [G]* |
| 228601 | *Bacteroides [G]* |
| 193859 | *Bacteroides [G]* |
| 308308 | *Clostridiales [O]* |
| 4457872 | *Bacteroides [G]* |
| 348404 | *Clostridiales [O]* |
| 309524 | *Ruminococcaceae [F]* |
| 826541 | *Clostridiales [O]* |
| 260410 | *Bacteroides [G]* |
| 640999 | *Clostridiales [O]* |
| 287879 | *Clostridiales [O]* |
| 539688 | *Clostridiales [O]* |
| 4480176 | *Clostridiales [O]* |
| 1517774 | *Clostridiales [O]* |
| 3998912 | *Clostridiales [O]* |
| 1111350 | *Ruminococcaceae [F]* |
| 355187 | *Clostridiales [O]* |
| 4455677 | *Clostridiales [O]* |
| 835900 | *Odoribacter [G]* |
| 4432841 | *RF39 [O]* |
| 260454 | *Clostridiales [O]* |
| 355305 | *Lactobacillus reuteri [S]* |
| 190026 | *AF12 [G]* |
| 4405128 | *YS2 [O]* |
| 182762 | *Ruminococcaceae [F]* |
| 354911 | *Lactobacillus reuteri [S]* |

FIG. 21

FIG. 22A

FIG. 22B

FIG. 22E

FIG. 22C

FIG. 22D

FIG. 22F

FIG. 22G

EP 3 058 085 B1

**FIG. 23A**

*Bacteroides vulgatus*

Saccharin      Glucose      Water

**FIG. 23B**

*Akkermansia muciniphila*

Saccharin      Glucose      Water

**FIG. 23C**

| | | |
|---|---|---|
| Glucose | PtsG \| Crr | Glucose 6-phosphate |
| N-Acetyl-D-glucosamine | NagE | N-Acetyl-D-glucosamine 6-phosphate |
| Maltose | MalX \| Crr | Maltose 6-phosphate |
| D-glucosamine | GamP | D-glucosamine 6-phosphate |
| Sucrose | ScrA | Sucrose-6-phosphate |
| β-glucosides | BglF | Phospho-β-glucoside |
| Arbutin / Salicin | AscF \| Crr | Arbutin 6-phosphate / Salicin 6-phosphate |
| Trehalose | TreB \| Crr | Trehalose 6-phosphate |
| N-Acetyl-muramic acid | MurP \| Crr | N-Acetylmuramic acid 6-phosphate |

-0.5   0   1
Fold change

**FIG. 23D**

| | |
|---|---|
| ko01053 | Biosynthesis of siderophore group nonribosomal peptides |
| ko02040 | Flagellar assembly |
| ko00643 | Styrene degradation |
| ko00053 | Ascorbate and aldarate metabolism |
| ko00633 | Nitrotoluene degradation |
| ko05111 | Vibrio cholerae pathogenic cycle |
| ko00930 | Caprolactam degradation |
| ko00380 | Tryptophan metabolism |
| ko00565 | Ether lipid metabolism |
| ko00410 | beta-Alanine metabolism |
| ko00281 | Geraniol degradation |
| ko00791 | Atrazine degradation |
| ko02030 | Bacterial chemotaxis |
| ko00440 | Phosphonate and phosphinate metabolism |
| ko00540 | Lipopolysaccharide biosynthesis |

-0.2   1   0.3
Fold change
(Week 11/Week 0)

FIG. 24A

FIG. 24B

FIG. 24C

## FIG. 25A

Transplanted culture:
- Saccharin-treated
- Control

## FIG. 25B

| | | level identified | |
|---|---|---|---|
| 4390755 | Anaeroplasma [G] | |
| 1135084 | Bacteroides [G] | |
| 260410 | Bacteroides [G] | |
| 178064 | Bacteroides [G] | |
| 311482 | Bacteroides [G] | |
| 4308591 | Bacteroides acidifaciens [S] | |
| 355305 | Parabacteroides [G] | |
| 276149 | Lactobacillus reuteri [S] | |
| 419601 | Candidatus Arthromitus [G] | |
| 176061 | Bacteroides [G] | |
| 190473 | Clostridiales [O] | |
| 184753 | Bacteroides [G] | |
| 234912 | Clostridium [G] | |
| 3750380 | Clostridiales [O] | |

-2   1   2

Fold difference
(Saccharin-treated / Control)

## FIG. 25C

KEGG pathway enrichment

r=0.38
p<10⁻⁴

Sphingolipid metabolism
Glycosphingolipid biosynthesis
Glycosaminoglycan degradation
Other glycan degradation
PTS

EP 3 058 085 B1

FIG. 26A

FIG. 26B

FIG. 26C

FIG. 26D

FIG. 26E

FIG. 26F

FIG. 26G

FIG. 26H

FIG. 27A

FIG. 27B

FIG. 27C

FIG. 27D

FIG. 27E

FIG. 27F

EP 3 058 085 B1

FIG. 27G

FIG. 27H

FIG. 27I

Transplanted sample from responder R4:
Day 7
Day 1

Transplanted sample from non-responder NR2:
Day 7
Day 1

FIG. 27J

FIG. 27K

FIG. 27L

FIG. 28A

FIG. 28B

FIG. 28C

FIG. 28D

FIG. 28E

FIG. 28F

FIG. 28G

FIG. 28H

FIG. 28I

## FIG. 29A

## FIG. 29B

## FIG. 29C

FIG. 30A

first 24 hrs

FIG. 30B

second 24 hrs

FIG. 30C

FIG. 30D

FIG. 30E

EP 3 058 085 B1

FIG. 30F
Mucispirillum schaederi

FIG. 30G
Ruminococcus gnavus

FIG. 30H
Lactobacillus reuteri

FIG. 30I
Bacteroides acidifaciens

No. of bacteria/cm of tissue
Time (ZT)

FIG. 31A

FIG. 31B

FIG. 31C

FIG. 31D

FIG. 31E

FIG. 31F

EP 3 058 085 B1

FIG. 31G

*Asparagine*

FIG. 31I

Metagenome

FIG. 31J

Colonic transcriptome

FIG. 31K

Metabolome

FIG. 31H

*Lipids*

*Beta-muricholate*     *Hyocholate*

EP 3 058 085 B1

FIG. 32A — Nucleotides; Cytidine, Cytosine

FIG. 32B — Cytosine

FIG. 32C — Peptides; Valyglutamine

FIG. 32D — Valyglutamate

FIG. 32E — Xenobiotics; Ergothioneine; 2-Isopropylmalate

FIG. 32F — Ergothioneine

FIG. 32G — bioD

FIG. 32H — bioB

EP 3 058 085 B1

FIG. 33A

Antibiotics vs. control

ZT12 ZT18 ZT0 ZT6 ZT12 ZT18 ZT0 ZT6 ZT12

.Bio-geography

-Host transcriptome

(Colon, Fig. 3) (Liver, Fig. 7)

FIG. 33B

Control
Antibiotics

No. of bacteria/cm of tissue

Time (ZT)

FIG. 33C

ZT0

Control

Antibiotics

FIG. 33D

p-value

Control
Antibiotics

Amplitude (rel. abundance)

FIG. 33E

Lactobacillus reuteri

Control
Antibiotics

Relative abundance (%)

Time (ZT)

FIG. 33F

Control          Antibiotics

2102     1031     1194

Control    Antibiotics

Transcripts

FIG. 33G

Control    Antibiotics

Transcripts

FIG. 33H

Control    Antibiotics

Transcripts

FIG. 33I

KEGG pathways

Circadian rhythm
Fructose metab.
Glycerolipid metab.
PPAR signaling
Pentose metab.
ECM-receptor
Amino sugar metab.
Drug metabolism
Galactose metab.
Glutathione metab.

p-value (-Log$_{10}$)

FIG. 33J

KEGG pathways

DNA replication
Nucleotide repair
Mismatch repair
Propanoate metab.
Galactose metab.
N-Glycan biosyn.
Cell cycle
Glycerophospholipids
p53 signaling
TLR signaling

p-value (-Log$_{10}$)

FIG. 33K

KEGG pathways

Ox. phosphorylation
Glutathione metab.
TCA cycle
Pyruvate metabolism
Insulin signaling
Focal adhesion
MAPK signaling
GnRH signaling
Taurine metab.

p-value (-Log$_{10}$)

FIG. 33L

EP 3 058 085 B1

FIG. 34A

FIG. 34B

FIG. 34C

FIG. 34D

FIG. 34E

FIG. 35A

FIG. 35E

FIG. 35B *Nr1d2*

FIG. 35C  FIG. 35D  FIG. 35F  FIG. 35G  FIG. 35H  FIG. 35I

FIG. 36A FIG. 36B FIG. 36C FIG. 36D FIG. 36E FIG. 36F

FIG. 36G

Chondoitin sulfate degradation

FIG. 36H

Ad libitum-fed    Light phase-fed

Pathways/Modules:
Lipopolysaccharide biosynthesis
CheA-CheYBV two-component regulatory system
Bacterial chemotaxis
Two-component system
Bacterial secretion system
Flagellar assembly

FIG. 36I

Dbp

FIG. 36J

Cldn4

FIG. 37A

WT, timed feeding (Fig. 4)
+/- antibiotics (Fig .5)

ad libitum
dark phase
light phase

- Metagenome
. Bio-geography
- Host transcriptome

FIG. 37B

p-value

ad libitum-fed
light phase-fed

Amplitude (rel. abundance)

FIG. 37C

Ad libitum-fed          Light phase-fed

Genes

FIG. 37D

Relative abundance

ad libitum-fed
light phase-fed

Time (ZT)

FIG. 37E
Keratan sulfate degradation

Relative abundance (%)

Ad libitum-fed
Light phase-fed

Time (ZT)

FIG. 37F
Glycosaminoglycan degradation

Relative abundance (%)

ad libitum-fed
light phase-fed

Time (ZT)

FIG. 37G

FIG. 37H

FIG. 37I

FIG. 37J

FIG. 37K

FIG. 37L

FIG. 38A

dark phase-fed + antibiotics

dark phase-fed

856

210

956

75    43    73

44    39

492

26

300

83    112

122

1131

light phase-fed + antibiotics

light phase-fed

☐ Unique
▨ Double shared
▨ Triple shared
▨ Quadruple shared

KEGG analysis of quandruple shared transcripts

Circadian rhythm

0.0    0.5    1.0    1.5    2.0
p-value (-Log₁₀)

FIG. 38B

Dark phase-fed    Light phase-fed    Dark phase-fed+Abx    Light phase-fed+Abx

Transcripts

FIG. 38C    Ifngr1 Control

▬ dark phase-fed
▬ light phase-fed

300

250

200

12 18 0 6 12 18 0 6 12
Time (ZT)

Relative abundance

FIG. 38D    Ifngr1 Antibiotics

▬ dark phase-fed
▬ light phase-fed

400

350

300

250

200

150

12 18 0 6 12 18 0 6 12
Time (ZT)

Relative abundance

EP 3 058 085 B1

FIG. 38E  *Cldn2*  Control

FIG. 38F  *Cldn2*  Antibiotics

FIG. 38G  *Cry1*  Control

FIG. 38H  *Cry1*  Antibiotics

FIG. 38I  *Per2*  Control

FIG. 38J  *Per2*  Antibiotics

FIG. 39A

FIG. 39B

FIG. 39C

FIG. 39D

FIG. 39E

FIG. 39F

FIG. 39G

FIG. 40A

Oscillating in control    Oscillating in antibiotic-treated

629    1167    1825

FIG. 40B

Control    Antibiotics

Transcripts

FIG. 40C

Control    Antibiotics

Transcripts

FIG. 40D

KEGG pathways

Ox. phosphorylation
Ribosome
Starch metabolism
Wnt singaling
Endocytosis
Adherence junction
Pentose-P pathway
Drug metabolism
Lysosome
Ascorbate metabolism

0    1    2    3    4    5
p-value ($-Log_{10}$)

FIG. 40E

Control    Antibiotics

Transcripts

FIG. 40F

KEGG pathways

Fatty acid metab.
Aminoacyl-tRNA biosyn.
Nucleotide sugar metab.
PPAR signaling
V, L, I degradation
Alanine metabolism
Pyrimidine metab.
Xenobiotic metab.
Drug metabolism
Retinol metab.

0    1    2    3    4
p-value ($-Log_{10}$)

EP 3 058 085 B1

FIG. 40G

Control
Antibiotics

FIG. 40H

FIG. 41A

FIG. 41B

FIG. 41C    Per2

FIG. 41D    Dbp

FIG. 41E    Cyp2e1

FIG. 41F

Cyp2b10

FIG. 41H

Network of core clock transcription factors

Genetic control

Environmental control

Feeding time    Microbiota    Type of food

Transcription of target genes

FIG. 41G

Gpi1

# FIG. 42

FIG. 43

FIG. 44A

Normalizing postprandial iAUCmed levels by meal calories

Legend:
- Prediabetic Breakfast
- Prediabetic Lunch
- Prediabetic Dinner
- Non diabetic Breakfast
- Non diabetic Lunch
- Non diabetic Dinner

Y-axis: iAUCmed of every meal- Good week
X-axis: iAUCmed of every meal- Bad week

FIG. 44B

Normalizing postprandial iAUCmed levels by meal carbs

Legend:
- Prediabetic Breakfast
- Prediabetic Lunch
- Prediabetic Dinner
- Non diabetic Breakfast
- Non diabetic Lunch
- Non diabetic Dinner

Y-axis: iAUCmed of every meal- Good week
X-axis: iAUCmed of every meal- Bad week

EP 3 058 085 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100172874 A
- US 6566495 B
- US 6190591 B
- US 4666828 A
- US 4683202 A
- US 4801531 A
- US 5192659 A
- US 5272057 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A
- US 62048065 B
- US 62050939 B
- US 61984944 B

**Non-patent literature cited in the description**

- **PAYNE et al.** *obesity reviews,* 2012, vol. 13, 799-809
- **COWEN et al.** *The FASEB Journal,* 2013, vol. 27, 224
- **A. N. PAYNE et al.** Gut microbial adaptation to dietary consumption of fructose, artificial sweeteners and sugar alcohols: implications for host-microbe interactions contributing to obesity. *OBESITY REVIEWS,* 11 June 2012, vol. 13 (9), ISSN 1467-7881, 799-809
- **THERESA E COWAN et al.** *Artificial sweetener consumption differentially affects the gut microbiota-host metabolic interactions*
- **COWAN et al.** *The FASEB Journal", THE FASEB JOURNAL,* 01 April 2013, vol. 27 (1), ISSN 0892-6638, 224.7
- **THOMPSON-CHAGOYAN O C et al.** Faecal microbiota and short-chain fatty acid levels in faeces from infants with cow's milk protein allergy. *INTERNATIONAL ARCHIVES OF ALLERGY AND IMMUNOLOGY,* 01 January 2011, vol. 156 (3), ISSN 1018-2438, 325-332
- The role of commensal bacteria in the regulation of sensitization to food allergens. **CAO SEVERINE et al.** FEBS LETTERS. ELSEVIER, 01 May 2014, vol. 588, 4258-4266
- **JOTHAM SUEZ et al.** Artificial sweeteners induce glucose intolerance by altering the gut microbiota. *NATURE,* 17 September 2014, ISSN 0028-0836
- Non-caloric artificial sweeteners and the microbiome: Findings and challenges. **SUEZ J et al.** GUT MICROBES 20150401. TAYLOR AND FRANCIS INC, 01 April 2015, vol. 6, 149-155
- **MARIE S. A. PALMNÄS et al.** Low-Dose Aspartame Consumption Differentially Affects Gut Microbiota-Host Metabolic Interactions in the Diet-Induced Obese Rat. *PLOS ONE,* 14 October 2014, vol. 9 (10), e109841
- Transkingdom Control of Microbiota Diurnal Oscillations Promotes Metabolic Homeostasis. **THAISS CHRISTOPH et al.** CELL. CELL PRESS, 16 October 2014, vol. 159, 514-529
- A day in the life of the meta-organism: Diurnal rhythms of the intestinal microbiome and its host. **THAISS C A et al.** GUT MICROBES 20150422. TAYLOR AND FRANCIS INC, 22 April 2015, vol. 6, 137-142
- Current Protocols in Molecular Biology. Wiley, 1998
- **HAZEN et al.** Deep-sea oil plume enriches indigenous oil-degrading bacteria. *Science,* 2010, vol. 330, 204-208
- **KARLIN ; ALTSCHUL.** *Proc. Natl. Acad. Sci. USA,* 1993, vol. 87, 2264-2268
- **ALTSCHUL et al.** *J. Mol. Biol.,* 1990, vol. 215, 403-410
- **ALTSCHUL et al.** *Nucleic Acids Res.,* 1997, vol. 25, 3389-3402

- **ROCKETT ; DIX.** DNA arrays: technology, options and toxicological applications. *Xenobiotica,* vol. 30 (2), 155-177
- **SAMBROOK et al.** Molecular Cloning: A laboratory Manual. 1989
- Current Protocols in Molecular Biology. 1994, vol. I-III
- **AUSUBEL et al.** Current Protocols in Molecular Biology. John Wiley and Sons, 1989
- **PERBAL.** A Practical Guide to Molecular Cloning. John Wiley & Sons, 1988
- **WATSON et al.** Recombinant DNA. *Scientific American Books*
- Genome Analysis: A Laboratory Manual Series. Cold Spring Harbor Laboratory Press, 1998, vol. 1-4
- Cell Biology: A Laboratory Handbook. 1994, vol. I-III
- **FRESHNEY.** Culture of Animal Cells - A Manual of Basic Technique. Wiley-Liss, 1994
- Current Protocols in Immunology. 1994, vol. I-III
- Basic and Clinical Immunology. Appleton & Lange, 1994
- Selected Methods in Cellular Immunology. W. H. Freeman and Co, 1980
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **PERBAL, B.** *A Practical Guide to Molecular Cloning,* 1984
- Methods in Enzymology. Academic Press, vol. 1
- PCR Protocols: A Guide To Methods And Applications. Academic Press, 1990
- **MARSHAK et al.** Strategies for Protein Purification and Characterization - A Laboratory Course Manual. CSHL Press, 1996
- **ADAMOVICH, Y. ; ROUSSO-NOORI, L. ; ZWIGHAFT, Z. ; NEUFELD-COHEN, A. ; GOLIK, M. ; KRAUT-COHEN, J. ; WANG, M. ; HAN, X. ; ASHER, G.** Circadian clocks and feeding time regulate the oscillations and levels of hepatic triglycerides. *Cell metabolism,* 2014, vol. 19, 319-330
- **AKHTAR, R.A. ; REDDY, A.B. ; MAYWOOD, E.S. ; CLAYTON, J.D. ; KING, V.M. ; SMITH, A.G. ; GANT, T.W. ; HASTINGS, M.H. ; KYRIACOU, C.P.** Circadian cycling of the mouse liver transcriptome, as revealed by cDNA microarray, is driven by the suprachiasmatic nucleus. *Current biology,* 2002, vol. 12, 540-550
- **ARCHER, S.N. ; LAING, E.E. ; MOLLER-LEVET, C.S. ; VAN DER VEEN, D.R. ; BUCCA, G. ; LAZAR, A.S. ; SANTHI, N. ; SLAK, A. ; KABILJO, R. ; VON SCHANTZ, M. et al.** From the Cover: Mistimed sleep disrupts circadian regulation of the human transcriptome. *Proceedings of the National Academy of Sciences of the United States of America,* 2014, vol. 111, E682-691
- **ASHER, G. ; REINKE, H. ; ALTMEYER, M. ; GUTIERREZ-ARCELUS, M. ; HOTTIGER, M.O. ; SCHIBLER, U.** Poly(ADP-ribose) polymerase 1 participates in the phase entrainment of circadian clocks to feeding. *Cell,* 2010, vol. 142, 943-953
- **BASS, J.** Circadian topology of metabolism. *Nature,* 2012, vol. 491, 348-356
- **BUXTON, O.M. ; CAIN, S.W. ; O'CONNOR, S.P. ; PORTER, J.H. ; DUFFY, J.F. ; WANG, W. ; CZEISLER, C.A. ; SHEA, S.A.** Adverse metabolic consequences in humans of prolonged sleep restriction combined with circadian disruption. *Science translational medicine,* 2012, vol. 4, 129ra143
- **CAPORASO, J.G ; KUCZYNSKI, J. ; STOMBAUGH, J. ; BITTINGER, K. ; BUSHMAN, F.D. ; COSTELLO, E.K. ; FIERER, N. ; PENA, A.G. ; GOODRICH, J.K. ; GORDON, J.I. et al.** QIIME allows analysis of high-throughput community sequencing data. *Nature methods,* 2010, vol. 7, 335-336
- **CLEMENTE, J.C. ; URSELL, L.K. ; PARFREY, L.W. ; KNIGHT, R.** The impact of the gut microbiota on human health: an integrative view. *Cell,* 2012, vol. 148, 1258-1270
- **DAMIOLA, F. ; LE MINH, N. ; PREITNER, N. ; KORNMANN, B. ; FLEURY-OLELA, F. ; SCHIBLER, U.** Restricted feeding uncouples circadian oscillators in peripheral tissues from the central pacemaker in the suprachiasmatic nucleus. *Genes & development,* 2000, vol. 14, 2850-2961
- **DIBNER, C. ; SCHIBLER, U. ; ALBRECHT, U.** The mammalian circadian timing system: organization and coordination of central and peripheral clocks. *Annual review of physiology,* 2010, vol. 72, 517-549
- **EDGAR, R.S. ; GREEN, E.W. ; ZHAO, Y. ; VAN OOIJEN, G. ; OLMEDO, M. ; QIN, X. ; XU, Y. ; PAN, M. ; VALEKUNJA, U.K. ; FEENEY, K.A. et al.** Peroxiredoxins are conserved markers of circadian rhythms. *Nature,* 2012, vol. 485, 459-464
- **ELINAV, E. ; STROWIG, T. ; KAU, A.L. ; HENAO-MEJIA, J. ; THAISS, C.A. ; BOOTH, C.J. ; PEAPER, D.R. ; BERTIN, J. ; EISENBARTH, S.C. ; GORDON, J.I. et al.** NLRP6 inflammasome regulates colonic microbial ecology and risk for colitis. *Cell,* 2011, vol. 145, 745-757

- **FAGARASAN, S. ; MURAMATSU, M. ; SUZUKI, K. ; NAGAOKA, H. ; HIAI, H. ; HONJO, T.** Critical roles of activation-induced cytidine deaminase in the homeostasis of gut flora. *Science,* 2002, vol. 298, 1424-1427
- **FAITH, J.J. ; GURUGE, J.L. ; CHARBONNEAU, M. ; SUBRAMANIAN, S. ; SEEDORF, H. ; GOODMAN, A.L. ; CLEMENTE, J.C. ; KNIGHT, R. ; HEATH, A.C. ; LEIBEL, R.L. et al.** The long-term stability of the human gut microbiota. *Science,* 2013, vol. 341, 1237439
- **FONKEN, L.K. ; WORKMAN, J.L. ; WALTON, J.C. ; WEIL, Z.M. ; MORRIS, J.S. ; HAIM, A. ; NELSON, R.J.** Light at night increases body mass by shifting the time of food intake. *Proceedings of the National Academy of Sciences of the United States of America,* 2010, vol. 107, 18664-18669
- **GERHART-HINES, Z. ; FENG, D. ; EMMETT, M.J. ; EVERETT, L.J. ; LORO, E. ; BRIGGS, E.R. ; BUGGE, A. ; HOU, C. ; FERRARA, C. ; SEALE, P. et al.** The nuclear receptor Rev-erbalpha controls circadian thermogenic plasticity. *Nature,* 2013, vol. 503, 410-413
- **GORDON, J.I.** Honor thy gut symbionts redux. *Science,* 2012, vol. 336, 1251-1253
- **HENAO-MEJIA, J. ; ELINAV, E. ; JIN, C. ; HAO, L. ; MEHAL, W.Z. ; STROWIG, T. ; THAISS, C.A. ; KAU, A.L. ; EISENBARTH, S.C. ; JURCZAK, M.J. et al.** Inflammasome-mediated dysbiosis regulates progression of NAFLD and obesity. *Nature,* 2012, vol. 482, 179-185
- **HOGENESCH, J.B. ; UEDA, H.R.** Understanding systems-level properties: timely stories from the study of clocks. *Nature reviews Genetics,* 2011, vol. 12, 407-416
- **HOOGERWERF, W.A. ; HELLMICH, H.L. ; CORNELISSEN, G. ; HALBERG, F. ; SHAHINIAN, V.B. ; BOSTWICK, J. ; SAVIDGE, T.C. ; CASSONE, V.M.** Clock gene expression in the murine gastrointestinal tract: endogenous rhythmicity and effects of a feeding regimen. *Gastroenterology,* 2007, vol. 133, 1250-1260
- **HOOPER, L.V. ; LITTMAN, D.R. ; MACPHERSON, A.J.** Interactions between the microbiota and the immune system. *Science,* 2012, vol. 336, 1268-1273
- **HSIAO, E.Y. ; MCBRIDE, S.W. ; HSIEN, S. ; SHARON, G. ; HYDE, E.R. ; MCCUE, T. ; CODELLI, J.A. ; CHOW, J. ; REISMAN, S.E. ; PETROSINO, J.F. et al.** Microbiota modulate behavioral and physiological abnormalities associated with neurodevelopmental disorders. *Cell,* 2013, vol. 155, 1451-1463
- **HUANG, W. ; RAMSEY, K.M. ; MARCHEVA, B. ; BASS, J.** Circadian rhythms, sleep, and metabolism. *The Journal of clinical investigation,* 2011, vol. 121, 2133-2141
- **HUGHES, M.E. ; HOGENESCH, J.B. ; KORNACKER, K.** JTK_CYCLE: an efficient nonparametric algorithm for detecting rhythmic components in genome-scale data sets. *Journal of biological rhythms,* 2010, vol. 25, 372-380
- **HUMAN MICROBIOME PROJECT, C.** Structure, function and diversity of the healthy human microbiome. *Nature,* 2012, vol. 486, 207-214
- **JOHNSON, C.H. ; EGLI, M. ; STEWART, P.L.** Structural insights into a circadian oscillator. *Science,* 2008, vol. 322, 697-701
- **JOHNSON, C.H. ; STEWART, P.L. ; EGLI, M.** The cyanobacterial circadian system: from biophysics to bioevolution. *Annual review of biophysics,* 2011, vol. 40, 143-167
- **KAASIK, K. ; KIVIMAE, S. ; ALLEN, J.J. ; CHALKLEY, R.J. ; HUANG, Y. ; BAER, K. ; KISSEL, H. ; BURLINGAME, A.L. ; SHOKAT, K.M. ; PTACEK, L.J. et al.** Glucose sensor O-GlcNAcylation coordinates with phosphorylation to regulate circadian clock. *Cell metabolism,* 2013, vol. 17, 291-302
- **KANEHISA, M. ; GOTO, S.** KEGG: kyoto encyclopedia of genes and genomes. *Nucleic acids research,* 2000, vol. 28, 27-30
- **KANEHISA, M. ; GOTO, S. ; SATO, Y. ; KAWASHIMA, M. ; FURUMICHI, M. ; TANABE, M.** Data, information, knowledge and principle: back to metabolism in KEGG. *Nucleic acids research,* 2014, vol. 42, D199-D205
- **KARATSOREOS, I.N. ; BHAGAT, S. ; BLOSS, E.B. ; MORRISON, J.H. ; MCEWEN, B.S.** Disruption of circadian clocks has ramifications for metabolism, brain, and behavior. *Proceedings of the National Academy of Sciences of the United States of America,* 2011, vol. 108, 1657-1662
- **KELLER, M. ; MAZUCH, J. ; ABRAHAM, U. ; EOM, G.D. ; HERZOG, E.D. ; VOLK, H.D. ; KRAMER, A. ; MAIER, B.** A circadian clock in macrophages controls inflammatory immune responses. *Proceedings of the National Academy of Sciences of the United States of America,* 2009, vol. 106, 21407-21412
- **LENZ, P. ; SOGAARD-ANDERSEN, L.** Temporal and spatial oscillations in bacteria. *Nature reviews Microbiology,* 2011, vol. 9, 565-577
- **LEY, R.E. ; TURNBAUGH, P.J. ; KLEIN, S. ; GORDON, J.I.** Microbial ecology: human gut microbes associated with obesity. *Nature,* 2006, vol. 444, 1022-1023
- **LOZUPONE, C.A. ; STOMBAUGH, J.I. ; GORDON, J.I. ; JANSSON, J.K. ; KNIGHT, R.** Diversity, stability and resilience of the human gut microbiota. *Nature,* 2012, vol. 489, 220-230

- **MCCARTHY, J.J. ; ANDREWS, J.L. ; MCDEAR-MON, E.L. ; CAMPBELL, K.S. ; BARBER, B.K. ; MILLER, B.H. ; WALKER, J.R. ; HOGENESCH, J.B. ; TAKAHASHI, J.S. ; ESSER, K.A.** Identification of the circadian transcriptome in adult mouse skeletal muscle. *Physiological genomics,* 2007, vol. 31, 86-95
- **MOHAWK, J.A. ; GREEN, C.B. ; TAKAHASHI, J.S.** Central and peripheral circadian clocks in mammals. *Annual review of neuroscience,* 2012, vol. 35, 445-462
- **MUKHERJI, A. ; KOBIITA, A. ; YE, T. ; CHAMBON, P.** Homeostasis in intestinal epithelium is orchestrated by the circadian clock and microbiota cues transduced by TLRs. *Cell,* 2013, vol. 153, 812-827
- **NGUYEN, K.D. ; FENTRESS, S.J. ; QIU, Y. ; YUN, K. ; COX, J.S. ; CHAWLA, A.** Circadian gene Bmal1 regulates diurnal oscillations of Ly6C(hi) inflammatory monocytes. *Science,* 2013, vol. 341, 1483-1488
- **PANDA, S. ; ANTOCH, M.P. ; MILLER, B.H. ; SU, A.I. ; SCHOOK, A.B. ; STRAUME, M. ; SCHULTZ, P.G. ; KAY, S.A. ; TAKAHASHI, J.S. ; HOGENE-SCH, J.B.** Coordinated transcription of key pathways in the mouse by the circadian clock. *Cell,* 2002, vol. 109, 307-320
- **QIN, J. ; LI, R. ; RAES, J. ; ARUMUGAM, M. ; BURGDORF, K.S. ; MANICHANH, C. ; NIELSEN, T. ; PONS, N. ; LEVENEZ, F. ; YAMADA, T.** A human gut microbial gene catalogue established by metagenomic sequencing. *Nature,* 2010, vol. 464, 59-65
- **RAKOFF-NAHOUM, S. ; PAGLINO, J. ; ESLA-MI-VARZANEH, F. ; EDBERG, S. ; MEDZHITOV, R.** Recognition of commensal microflora by toll-like receptors is required for intestinal homeostasis. *Cell,* 2004, vol. 118, 229-241
- **RIDAURA, V.K. ; FAITH, J.J. ; REY, F.E. ; CHENG, J. ; DUNCAN, A.E. ; KAU, A.L. ; GRIFFIN, N.W. ; LOMBARD, V. ; HENRISSAT, B. ; BAIN, J.R. et al.** Gut microbiota from twins discordant for obesity modulate metabolism in mice. *Science,* 2013, vol. 341, 1241214
- **RUDIC, R.D. ; MCNAMARA, P. ; CURTIS, A.M. ; BOSTON, R.C. ; PANDA, S. ; HOGENESCH, J.B. ; FITZGERALD, G.A.** BMAL1 and CLOCK, two essential components of the circadian clock, are involved in glucose homeostasis. *PLoS biology,* 2004, vol. 2, e377
- **RUST, M.J. ; MARKSON, J.S. ; LANE, W.S. ; FISH-ER, D.S. ; O'SHEA, E.K.** Ordered phosphorylation governs oscillation of a three-protein circadian clock. *Science,* 2007, vol. 318, 809-812
- **SCHEER, F.A. ; HILTON, M.F. ; MANTZOROS, C.S. ; SHEA, S.A.** Adverse metabolic and cardiovascular consequences of circadian misalignment. *Proceedings of the National Academy of Sciences of the United States of America,* 2009, vol. 106, 4453-4458
- **SILVER, A.C. ; ARJONA, A. ; WALKER, W.E. ; FIKRIG, E.** The circadian clock controls toll-like receptor 9-mediated innate and adaptive immunity. *Immunity,* 2012, vol. 36, 251-261
- **SO, A.Y. ; BEMAL, T.U. ; PILLSBURY, M.L. ; YAMAMOTO, K.R. ; FELDMAN, B.J.** Glucocorticoid regulation of the circadian clock modulates glucose homeostasis. *Proceedings of the National Academy of Sciences of the United States of America,* 2009, vol. 106, 17582-17587
- **SOMMER, F. ; BACKHED, F.** The gut microbiota-masters of host development and physiology. *Nature reviews Microbiology,* 2013, vol. 11, 227-238
- **STECHER, B. ; HARDT, W.D.** Mechanisms controlling pathogen colonization of the gut. *Current opinion in microbiology,* 2011, vol. 14, 82-91
- **STOKKAN, K.A. ; YAMAZAKI, S. ; TEI, H. ; SAKAKI, Y. ; MENAKER, M.** Entrainment of the circadian clock in the liver by feeding. *Science,* 2001, vol. 291, 490-493
- **STORCH, K.F. ; LIPAN, O. ; LEYKIN, I. ; VISWA-NATHAN, N. ; DAVIS, F.C. ; WONG, W.H. ; WEITZ, C.J.** Extensive and divergent circadian gene expression in liver and heart. *Nature,* 2002, vol. 417, 78-83
- **SUWAZONO, Y. ; DOCHI, M. ; SAKATA, K. ; OKUBO, Y. ; OISHI, M. ; TANAKA, K. ; KOBA-YASHI, E. ; KIDO, T. ; NOGAWA, K.** A longitudinal study on the effect of shift work on weight gain in male Japanese workers. *Obesity,* 2008, vol. 16, 1887-1893
- **TUREK, F.W. ; JOSHU, C. ; KOHSAKA, A. ; LIN, E. ; IVANOVA, G. ; MCDEARMON, E. ; LAPOSKY, A. ; LOSEE-OLSON, S. ; EASTON, A. ; JENSEN, D.R. et al.** Obesity and metabolic syndrome in circadian Clock mutant mice. *Science,* 2005, vol. 308, 1043-1045
- **VOLLMERS, C. ; GILL, S. ; DITACCHIO, L. ; PULI-VARTHY, S.R. ; LE, H.D. ; PANDA, S.** Time of feeding and the intrinsic circadian clock drive rhythms in hepatic gene expression. *Proceedings of the National Academy of Sciences of the United States of America,* 2009, vol. 106, 21453-21458
- **YAMAGUCHI, Y. ; SUZUKI, T. ; MIZORO, Y. ; KORI, H. ; OKADA, K. ; CHEN, Y. ; FUSTIN, J.M. ; YAMA-ZAKI, F. ; MIZUGUCHI, N. ; ZHANG, J. et al.** Mice genetically deficient in vasopressin V1a and V1b receptors are resistant to jet lag. *Science,* 2013, vol. 342, 85-90

- **YU, X. ; ROLLINS, D. ; RUHN, K.A. ; STUBBLE-FIELD, J.J. ; GREEN, C.B. ; KASHIWADA, M. ; ROTHMAN, P.B. ; TAKAHASHI, J.S. ; HOOPER, L.V.** TH17 cell differentiation is regulated by the circadian clock. *Science,* 2013, vol. 342, 727-730
- **GARDNER, C. et al.** Nonnutritive Sweeteners: Current Use and Health Perspectives A Scientific Statement from the American Heart Association and the American Diabetes Association. *Diabetes care,* 2012, vol. 35, 1798-1808
- **FITCH, C. ; KEIM, K. S.** Position of the academy of nutrition and dietetics: use of nutritive and nonnutritive sweeteners. *Journal of the Academy of Nutrition and Dietetics,* 2012, vol. 112, 739-758
- **TORDOFF, M. G. ; ALLEVA, A. M.** Effect of drinking soda sweetened with aspartame or high-fructose corn syrup on food intake and body weight. *The American journal of clinical nutrition,* 1990, vol. 51, 963-969
- **HORWITZ, D. L. ; MCLANE, M. ; KOBE, P.** Response to single dose of aspartame or saccharin by NIDDM patients. *Diabetes Care,* 1988, vol. 11, 230-234
- **NETTLETON, J. A. et al.** Diet soda intake and risk of incident metabolic syndrome and type 2 diabetes in the Multi-Ethnic Study of Atherosclerosis (MESA). *Diabetes Care,* 2009, vol. 32, 688-694
- Sucralose metabolism and pharmacokinetics in man. **ROBERTS, A. ; RENWICK, A. G. ; SIMS, J. ; SNODIN, D. J.** Food and chemical toxicology : an international journal. British Industrial Biological Research Association, 2000, vol. 38, 31-41
- **BYARD, J. L. ; GOLDBERG, L.** The metabolism of saccharin in laboratory animals. *Food and cosmetics toxicology,* 1973, vol. 11, 391-402
- **CLEMENTE, J. C. ; URSELL, L. K. ; PARFREY, L. W. ; KNIGHT, R.** The impact of the gut microbiota on human health: an integrative view. *Cell,* 2012, vol. 148, 1258-1270
- **CLAESSON, M. J. et al.** Gut microbiota composition correlates with diet and health in the elderly. *Nature,* 2012, vol. 488, 178-184
- **MUEGGE, B. D. et al.** Diet drives convergence in gut microbiome functions across mammalian phylogeny and within humans. *Science,* 2011, vol. 332, 970-974
- **TURNBAUGH, P. J. et al.** An obesity-associated gut microbiome with increased capacity for energy harvest. *Nature,* 2006, vol. 444, 1027-1131
- **LEY, R. E. ; TURNBAUGH, P. J. ; KLEIN, S. ; GORDON, J. I.** Microbial ecology: human gut microbes associated with obesity. *Nature,* 2006, vol. 444, 1022-1023
- **QIN, J. et al.** A metagenome-wide association study of gut microbiota in type 2 diabetes. *Nature,* 2012, vol. 490, 55-60
- **HENAO-MEJIA, J. et al.** Inflammasome-mediated dysbiosis regulates progression of NAFLD and obesity. *Nature,* 2012, vol. 482, 179-185
- **DAVID, L. A. et al.** Diet rapidly and reproducibly alters the human gut microbiome. *Nature,* 2013
- **PETERSON, J. et al.** The NIH human microbiome project. *Genome research,* 2009, vol. 19, 2317-2323
- **KOROPATKIN, N. M. ; CAMERON, E. A. ; MARTENS, E. C.** How glycan metabolism shapes the human gut microbiota. Nature reviews. *Microbiology,* 2012, vol. 10, 323-335
- Microbiota and SCFA in lean and overweight healthy subjects. **SCHWIERTZ, A. et al.** Obesity. Silver Spring, 2010, vol. 18, 190-195
- **BERGMAN, E. N.** Energy contributions of volatile fatty acids from the gastrointestinal tract in various species. *Physiological reviews,* 1990, vol. 70, 567-590
- **KARLSSON, F. H. et al.** Gut metagenome in European women with normal, impaired and diabetic glucose control. *Nature,* 2013, vol. 498, 99-103
- **CONNOR, S. C. ; HANSEN, M. K. ; CORNER, A. ; SMITH, R. F. ; RYAN, T. E.** Integration of metabolomics and transcriptomics data to aid biomarker discovery in type 2 diabetes. *Molecular bioSystems,* 2010, vol. 6, 909-921
- **CANI, P. D. et al.** Changes in gut microbiota control metabolic endotoxemia-induced inflammation in high-fat diet-induced obesity and diabetes in mice. *Diabetes,* 2008, vol. 57, 1470-1481
- **MARKLE, J. G. et al.** Sex differences in the gut microbiome drive hormone-dependent regulation of autoimmunity. *Science,* 2013, vol. 339, 1084-1088
- **SONNENBURG, J. L. et al.** Glycan foraging in vivo by an intestine-adapted bacterial symbiont. *Science,* 2005, vol. 307, 1955-1959
- **CANI, P. D. et al.** Metabolic endotoxemia initiates obesity and insulin resistance. *Diabetes,* 2007, vol. 56, 1761-1772
- **SMITH, M. I. et al.** Gut microbiomes of Malawian twin pairs discordant for kwashiorkor. *Science,* 2013, vol. 339, 548-554
- **ARPAIA, N. ; CAMPBELL, C. ; FAN, X. ; DIKIY, S. ; VAN DER VEEKEN, J. ; DEROOS, P. ; LIU, H. ; CROSS, J.R. ; PFEFFER, K. ; COFFER, P.J. et al.** Metabolites produced by commensal bacteria promote peripheral regulatory T-cell generation. *Nature,* 2013, vol. 504, 451-455
- **ASHER, G. ; SASSONE-CORSI, P.** Time for Food: The Intimate Interplay between Nutrition, Metabolism, and the Circadian Clock. *Cell,* 2015, vol. 161, 84-92
- **AYRES, J.S. ; TRINIDAD, N.J. ; VANCE, R.E.** Lethal inflammasome activation by a multidrug-resistant pathobiont upon antibiotic disruption of the microbiota. *Nature medicine,* 2012, vol. 18, 799-806

- **COX, L.M. ; YAMANISHI, S. ; SOHN, J. ; ALEK-SEYENKO, A.V. ; LEUNG, J.M. ; CHO, I. ; KIM, S.G. ; LI, H. ; GAO, Z. ; MAHANA, D. et al.** Altering the intestinal microbiota during a critical developmental window has lasting metabolic consequences. *Cell,* 2014, vol. 158, 705-721
- **DAMIOLA, F. ; LE MINH, N. ; PREITNER, N. ; KORNMANN, B. ; FLEURY-OLELA, F. ; SCHIBLER, U.** Restricted feeding uncouples circadian oscillators in peripheral tissues from the central pacemaker in the suprachiasmatic nucleus. *Genes & development,* 2000, vol. 14, 2950-2961
- **DE VADDER, F. ; KOVATCHEVA-DATCHARY, P. ; GONCALVES, D. ; VINERA, J. ; ZITOUN, C. ; DUCHAMPT, A. ; BACKHED, F. ; MITHIEUX, G.** Microbiota-generated metabolites promote metabolic benefits via gut-brain neural circuits. *Cell,* 2014, vol. 156, 84-96
- **ECKEL-MAHAN, K.L. ; PATEL, V.R. ; DE MATEO, S. ; OROZCO-SOLIS, R. ; CEGLIA, N.J. ; SAHAR, S. ; DILAG-PENILLA, S.A. ; DYAR, K.A. ; BALDI, P. ; SASSONE-CORSI, P.** Reprogramming of the circadian clock by nutritional challenge. *Cell,* 2013, vol. 155, 1464-1478
- **ECKEL-MAHAN, K.L. ; PATEL, V.R. ; MOHNEY, R.P. ; VIGNOLA, K.S. ; BALDI, P. ; SASSONE-CORSI, P.** Coordination of the transcriptome and metabolome by the circadian clock. *Proceedings of the National Academy of Sciences of the United States of America,* 2012, vol. 109, 5541-5546
- **FUNG, T.C. ; ARTIS, D. ; SONNENBERG, G.F.** Anatomical localization of commensal bacteria in immune cell homeostasis and disease. *Immunological reviews,* 2014, vol. 260, 35-49
- **HUANG DA, W. ; SHERMAN, B.T. ; LEMPICKI, R.A.** Systematic and integrative analysis of large gene lists using DAVID bioinformatics resources. *Nature protocols,* 2009, vol. 4, 44-57
- **LAVIN, Y. ; WINTER, D. ; BLECHER-GONEN, R. ; DAVID, E. ; KEREN-SHAUL, H. ; MERAD, M. ; JUNG, S. ; AMIT, I.** Tissue-resident macrophage enhancer landscapes are shaped by the local microenvironment. *Cell,* 2014, vol. 159, 1312-1326
- **LOPEZ, C.A. ; KINGSBURY, D.D. ; VELAZQUEZ, E.M. ; BAUMLER, A.J.** Collateral damage: microbiota-derived metabolites and immune function in the antibiotic era. *Cell host & microbe,* 2014, vol. 16, 156-163
- **MINAMI, Y. ; KASUKAWA, T. ; KAKAZU, Y. ; IIGO, M. ; SUGIMOTO, M. ; IKEDA, S. ; YASUI, A. ; VAN DER HORST, G.T. ; SOGA, T. ; UEDA, H.R.** Measurement of internal body time by blood metabolomics. *Proceedings of the National Academy of Sciences of the United States of America,* 2009, vol. 106, 9890-9895
- **NG, K.M. ; FERREYRA, J.A. ; HIGGINBOTTOM, S.K. ; LYNCH, J.B. ; KASHYAP, P.C. ; GOPINATH, S. ; NAIDU, N. ; CHOUDHURY, B. ; WEIMER, B.C. ; MONACK, D.M. et al.** Microbiota-liberated host sugars facilitate post-antibiotic expansion of enteric pathogens. *Nature,* 2013, vol. 502, 96-99
- **QIN, N. ; YANG, F. ; LI, A. ; PRIFTI, E. ; CHEN, Y. ; SHAO, L. ; GUO, J. ; LE CHATELIER, E. ; YAO, J. ; WU, L. et al.** Alterations of the human gut microbiome in liver cirrhosis. *Nature,* 2014, vol. 513, 59-64
- **ROBERTSON, B.R. ; O'ROURKE, J.L. ; NEILAN, B.A. ; VANDAMME, P. ; ON, S.L. ; FOX, J.G. ; LEE, A.** Mucispirillum schaedleri gen. nov., sp. nov., a spiral-shaped bacterium colonizing the mucus layer of the gastrointestinal tract of laboratory rodents. *International journal of systematic and evolutionary microbiology,* 2005, vol. 55, 1199-1204
- **SHAPIRO, H. ; THAISS, C.A. ; LEVY, M. ; ELINAV, E.** The cross talk between microbiota and the immune system: metabolites take center stage. *Current opinion in immunology,* 2014, vol. 30, 54-62
- **SHARON, G. ; GARG, N. ; DEBELIUS, J. ; KNIGHT, R. ; DORRESTEIN, P.C. ; MAZMANIAN, S.K.** Specialized metabolites from the microbiome in health and disease. *Cell metabolism,* 2014, vol. 20, 719-730
- **SMITH, P.M. ; HOWITT, M.R. ; PANIKOV, N. ; MICHAUD, M. ; GALLINI, C.A. ; BOHLOOLY, Y.M. ; GLICKMAN, J.N. ; GARRETT, W.S.** The microbial metabolites, short-chain fatty acids, regulate colonic Treg cell homeostasis. *Science,* 2013, vol. 341, 569-573
- **THAISS, C.A. ; ELINAV, E.** Exploring new horizons in microbiome research. *Cell host & microbe,* 2014, vol. 15, 662-667
- **THAISS, C.A. ; LEVY, M. ; SUEZ, J. ; ELINAV, E.** The interplay between the innate immune system and the microbiota. *Current opinion in immunology,* 2014, vol. 26, 41-48
- **THAISS, C.A. ; ZEEVI, D. ; LEVY, M. ; ZILBERMAN-SCHAPIRA, G. ; SUEZ, J. ; TENGELER, A.C. ; ABRAMSON, L. ; KATZ, M.N. ; KOREM, T. ; ZMORA, N. et al.** Transkingdom control of microbiota diurnal oscillations promotes metabolic homeostasis. *Cell,* 2014, vol. 159, 514-529
- **VOIGT, R.M. ; FORSYTH, C.B. ; GREEN, S.J. ; MUTLU, E. ; ENGEN, P. ; VITATERNA, M.H. ; TUREK, F.W. ; KESHAVARZIAN, A.** Circadian disorganization alters intestinal microbiota. *PloS one,* 2014, vol. 9, e97500
- **ZARRINPAR, A. ; CHAIX, A. ; YOOSEPH, S. ; PANDA, S.** Diet and feeding pattern affect the diurnal dynamics of the gut microbiome. *Cell metabolism,* 2014, vol. 20, 1006-1017
- **ZEISSIG, S. ; BLUMBERG, R.S.** Life at the beginning: perturbation of the microbiota by antibiotics in early life and its role in health and disease. *Nature immunology,* 2014, vol. 15, 307-310

SEQUENCE LISTING

<110> Yeda Research And Development Co. Ltd. ELINAV, Eran SEGAL, Eran

<120> MICROBIOME RESPONSE TO AGENTS

<130> 62208

<150> US 62/048,065
<151> 2014-09-09

<150> US 62/050,939
<151> 2014-09-16

<150> US 61/984,944
<151> 2014-04-28

<160> 4

<170> PatentIn version 3.5

<210> 1
<211> 28
<212> DNA
<213> Artificial Sequence

<220>
<223> Single strand DNA oligonucleotide

<220>
<221> misc_feature
<222> (1)..(8)
<223> barcoded primer n represents a barcode base

<400> 1
nnnnnnnnag agtttgatcc tggctcag 28

<210> 2
<211> 19
<212> DNA
<213> Artificial Sequence

<220>
<223> Single strand DNA oligonucleotide

<400> 2
tgctgcctcc cgtaggagt 19

<210> 3
<211> 20
<212> DNA
<213> Artificial Sequence

<220>

<223> Single strand DNA oligonucleotide

<400> 3
agagtttgat cctggctcag 20

<210> 4
<211> 19
<212> DNA
<213> Artificial Sequence

<220>
<223> Single strand DNA oligonucleotide

<400> 4
tgctgcctcc cgtaggagt 19